(19) Europäisches Patentamt
European Patent Office
Office européen des brevets

(11) EP 3 822 263 A1

(12) EUROPEAN PATENT APPLICATION
published in accordance with Art. 153(4) EPC

(43) Date of publication:
**19.05.2021 Bulletin 2021/20**

(21) Application number: **19830650.8**

(22) Date of filing: **03.07.2019**

(51) Int Cl.:
*C07D 401/12* (2006.01) *A61K 31/517* (2006.01)
*A61P 35/00* (2006.01) *A61P 43/00* (2006.01)
*C07D 401/14* (2006.01) *C07D 413/14* (2006.01)
*C07D 417/14* (2006.01)

(86) International application number:
**PCT/JP2019/026483**

(87) International publication number:
**WO 2020/009156 (09.01.2020 Gazette 2020/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **04.07.2018 JP 2018127829**

(71) Applicant: **Daiichi Sankyo Company, Limited**
**Tokyo 103-8426 (JP)**

(72) Inventors:
- **YOSHIDA Kenichi**
**Tokyo 103-8426 (JP)**
- **TAKEUCHI Kosuke**
**Tokyo 103-8426 (JP)**
- **INOUE Hidekazu**
**Tokyo 103-8426 (JP)**
- **KAGEJI Hideaki**
**Tokyo 103-8426 (JP)**
- **MOMOSE Takayuki**
**Tokyo 103-8426 (JP)**
- **YOSHIDA Keisuke**
**Tokyo 103-8426 (JP)**
- **JIMBO Takeshi**
**Tokyo 103-8426 (JP)**
- **EGAMI Akiko**
**Tokyo 103-8426 (JP)**

(74) Representative: **Wallace, Sheila Jane**
**Marks & Clerk LLP**
**15 Fetter Lane**
**London EC4A 1BW (GB)**

(54) **BIARYL ETHER-TYPE QUINAZOLINE DERIVATIVE**

(57) The present invention provides a novel compound or a pharmaceutically acceptable salt thereof having an inhibitory action on an EGFR tyrosine kinase having an exon 20 insertion mutation and/or a HER2 tyrosine kinase having an exon 20 insertion mutation, a compound represented by Formula (I) or a pharmaceutically acceptable salt thereof wherein $R^1$, $R^2$, $R^3$, $R^4$, $R^5$ and $R^6$ in the Formula (I) are each as defined in the description.

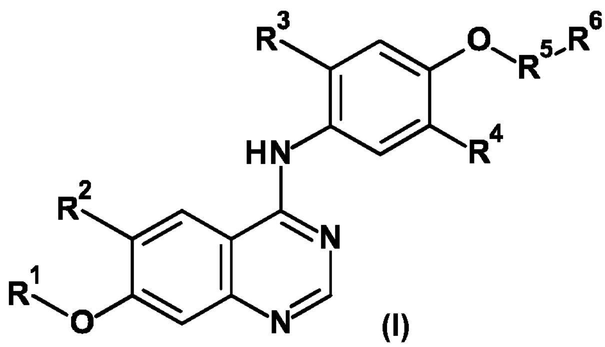

(I)

EP 3 822 263 A1

## Description

### Technical field

The present invention relates to an inhibitor of an EGFR tyrosine kinase having an exon 20 insertion mutation and/or a HER2 tyrosine kinase having an exon 20 insertion mutation.

### Background art

Epidermal growth factor receptor (EGFR) is a tyrosine kinase-type receptor that performs signaling by recognizing factors involved in cell proliferation (Non-patent Reference 1). EGFR is present on the cell membrane, and when this receptor is activated, cell differentiation and proliferation occur. EGFR can be seen in many cells, and excessive expression and gene mutation of EGFR lead to cancer, infiltration and metastasis. In various cancers including non-small cell lung cancer and colon cancer, EGFR is genetically amplified or mutated in the cancer cells, and proliferation of the cancer cells is active. Furthermore, it is known that cells that are genetically amplified or mutated exhibit higher metastaticity than cells that are not. Inhibition of phosphorylation of EGFR tyrosine kinases can block signaling necessary for proliferation of cancer cells, thereby suppressing proliferation of cancer cells. For mutations in the EGFR gene, it is known that activation mutations such as a mutation in which leucine 858 of the coding protein is substituted by arginine (L858R), a deletion mutation in exon 19 (exon19del), a mutation in which glycine 719 of the coding protein is substituted by another amino acid (G719X), a mutation in which leucine 861 of the coding protein is substituted by glutamine (L861Q) can be seen in non-small cell lung cancer and the like (Non-patent References 2 to 4). It is also known that, in cancers resistant to so-called first-generation EGFR tyrosine kinase inhibitors such as gefitinib and erlotinib, a mutation in which threonine 790 of the coding protein is substituted by methionine (T790M) occurs (Non-patent Reference 5).

Meanwhile, it is known that an exon 20 insertion mutation in the EGFR gene occurs in lung cancer or the like. As a result of the mutation, mutations of insertion of 1 amino acid residue to 4 amino acid residues are found in EGFR protein, and insertion mutations of 3 amino acid residues, such as V769_D770ins.ASV, D770_N771ins.SVD, and H773_V774ins.NPH, are frequently found (Non-patent References 6 to 8).

Human epidermal growth factor receptor 2 (HER2) is one of the representative growth factor receptor-type oncogene products identified as a human epithelial cell growth factor receptor-type 2 associated oncogene, and is a transmembrane receptor protein having a tyrosine kinase domain with a molecular weight of 185 kDa (Non-patent Reference 9).

HER2 (neu, ErbB-2) is one of the EGFR family members, and it is known that intracellular tyrosine residues in HER2 are autophosphorylated by formation of a homodimer or heterodimer with HER1 (EGFR, ErbB-1), HER3 (ErbB-3),or HER4 (ErbB-4), which are other EGFR receptors, to be activated, thereby HER2 plays an important role in cell proliferation, differentiation, and survival in normal cells and cancer cells. HER2 is overexpressed in various cancer species such as breast cancer, stomach cancer, ovarian cancer, and the like (Non-patent References 10 to 15). It is known that an exon 20 insertion mutation in the HER2 gene occurs in lung cancer, breast cancer, bladder cancer, ovarian cancer, and the like. The mutation results in mutations of insertion of 1 amino acid residue to 4 amino acid residues, and mutations of insertion of 4 amino acid residues, such as A775_G776 ins. YVMA, E770_A771 ins. AYVM, A771_Y772 ins. YVMA, Y772_A775dup, are most frequently found (Non-patent References 6, 7, and 16).

Pozionitib is known as a compound having a quinazoline backbone and being developed as a therapeutic agent for cancers (Patent Reference 1).

### Prior art references

#### Patent references

Patent Reference 1: WO 2008150118

#### Non-patent references

Non-patent Reference 1: Das, M. et al., Proc. Natl Acad.Sci. USA, 74, 2790-2794 (1977).
Non-patent Reference 2: Kancha, R. et al., Clin. Cancer Res. 15, 460-467 (2009).
Non-patent Reference 3: Lee, C. K. et al., J. Clin. Oncol. 33, 1958-1965 (2015).
Non-patent Reference 4: Watanabe, S. et al., J. Thorac. Oncol. 9, 189-194 (2014).
Non-patent Reference 5: Pao, W. et al., PLoS Med. 2, e73 (2005) .

Non-patent Reference 6: Maria E. A. et al., Clin Cancer Res; 18(18) September 15, 2012
Non-patent Reference 7: Oxnard G.R., J Thorac Oncol. 2013 Feb;8(2):179-184.
Non-patent Reference 8: Yoshihisa K. et al., Cancer Sci, September 2016, 107 (9), 1179-1186
Non-patent Reference 9: Coussens L, et al., Science. 1985; 230(4730): 1132-1139.
Non-patent Reference 10: Hardwick R, et al., Eur. J Surg Oncol. 1997 (23):30-35.
Non-patent Reference 11: Korkaya H, et al., Oncogene. 2008; 27(47): 6120-6130.
Non-patent Reference 12: Yano T, et al., OncolRep. 2006;15(1):65-71.
Non-patent Reference 13: Slamon DJ, et al., Science. 1987; 235: 177-182.
Non-patent Reference 14: Gravalos C, et al., Ann Oncol 19: 1523-1529, 2008.
Non-patent Reference 15: Fukushige S et al., Mol Cell Biol 6: 955-958, 1986.
Non-patent Reference 16: Hyman D.M., et al., Nature. 2018 Feb 8;554 (7691) : 189-194

Disclosure of the invention

Object of the invention

The present invention provides a novel compound having an inhibitory action on an EGFR tyrosine kinase having an exon 20 insertion mutation and/or a HER2 tyrosine kinase having an exon 20 insertion mutation, or a pharmaceutically acceptable salt.

Means for achieving the object

The present invention relates to the following (1) to (49).

(1) A compound represented by Formula (I) or a pharmaceutically acceptable salt thereof:

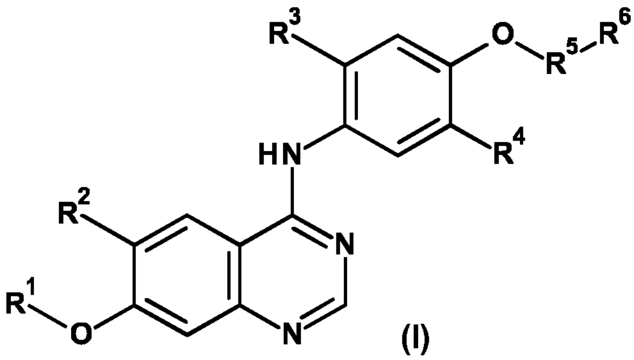

(I)

wherein

$R^1$ represents a $C_1$-$C_3$ alkyl group optionally substituted with 1 to 3 halogen atoms,
$R^2$ represents Formula (II) below:

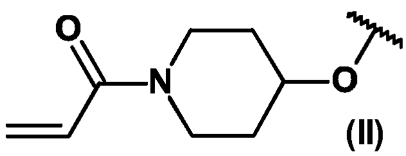

(II)

or -NH-CO-CH=CH-$CH_2$-X;
X represents an amino group optionally having 1 or 2 substituents independently selected from Group A below, a pyrrolidinyl group optionally having 1 or 2 substituents independently selected from Group A below, an azetidinyl group optionally having 1 or 2 substituents independently selected from Group A below, or a morpholyl group;
$R^3$ represents a halogen atom;
$R^4$ represents a hydrogen atom or a halogen atom;
$R^5$ represents a benzene ring, a thiazole ring, or a pyrazole ring optionally having 1 or 2 substituents independently selected from the group consisting of a halogen atom and a $C_1$-$C_3$ alkyl group;

$R^6$ represents an oxadiazolyl group optionally having 1 or 2 substituents independently selected from Group B below, a triazolyl group optionally having 1 or 2 substituents independently selected from Group B below, a pyridyl group optionally having 1 or 2 substituents independently selected from Group B below, a pyrimidyl group optionally having 1 or 2 substituents independently selected from Group B below, a thiadiazolyl group optionally having 1 or 2 substituents independently selected from Group B below, -CO-N(Y)(Z), or $-CH_2$-CON(Y)(Z) ;

Y and Z each independently represents a hydrogen atom, a $C_1$-$C_6$ alkyl group optionally substituted with 1 to 3 halogen atoms, or a $C_3$-$C_6$ cycloalkyl group substituted with a $C_1$-$C_3$ alkyl group optionally substituted with 1 to 3 halogen atoms, or

Y, Z and the nitrogen atom to which they are bonded may be taken together to form a 4- to 6-membered nitrogen-containing saturated heterocyclic ring,

Group A: a halogen atom, a $C_1$-$C_3$ alkyl group, a $C_1$-$C_3$ alkoxy group, and a tetrahydrofuryl group, and

Group B: a halogen atom, a $C_1$-$C_3$ alkyl group, a $C_3$-$C_6$ cycloalkyl group, and a $C_1$-$C_3$ alkoxy group.

(2) The compound or a pharmaceutically acceptable salt thereof according to (1), wherein $R^1$ is a methyl group.

(3) The compound or a pharmaceutically acceptable salt thereof according to (1) or (2), wherein $R^2$ is a group represented by Formula (II).

(4) The compound or a pharmaceutically acceptable salt thereof according to any one of (1) to (3), wherein $R^3$ is a chlorine atom or a fluorine atom, and $R^4$ is a hydrogen atom.

(5) The compound or a pharmaceutically acceptable salt thereof according to any one of (1) to (4), wherein $R^5$ is a benzene ring or a pyrazole ring.

(6) The compound or a pharmaceutically acceptable salt thereof according to any one of (1) to (5), wherein $R^6$ is an oxadiazolyl group optionally substituted with a methyl group, a pyridyl group optionally substituted with 1 or 2 substituents independently selected from the group consisting of a fluorine atom and a methyl group, or -CO-NH-Y; and

Y is a tert-butyl group optionally substituted with 1 to 3 fluorine atoms.

(7) A compound selected from the group consisting of:

N-tert-butyl-3-{3-fluoro-4-[(7-methoxy-6-{[1-(prop-2-enoyl)piperidin-4-yl]oxy}quinazolin-4-yl)amino]phenoxy}-1H-pyrazole-1-carboxamide,

3-{3-fluoro-4-[(7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide,

1-{4-[(4-{2-chloro-4-[3-(5-methyl-1,3,4-oxadiazol-2-yl)phenoxy]anilino}-7-methoxyquinazolin-6-yl)oxy]piperidin-1-yl}prop-2-en-1-one,

1-{4-[(4-{2-fluoro-4-[3-(5-methyl-1,3,4-oxadiazol-2-yl)phenoxy]anilino}-7-methoxyquinazolin-6-yl)oxy]piperidin-1-yl}prop-2-en-1-one,

1-(4-{[4-(2-fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one; and

1-(4-{[4-(2-fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one,

or a pharmaceutically acceptable salt thereof.

(8) N-tert-Butyl-3-{3-fluoro-4-[(7-methoxy-6-{[1-(prop-2-enoyl)piperidin-4-yl]oxy}quinazolin-4-yl)amino]phenoxy}-1H-pyrazole-1-carboxamide, or a pharmaceutically acceptable salt thereof.

(9) N-tert-Butyl-3-{3-fluoro-4-[(7-methoxy-6-{[1-(prop-2-enoyl)piperidin-4-yl]oxy}quinazolin-4-yl)amino]phenoxy}-1H-pyrazole-1-carboxamide methanesulfonate.

(10) 3-{3-Fluoro-4-[((7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide, or a pharmaceutically acceptable salt thereof.

(11) 3-{3-Fluoro-4-[(7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide 1,5-naphthalenedisulfonate.

(12) 1-{4-[(4-{2-Chloro-4-[3-(5-methyl-1,3,4-oxadiazol-2-yl)phenoxy]anilino}-7-methoxyquinazolin-6-yl)oxy]piperidin-1-yl}prop-2-en-1-one, or a pharmaceutically acceptable salt thereof.

(13) 1-{4-[(4-{2-Chloro-4-[3-(5-methyl-1,3,4-oxadiazol-2-yl)phenoxy]anilino}-7-methoxyquinazolin-6-yl)oxy]piperidin-1-yl}prop-2-en-1-one methanesulfonate.

(14) 1-{4-[(4-{2-Fluoro-4-[3-(5-methyl-1,3,4-oxadiazol-2-yl)phenoxy]anilino}-7-methoxyquinazolin-6-yl)oxy]piperidin-1-yl}prop-2-en-1-one, or a pharmaceutically acceptable salt thereof.

(15) 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one, or a pharmaceutically acceptable salt thereof.

(16) 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one benzenesulfonate.

(17) 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one tartrate.

(18) 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one citrate.

(19) 1-(4-1[4-(2-Fluoro-4-1[1-(6-methylpyridin-3-yl)-1H-pyrazol-3-yl]oxy}anilino)-7-methoxyquinazolin-6-yl]oxy}piperidin-1-yl)prop-2-en-1-one, or a pharmaceutically acceptable salt thereof.

(20) 1-(4-{[4-(2-Fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one hydrochloride.

(21) 1-(4-1[4-((2-Fluoro-4-1[1-(6-methylpyridin-3-yl)-1H-pyrazol-3-yl]oxy}anilino)-7-methoxyquinazolin-6-yl]oxy}piperidin-1-yl)prop-2-en-1-one 1,5-naphthalenedisulfonate.

(22) 1-(4-{[4-(2-Fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one citrate.

(23) A crystal of 3-{3-fluoro-4-[(7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide, having at least five peaks at diffraction angles (2θ) selected from 5.78±0.2, 15.48±0.2, 16.38±0.2, 17.24±0.2, 19.28±0.2, 19.90±0.2, 20.42±0.2, 20.82±0.2, 22.04±0.2, and 24.50±0.2 in powder X-ray diffraction with CuKα radiation.

(24) A crystal of 3-{3-fluoro-4-[(7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide 1,5-naphthalenedisulfonate, having at least five peaks at diffraction angles (2θ) selected from 5.74±0.2, 10.32±0.2, 11.58±0.2, 14.62±0.2, 14.94±0.2, 18.72±0.2, 19.60±0.2, 20.84±0.2, 22.96±0.2 and 26.42±0.2 in powder X-ray diffraction with CuKα radiation.

(25) A crystal of 1-{4-[(4-{2-chloro-4-[3-(5-methyl-1,3,4-oxadiazol-2-yl)phenoxy]anilino}-7-methoxyquinazolin-6-yl)oxy]piperidin-1-yl}prop-2-en-1-one, having at least five peaks at diffraction angles (2θ) selected from 4.26±0.2, 8.66±0.2, 13.64±0.2, 14.34±0.2, 14.98±0.2, 17.60±0.2, 19.08±0.2, 22.10±0.2, 23.02±0.2 and 25.88±0.2 in powder X-ray diffraction with CuKα radiation.

(26) A crystal of 1-{4-[(4-{2-chloro-4-[3-(5-methyl-1,3,4-oxadiazol-2-yl)phenoxy]anilino}-7-methoxyquinazolin-6-yl)oxy]piperidin-1-yl}prop-2-en-1-one methanesulfonate, having at least five peaks at diffraction angles (2θ) selected from 3.56±0.2, 7.24±0.2, 15.02±0.2, 16.84±0.2, 17.68±0.2, 20.26±0.2, 21.88±0.2, 22.92±0.2, 25.76±0.2 and 27.08±0.2 in powder X-ray diffraction with CuKα radiation.

(27) A crystal of 1-(4-{[4-(2-fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one, having at least five peaks at diffraction angles (2θ) selected from 8.08±0.2, 10.32±0.2, 12.90±0.2, 13.48±0.2, 13.82±0.2, 15.44±0.2, 19.76±0.2, 23.60±0.2, 24.24±0.2 and 25.90±0.2 in powder X-ray diffraction with CuKα radiation.

(28) A crystal of 1-(4-{[4-(2-fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one benzenesulfonate, having at least five peaks at diffraction angles (2θ) selected from 6.22±0.2, 12.16±0.2, 13.60±0.2, 16.26±0.2, 18.50±0.2, 19.58±0.2, 20.58±0.2, 21.06±0.2, 23.30±0.2, and 25.76±0.2 in powder X-ray diffraction with CuKα radiation.

(29) A crystal of 1-(4-{[4-(2-fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one tartrate, having at least five peaks at diffraction angles (2θ) selected from 3.58±0.2, 14.50±0.2, 16.50±0.2, 24.28±0.2, 24.70±0.2, 24.98±0.2, 25.76±0.2, 26.12±0.2, 26.60±0.2 and 27.40±0.2 in powder X-ray diffraction with CuKα radiation.

(30) A crystal of 1-(4-{[4-(2-fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one citrate, having at least five peaks at diffraction angles (2θ) selected from 7.36±0.2, 8.74±0.2, 13.62±0.2, 15.32±0.2, 16.32±0.2, 17.56±0.2, 19.02±0.2, 19.44±0.2, 21.28±0.2, and 25.02±0.2 in powder X-ray diffraction with CuKα radiation.

(31) A crystal of 1-(4-{[4-(2-fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one, having at least five peaks at diffraction angles (2θ) selected from 8.14±0.2, 10.56±0.2, 13.10±0.2, 15.16±0.2, 15.50±0.2, 15.92±0.2, 19.30±0.2, 20.18±0.2, 23.92±0.2, and 25.54±0.2 in powder X-ray diffraction with CuKα radiation.

(32) A crystal of 1-(4-{[4-(2-fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one hydrochloride, having at least five peaks at diffraction angles (2θ) selected from 5.28±0.2, 5.98±0.2, 7.70±0.2, 8.28±0.2, 10.64±0.2, 12.60±0.2, 13.48±0.2, 16.68±0.2, 17.66±0.2 and 20.80±0.2 in powder X-ray diffraction with CuKα radiation.

(33) A crystal of 1-(4-{ [4-(2-fluoro-4-{ [1-(6-methylpyridin-3-yl)-1H-pyrazol-3-yl]oxy}anilino)-7-methoxyquinazolin-6-yl]oxy}piperidin-1-yl)prop-2-en-1-one 1,5-naphthalenedisulfonate, having at least five peaks at diffraction angles (2θ) selected from 8.50±0.2, 13.98±0.2, 15.56±0.2, 16.94±0.2, 18.28±0.2, 19.52±0.2, 20.04±0.2, 25.16±0.2, 25.44±0.2 and 26.10±0.2 in powder X-ray diffraction with CuKα radiation.

(34) A crystal of 1-(4-{[4-(2-fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one citrate, having at least five peaks at diffraction angles (2θ) selected from 5.34±0.2, 7.32±0.2, 7.86±0.2, 8.68±0.2, 13.56±0.2, 16.26±0.2, 17.50±0.2, 19.36±0.2, 21.22±0.2 and 24.90±0.2 in powder X-ray diffraction with CuKα radiation.

(35) A crystal of N-tert-butyl-3-{3-fluoro-4-[(7-methoxy-6-{ [1-(prop-2-enoyl)piperidin-4-yl]oxy}quinazolin-4-yl)amino]phenoxy}-1H-pyrazole-1-carboxamide methanesulfonate, having at least five peaks at diffraction angles (2θ) selected from 6.72±0.2, 8.38±0.2, 11.10±0.2, 13.62±0.2, 16.28±0.2, 17.92±0.2, 19.02±0.2, 21.66±0.2, 22.40±0.2 and 25.64±0.2 in powder X-ray diffraction with CuKα radiation.

(36) A pharmaceutical composition, comprising as an active ingredient a compound or a pharmaceutically acceptable salt thereof according to any one of (1) to (22), or a crystal according to any one of (23) to (35).

(37) An inhibitor of an EGFR tyrosine kinase having an exon 20 insertion mutation and/or a HER2 tyrosine kinase having an exon 20 insertion mutation, comprising as an active ingredient a compound or a pharmaceutically acceptable salt thereof according to any one of (1) to (22), or a crystal according to any one of (23) to (35).

(38) An antitumor agent, comprising as an active ingredient a compound or a pharmaceutically acceptable salt thereof according to any one of (1) to (22), or a crystal according to any one of (23) to (35).

(39) The antitumor agent according to (38), wherein the tumor is lung cancer, breast cancer, bladder cancer, or ovarian cancer.

(40) The antitumor agent according to (38) or (39), wherein the tumor is a tumor having an exon 20 insertion mutation of an EGFR tyrosine kinase and/or an exon 20 insertion mutation of a HER2 tyrosine kinase.

(41) A method for treating a tumor, comprising administering a compound or a pharmaceutically acceptable salt thereof according to any one of (1) to (22), or a crystal according to any one of (23) to (35).

(42) The method according to (41), wherein the tumor is lung cancer, breast cancer, bladder cancer, or ovarian cancer.

(43) The method according to (41) or (42), wherein the tumor is a tumor having an exon 20 insertion mutation of an EGFR tyrosine kinase and/or an exon 20 insertion mutation of a HER2 tyrosine kinase.

(44) A compound or a pharmaceutically acceptable salt thereof according to any one of (1) to (22), or a crystal according to any one of (23) to (35), for the treatment of a tumor.

(45) The compound or a pharmaceutically acceptable salt thereof, or the crystal according to (44), wherein the tumor is lung cancer, breast cancer, bladder cancer, or ovarian cancer.

(46) The compound or a pharmaceutically acceptable salt thereof, or the crystal according to (44) or (45), wherein the tumor is a tumor having an exon 20 insertion mutation of an EGFR tyrosine kinase and/or an exon 20 insertion mutation of a HER2 tyrosine kinase.

(47) Use of a compound or a pharmaceutically acceptable salt thereof according to any one of (1) to (22), or a crystal according to any one of (23) to (35), in the manufacture of a pharmaceutical composition for use in treating a tumor.

(48) Use according to (47), wherein the tumor is lung cancer, breast cancer, bladder cancer, or ovarian cancer.

(49) Use according to (47) or (48), wherein the tumor is a tumor having an exon 20 insertion mutation of an EGFR tyrosine kinase and/or an exon 20 insertion mutation of a HER2 tyrosine kinase.

Effects of the invention

The compound or a pharmaceutically acceptable salt of the present invention has an inhibitory activity on an EGFR tyrosine kinase having an exon 20 insertion mutation and/or a HER2 tyrosine kinase having an exon 20 insertion mutation, and suppresses cell proliferation. Thus, the compound or a pharmaceutically acceptable salt thereof of the present invention is useful as an antitumor agent, in particular as a therapeutic agent for a tumor such as lung cancer, breast cancer, bladder cancer, and/or ovarian cancer, and among those tumors, is effective as a therapeutic agent for a tumor that can be treated by inhibiting an EGFR tyrosine kinase having an exon 20 insertion mutation and/or a HER2 tyrosine kinase having an exon 20 insertion mutation.

Brief description of the figures

[Figure 1] Figure 1 is a diagram showing the results of proliferation suppression tests with Ba/F3-EGFR ins. ASV cells for the compound of Example 5.

[Figure 2] Figure 2 is a diagram showing the results of proliferation suppression tests with Ba/F3-EGFR ins. ASV cells for the compound of Example 13.

[Figure 3] Figure 3 is a diagram showing the results of proliferation suppression tests with Ba/F3-EGFR ins. ASV cells for the compound of Example 27.

[Figure 4] Figure 4 is a diagram showing the results of proliferation suppression tests with Ba/F3-EGFR ins. ASV

cells for the compound of Example 28.

[Figure 5] Figure 5 is a diagram showing the results of proliferation suppression tests with Ba/F3-EGFR ins. ASV cells for the compound of Example 33.

[Figure 6] Figure 6 is a diagram showing the results of proliferation suppression tests with Ba/F3-EGFR ins. ASV cells for the compound of Example 40.

[Figure 7] Figure 7 is a diagram showing the results of proliferation suppression tests with Ba/F3-EGFR ins. ASV cells for the compound of Example 49.

[Figure 8] Figure 8 is a diagram showing the results of proliferation suppression tests with Ba/F3-EGFR ins. ASV cells for the compound of Example 55.

[Figure 9] Figure 9 is a diagram showing the results of proliferation suppression tests with Ba/F3-HER2 ins. YVMA cells for the compound of Example 4.

[Figure 10] Figure 10 is a diagram showing the results of proliferation suppression tests with Ba/F3-HER2 ins. YVMA cells for the compound of Example 5.

[Figure 11] Figure 11 is a diagram showing the results of proliferation suppression tests with Ba/F3-HER2 ins. YVMA cells for the compound of Example 27.

[Figure 12] Figure 12 is a diagram showing the results of proliferation suppression tests with Ba/F3-HER2 ins. YVMA cells for the compound of Example 28.

[Figure 13] Figure 13 shows a powder X-ray diffraction pattern of the crystal of Example 64. The vertical axis of the figure shows the diffraction intensity as the relative ray intensity, and the horizontal axis shows the value of the diffraction angle 2θ.

[Figure 14] Figure 14 shows a powder X-ray diffraction pattern of the crystal of Example 65. The vertical axis of the figure shows the diffraction intensity as the relative ray intensity, and the horizontal axis shows the value of the diffraction angle 2θ.

[Figure 15] Figure 15 shows a powder X-ray diffraction pattern of the crystal of Example 66. The vertical axis of the figure shows the diffraction intensity as the relative ray intensity, and the horizontal axis shows the value of the diffraction angle 2θ.

[Figure 16] Figure 16 shows a powder X-ray diffraction pattern of the crystal of Example 67. The vertical axis of the figure shows the diffraction intensity as the relative ray intensity, and the horizontal axis shows the value of the diffraction angle 2θ.

[Figure 17] Figure 17 shows a powder X-ray diffraction pattern of the crystal of Example 68. The vertical axis of the figure shows the diffraction intensity as the relative ray intensity, and the horizontal axis shows the value of the diffraction angle 2θ.

[Figure 18] Figure 18 shows a powder X-ray diffraction pattern of the crystal of Example 69. The vertical axis of the figure shows the diffraction intensity as the relative ray intensity, and the horizontal axis shows the value of the diffraction angle 2θ.

[Figure 19] Figure 19 shows a powder X-ray diffraction pattern of the crystal of Example 70. The vertical axis of the figure shows the diffraction intensity as the relative ray intensity, and the horizontal axis shows the value of the diffraction angle 2θ.

[Figure 20] Figure 20 shows a powder X-ray diffraction pattern of the crystal of Example 71. The vertical axis of the figure shows the diffraction intensity as the relative ray intensity, and the horizontal axis shows the value of the diffraction angle 2θ.

[Figure 21] Figure 21 shows a powder X-ray diffraction pattern of the crystal of Example 72. The vertical axis of the figure shows the diffraction intensity as the relative ray intensity, and the horizontal axis shows the value of the diffraction angle 2θ.

[Figure 22] Figure 22 shows a powder X-ray diffraction pattern of the crystal of Example 73. The vertical axis of the figure shows the diffraction intensity as the relative ray intensity, and the horizontal axis shows the value of the diffraction angle 2θ.

[Figure 23] Figure 23 shows a powder X-ray diffraction pattern of the crystal of Example 74. The vertical axis of the figure shows the diffraction intensity as the relative ray intensity, and the horizontal axis shows the value of the diffraction angle 2θ.

[Figure 24] Figure 24 shows a powder X-ray diffraction pattern of the crystal of Example 75. The vertical axis of the figure shows the diffraction intensity as the relative ray intensity, and the horizontal axis shows the value of the diffraction angle 2θ.

[Figure 25] Figure 25 shows a powder X-ray diffraction pattern of the crystal of Example 76. The vertical axis of the figure shows the diffraction intensity as the relative ray intensity, and the horizontal axis shows the value of the diffraction angle 2θ.

Mode for carrying out the invention

In the present invention, a "halogen atom" is, for example, a fluorine atom, a chlorine atom, a bromine atom, or an iodine atom.

In the present invention, a "$C_1$-$C_3$ alkyl group" is a linear or branched chain alkyl group having 1 to 3 carbon atoms, and examples thereof include a methyl group, an ethyl group, a propyl group, and an isopropyl group.

In the present invention, a "$C_1$-$C_6$ alkyl group" is a linear or branched chain alkyl group having 1 to 6 carbon atoms, and examples thereof include a methyl group, an ethyl group, a propyl group, an isopropyl group, a butyl group, an isobutyl group, a s-butyl group, a t-butyl group, a pentyl group, an isopentyl group, a 2-methylbutyl group, a neopentyl group, a 1-ethylpropyl group, a hexyl group, an isohexyl group, and a 4-methylpentyl group.

In the present invention, a "$C_1$-$C_3$ alkoxy group" is a group formed of a $C_1$-$C_3$ alkyl group as described above and an oxy group, and examples thereof include a methoxy group, an ethoxy group, a propoxy group, and an isopropoxy group.

In the present invention, a "$C_3$-$C_6$ cycloalkyl group" is, for example, a cyclopropyl group, a cyclobutyl group, a cyclopentyl group, or a cyclohexyl group.

In the present invention, a "$C_1$-$C_3$ alkyl group optionally substituted with 1 to 3 halogen atoms" is a group in which a $C_1$-$C_3$ alkyl group as described above is substituted with a halogen atom, and examples thereof include substituents such as a fluoromethyl group, a difluoromethyl group, and a trifluoromethyl group.

In the present invention, a "4- to 6-membered nitrogen-containing saturated heterocyclic ring" is a saturated ring containing a nitrogen atom in the ring, and examples thereof include an azetidine ring, a pyrrolidine ring, and a piperidine ring.

Next, suitable substituents in Formula (I) are described.

In a suitable combination of $R^1$, $R^2$, $R^3$, and $R^4$, $R^1$ is a methyl group, $R^2$ is Formula (II) as described above, $R^3$ is a fluorine atom, and $R^4$ is a hydrogen atom.

In a suitable combination of $R^5$ and $R^6$, $R^5$ is a benzene ring or a pyrazole ring; $R^6$ is an oxadiazolyl group optionally substituted with a methyl group, a pyridyl group optionally substituted with 1 or 2 substituents independently selected from the group consisting of a fluorine atom and a methyl group, or -CO-NH-Y; and Y is a tert-butyl group optionally substituted with one fluorine atom.

In another embodiment, a preferred $R^5$ is any one of $R^{51}$ to $R^{54}$ below.

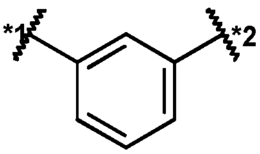
$R^{51}$

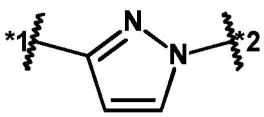
$R^{52}$

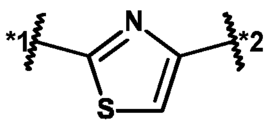
$R^{53}$

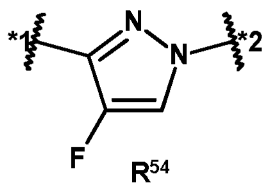
$R^{54}$

*1 is bonded to an oxygen atom, and *2 is bonded to $R^6$.

In another embodiment, a more preferred $R^5$ is $R^{52}$ as described above.

In another embodiment, a preferred $R^6$ is any one of $R^{61}$ to $R^{632}$ below.

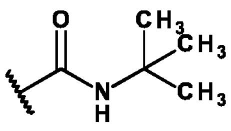
$R^{61}$

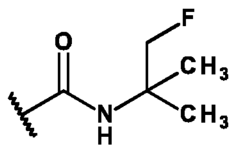
$R^{62}$

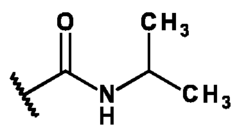
$R^{63}$

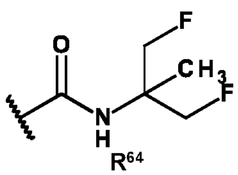
$R^{64}$

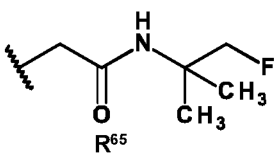
$R^{65}$

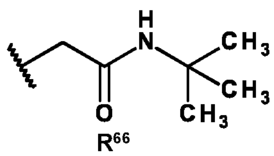
$R^{66}$

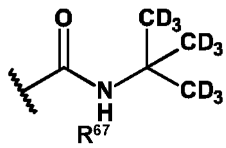
$R^{67}$

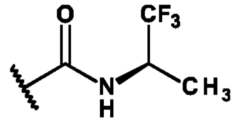
$R^{68}$

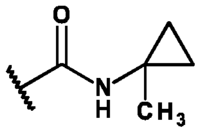
$R^{69}$

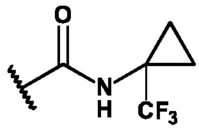
$R^{610}$

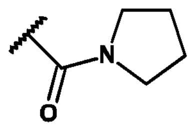
$R^{611}$

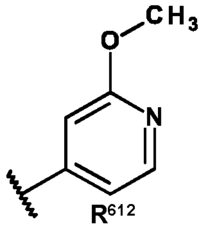
$R^{612}$

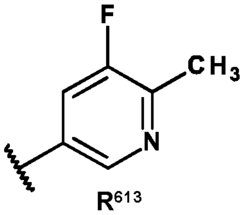
$R^{613}$

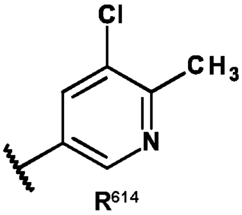
$R^{614}$

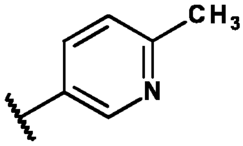
$R^{615}$

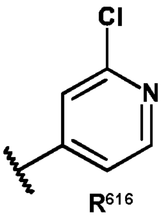
$R^{616}$

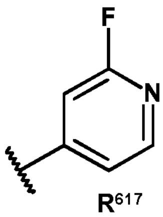
$R^{617}$

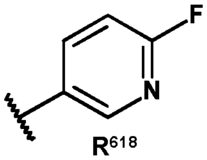
$R^{618}$

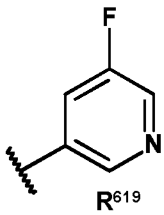
$R^{619}$

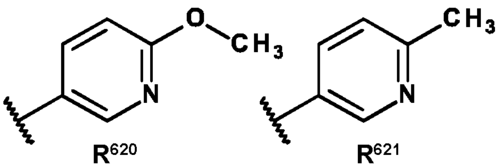
$R^{620}$ $R^{621}$

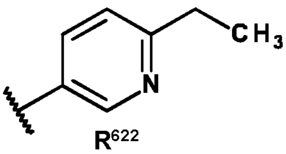
$R^{622}$

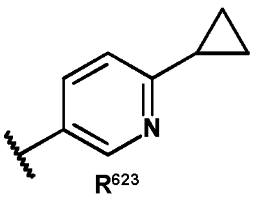
$R^{623}$

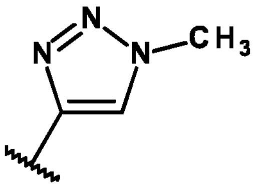
$R^{624}$

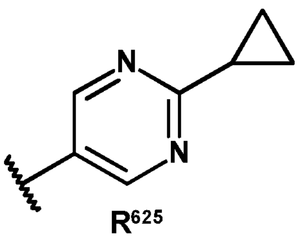
$R^{625}$

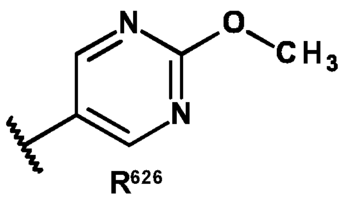
$R^{626}$

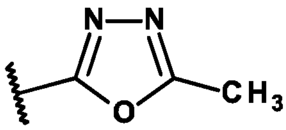
$R^{627}$

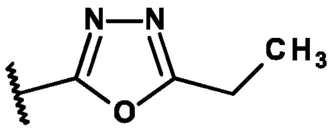
$R^{628}$

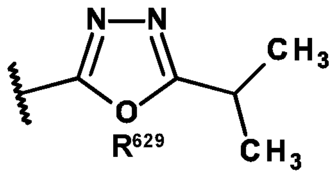
$R^{629}$

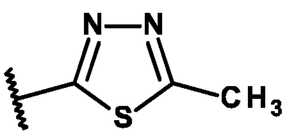
$R^{630}$

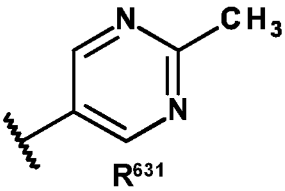
$R^{631}$

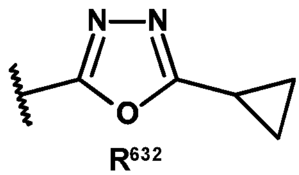
$R^{632}$

In another embodiment, a more preferred $R^6$ is $R^{613}$ as described above.

In another embodiment, a preferred combination of $R^5$ and $R^6$ is any one of $R^{71}$ to $R^{79}$ below.

[Chemical Formula 7]

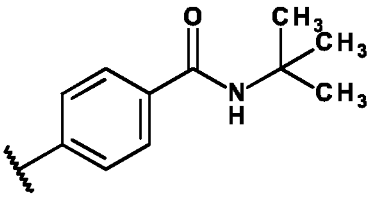

$R^{71}$

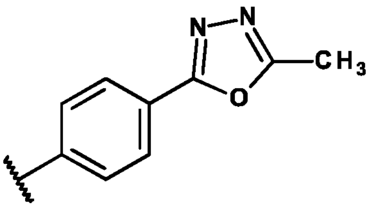

$R^{72}$

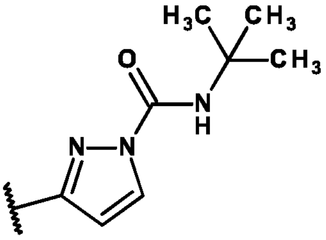

$R^{73}$

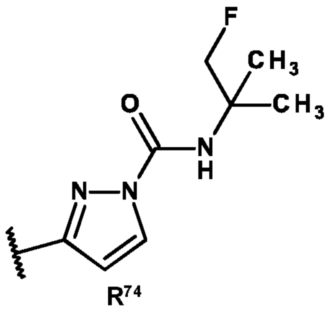

$R^{74}$

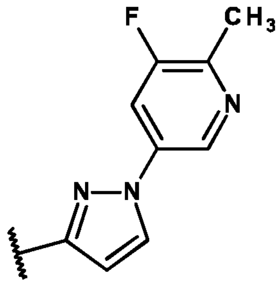

$R^{75}$

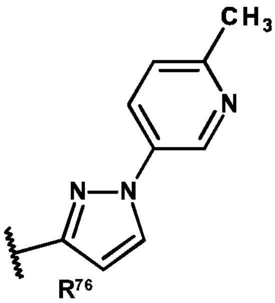

$R^{76}$

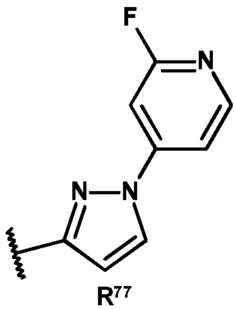

$R^{77}$

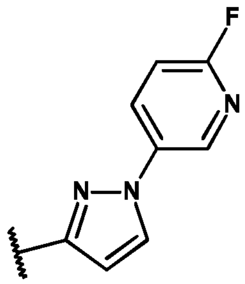

$R^{78}$

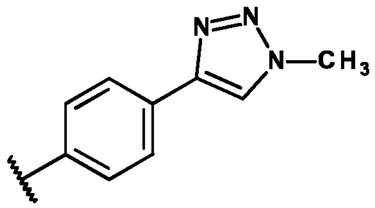

$R^{79}$

In another embodiment, a more preferred combination of $R^5$ and $R^6$ is $R^{75}$ as described above.

In another embodiment, in a suitable combination of $R^1$, $R^2$, $R^3$, $R^4$, $R^5$ and $R^6$, $R^1$ is a methyl group, $R^2$ is a group represented by Formula (II) as described above, $R^3$ is a fluorine atom, $R^4$ is a hydrogen atom, $R^5$ is a benzene ring, and $R^6$ is an oxadiazolyl group optionally substituted with a methyl group, or $-CO-NH-C(CH_3)_3$.

In another embodiment, in a suitable combination of $R^1$, $R^2$, $R^3$, $R^4$, $R^5$ and $R^6$, $R^1$ is a methyl group, $R^2$ is a group represented by Formula (II) as described above, $R^3$ is a fluorine atom, $R^4$ is a hydrogen atom, $R^5$ is a pyrazole ring, $R^6$ is a pyridyl group optionally substituted with 1 or 2 substituents independently selected from the group consisting of a fluorine atom and a methyl group, or -CO-NH-Y; and Y is a tert-butyl group optionally substituted with one fluorine atom.

The compound represented by Formula (I) of the present invention can form a pharmaceutically acceptable salt, if desired. The term "pharmaceutically acceptable salt" refers to a salt which is not significantly toxic and can be used as a medicament. The compound represented by Formula (I) of the present invention can form a salt by reacting with an acid when the compound has a basic group.

When the compound represented by Formula (I) of the present invention has a basic group, the compound can form an acid addition salt by being combined in any proportion with, for example, an acid selected from the group consisting of a hydrohalic acid such as hydrofluoric acid, hydrochloric acid, hydrobromic acid or hydroiodic acid; an inorganic acid such as nitric acid, perchloric acid, sulfuric acid or phosphoric acid; a $C_1$-$C_6$ alkyl sulfonic acid such as methanesulfonic acid, trifluoromethanesulfonic acid or ethanesulfonic acid; an aryl sulfonic acid such as benzenesulfonic acid or p-toluenesulfonic acid; an organic acid such as acetic acid, malic acid, fumaric acid, succinic acid, citric acid, ascorbic acid, tartaric acid, oxalic acid, adipic acid or maleic acid; and an amino acid such as glycine, lysine, arginine, ornithine, glutamic acid or aspartic acid. For example, a hydrochloride includes salts that may be formed, such as monohydrochloride, dihydrochloride, and trihydrochloride; a fumarate includes salts that may be formed, such as mono-fumarate and one-half fumarate; and a citrate includes salts that may be formed, such as mono-citrate, two-third citrate, and one-third citrate.

A pharmaceutically acceptable salt of the compound represented by Formula (I) of the present invention includes both of (i) a salt formed from a compound represented by Formula (I) in which the basic group has been protonated and an acid in which the proton has dissociated, and (ii) an adduct formed from a compound represented by Formula (I) in which the basic group has not been protonated and an acid in which the proton has not dissociated. A "pharmaceutically acceptable salt" of the present invention may mean each one of (i) or (ii) above.

The compound represented by Formula (I) or a pharmaceutically acceptable salt thereof of the present invention may take up a water molecule by being left in the air or by re-crystallization to form a hydrate. Such a hydrate is also included in the compound or salt of the present invention.

The compound represented by Formula (I) or a pharmaceutically acceptable salt thereof of the present invention may absorb a certain kind of solvent by being left in the solvent or by re-crystallization in the solvent to form a solvate. Such a solvate is also included in the compound or salt of the present invention.

Furthermore, a compound that is converted to a compound represented by Formula (I), which is an active ingredient of a pharmaceutical composition of the present invention, by reaction with an enzyme or gastric acid or the like under physiological conditions in vivo, in other words a compound that undergoes enzymatic oxidation, reduction, hydrolysis or the like to change to a compound represented by Formula (I), or a compound that undergoes hydrolysis or the like by gastric acid or the like to change to a compound represented by Formula (I), is included in the present invention as a "pharmaceutically acceptable prodrug compound".

When the compound represented by Formula (I) has an amino group, examples of the prodrug as described above include a compound in which the amino group is acylated, alkylated or phosphorylated (for example, a compound in which the amino group is eicosanoylated, alanylated, pentylaminocarbonylated, (5-methyl-2-oxo-1,3-dioxolen-4-yl)methoxycarbonylated, tetrahydrofuranylated, pyrrolidyl methylated, pivaloyloxymethylated or tert-butylated). When the compound represented by Formula (I) has a hydroxy group, examples of the prodrug include a compound in which the hydroxy group is acylated, alkylated, phosphorylated or borated (for example, a compound in which the hydroxy group is acetylated, palmitoylated, propanoylated, pivaloylated, succinylated, fumarylated, alanylated or dimethylaminomethylcarbonylated). When the compound represented by Formula (I) has a carboxy group, examples of the prodrug include a compound in which the carboxy group is esterified or amidated (for example, a compound in which the carboxy group is ethyl-esterified, phenyl-esterified, carboxymethyl-esterified, dimethylaminomethyl-esterified, pivaloyloxymethyl-esterified, ethoxycarbonyloxyethyl-esterified or methylamidated) .

The prodrug in the present invention can be produced from the compound represented by Formula (I) by a known method. The prodrug in the present invention also includes a compound that changes to the compound represented by Formula (I) under physiological conditions, as described in "Iyakuhin no kaihatsu (Drug development)", volume 7 Molecular Design, pp. 163-198, 1990, published by Hirokawa-Shoten Ltd.

The compound represented by Formula (I) or a pharmaceutically acceptable salt thereof of the present invention encompasses all stereoisomers thereof.

In the compound represented by Formula (I) or a pharmaceutically acceptable salt thereof of the present invention, these isomers and mixtures of these isomers are all represented by a single formula, i.e., Formula (I). Thus, the present invention includes all of these isomers and mixtures of these isomers in any proportion.

The compound represented by Formula (I) or a pharmaceutically acceptable salt thereof of the present invention may also contain an atomic isotope at a non-natural proportion in one or more of the atoms that constitute the compound. Examples of the atomic isotope include deuterium ($^{2}H$) , tritium ($^{3}H$) , iodine-125 ($^{125}I$) and carbon-14 ($^{14}C$). Furthermore, the compound may be radiolabeled with radioisotopes such as tritium ($^{3}H$), iodine-125 ($^{125}I$) or carbon-14 ($^{14}C$) . The radiolabeled compounds are useful as a therapeutic or prophylactic agent, a research reagent such as an assay reagent, and a diagnostic agent such as an in vivo imaging diagnostic agent. All isotopic variants of the compound of the present invention, whether they are radioactive or not, are encompassed within the scope of the present invention.

Another aspect of the present invention is a crystal of the compound represented by Formula (I) or a pharmaceutically acceptable salt thereof.

In the present invention, the term "crystal" refers to a solid whose internal structure consists of regular repetitions of constituent atoms or molecules in three dimensions, and is distinguished from an amorphous solid or non-crystalline body that does not have such a regular internal structure.

In the present invention, a crystal encompasses a crystal of the compound represented by Formula (I), a crystal of a hydrate of the compound represented by Formula (I), a crystal of a solvate of the compound represented by Formula (I), a crystal of a pharmaceutically acceptable salt of the compound represented by Formula (I), a crystal of a hydrate of a pharmaceutically acceptable salt of the compound represented by Formula (I), and a crystal of a solvate of a pharmaceutically acceptable salt of the compound represented by Formula (I).

The crystal of a hydrate of the present invention can take the form of, for example, a 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9 or 5.0 hydrate, and the amount of hydrated water may be increased or decreased according to humidity.

In the present invention, confirmation that the compound represented by Formula (I) or a pharmaceutically acceptable salt thereof is in the form of crystal can be performed by observation with a polarization microscope, by powder X-ray crystallography analysis, or by single-crystal X-ray diffraction measurements. Furthermore, the crystal type can be identified by comparing the crystal characteristics with data based on each indicator which has been measured in advance. According to a preferred aspect of the present invention, the crystal in the present invention is one that can be confirmed to be a crystal using such measurement means.

In the present invention, not only crystals whose diffraction angles in powder X-ray diffraction match perfectly, but also crystals whose diffraction angles match within the range of ±0.2 are included in the present invention. This is a common practice because there are inherent variations in peak values due to differences in instrumentation, samples, and sample preparation. Since an error in the diffraction angle (2θ) in powder X-ray diffraction may occur within the range of ±0.2, it is necessary to understand that the value of the above diffraction angle includes a numerical value within the range of ±0.2.

The crystals of the present invention (hereinafter sometimes referred to as "crystal of Example 64 of the present invention", "crystal of Example 65 of the present invention", "crystal of Example 66 of the present invention", "crystal of Example 67 of the present invention", "crystal of Example 68 of the present invention", "crystal of Example 69 of the present invention", "crystal of Example 70 of the present invention", "crystal of Example 71 of the present invention", "crystal of Example 72 of the present invention", "crystal of Example 73 of the present invention", "crystal of Example 74 of the present invention", "crystal of Example 75 of the present invention", and "crystal of Example 76 of the present invention", respectively) can be supplied stably as a crystal of a drug substance used in the production of a medicament, and have excellent hygroscopicity or stability. The differences in these crystalline forms are distinguished, in particular, by powder X-ray diffraction.

The crystal of Example 64 of the present invention has peaks at diffraction angles (2θ) of 5.78±0.2, 15.48±0.2, 16.38±0.2, 17.24±0.2, 19.28±0.2, 19.90±0.2, 20.42±0.2, 20.82±0.2, 22.04±0.2, and 24.50±0.2 in powder X-ray diffraction with CuKα radiation.

The crystal of Example 64 of the present invention can take the form of, for example, a 0.1, 0.2, 0.3, 0.4, 0.5, 0.6, 0.7, 0.8, 0.9, or 1.0 hydrate. Preferably, the crystal of Example 64 is in the form of a 0.2 hydrate.

The crystal of Example 65 of the present invention has peaks at diffraction angles (2θ) of 4.26±0.2, 8.66±0.2, 13.64±0.2, 14.34±0.2, 14.98±0.2, 17.60±0.2, 19.08±0.2, 22.10±0.2, 23.02±0.2 and 25.88±0.2 in powder X-ray diffraction with CuKα radiation.

The crystal of Example 65 of the present invention can take the form of, for example, a 0.5, 0.6, 0.7, 0.8, 0.9, 1.0, 1.1, 1.2, 1.3, 1.4, or 1.5 hydrate. Preferably, the crystal of Example 65 is in the form of a 1.0 hydrate.

The crystal of Example 66 of the present invention has peaks at diffraction angles (2θ) of 8.08±0.2, 10.32±0.2, 12.90±0.2, 13.48±0.2, 13.82±0.2, 15.44±0.2, 19.76±0.2, 23.60±0.2, 24.24±0.2 and 25.90±0.2 in powder X-ray diffraction with CuKα radiation.

The crystal of Example 66 of the present invention can take the form of, for example, a 1.0, 1.1, 1.2, 1.3, 1.4, 1.5, 1.6, 1.7, 1.8, 1.9, or 2.0 hydrate. Preferably, the crystal of Example 66 is in the form of a 1.5 hydrate.

The crystal of Example 67 of the present invention has peaks at diffraction angles (2θ) of 8.14±0.2, 10.56±0.2, 13.10±0.2, 15.16±0.2, 15.50±0.2, 15.92±0.2, 19.30±0.2, 20.18±0.2, 23.92±0.2, and 25.54±0.2 in powder X-ray diffraction with CuKα radiation.

The crystal of Example 67 of the present invention can take the form of, for example, a 0.5, 0.6, 0.7, 0.8, 0.9, 1.0, 1.1, 1.2, 1.3, 1.4, or 1.5 hydrate. Preferably, the crystal of Example 67 is in the form of a 1.0 hydrate.

The crystal of Example 68 of the present invention has peaks at diffraction angles (2θ) of 6.72±0.2, 8.38±0.2, 11.10±0.2, 13.62±0.2, 16.28±0.2, 17.92±0.2, 19.02±0.2, 21.66±0.2, 22.40±0.2 and 25.64±0.2 in powder X-ray diffraction with CuKα radiation.

The crystal of Example 68 of the present invention can take the form of, for example, a 2.5, 2.6, 2.7, 2.8, 2.9, 3.0, 3.1, 3.2, 3.3, 3.4, or 3.5 hydrate. Preferably, the crystal of Example 68 is in the form of a 3.0 hydrate.

The crystal of Example 68 of the present invention can take the form of, for example, mono-, di-, or trimethanesulfonate. Preferably, the crystal of Example 68 is in the form of a monomethanesulfonate.

The crystal of Example 69 of the present invention has peaks at diffraction angles (2θ) of 5.74±0.2, 10.32±0.2, 11.58±0.2, 14.62±0.2, 14.94±0.2, 18.72±0.2, 19.60±0.2, 20.84±0.2, 22.96±0.2 and 26.42±0.2 in powder X-ray diffraction with CuKα radiation.

The crystal of Example 69 of the present invention can take the form of, for example, a 0.1, 0.2, 0.3, 0.4, 0.5, 0.6, 0.7, 0.8, 0.9, or 1.0 hydrate. Preferably, the crystal of Example 69 is in the form of a 0.2 hydrate.

The crystal of Example 69 of the present invention can take the form of, for example, 1/2- or mono-1,5-naphthalenedisulfonate. Preferably, the crystal of Example 69 is in the form of a 1/2 1,5-naphthalenedisulfonate.

The crystal of Example 70 of the present invention has peaks at diffraction angles (2θ) of 3.56±0.2, 7.24±0.2, 15.02±0.2, 16.84±0.2, 17.68±0.2, 20.26±0.2, 21.88±0.2, 22.92±0.2, 25.76±0.2 and 27.08±0.2 in powder X-ray diffraction with CuKα radiation.

The crystal of Example 70 of the present invention can take the form of, for example, a 0.5, 0.6, 0.7, 0.8, 0.9, 1.0, 1.1, 1.2, 1.3, 1.4, or 1.5 hydrate. Preferably, the crystal of Example 70 is in the form of a 1.0 hydrate.

The crystal of Example 70 of the present crystal can take the form of, for example, mono-, di-, or trimethanesulfonate. Preferably, the crystal of Example 70 is in the form of a monomethanesulfonate.

The crystal of Example 71 of the present invention has peaks at diffraction angles (2θ) of 6.22±0.2, 12.16±0.2, 13.60±0.2, 16.26±0.2, 18.50±0.2, 19.58±0.2, 20.58±0.2, 21.06±0.2, 23.30±0.2, and 25.76±0.2 in powder X-ray diffraction with CuKα radiation.

The crystal of Example 71 of the present invention can take the form of, for example, a 1.0, 1.1, 1.2, 1.3, 1.4, 1.5, 1.6, 1.7, 1.8, 1.9, or 2.0 hydrate. Preferably, the crystal of Example 71 is in the form of a 1.4 hydrate.

The crystal of Example 71 of the present invention can take the form of, for example, mono-, di-, tri-, or tetrabenzenesulfonates. Preferably, the crystal of Example 71 is in the form of a monobenzenesulfonate.

The crystal of Example 72 of the present invention has peaks at diffraction angles (2θ) of 3.58±0.2, 14.50±0.2, 16.50±0.2, 24.28±0.2, 24.70±0.2, 24.98±0.2, 25.76±0.2, 26.12±0.2, 26.60±0.2 and 27.40±0.2 in powder X-ray diffraction with CuKα radiation.

The crystal of Example 72 of the present invention can take the form of, for example, a 3.5, 3.6, 3.7, 3.8, 3.9, 4.0, 4.1, 4.2, 4.3, 4.4, or 4.5 hydrate. Preferably, the crystal of Example 72 is in the form of a 3.8 hydrate.

The crystal of Example 72 of the present invention can take the form of, for example, 1/2-, mono-, 3/2-, or ditartrates. Preferably, the crystal of Example 72 is in the form of a monotartrate.

The crystal of Example 73 of the present invention has peaks at diffraction angles (2θ) of 7.36±0.2, 8.74±0.2, 13.62±0.2, 15.32±0.2, 16.32±0.2, 17.56±0.2, 19.02±0.2, 19.44±0.2, 21.28±0.2, and 25.02±0.2 in powder X-ray diffraction with CuKα radiation.

The crystal of Example 73 of the present invention can take the form of, for example, a 1.0, 1.1, 1.2, 1.3, 1.4, 1.5, 1.6, 1.7, 1.8, 1.9, or 2.0 hydrate. Preferably, the crystal of Example 73 is in the form of a 1.2 hydrate.

The crystal of Example 73 of the present invention can take the form of, for example, 1/2-, mono-, 3/2-, or dicitrate. Preferably, the crystal of Example 73 is in the form of a monocitrate.

The crystal of Example 74 of the present invention has peaks at diffraction angles (2θ) of 5.28±0.2, 5.98±0.2, 7.70±0.2, 8.28±0.2, 10.64±0.2, 12.60±0.2, 13.48±0.2, 16.68±0.2, 17.66±0.2, and 20.80±0.2 in powder X-ray diffraction with CuKα radiation.

The crystal of Example 74 of the present invention can take the form of, for example, a 3.5, 3.6, 3.7, 3.8, 3.9, 4.0, 4.1, 4.2, 4.3, 4.4, or 4.5 hydrate. Preferably, the crystal of Example 74 is in the form of a 4.0 hydrate.

The crystal of Example 74 of the present invention can take the form of, for example, mono-, di-, tri-, or tetrahydrochloride. Preferably, the crystal of Example 74 is in the form of a monohydrochloride.

The crystal of Example 75 of the present invention has peaks at diffraction angles (2θ) of 8.50±0.2, 13.98±0.2, 15.56±0.2, 16.94±0.2, 18.28±0.2, 19.52±0.2, 20.04±0.2, 25.16±0.2, 25.44±0.2, and 26.10±0.2 in powder X-ray diffraction with CuKα radiation.

The crystal of Example 75 of the present invention can take the form of, for example, a 1.5, 1.6, 1.7, 1.8, 1.9, 2.0, 2.1, 2.2, 2.3, 2.4, or 2.5 hydrate. Preferably, the crystal of Example 75 is in the form of a 2.0 hydrate.

The crystal of Example 75 of the present invention can take the form of, for example, 1/2-, mono-, 3/2-, or di-1,5-naphthalenedisulfonate. Preferably, the crystal of Example 75 is in the form of a 1/2 1,5-naphthalenedisulfonate.

The crystal of Example 76 of the present invention has peaks at diffraction angles (2θ) of 5.34±0.2, 7.32±0.2, 7.86±0.2, 8.68±0.2, 13.56±0.2, 16.26±0.2, 17.50±0.2, 19.36±0.2, 21.22±0.2, and 24.90±0.2 in powder X-ray diffraction with CuKα radiation.

The crystal of Example 76 of the present invention can take the form of, for example, a 1.5, 1.6, 1.7, 1.8, 1.9, 2.0, 2.1, 2.2, 2.3, 2.4, or 2.5 hydrate. Preferably, the crystal of Example 76 is in the form of a 2.0 hydrate.

The crystal of Example 76 of the present invention can take the form of, for example, 1/2-, mono-, 3/2-, or dicitrate. Preferably, the crystal of Example 76 is in the form of a monocitrate.

In the present invention, the term "tumor" is not limited to a malignant tumor, and includes all types of tumors such as carcinoma, sarcoma, and a benign tumor. In particular, a malignant tumor is sometimes referred to as "cancer".

As used herein, "treat" and derivative terms thereof mean remission, amelioration and/or a delay in worsening of a clinical symptom of cancer in a patient developing the cancer.

EGFR is a tyrosine kinase-type receptor that performs signaling by recognizing factors involved in cell proliferation. EGFR is present on the cell membrane, and when this receptor is activated, cell differentiation and proliferation occur. EGFR can be seen in many cells, and excessive expression and gene mutation of EGFR can lead to cancer, infiltration and metastasis. In various cancers including non-small cell lung cancer and colon cancer, EGFR is overexpressed or genetically mutated in the cancer cells, and proliferation of the cancer cells is active. Furthermore, it is known that cells that are overexpressed or genetically mutated exhibit higher metastaticity than cells that are not. Inhibition of phosphorylation of EGFR tyrosine kinases can block the signaling necessary for proliferation of cancer cells, thereby suppressing proliferation of cancer cells.

For mutations in the EGFR gene, it is known that activation mutations such as mutations in which leucine 858 of the coding protein in the EGFR protein is substituted by arginine (L858R), deletion mutations in exon 19 (exon19del), mutations in which glycine 719 of the coding protein is substituted by another amino acid (G719X), mutations in which leucine 861 of the coding protein is substituted by glutamine (L861Q), can be seen in non-small cell lung cancer and the like.

The exon 20 insertion mutation in an EGFR gene is a mutation that occurs by insertion of a base into an exon 20 region of the EGFR gene. As a result, EGFR proteins in which 1 amino acid residue to 4 amino acid residues are inserted are generated. Insertion mutations of 3 amino acid residues are frequently found, and for example, V769_D770ins.ASV in which ASV is inserted between valine 769 and aspartic acid 770 of the coding protein, D770_N771ins.SVD in which SVD is inserted between aspartic acid 770 and aspartic acid 771, and H773_V774ins.NPH in which NPH is inserted between histidine 773 and valine 774 of the coding protein and the like are known.

HER2 is one of the representative growth factor receptor-type oncogene products identified as a human epithelial cell growth factor receptor-type 2 associated oncogene, and is a transmembrane receptor protein having a tyrosine kinase domain with a molecular weight of 185 kDa. HER2 is one of the EGFR family members consisting of HER1 (EGFR, ErbB-1), HER2 (neu, ErbB-2), HER3 (ErbB-3), and HER4 (ErbB-4). It is known that intracellular tyrosine residues in HER2 are autophosphorylated by formation of a homodimer or heterodimer with HER1, HER3, or HER4, which are other EGFR to be activated, thereby HER2 plays an important role in cell proliferation, differentiation, and survival in normal cells and tumor cells.

The exon 20 insertion mutation in a HER2 gene is a mutation that occurs by insertion of a base into an exon 20 region of the HER2 gene. As a result, HER2 proteins in which 1 amino acid residue to 4 amino acid residues are inserted are generated. Insertion mutations of 4 amino acid residues are frequently found. Examples thereof include A775_G776 ins. YVMA in which YVMA is inserted between alanine 775 and glycine 776; E770_A771 ins.AYVM in which AYVM is inserted between glutamic acid 770 and alanine 771; A771_Y772 ins.YVMA in which YVMA is inserted between alanine 771 and tyrosine 772; and Y772_A775dup in which residues from tyrosine 772 to alanine 775 are duplicated.

The inhibitory effect on an EGFR tyrosine kinase and/or a HER2 tyrosine kinase in the present invention can be measured by a method for measuring the kinase inhibitory activity ordinarily used by a person skilled in the art.

The cell proliferation inhibitory activity of the compound represented by Formula (I) or a pharmaceutically acceptable salt thereof of the present invention can be examined using a cell proliferation inhibition test method ordinarily used by a person skilled in the art. For example, the cell proliferation inhibitory activity can be measured by the method of Test Example 1.

Furthermore, the antitumor activity in vivo can be examined using an antitumor test method ordinarily used by a person skilled in the art. For example, the antitumor activity can be measured by the method of Test Example 2.

The compound represented by Formula (I) or a pharmaceutically acceptable salt thereof of the present invention can be used for treating a tumor. For example, the compound represented by Formula (I) or a pharmaceutically acceptable salt thereof can be used for treating lung cancer, breast cancer, bladder cancer, ovarian cancer or the like. In particular, the compound represented by Formula (I) or a pharmaceutically acceptable salt thereof can be used for treating a tumor having exon 20 insertion mutations in an EGFR gene and/or a HER2 gene.

The presence of a mutation in the EGFR gene and/or the HER2 gene can be confirmed, for example, by examining the base sequence of genomic DNA.

The compound represented by Formula (I) or a pharmaceutically acceptable salt thereof of the present invention may be used in combination with another antitumor agent. Examples of another antitumor agent include an alkylating agent, an antimetabolite, an antitumor antibiotic, an antineoplastic plant component, a biological response modifier (BRM), a hormone, a vitamin, an antineoplastic antibody, a molecular targeting agent, and other antitumor agents.

More specifically, examples of the alkylating agent include alkylating agents such as nitrogen mustard, nitrogen mustard-N-oxide, or chlorambutil; aziridine-based alkylating agents such as carbocone or thiotepa; epoxide-based alkylating agents such as dibromomannitol or dibromodulcitol; nitrosourea-based alkylating agents such as carmustine, lomustine, semustine, nimustine hydrochloride, streptozocin, chlorzotocin, and ranimustine; busulfan; improsulfan tosylate; and dacarbazine.

Examples of the antimetabolite include purine antimetabolites such as 6-mercaptopurine, 6-thioguanine, or thioinosine; pyrimidine antimetabolites such as fluorouracil, tegafur, tegafur uracil, carmofur, doxifluridine, broxuridine, cytarabine, or enocitabine; and folate antimetabolites such as methotrexate or trimetrexate.

Examples of the antitumor antibiotic include anthracycline-based antibiotic antitumor agents such as mitomycin C, bleomycin, peplomycin, daunorubicin, aclarubicin, doxorubicin, pirarubicin, THP-adriamycin, 4'-epidoxorubicin and epirubicin; chromomycin A3; and actinomycin D.

Examples of the antineoplastic plant component include vinca alkaloids such as vindesine, vincristine or vinblastine; taxanes such as paclitaxel and docetaxel; and epipodophyllotoxins such as etoposide and teniposide.

Examples of the BRM include tumor necrosis factors and indomethacin.

Examples of the hormone include hydrocortisone, dexamethasone, methylprednisolone, prednisolone, prasterone, betamethasone, triamcinolone, oxymetholone, nandrolone, methenolone, fosfestrol, ethinyl estradiol, chlormadinone, and medroxyprogesterone.

Examples of the vitamin include vitamin C and vitamin A.

Examples of the antineoplastic antibody and the molecular targeting agent include trastuzumab, rituximab, cetuximab, nimotuzumab, denosumab, bevacizumab, infliximab, imatinib, gefitinib, erlotinib, sunitinib, lapatinib, sorafenib, dasatinib, nilotinib, vemurafenib, and osimertinib.

Examples of the other antitumor agents include cisplatin, carboplatin, oxaliplatin, tamoxifen, camptothecin, ifosfamide, cyclophosphamide, melphalan, L-asparaginase, aceclatone, schizophyllan, picibanil, procarbazine, pipobroman, neocarzinostatin, hydroxyurea, ubenimex, and krestin.

The compound represented by Formula (I) or a pharmaceutically acceptable salt thereof of the present invention can be administered in various forms. Examples of such administration forms include oral administration with tablets, capsules, granules, emulsions, pills, powders, syrups (liquids) or the like, or parenteral administration with injectables (intravenous, intramuscular, subcutaneous, or intraperitoneal administration), infusions, suppositories (rectal administration) or the like. These various formulations can be formulated in accordance with ordinary methods by using the principal agent with auxiliary agents that are ordinarily used in the technical field of formulation of medicaments, such as an excipient, a binder, a disintegrant, a lubricant, a flavor modifier, a dissolution aid, a suspending agent, and a coating agent.

When used as tablets, examples of the usable carrier include excipients such as lactose, white sugar, sodium chloride, glucose, urea, starch, calcium carbonate, kaolin, crystalline cellulose, and silicic acid; binders such as water, ethanol, propanol, simple syrup, liquid glucose, liquid starch, gelatin solution, carboxymethylcellulose, shellac, methylcellulose, potassium phosphate, and polyvinylpyrrolidone; disintegrating agents such as dry starch, sodium alginate, agar powder, laminaran powder, sodium hydrogen carbonate, calcium carbonate, polyoxyethylene sorbitan fatty acid ester, sodium lauryl sulfate, stearic acid monoglyceride, starch, and lactose; disintegration inhibiting agents such as white sugar, stearin, cocoa butter, and hydrogenated oil; absorption promoting agents such as quaternary ammonium salts and sodium lauryl sulfate; moisturizing agents such as glycerin and starch; adsorbents such as starch, lactose, kaolin, bentonite, and colloidal silica; and lubricating agents such as purified talc, stearic acid salts, boric acid powder, and polyethylene glycol. When necessary, tablets that have been coated with a normal coating agent, such as sugar-coated tablets, gelatin-coated tablets, enteric-coated tablets, film-coated tablets, or double-layer tablets, or multi-layer tablets, can be used.

When used as pills, examples of the usable carrier include excipients such as glucose, lactose, cocoa butter, starch, hardened vegetable oil, kaolin, and talc; binders such as gum arabic powder, tragacanth powder, gelatin, and ethanol; and disintegrants such as laminaran and agar.

When used as suppositories, materials that are conventionally known in the art as carriers can be widely used, and examples thereof include polyethylene glycol, cocoa butter, higher alcohols, esters of higher alcohols, gelatin, and semi-synthetic glycerides.

When used as injections, use as a liquid, emulsion or suspension agent may be possible. It is preferred that such a liquid, emulsion or suspension agent is sterilized, and isotonic with blood. The solvent used in the production of the liquid, emulsion or suspension agent is not particularly limited as long as it can be used as a medical diluent, and examples thereof include water, ethanol, propylene glycol, ethoxylated isostearyl alcohol, polyoxylated isostearyl alcohol, and polyoxyethylene sorbitan fatty acid esters. In this case, the formulation may contain a sufficient amount of salt, glucose or glycerin to prepare an isotonic solution, and may contain a normal dissolution aid, a buffer, a soothing agent, or the like.

The above formulations can also contain colorants, preservatives, fragrances, flavors, sweeteners, and the like, as needed, as well as other pharmaceuticals.

The amount of the compound contained in the formulation is not particularly limited and is suitably selected in a wide range, but the compound is usually contained at 0.5 to 70% by weight, preferably 1 to 30% by weight, of the total composition.

The amount used varies depending on the symptoms, age, or the like of the patient (warm-blooded animal, especially human), and in the case of oral administration, it is preferred that a dose with 2000 mg (preferably 100 mg) as the upper limit and 0.1 mg (preferably 1 mg, even more preferably 10 mg) as the lower limit is administered once to six times per day to a human adult depending on the symptoms.

Next, representative methods for producing the compound represented by Formula (I) are described. The compound of the present invention can be produced by various production methods. The following production methods are examples and the present invention is not to be construed as limited thereto.

The compound represented by Formula (I) or a pharmaceutically acceptable salt thereof of the present invention can be produced by applying a variety of known production methods according to a characteristic based on the basic backbone or the type of the substituent of the compound. Examples of known methods include those described in "ORGANIC FUNCTIONAL GROUP PREPARATIONS", 2nd edition, ACADEMIC PRESS, INC., 1989, "Comprehensive

Organic Transformations", VCH Publishers Inc., 1989 and the like.

Depending on the type of functional group present in the compound, it may be effective in terms of production technique to protect the functional group with an appropriate protecting group at the stage of the raw materials or intermediates, or to substitute the functional group with a group capable of being easily converted to the functional group.

Examples of such functional groups include an amino group, a hydroxy group, a carboxy group, and the like, and examples of their protecting groups include the protecting groups described in T.W. Greene and P.G. Wuts, "Greene's Protective Groups in Organic Synthesis (4th Edition, John Wiley & Sons, Inc., 2006)."

The protecting group or the group capable of being easily converted to the functional group may be appropriately selected depending on each reaction condition of the production methods for producing the compounds.

With such a method, the desired compound can be obtained by introducing the corresponding group and carrying out the reaction, and then removing the protecting group from the corresponding group or converting the corresponding group to a desired group as necessary.

A prodrug of the compound can also be produced by introducing a specific group at the stage of the raw materials or intermediates, or carrying out a reaction using the resulting compound, in the same manner as the protecting group described above. The reaction for producing the prodrug can be performed by applying methods known to a person skilled in the art, such as normal esterification, amidation, dehydration, hydrogenation, or the like.

[Method A]

Compound (a15), the compound represented by Formula (I) wherein $R^2$ is Formula (II), can be produced by the method shown below. Each step need not necessarily be performed in the order shown below as long as it does not affect the reaction substrates and the reaction products.

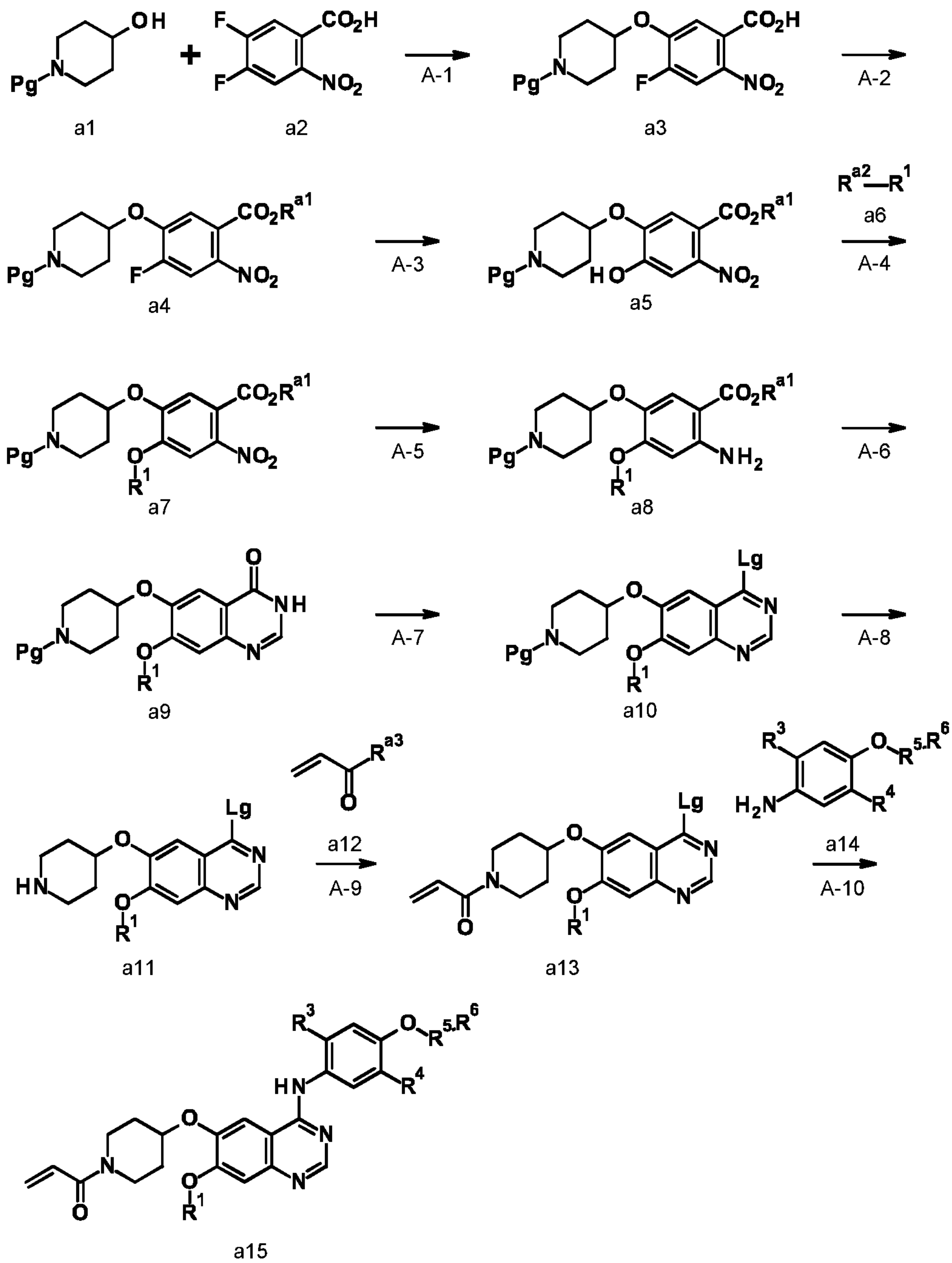


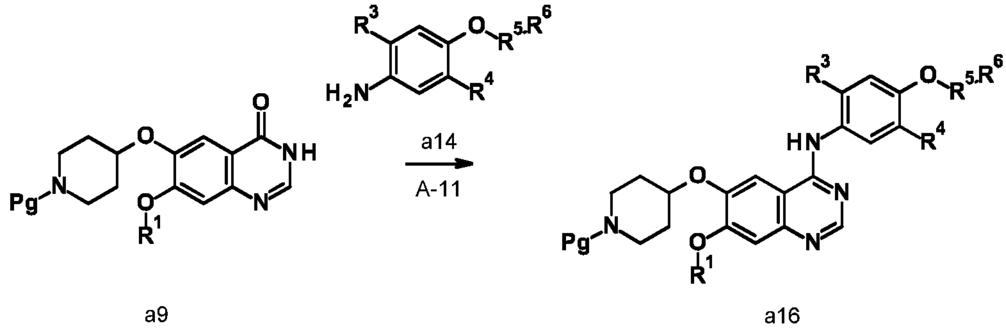


[wherein, $R^1$ is the same as defined in (1) described above; Pg in Method A represents a protecting group for a nitrogen atom, for example, a tert-butoxycarbonyl group; Lg in Method A represents a leaving group, for example, a halogen atom; $R^{a1}$ represents a $C_1$-$C_6$ alkyl group; $R^{a2}$ represents Lg or a hydroxy group; $R^{a3}$ represents a substituent that can be used in a condensation reaction to a nitrogen atom, for example, a halogen atom or a hydroxy group.]

Step A-1 is a step of obtaining Compound a3 from Compound a1 and Compound a2 by a nucleophilic substitution reaction. The nucleophilic substitution reaction in this step can be carried out, for example, by reacting with a base such as sodium hydride in a solvent such as N,N-dimethyldimethylformamide.

Step A-2 is a step of obtaining Compound a4 by protecting the carboxy group of Compound a3 with an ester. The esterification in this step can be carried out, for example, by reacting with an alkylating agent such as methyl iodide in the presence of a base such as potassium carbonate in a solvent such as N,N-dimethyldimethylformamide.

Step A-3 is a step of obtaining Compound a5 by converting the fluorine atom of Compound a4 to a hydroxy group. This step can be carried out, for example, by reacting with N-hydroxyacetamide in the presence of a base such as potassium carbonate in a solvent such as dimethylsulfoxide, and heating.

Step A-4 is a step of obtaining Compound a7 from Compound a5 by alkylating the hydroxy group. This step can be carried out, for example, by reacting with an alkylating agent a6 such as methyl iodide in the presence of a base such as potassium carbonate or sodium hydride in a solvent such as N,N-dimethyldimethylformamide. This step can also be carried out by a Mitsunobu reaction in which alcohol a6 is reacted in the coexistence of a phosphine such as triphenylphosphine and an azodicarboxylic acid ester such as bis(2-methoxyethyl) azodicarboxylate in a solvent such as tetrahydrofuran. Compound a7 can also be obtained by first introducing a substituent having a leaving group into the hydroxy group, and then performing a substitution reaction again.

Step A-5 is a step of converting Compound a7 to Compound a8. The reducing reaction in this step can be carried out, for example, by contact-hydrogen reduction with a noble metal catalyst such as palladium carbon in a solvent such as ethanol or by Bechamp reduction with a metal such as zinc in the presence of an acid such as acetic acid in a solvent such as methanol.

Step A-6 is a step of producing Compound a9 by a cyclization reaction of Compound a8. The cyclization reaction in this step can be carried out, for example, by reacting with a compound such as formamidine acetate in a solvent such as 2-methoxyethanol, and heating.

Step A-7 is a step of introducing a leaving group into Compound a9 to convert to Compound a10. The introduction of the leaving group in this step can be carried out, for example, by reacting with a halogenating agent such as thionyl chloride in a solvent such as N,N-dimethylformamide, and heating.

Step A-8 is a step of obtaining Compound a11 by deprotecting the amino group of Compound a10. The deprotecting reaction in this step can employ a method ordinarily used to deprotect an amino group. For this first line of removal method (reagent, solvent, reaction conditions), any method can be used as long as the method is a deprotection method appropriate for the protecting group used, and does not affect the reaction substrates and the reaction products.

Step A-9 is a step of obtaining Compound a13 by acylating an amino group of Compound a11. The acylation reaction in this step can be carried out by the use of an acylating agent such as an acid chloride or acid anhydride, or a condensation with a carboxylic acid component using a condensing agent, and any method can be used as long as the solvent and the reaction conditions of such method are appropriate for the reaction conditions of this acylation reaction.

Step A-10 is a step of obtaining Compound a15 by introducing Compound a14 into Compound a13. For this step, any method can be used as long as the solvent and the reaction conditions are appropriate for the reaction substrate. For example, this step can be carried out by using Compound a14 or a synthetic intermediate capable of being converted to Compound a14 as a substrate in the coexistence of an acid such as hydrochloric acid or trifluoroacetic acid. This step can also be carried out in the presence of a base such as potassium carbonate or cesium carbonate. Alternatively, even when they are not added, the step can be carried out, for example, in a solvent such as 2-propanol or dimethylsulfoxide.

Step A-11 is a step of obtaining Compound a16 by introducing Compound a14 into Compound a9. The nucleophilic substitution reaction in this step can be carried out using Compound a14 or a synthetic intermediate capable of being converted to Compound a14 as a substrate, for example, by reacting with a phosphonium-based condensing agent such as 1H-benzotriazol-1-yloxy-tris(dimethylamino)phosphonium hexafluorophosphate in the coexistence of a base such as 1,8-diazabicyclo[5,4,0]-7-undecene in a solvent such as acetonitrile.

In Step A-10 or A-11, when a synthetic intermediate capable of being converted to Compound a14 is used as a substrate, the conversion to Compound a15 can be achieved by using the method for producing the compound represented by Formula (III) described below at any synthetic stage that does not affect the reaction substrates and the reaction products.

[Method B]

Compound (b11), the compound represented by Formula (I) wherein $R^2$ is -NH-CO-CH=CH-$CH_2$-X, can be produced by the method shown below. Each step need not necessarily be performed in the order shown below, as long as it does not affect the reaction substrates and the reaction products.

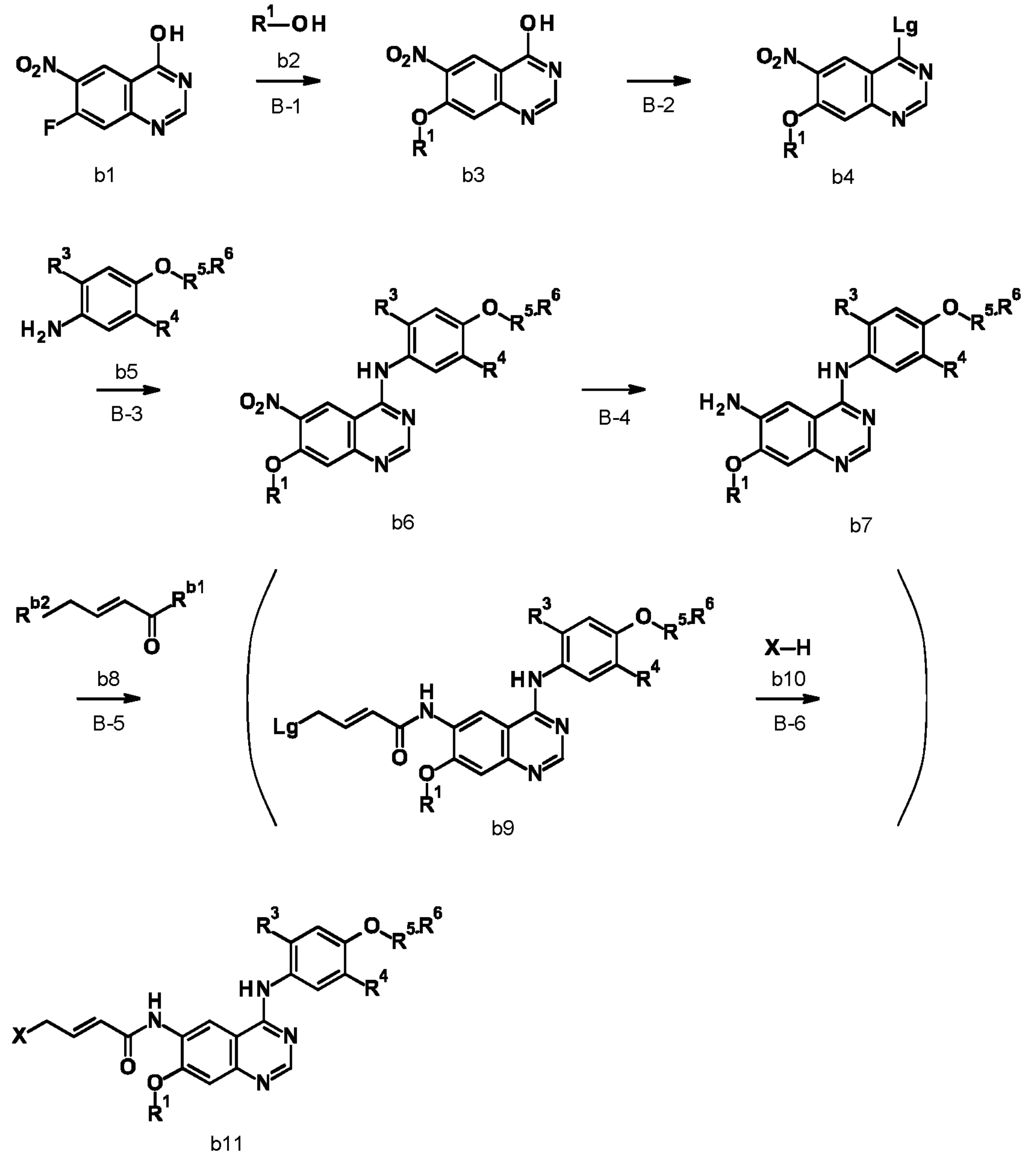


[wherein $R^1$, $R^3$, $R^4$, $R^5$, $R^6$, and X are the same as defined in (1) described above. Lg in Method B represents a leaving group, for example, a halogen atom. $R^{b1}$ represents a substituent that can be used in a condensation reaction with a nitrogen atom, for example, a halogen atom or a hydroxy group. $R^{b2}$ represents X, Lg, or a functional group that can be converted to Lg, for example, a halogen atom or a hydroxy group].

Step B-1 is a step of obtaining Compound b3 from Compound b1 by a nucleophilic substitution reaction. The nucleophilic substitution reaction in this step can be carried out, for example, by reacting with alcohol b2 in the presence of a base such as sodium hydride in a solvent such as N,N-dimethylformamide or tetrahydrofuran, and heating.

Step B-2 is a step of introducing a leaving group into Compound b3 to convert to Compound b4. This step can be performed under the same conditions as in step A-7.

Step B-3 is a step of obtaining Compound b6 by introducing Compound b5 into Compound b4 by a nucleophilic substitution reaction. This step can be carried out under the same conditions as in step A-10 using Compound b5 or a synthetic intermediate capable of being converted to Compound b5 as a substrate.

In step B-3, when a synthetic intermediate capable of being converted to Compound b5 is used as a substrate, the conversion to Compound b11 can be achieved by the method of producing the compound represented by Formula (III) described below at any synthetic stage that does not affect the reaction substrates and the reaction products.

Step B-4 is a step of converting Compound b6 to Compound b7. This step can be carried out under the same conditions as in Step A-5.

Step B-5 is a step of obtaining Compound b11 directly or a synthetic precursor b9 of Compound b11 by condensing Compound b7 and Compound b8. For the acylation reaction in this step, any method can be used as long as the solvent and reaction conditions are appropriate for the reaction substrates. For example, an acylation with an acylating agent such as an acid chloride, acid anhydride or the like, or a condensation reaction with a carboxylic acid using a condensing agent can be used.

Step B-6 is a step of obtaining Compound b11 by carrying out a substitution reaction on Compound b9. The substitution reaction in this step can be carried out, for example, by reacting with a desired amine b10 in the presence of a base such as N,N-diisopropylethylamine in a solvent such as N,N-dimethylformamide.

Next, a method for producing a compound represented by Formula (III) is shown.

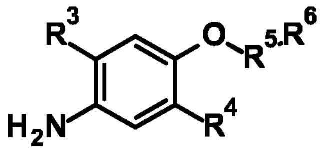

(III)

[wherein $R^3$, $R^4$, $R^5$, and $R^6$ are the same as defined in (1) described above].

A general production method for each reaction site is shown in order, but each step need not necessarily be performed in the order shown below, as long as it does not affect the reaction substrates and the reaction products. In addition, for a compound in which a synthetic intermediate capable of being converted to the compound represented by Formula (III) is introduced into Compound a9, a10, a11, a13, b4 or the like in any step in Methods A and B, the steps below can be carried out in any order as long as they do not affect the reaction substrates and the reaction products.

[Method C]

In the compound represented by Formula (III), the biaryl ether moiety can be produced by Method C.

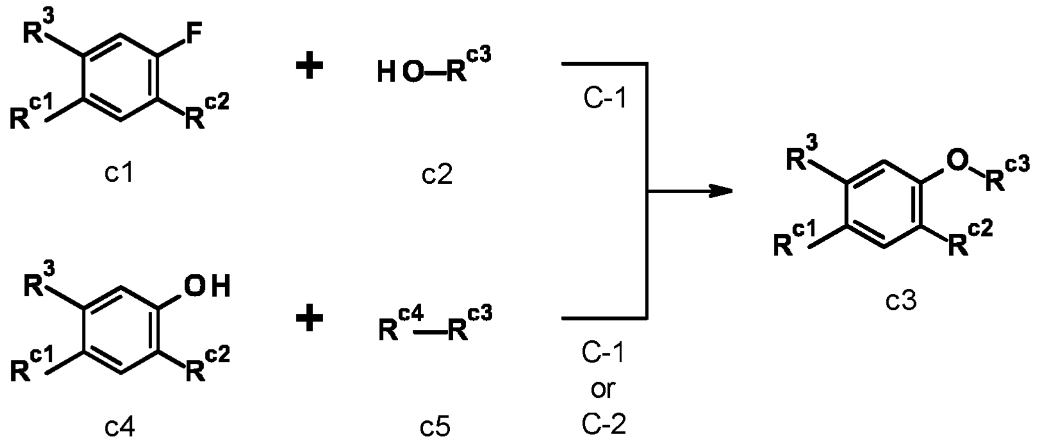


[wherein $R^{c1}$ represents an amino group or a substituent capable of being converted to an amino group, for example, an amino group substituted by a protecting group, a nitro group, an ester group, or a carboxy group. $R^{c2}$ represents $R^4$ or a substituent capable of being converted to $R^4$, for example, a hydrogen atom or a halogen atom. $R^{c3}$ represents $-R^5-R^6$ or a substituent capable of being converted to $-R^5-R^6$, for example, a substituted phenyl group, a substituted pyrazolyl group, or a substituted thiazolyl group. $R^{c4}$ represents a halogen atom. $R^4$, $R^5$, and $R^6$ are the same as defined in (1) described above.]

Step C-1 is a step of obtaining Compound c3 from Compound c1 and Compound c2, or a step of obtaining Compound c3 from Compound c4 and Compound c5. The nucleophilic substitution reaction in this step can be carried out, for example, by reacting with a base such as cesium carbonate, potassium carbonate, or N,N-diisopropylethylamine in a solvent such as dimethylsulfoxide, N,N-dimethylformamide or tetrahydrofuran at a reaction temperature depending on the substrate.

Compound c3 can also be obtained by Step C-2. Step C-2 is a step of obtaining Compound c3 by Ullmann type coupling of Compound c4 and Compound c5. The coupling reaction in this step can be carried out, for example, by reacting with a catalyst such as copper(I) iodide in the coexistence of a ligand such as trans-N,N'-dimethylcyclohexane-1,2-diamine or N,N-dimethylglycine using a base such as cesium carbonate or potassium carbonate in a solvent such

as butyronitrile or 1,4-dioxane, and heating.

[Method D]

In the compound represented by Formula (I) wherein $R^6$ is -CO-N(Y)(Z) or $-CH_2$-CO-N(Y) (Z), the $R^6$ moiety can be produced by Method D.

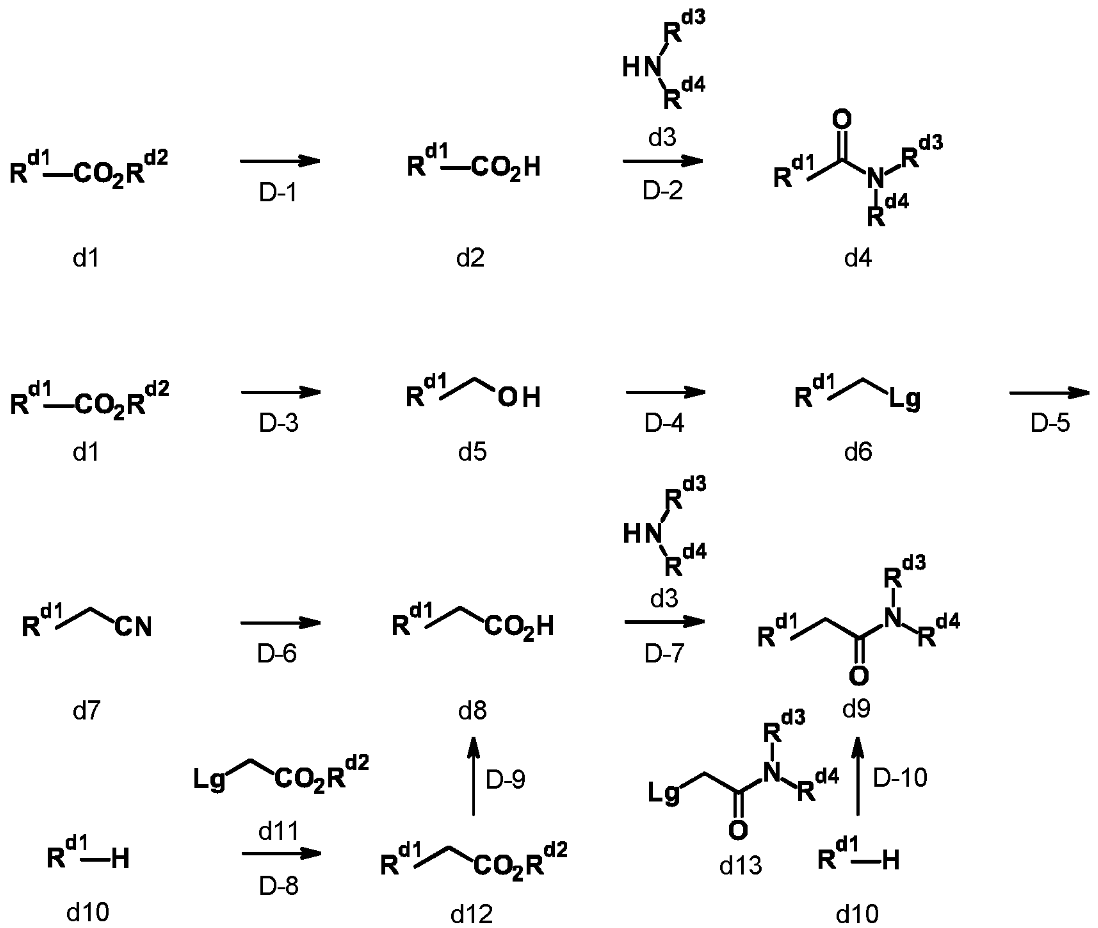


d1 d2 d4

d1 d5 d6

d7 d8 d9

d13

d10 d12 d10

[wherein Lg in Method D represents a leaving group such as a methanesulfonyloxy group. $R^{d1}$ represents a group represented by Formula (IV):

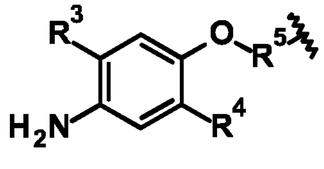
(IV)

[wherein $R^3$, $R^4$, and $R^5$ are the same as defined in (1) described above].
or a substituent capable of being converted to a group represented by Formula (IV). $R^{d2}$ represents a protecting group for a carboxy group, such as an ethyl group or a tert-butyl group. $R^{d3}$ and $R^{d4}$ represent Y, Z, a substituent capable of being converted to Y, or a substituent capable of being converted to Z. Y and Z are the same as defined in (1) described above.]

Step D-1 is a step of converting Compound d1 to Compound d2. This step can be carried out, for example, when $R^{d2}$ is an ethyl group or the like, by reacting with a base such as aqueous sodium hydroxide solution in a solvent such as tetrahydrofuran. This step can be carried out, when $R^{d2}$ is a tert-butyl group, by reacting with an acid such as trifluoroacetic acid in a solvent such as dichloromethane.

Step D-2 is a step of obtaining Compound d4 by condensing Compound d2 and Compound d3. The amidation in this step can be carried out, for example, by reacting with a condensing agent such as 1-[bis(dimethylamino)methylene]-1H-1,2,3-triazolo[4,5-b]pyridinium 3-oxide hexafluorophosphate in the coexistence of a base such as N,N-diisopropylethylamine in a solvent such as N,N-dimethylformamide.

Step D-3 is a step of obtaining Compound d5 by reducing Compound d1. The reducing reaction in this step can be carried out, for example, by reacting with a reducing agent such as diisobutylaluminium hydride in a solvent such as dichloromethane.

Step D-4 is a step of obtaining Compound d6 by converting a hydroxy group of Compound d5 to a leaving group. Examples of the leaving group in this step include a methanesulfonyloxy group and a p-toluenesulfonyloxy group, which can be introduced by a ordinarily used method (reagent, solvent, reaction conditions, or the like).

Step D-5 is a step of obtaining Compound d7 by introducing a cyano group into Compound d6 by a substitution reaction. This step can be carried out, for example, by reacting with sodium cyanide or the like in a solvent such as dimethylsulfoxide, and heating.

Step D-6 is a step of converting Compound d7 to Compound d8. The hydrolysis in this step can be carried out, for example, by reacting with a base such as aqueous sodium hydroxide solution in a solvent such as ethanol, and heating.

Step D-7 is a step of obtaining Compound d9 by condensing Compound d8 and Compound d3. This step can be carried out under the same conditions as in Step D-2.

Step D-8 is a step of obtaining Compound d12 by alkylating Compound d10 with Compound d11. The alkylation in this step can be carried out, for example, by reacting with an alkylating agent such as tert-butyl chloroacetate in the presence of a base such as potassium carbonate in a solvent such as N,N-dimethylformamide.

Step D-9 is a step of obtaining Compound d8 from Compound d12. This step can be carried out under the same conditions as in Step D-1.

Step D-10 is a step of obtaining Compound d9 by alkylating Compound d10 with Compound d13. The alkylation in this step can be carried out, for example, by reacting with an alkylating agent such as N-tert-butyl-2-chloroacetamide in the presence of a base such as potassium carbonate in a solvent such as N,N-dimethylformamide.

[Method E]

In the compound represented by Formula (I) wherein $R^5$ is a triazole ring, the $R^5$-$R^6$ moiety can be produced by Method E.

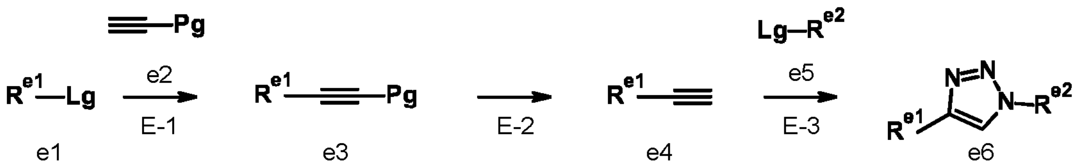


[wherein $R^{e1}$ represents a group represented by Formula (IV) or a substituent capable of being converted to a group represented by Formula (IV). $R^{e2}$ represents an alkyl group. Pg in Method E represents a protecting group for a terminal acetylene, such as a trimethylsilyl group. Lg in Method E represents a leaving group, for example, a halogen atom or a trifluoromethanesulfonyloxy group.]

Step E-1 is a step of introducing an alkynyl group into Compound e1 by a Sonogashira reaction. The Sonogashira reaction in this step can be carried out, for example, by adding Compound e2 in the coexistence of a palladium catalyst such as bis(triphenylphosphine)palladium(II) and a copper catalyst such as copper(I) iodide in a solvent such as triethylamine, and heating.

Step E-2 is a step of obtaining Compound e4 by deprotecting the terminal acetylene. The deprotecting reaction in this step can be carried out by a method ordinarily used to deprotect a terminal acetylene. For example, when Pg is a trimethylsilyl group, the deprotecting reaction can be carried out by reacting with a base such as potassium carbonate in a solvent such as methanol.

Step E-3 is a step of obtaining Compound e6 by a 1,3-dipolar cycloaddition reaction of Compound e4 and an azide compound. The 1,3-dipolar cycloaddition reaction in this step can be carried out, for example, by reacting with an azide source such as sodium azide, Compound e5 (e.g., an alkylating agent such as iodomethane) and a catalyst such as copper(I) iodide in a solvent such as acetonitrile under heating.

[Method F]

In the compound represented by Formula (I) wherein $R^5$ is a 1,3,4-oxadiazole ring or a 1,3,4-thiadiazole ring, the $R^5$-$R^6$ moiety can be produced by Method F.

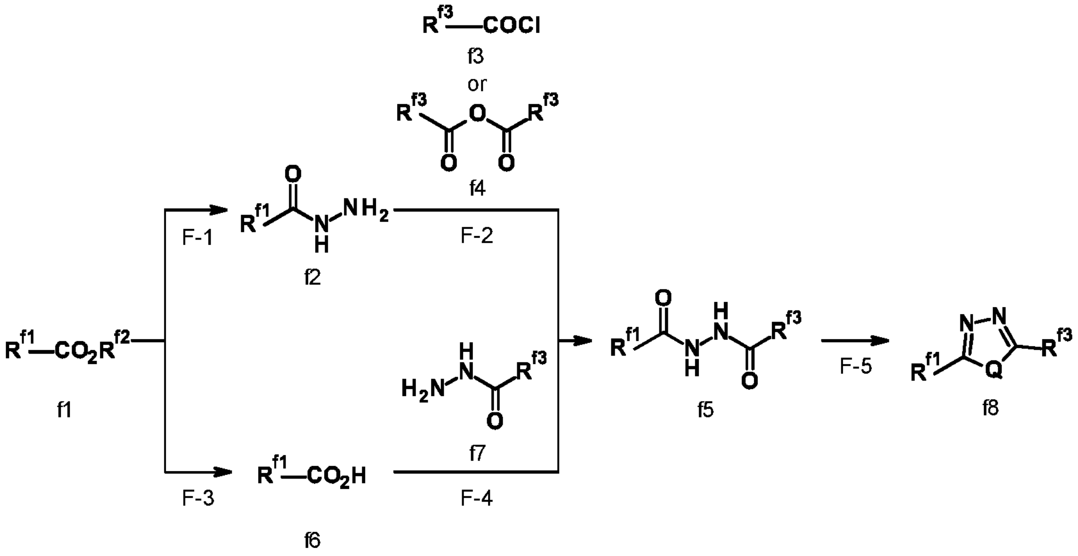


[wherein $R^{f1}$ represents a group represented by Formula (IV) or a substituent capable of being converted to a group represented by Formula (IV) . $R^{f2}$ represents a $C_1$-$C_6$ alkyl group. $R^{f3}$ represents a group described in Group B. Q represents an oxygen atom or a sulfur atom. Group B is the same as defined in (1) described above.]

Step F-1 is a step of obtaining Compound f2 by reacting Compound f1 with hydrazine. This step can be carried out, for example, by reacting with hydrazine monohydrate in a solvent such as ethanol under heating.

Step F-2 is a step of obtaining Compound f5 by acylating Compound f2. This step can be carried out, for example, by reacting with Compound f3 or Compound f4 in a solvent such as dichloromethane.

Step F-3 is a step of obtaining Compound f6 from Compound f1. This step can be carried out under the same conditions as in Step D-1.

Step F-4 is a step of obtaining Compound f5 by reacting Compound f6 with Compound f7. This step can be carried out under the same conditions as in Step D-2.

Step F-5 is a step of obtaining Compound f8 from Compound f5 by a Paal-Knorr type cyclization reaction. In this step, the 1,3,4-oxadiazole ring can be obtained, for example, by reacting with p-toluenesulfonyl chloride in the coexistence of triethylamine in a solvent such as dichloromethane, and heating. The 1,3,4-thiadiazole ring can be obtained, for example, by reacting with Lawesson's reagent in a solvent such as toluene, and heating.

[Method G]

In the compound represented by Formula (I) wherein $R^5$ is a pyrazole ring and $R^6$ is -CO-N(Y)(Z), the $R^6$ moiety can be produced by Method G.

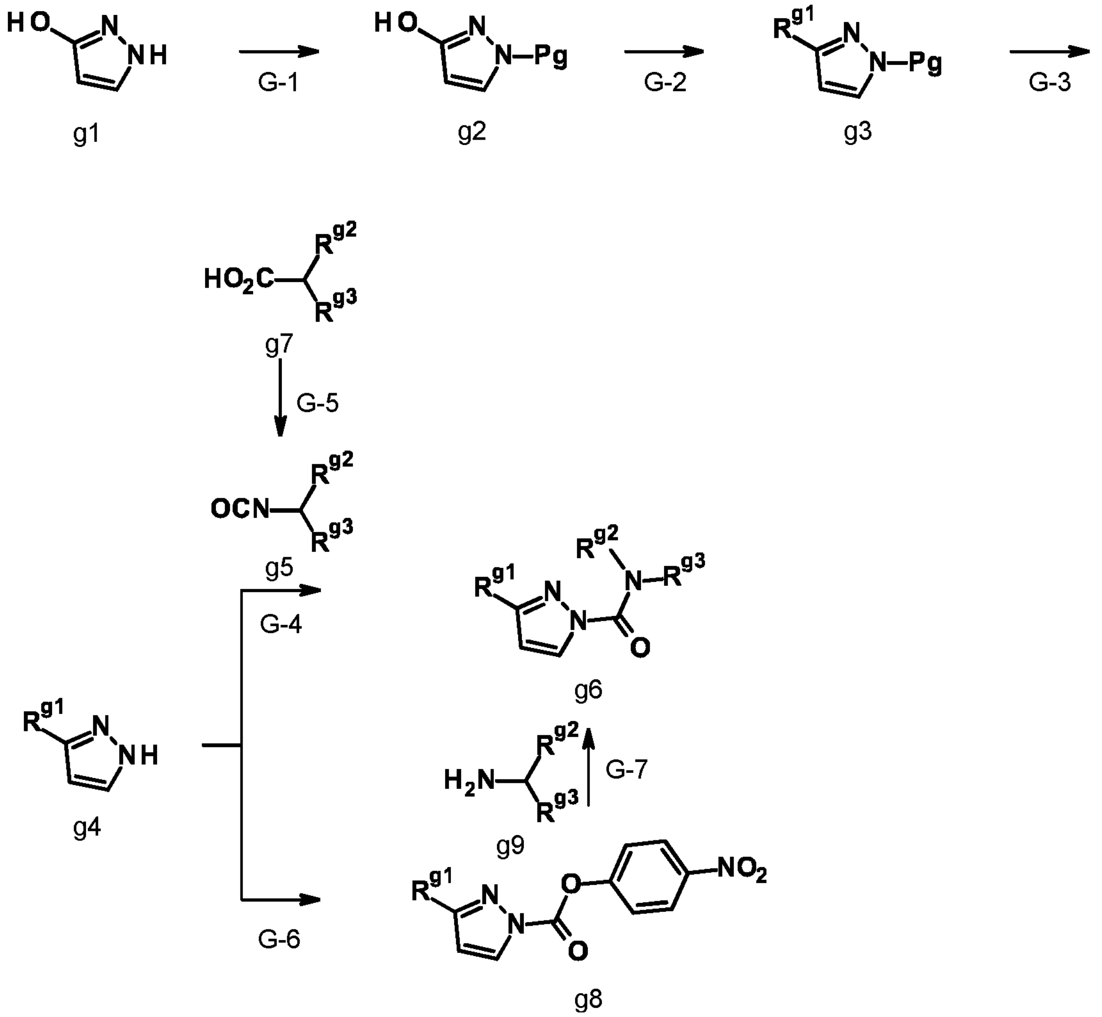


[wherein Pg in Method G represents a protecting group for a pyrazole nitrogen atom, such as a tert-butoxycarbonyl group. $R^{g1}$ represents a group represented by Formula (V):

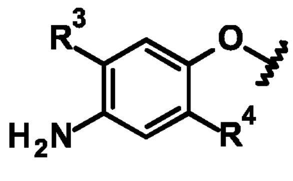 (V)

[wherein $R^3$ or $R^4$ is the same as defined in (1) described above.]
or a substituent capable of being converted to a group represented by Formula (V) . $R^{g2}$ and $R^{g3}$ represent Y, Z, a substituent capable of being converted to Y, or a substituent capable of being converted to Z. Y and Z are the same as defined in (1) described above.]

Step G-1 is a step of converting Compound g1 to Compound g2. For the protection of the nitrogen atom in this step, a method ordinarily used can be employed.

Step G-2 is a step of obtaining Compound g3 by modifying a hydroxy group of Compound g2. This step can be carried out under the same conditions as in Step C-1.

Step G-3 is a step of deprotecting the nitrogen atom of Compound g3 to convert to Compound g4. For the deprotection of the nitrogen atom in this step, a method ordinarily used can be employed.

Step G-4 is a step of reacting Compound g4 with Compound g5 to convert to Compound g6. The carbamoylation in this step can be carried out, for example, by heating in the coexistence of a base such as triethylamine in a solvent such as 1,2-dichloroethane.

Step G-5 is a step of converting Compound g7 to Compound g5. The acid azidation and subsequent Curtius

rearrangement reaction in this step can be carried out, for example, by reacting with diphenylphosphoryl azide in the presence of a base such as triethylamine in a solvent such as toluene, and heating.

Step G-6 is a step of obtaining Compound g8 by reacting Compound g4 with 4-nitrophenyl chloroformate. The urethanization in this step can be carried out, for example, in the coexistence of a base such as triethylamine in a solvent such as dichloromethane.

Step G-7 is a step of reacting Compound g9 with Compound g8 to convert to Compound g6. The carbamoylation in this step can be carried out, for example, in the coexistence of a base such as triethylamine in a solvent such as dichloromethane.

[Method H]

In the compound represented by Formula (I) wherein $R^5$ is Compound h2 below, the $R^5$ moiety can be produced by Method H.

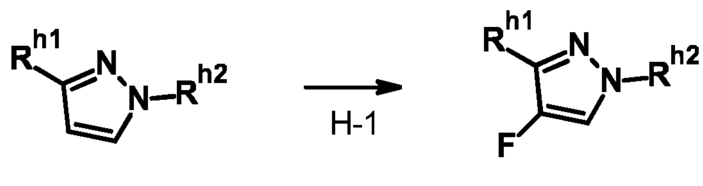


h1 h2

[wherein $R^{h1}$ represents a group represented by Formula (V) or a substituent capable of being converted to a group represented by Formula (V). $R^{h2}$ represents $R^6$ or a substituent capable of being converted to $R^6$. $R^6$ is the same as defined in (1) described above.]

Step H-1 is a step of obtaining Compound h2 by fluorinating at the 4-position of Compound h1. The fluorination in this step can be carried out, for example, by reacting with N-fluoro-N'-(chloromethyl)triethylenediamine bis(tetrafluoroborate) in a solvent such as acetonitrile, and heating.

[Method I]

In the compound represented by Formula (I) wherein $R^5$ is Compound i3 below, the $R^5$-$R^6$ moiety can be produced by Method I.

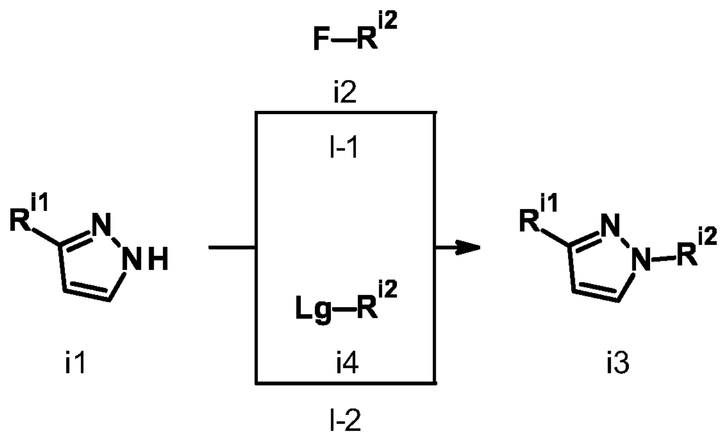


i1 i2 i4 i3

[wherein Lg in Method I represents a leaving group such as an iodo group or a bromo group. $R^{11}$ represents a group represented by Formula (V) or a substituent capable of being converted to a group represented by Formula (V). $R^{i2}$ represents $R^6$ or a substituent capable of being converted to $R^6$. $R^6$ is the same as defined in (1) described above.]

Step I-1 is a step of obtaining Compound i3 by a nucleophilic substitution reaction of Compound i1 and Compound i2. The nucleophilic substitution reaction in this step can be carried out, for example, in a solvent such as dimethylsulfoxide in the presence of a base such as cesium carbonate.

Step 1-2 is a step of obtaining Compound i3 by Ullmann type coupling of Compound i1 and Compound i4. The coupling reaction in this step can be carried out, for example, when Lg is a bromo group, by reacting with a metal catalyst such as copper(I) iodide in the coexistence of a base such as potassium carbonate and a ligand such as trans-N,N'-dimethylcyclohexane-1,2-diamine or N,N-dimethylglycine in a solvent such as dimethylsulfoxide or toluene, and heating.

[Method J]

In the compound represented by Formula (I) wherein $R^5$ is Compound j3 below, the $R^5$-$R^6$ moiety can be produced by Method J.

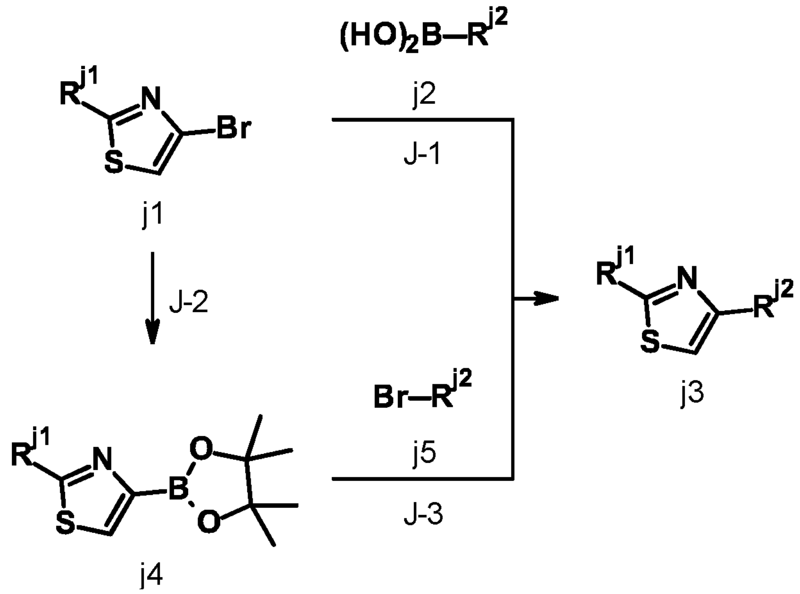


[wherein $R^{j1}$ represents a group represented by Formula (V) or a substituent capable of being converted to a group represented by Formula (V). $R^{j2}$ represents $R^6$ or a substituent capable of being converted to $R^6$. $R^6$ is the same as defined in (1) described above.]

Step J-1 is a step of obtaining Compound j3 from Compound j1 and Compound j2. The coupling reaction in this step can be carried out, for example, by heating Compound j1 and Compound j2 in the presence of a base such as cesium carbonate and a metal catalyst such as tetrakis (triphenylphosphine)palladium(0) or [1,1'-bis(diphenylphosphino)ferrocene]palladium(II) dichloride dichloromethane adduct in a solvent such as hydrous 1,2-dimethoxyethane.

Step J-2 is a step of obtaining Compound j4 from Compound j1. The coupling reaction in this step can be carried out by reacting Compound j1, for example, with bis(pinacolato)diboron in the presence of a base such as potassium acetate and a metal catalyst such as [1,1'-bis(diphenylphosphino)ferrocene]palladium(II) dichloride dichloromethane adduct under heating in a solvent such as 1,4-dioxane.

Step J-3 is a step of obtaining Compound j3 from Compound j4 and Compound j5. The coupling reaction in this step can be carried out by heating Compound j4 and Compound j5, for example, in the presence of a base such as cesium carbonate and a metal catalyst such as tetrakis(triphenylphosphine)palladium(0) or [1,1'-bis(diphenylphosphino)ferrocene]palladium(II) dichloride dichloromethane adduct in a solvent such as hydrous 1,2-dimethoxyethane.

[Method K]

The aromatic amine moiety in the compound represented by Formula (III) can be produced by Method K.

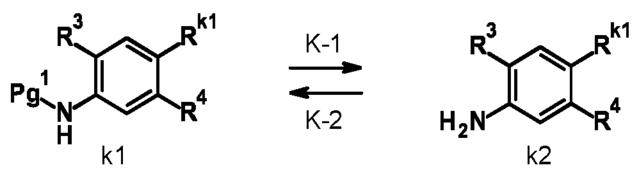

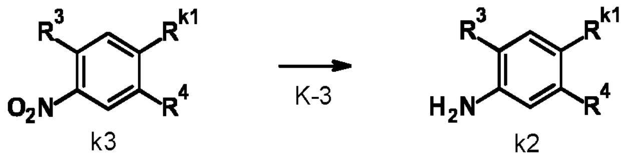


k3 k2

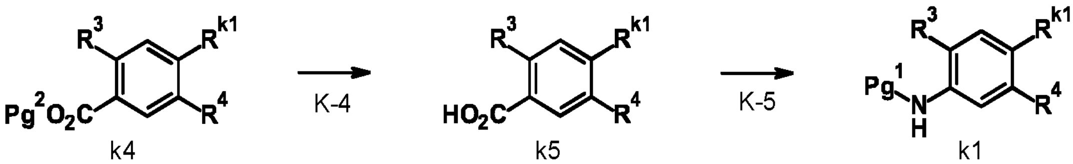


k4 k5 k1

[wherein $R^3$ and $R^4$ are the same as defined in (1) described above. $Pg^1$ represents a protecting group for a nitrogen atom, such as a tert-butoxycarbonyl group. $Pg^2$ represents a protecting group for a carboxy group, such as a methyl group or tert-butyl group. $R^{k1}$ is a group represented by Formula (VI):

[Chemical Formula 22]

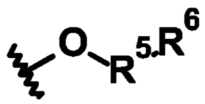 (VI)

[wherein $R^5$ and $R^6$ are the same as defined in (1) described above.]
or a substituent capable of being converted to a group represented by Formula (VI).]

Step K-1 is a step of obtaining Compound k2 by deprotecting the amino group of Compound k1. The deprotecting reaction in this step can be carried out by a method ordinarily used to deprotect an amino group. For example, when $Pg^1$ is a tert-butoxycarbonyl group, the reaction can be carried out by reacting with an acid such as trifluoroacetic acid in a solvent such as dichloromethane.

Step K-2 is a step of obtaining Compound k1 by protecting the amino group of Compound k2. The protection of the amino group in this step can be carried out by a method ordinarily used to protect an amino group. For example, when $Pg^1$ is a tert-butoxycarbonyl group, the reaction can be carried out by reacting with di-tert-butyl dicarbonate or the like in a solvent such as tetrahydrofuran.

Step K-3 is a step of obtaining Compound k2 from Compound k3. This step can be carried out under the same conditions as in Step A-5.

Step K-4 is a step of obtaining Compound k5 from Compound k4. The deprotecting reaction in this step can be carried out by a method ordinarily used to deprotect a carboxy group. For example, when $Pg^2$ is a tert-butyl group, the reaction can be carried out by reacting with an acid such as trifluoroacetic acid in a solvent such as dichloromethane.

Step K-5 is a step of obtaining Compound k1 from Compound k5. The acid azidation and subsequent carbamation through a Curtius rearrangement reaction in this step can be carried out, for example, when $Pg^1$ is a tert-butoxycarbonyl group, by reacting Compound k5 with diphenylphosphoryl azide in the presence of a base such as triethylamine in a solvent such as toluene and heating to obtain an isocyanic acid ester, and then reacting with tert-butanol under heating.

The compounds produced by the above methods can be isolated and purified by known methods, such as extraction, precipitation, distillation, chromatography, fractional recrystallization, and recrystallization.

Examples

Hereinafter, the present invention is described in further detail with Reference Examples and Examples, but the present invention is not limited thereto, and these are not to be construed in any sense as limiting. Reagents, solvents, and starting materials not specifically described in the description are readily available from commercially available sources.

The proton nuclear magnetic resonance spectrum ($^1H$-NMR) was measured with a 400 MHz Nuclear Magnetic Resonance Spectrometer manufactured by JEOL Ltd. or a 400 MHz Nuclear Magnetic Resonance Spectrometer manufactured by Varian, Inc. Spectral data are indicated with significant peaks, and shown in chemical shifts (indicated as relative ppm ($\delta$) using tetramethylsilane as a standard substance); the number of protons; multiplicity of peak splitting (indicated as s: singlet, d: doublet, t: triplet, q: quartet, m: multiplet, br: broad, and the like); and, when clear specification was possible, the spin coupling constants as J values (in Hz) .

The mass spectrum (MS m/z) was measured by an electro spray ionization (ESI) method or an atmospheric pressure chemical ionization (APCI) method. The mass spectrum data are shown with the maximum ionization peak (in most cases corresponding to maximum UV absorption peak) measured after being passed through a reverse phase high-performance liquid chromatography column (Agilent system; column: Develosil Combi-RP-5, 2.0 x 50 mm, Cadenza CD-C18, 3.0 x 75 mm, or ZORBAX SB-C18, 1.8 μm, 2.1 x 50 mm; solvent: 0.1% formic acid-containing acetonitrile/water system, or 0.01% trifluoroacetic acid-containing acetonitrile/water system).

Silica gel column chromatography was performed with commercially available pre-packed columns and automated preparatory purification systems (SP1 manufactured by Biotage AB, EPCLC-W-Prep2XY manufactured by Yamazen CORPORATION, Purif-α2 manufactured by Shoko Science Co. Ltd. or the like). Only multiple solvents which were used for the mobile phase are described. Elution was performed under observation by thin layer chromatography (TLC). Silica gel 60 $F_{254}$ or 60 $NH_2F_{254}S$ manufactured by Merck KGaA, a $NH_2$ silica gel 60 $F_{254}$ plate manufactured by FUJIFILM Wako Pure Chemical Corporation, or CHROMATOREX NH TLC manufactured by Fuji Silysia Chemical Ltd. was employed as the TLC plate. The mobile phase used for the column chromatography was employed as the developing solvent. A UV detector or staining reagent was employed as the detection method.

Preparative thin layer chromatography (PTLC) was performed with a silica gel 60 $F_{254}$ plate manufactured by Merck KGaA, or a silica gel 70 $PF_{254}$ plate or $NH_2$ silica gel 60 $F_{254}$ plate manufactured by FUJIFILM Wako Pure Chemical Corporation. Only multiple solvents which were used for the mobile phase are described.

Preparative high-performance liquid chromatography (prep HPLC) was performed with a reverse phase column (Develosil Combi-RP-5) manufactured by Nomura Chemical Co., Ltd., and 0.1% formic acid-containing acetonitrile/water system was used for the mobile phase.

The amount of solvent or the ratio of solvents, the timing of its change, and the gradient method used in these chromatography methods are not shown, but it is considered that the purification and separation methods used here can be easily reproduced using common knowledge and techniques of chemical synthesis.

The instruments and measurement conditions in the powder X-ray diffraction measurements in the Examples are as follows.

Machine model: Rigaku Rint TTR-III
Sample holder: Non-reflective sample holder:

Samples: Appropriate amount
X-ray generation conditions: 50 kV, 300 mA
Wavelength: 1.54 Å (Copper Kα Ray)
Measurement temperature: room temperature
Scan Speed: 20°/min
Scan Range: 2 to 40°
Sampling width: 0.02°
Sample Preparation: Several mg of the crystals were collected with a spatula, placed on a non-reflective sample holder, and flattened with powder paper. Thereafter, the peak pattern was analyzed under the conditions described above.

The abbreviations used in the Examples have the following meanings:
Ac: acetyl group, Bn: benzyl group, Boc: tert-butoxycarbonyl group, tBu: tert-butyl group, Cbz: benzyloxycarbonyl group, $CDCl_3$: deuterated chloroform, $CD_3OD$: deuterated methanol, DMSO: dimethyl sulfoxide, Et: ethyl group, Me: methyl group, Ms: methanesulfonyl group, TMS: trimethylsilyl group.

Unless otherwise stated in the Reference Examples and Examples, hexane represents n-hexane.

[Reference Example A1] 4-Chloro-7-methoxy-6-[(piperidin-4-yl)oxy]quinazoline dihydrochloride

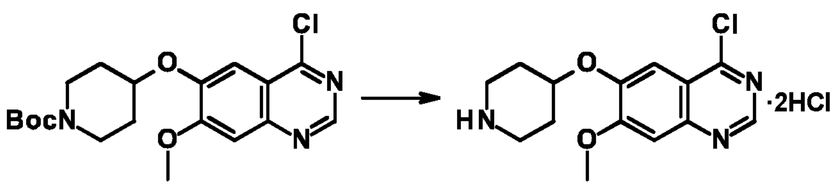

To a solution of tert-butyl 4-[(4-chloro-7-methoxyquinazolin-6-yl)oxy]piperidine-1-carboxylate (ACS Med. Chem.

Lett. 2013, 4, 742-746) (5.37 g, 13.6 mmol) in tetrahydrofuran (55 mL), a 4 mol/L hydrogen chloride/1,4-dioxane solution (17 mL) was added, and the mixture was stirred at room temperature for 4 hours. A 4 mol/L hydrogen chloride/1,4-dioxane solution (38 mL) was further added, and the mixture was stirred at room temperature for 14 hours. The reaction solution was diluted with hexane. The precipitated solid was collected by filtration and dried under reduced pressure at 50°C to obtain the unpurified title compound (5.14 g). $^1$H-NMR (DMSO-$D_6$) δ: 1.82-2.01 (2H, m), 2.08-2.26 (2H, m), 3.02-3.34 (4H, m), 4.03 (3H, s), 4.95-5.08 (1H, m), 7.52 (1H, s), 7.57 (1H, s), 8.71-8.96 (1H, m), 8.91 (1H, s). MS m/z: 294 $[M+H]^+$.

[Reference Example A2] 1-{4-[ (4-Chloro-7-methoxyquinazolin-6-yl)oxy]piperidin-1-yl}prop-2-en-1-one

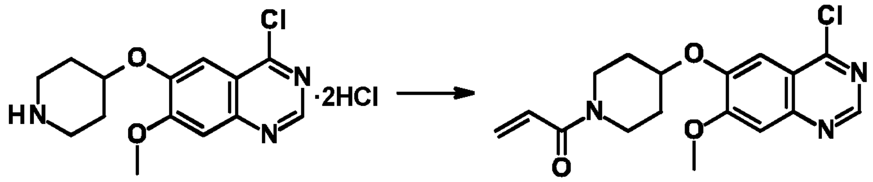

To a suspension of 4-chloro-7-methoxy-6-[(piperidin-4-yl)oxy]quinazoline dihydrochloride (5.14 g) in dichloromethane (250 mL) manufactured in Reference Example A1, triethylamine (7.54 mL, 54.1 mmol) was added dropwise at 0°C, and the mixture was stirred at 0°C for 30 minutes. A solution of acryloyl chloride (1.32 mL, 16.2 mmol) in dichloromethane (16.3 mL) was added dropwise at 0°C, and the mixture was stirred at 0°C for 1 hour. The reaction solution was diluted with saturated aqueous sodium bicarbonate solution and extracted with dichloromethane (x 3). The organic layer was dried over anhydrous sodium sulfate, filtered, and then concentrated under reduced pressure. The residue was azeotropically concentrated with hexane to obtain the title compound (4.63 g, 13.3 mmol, 2-step yield 97.8%). $^1$H-NMR ($CDCl_3$) δ: 1.93-2.15 (4H, m), 3.55-3.67 (1H, m), 3.76-3.95 (3H, m), 4.05 (3H, s), 4.80-4.89 (1H, m), 5.73 (1H, dd, J = 10.9, 1.8 Hz), 6.32 (1H, dd, J = 17.0, 1.8 Hz), 6.63 (1H, dd, J = 17.0, 10.9 Hz), 7.36 (1H, s), 7.46 (1H, s), 8.88 (1H, s). MS m/z: 348 $[M+H]^+$.

[Reference Example A3] tert-Butyl 4-[2-hydroxy-5-(methoxycarbonyl)-4-nitrophenoxy]piperidine-1-carboxylate

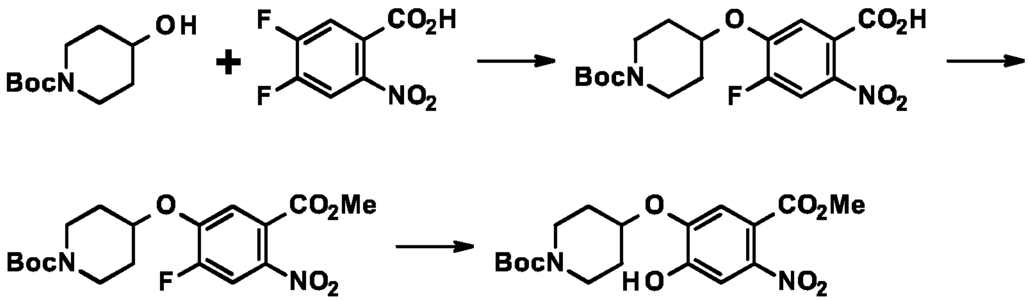

To a solution of 4,5-difluoro-2-nitrobenzoic acid (2.03 g, 10.0 mmol) and tert-butyl 4-hydroxypiperidine-1-carboxylate (4.03 g, 20.0 mmol) in N,N-dimethylformamide (20 mL), sodium hydride (60% in oil, 800 mg, 20.0 mmol) was added under ice cooling, and the mixture was gradually warmed to room temperature, and stirred for 19 hours. To the reaction solution, 1 mol/L hydrochloric acid was added, and the reaction solution was extracted with ethyl acetate. The organic layer was washed with saturated saline, and then dried over anhydrous sodium sulfate. The insoluble materials were filtered off and then the filtrate was concentrated under reduced pressure to obtain 5-{[1-(tert-butoxycarbonyl)piperidin-4-yl]oxy}-4-fluoro-2-nitrobenzoic acid as a crude product. The crude product was used in the next step without further purification. To a solution of 5-{[1-(tert-butoxycarbonyl)piperidin-4-yl]oxy}-4-fluoro-2-nitrobenzoic acid in N,N-dimethylformamide (20 mL), methyl iodide (1.24 mL, 20.0 mmol) and potassium carbonate (2.76 g, 20.0 mmol) were added, and the mixture was stirred at room temperature for 4 hours. The reaction solution was diluted with ethyl acetate, washed with saturated saline, and then dried over anhydrous sodium sulfate. The insoluble materials were filtered off and then the filtrate was concentrated under reduced pressure to obtain tert-butyl 4-[2-fluoro-5-(methoxycarbonyl)-4-nitrophenoxy]piperidine-1-carboxylate as a crude product. The crude product was used in the next step without further

purification. To a solution of tert-butyl 4-[2-fluoro-5-(methoxycarbonyl)-4-nitrophenoxy]piperidine-1-carboxylate in dimethyl sulfokide (20 mL), N-hydroxyacetamide (2.25 g, 30.0 mmol) and potassium carbonate (6.90 g, 50.0 mmol) were added, and the mixture was stirred at 80°C for 1 hour. To the reaction solution after left to cool, 1 mol/L hydrochloric acid was added, and the mixture was extracted with ethyl acetate. The organic layer was washed with saturated saline, then dried over anhydrous sodium sulfate. The insoluble materials were filtered off, then the filtrate was concentrated under reduced pressure. To the residue, ethyl acetate and 2-propanol were added, and the precipitated solid was collected by filtration to obtain the title compound (2.9 g, 7.3 mmol, 73% yield).
$^1$H-NMR ($CDCl_3$) δ: 1.48 (9H, s), 1.76-1.79 (2H, m), 2.04-2.06 (2H, m), 3.22-3.28 (2H, m), 3.84-3.88 (5H, m), 4.61-4.67 (1H, m), 6.09 (1H, br s), 7.14 (1H, s), 7.49 (1H, s). MS m/z: 297 [M-Boc+H]$^+$.

[Reference Example A4] tert-Butyl 4-[2-ethoxy-5-(methoxycarbonyl)-4-nitrophenoxy]piperidine-1-carboxylate

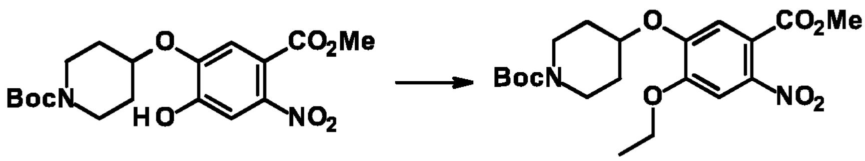

To a solution of tert-butyl 4-[2-hydroxy-5-(methoxycarbonyl)-4-nitrophenoxy]piperidine-1-carboxylate (198 mg, 0.500 mmol) in N,N-dimethylformamide (3 mL), ethyl iodide (0.080 mL, 1.0 mmol) and potassium carbonate (104 mg, 0.750 mmol) were added, and the mixture was stirred at room temperature for 4 hours. Water was added to the reaction solution, and the mixture was extracted with ethyl acetate. The organic layer was washed with saturated saline and then dried over anhydrous sodium sulfate. The insoluble materials were filtered off and then the filtrate was concentrated under reduced pressure and purified by silica gel column chromatography (ethyl acetate/hexane) to obtain the title compound (210 mg, 0.494 mmol, yield 99.0%).
$^1$H-NMR ($CDCl_3$) δ: 1.47 (12H, br s), 1.83 (2H, br s), 1.93 (2H, br s), 3.39 (2H, br s), 3.69 (2H, br s), 3.90 (3H, br s), 4.15 (2H, br s), 4.60 (1H, br s), 7.11 (1H, br s), 7.43 (1H, br s). MS m/z: 425 [M+H]$^+$.

By a similar method, the following compounds were synthesized using the corresponding alkyl halides.

[Table 1]

| Reference Example | Compound | Alkyl halide |
|---|---|---|
| A5 | tert-Butyl 4-[2-(benzyloxy)-5-(methoxycarbonyl)-4-nitrophenoxy]piperidine-1-carboxylate 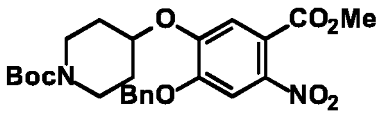 $^1$H-NMR ($CDCl_3$) δ: 1.47 (9H, s), 1.83-1.84 (2H, m), 1.92-1.94 (2H, m), 3.36-3.38 (2H, m), 3.65-3.70 (2H, m), 3.90 (3H, s), 4.59-4.68 (1H, m), 5.19 (2H, s), 7.11 (1H, s), 7.37-7.40 (5H, m), 7.53 (1H, s). MS m/z: 387 [M-Boc+H]+. | Benzylbromide (stirred at 100°C) |
| A6 | tert-Butyl 4-[5-( methoxycarbonyl)-2 - [($^2H_3$) methyloxy] -4-nitrophenoxy]piperidine-1-carboxylate 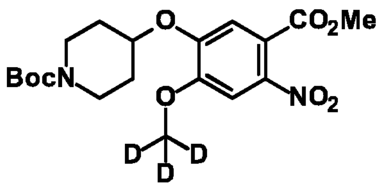 MS m/z: 314 [M-Boc+H]$^+$. | Methyl-$d_3$ iodide |

[Reference Example A7] tert-Butyl 4-{5-(methoxycarbonyl)-2-[(methylsulfanyl)methoxy]-4-nitrophenoxy}piperidine-1-carboxylate

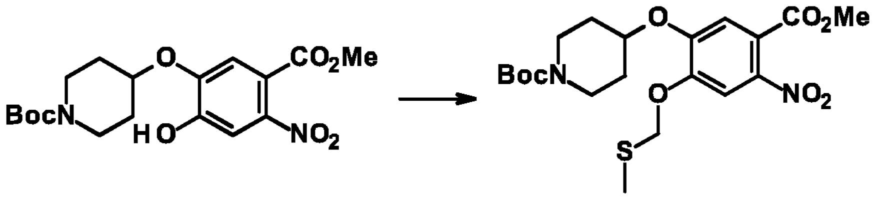

To a solution of tert-butyl 4-[2-hydroxy-5-(methoxycarbonyl)-4-nitrophenoxy]piperidine-1-carboxylate (119 mg, 0.300 mmol) in N,N-dimethylformamide (3 mL), chloromethyl methyl sulfide (0.050 mL, 0.60 mmol) and sodium hydride (60% in oil, 24 mg, 0.60 mmol) were added, and the mixture was stirred at room temperature for 16 hours. The reaction solution was diluted with water, and then extracted with ethyl acetate. The organic layer was washed with saturated saline and then dried over anhydrous sodium sulfate. The insoluble materials were filtered off and then the filtrate was concentrated under reduced pressure and purified by silica gel column chromatography (ethyl acetate/hexane) to obtain the title compound (133 mg, 0.291 mmol, yield 97.1%). $^1$H-NMR ($CDCl_3$) δ: 1.47 (9H, s), 1.79-1.85 (2H, m), 1.95-1.96 (2H, m), 2.26 (3H, s), 3.34-3.40 (2H, m), 3.70-3.72 (2H, m), 3.91 (3H, s), 4.60-4.64 (1H, m), 5.28 (2H, s), 7.11 (1H, s), 7.57 (1H, s). MS m/z: 357 $[M+H]^+$.

[Reference Example A8] tert-Butyl 4-[2-(fluoromethoxy)-5-(methoxycarbonyl)-4-nitrophenoxy]piperidine-1-carboxylate

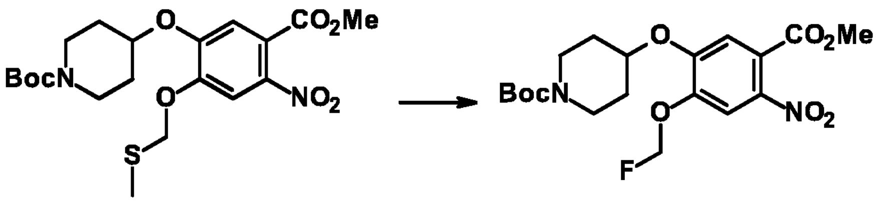

To a solution of tert-butyl 4-{5-(methoxycarbonyl)-2-[(methylsulfanyl)methoxy]-4-nitrophenoxy}piperidine-1-carboxylate (133 mg, 0.291 mmol) in dichloromethane (3 mL), sulfuryl chloride (0.035 mL, 0.44 mmol) was added, and the mixture was stirred at room temperature for 30 minutes. The reaction solution was concentrated under reduced pressure. To a solution of the obtained residue in dichloromethane (3 mL), tetra-n-butylammonium fluoride (1.0 mol/L tetrahydrofuran solution, 0.583 mL, 0.583 mmol) was added, and the mixture was stirred at room temperature for 4 hours. The reaction solution was concentrated under reduced pressure and purified by silica gel column chromatography (ethyl acetate/hexane) to obtain the title compound (53 mg, 0.12 mmol, yield 43%) .
$^1$H-NMR ($CDCl_3$) δ: 1.47 (9H, s), 1.83-1.84 (2H, m), 1.95-1.97 (2H, m), 3.34-3.40 (2H, m), 3.70-3.72 (2H, m), 3.92 (3H, s), 4.64-4.65 (1H, m), 5.77 (2H, d, J = 53.5 Hz), 7.13 (1H, s), 7.79 (1H, s).

[Reference Example A9] tert-Butyl 4-[(7-ethoxy-4-oxo-3, 4-dihydroquinazolin-6-yl) oxy]piperidine-1-carboxylate

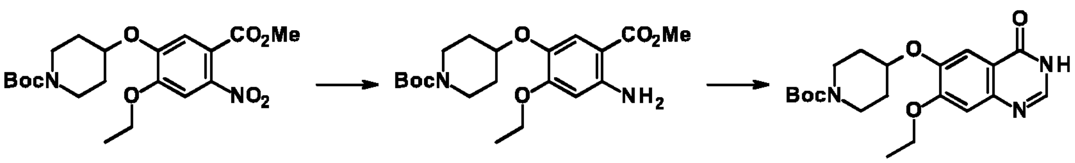

To a solution of tert-butyl 4-[2-ethoxy-5-(methoxycarbonyl)-4-nitrophenoxy]piperidine-1-carboxylate (210 mg, 0.494 mmol) in ethanol (5 mL), 10% palladium on carbon (M) Wet (21 mg) was added, and the mixture was stirred at

room temperature for 7 hours under a hydrogen atmosphere at normal pressure. The insoluble materials were filtered off and then the filtrate was concentrated under reduced pressure to obtain tert-butyl 4-[4-amino-2-ethoxy-5-(methoxycarbonyl)phenoxy]piperidine-1-carboxylate as a crude product. The crude product was used in the next step without further purification. To a solution of tert-butyl 4-[4-amino-2-ethoxy-5-(methoxycarbonyl)phenoxy]piperidine-1-carboxylate in 2-methoxyethanol (3 mL), formamidine acetate (154 mg, 1.48 mmol) was added, and the mixture was heated to reflux for 3 hours. To the reaction solution after left to cool, water was added, and the mixture was extracted with ethyl acetate. The organic layer was washed with saturated saline and then dried over anhydrous sodium sulfate. The insoluble materials were filtered off and then the filtrate was concentrated under reduced pressure. The precipitated solid was collected by filtration to obtain the title compound (145 mg, 0.372 mmol, yield 75.3%) . $^1$H-NMR ($CDCl_3$) δ: 1.49-1.51 (12H, br m), 1.87 (2H, br s), 1.96 (2H, br s), 3.39 (2H, br s), 3.74 (2H, br s), 4.20 (2H, br s), 4.63 (1H, br s), 7.15 (1H, br s), 7.67 (1H, br s), 7.99 (1H, br s), 10.69 (1H, br s). MS m/z: 390 [M+H] $^+$.

[Reference Example A10] tert-Butyl 4-{[7-(fluoromethoxy)-4-oxo-3,4-dihydroquinazolin-6-yl]oxy}piperidine-1-carboxylate

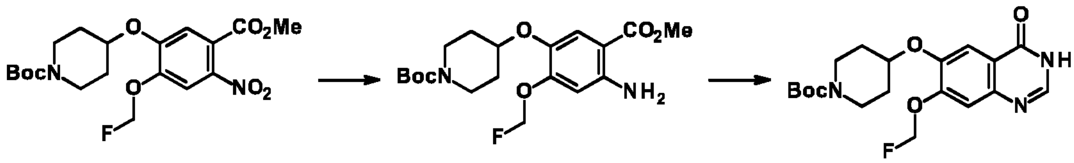

To a solution of tert-butyl 4-[2-(fluoromethoxy)-5-(methoxycarbonyl)-4-nitrophenoxy]piperidine-1-carboxylate (53 mg, 0.12 mmol) in methanol (4 mL), acetic acid (1 mL) and zinc (40 mg, 0.62 mmol) were added, and the mixture was stirred at room temperature for 16 hours. To the reaction solution, saturated aqueous sodium bicarbonate solution was added, and the mixture was extracted with dichloromethane. The organic layer was washed with saturated saline, and then dried over anhydrous sodium sulfate. The insoluble materials were filtered off and then the filtrate was concentrated under reduced pressure to obtain tert-butyl 4-[4-amino-2-(fluoromethoxy)-5-(methoxycarbonyl)phenoxy]piperidine-1-carboxylate as a crude product. The crude product was used in the next step without further purification. To a solution of tert-butyl 4-[4-amino-2-(fluoromethoxy)-5-(methoxycarbonyl)phenoxy]piperidine-1-carboxylate in 2-methoxyethanol (3 mL), formamidine acetate (39 mg, 0.37 mmol) was added, and the mixture was heated to reflux for 3 hours. To the reaction solution after left to cool, water was added, and the mixture was extracted with ethyl acetate. The organic layer was washed with saturated saline, and then dried over anhydrous sodium sulfate. The insoluble materials were filtered off and then the filtrate was concentrated under reduced pressure, and purified by silica gel column chromatography (ethyl acetate/hexane and dichloromethane/methanol) to obtain the title compound (38 mg, 0.097 mmol, yield 78%). $^1$H-NMR ($CDCl_3$) δ: 1.48 (9H, br s), 1.85 (2H, br s), 2.00 (2H, br s), 3.37 (2H, br s), 3.76 (2H, br s), 4.68 (1H, br s), 5.86 (2H, d, J = 52.7 Hz), 7.48 (1H, br s), 7.71 (1H, br s), 8.02 (1H, br s), 10.94 (1H, br s). MS m/z: 394 [M+H]$^+$.

By a similar method, the following compounds were synthesized from the corresponding intermediates.

[Table 2]

| Reference Example | Compound | Intermediate |
|---|---|---|
| A11 | tert-Butyl 4-{[7-(benzyloxy)-4-oxo-3,4-dihydroquinazolin-6-yl]oxy}piperidine-1-carboxylate 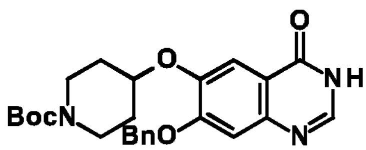 $^1$H-NMR($CDCl_3$) δ: 1.48 (9H, s), 1.87-1.88 (2H, m), 1.95-1.98 (2H, m), 3.38-3.40 (2H, m), 3.71-3.73 (2H, m), 4.66-4.66 (1H, m), 5.25 (2H, s), 7.22 (1H, s), 7.35-7.44 (6H, m), 7.68 (1H, s), 7.96 (1H, s). MS m/z: 396 [M-tBu+H]+. | Reference Example A5 |

(continued)

| Reference Example | Compound | Intermediate |
|---|---|---|
| A12 | tert-Butyl 4-({7-[($^2H_3$)methyloxy]-4-oxo-3,4-dihydroquinazolin-6-yl}oxy)piperidine-1-carboxylate 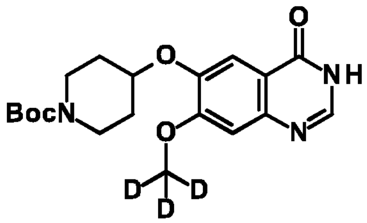 $^1$H-NMR ($CDCl_3$) δ: 1.47 (9H, s), 1.82-1.85 (2H, m), 2.01 (2H, br s), 3.26-3.32 (2H, m), 3.84 (2H, brs), 4.62-4.66 (1H, m), 7.15 (1H, s), 7.65 (1H, s), 7.95 (1H, d, J = 3.1 Hz), 9.51 (1H, br s). MS m/z: 379 $[M+H]^+$. | Reference Example A6 |

[Reference Example A13] 4-Chloro-7-methoxy-6-nitroquinazoline hydrochloride

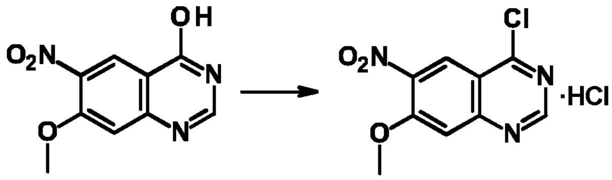

To a suspension of 7-methoxy-6-nitroquinazolin-4-ol (407 mg, 1.84 mmol) in thionyl chloride (4 mL), N,N-dimethylformamide (0.007 mL, 0.09 mmol) was added, and the mixture was stirred while being heated to reflux for 3 hours. The reaction solution was left to cool, and then concentrated under reduced pressure. To the residue, diethyl ether was added, and the insoluble materials were collected by filtration, and then further washed with diethyl ether. The resultant was dried under reduced pressure at 60°C to obtain the title compound (381 mg, 1.38 mmol, yield 75.0%).
$^1$H-NMR (DMSO-$D_6$) δ: 4.05 (3H, s), 7.45 (1H, s), 8.31 (1H, s), 8.54 (1H, s). MS m/z: 240 $[M+H]^+$.

[Reference Example B1] 3-(Benzyloxy)-N'-propanoylbenzohydrazide

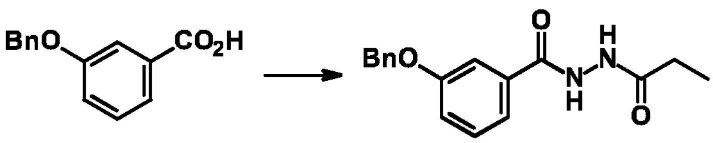

3-Benzyloxybenzoic acid (2.00 g, 8.76 mmol) and 1-hydroxybenzotriazole (355 mg, 2.63 mmol) were dissolved in N,N-dimethylformamide (25 mL) . Propanohydrazide (772 mg, 8.76 mmol) and 1-(3-dimethylaminopropyl)-3-ethylcarbodiimide hydrochloride (2.18 g, 11.4 mmol) were added, and the mixture was stirred for 19 hours. After dilution with ethyl acetate, the mixture was washed with water and saline, and the organic layer was dried over anhydrous sodium sulfate. After concentration, the obtained solid was washed with a mixed solvent of ethyl acetate/hexane (1:2) to obtain the title compound (2.10 g, 7.04 mmol, yield 80.3%) .
$^1$H-NMR (DMSO-$D_6$) δ: 1.06 (3H, t, J = 7.6 Hz), 2.19 (2H, q, J = 7.6 Hz), 5.17 (2H, s), 7.21 (1H, dd, J = 7.9, 1.8 Hz), 7.32-7.36 (1H, m), 7.38-7.43 (3H, m), 7.51-7.44 (4H, m), 9.84 (1H, s), 10.27 (1H, s). MS m/z: 299 $[M+H]^+$.

[Reference Example B2] 2-[3-(Benzyloxy)phenyl]-5-ethyl-1,3,4-oxadiazole

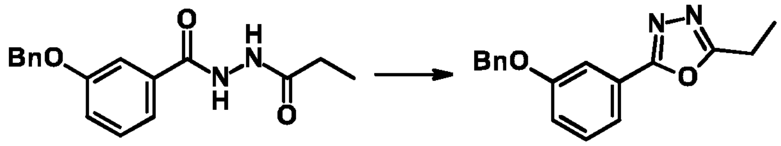

3-(Benzyloxy)-N'-propanoylbenzohydrazide (2.10 g, 7.04 mmol) was dissolved in dichloromethane (50 mL), and triethylamine (1.96 mL, 14.1 mmol) and p-toluenesulfonyl chloride (2.01 g, 10.6 mmol) were added thereto, and the mixture was stirred for 18 hours. The reaction solution was diluted with dichloromethane, and then washed with saline. The organic layer was dried over anhydrous sodium sulfate and then concentrated and purified by silica gel column chromatography (ethyl acetate/hexane) to obtain the title compound (1.75 g, 6.24 mmol, yield 88.7%) .
$^1$H-NMR ($CDCl_3$) δ: 1.44 (3H, t, J = 7.6 Hz), 2.96 (2H, q, J = 7.6 Hz), 5.14 (2H, s), 7.13 (1H, dd, J = 8.5, 2.4 Hz), 7.32-7.37 (1H, m), 7.39-7.47 (5H, m), 7.61-7.64 (1H, m), 7.68-7.67 (1H, m). MS m/z: 281 [M+H]$^+$.

[Reference Example B3] 3-(5-Ethyl-1,3,4-oxadiazol-2-yl)phenol

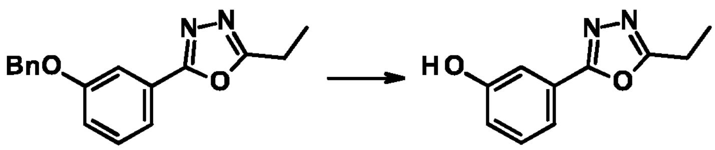

2-[3-(Benzyloxy)phenyl]-5-ethyl-1,3,4-oxadiazole (1.75 g, 6.24 mmol) was dissolved in ethyl acetate (8 mL) and ethanol (8 mL), and 10% palladium on carbon (M) Wet (300 mg) was added thereto. The mixture was stirred under a hydrogen atmosphere at normal pressure for 1.5 hours, then the catalyst was removed by filtration with Celite. The filtrate was concentrated, and the resulting solid was washed with a mixed solvent of ethyl acetate/hexane (1:3) to obtain the title compound (1.02 g, 5.36 mmol, yield 85.9%).
$^1$H-NMR ($CDCl_3$) δ: 1.45 (3H, t, J = 7.7 Hz), 2.97 (2H, q, J = 7.7 Hz), 6.48 (1H, s), 7.05 (1H, dd, J = 8.2, 2.7 Hz), 7.38 (1H, t, J = 7.9 Hz), 7.53 (1H, d, J = 7.3 Hz), 7.95 (1H, s). MS m/z: 191 [M+H]$^+$.

[Reference Example B4] tert-Butyl 3-hydroxy-1H-pyrazole-1-carboxylate

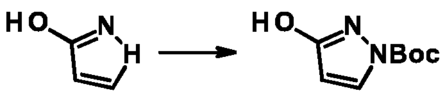

To a suspension of 1H-pyrazol-3-ol (3.00 g, 35.7 mmol) and triethylamine (7.5 mL, 54.1 mmol) in dichloromethane (50 mL), a solution of di-tert-butyl dicarbonate (8.60 g, 39.4 mmol) in dichloromethane (50 mL) was added dropwise, and the mixture was left at room temperature overnight. The solvent was distilled off under reduced pressure and the residue was purified by silica gel column chromatography (ethyl acetate/methanol). The obtained product was further washed with hexane to obtain the title compound (4.90 g, 26.6 mmol, yield 74.6%).
$^1$H-NMR ($CDCl_3$) δ: 1.63 (9H, s), 5.90 (1H, d, J = 3.1 Hz), 7.82 (1H, br s). MS m/z: 129 [M-tBu+H]$^+$

[Reference Example C1] Methyl 3-(3-chloro-4-nitrophenoxy)benzoate

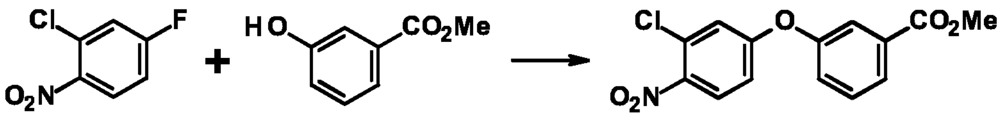

To a solution of 2-chloro-4-fluoronitrobenzene (16.1 g, 91.7 mmol) and methyl 3-hydroxybenzoate (14.1 g, 92.5 mmol) in dimethyl sulfoxide (100 mL), cesium carbonate (44.8 g, 138 mmol) was added at room temperature, and the reaction mixture was stirred at room temperature overnight. The reaction mixture was diluted by addition of ethyl acetate, and the insoluble materials were filtered with Celite, and washed with ethyl acetate. The washing solution was washed with water (x 3) and saturated saline, and the organic layer was dried over anhydrous sodium sulfate, then passed through a pad of silica gel, and eluted with ethyl acetate. The eluted solution was concentrated under reduced pressure. To the obtained residue, methanol was added to form a slurry. The solid was collected by filtration and then dried under reduced pressure to obtain the title compound (27.2 g, 88.5 mmol, yield 96.5%).
$^1$H-NMR ($CDCl_3$) δ: 3.94 (3H, s), 6.91-6.96 (1H, m), 7.07 (1H, d, J = 2.4 Hz), 7.28-7.32 (1H, m), 7.54 (1H, t, J = 8.2 Hz), 7.73-7.76 (1H, m), 7.94-8.01 (2H, m).

[Reference Example C2] 2-Fluoro-4-[3-(methoxycarbonyl)phenoxy]benzoic acid

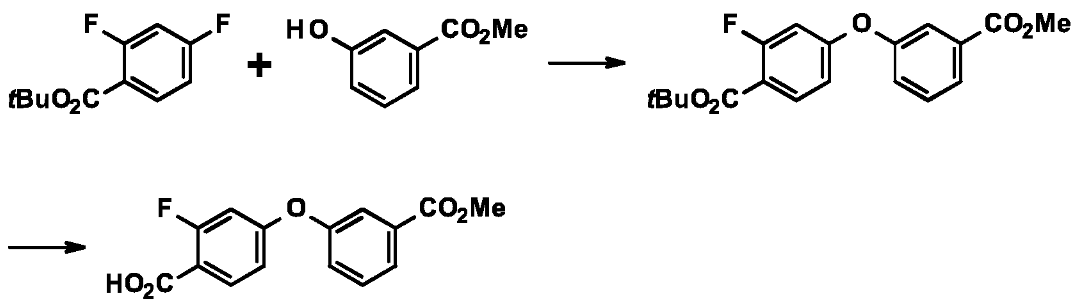

To a solution of tert-butyl 2,4-difluorobenzoate (J. Org. Chem. 2003, 68, 770-778) (6.35 g, 29.6 mmol) and methyl 3-hydroxybenzoate (4.65 g, 30.6 mmol) in dimethyl sulfoxide (40 mL), cesium carbonate (14.5 g, 44.5 mmol) was added at room temperature, and the mixture was stirred at 40°C for 10 hours. The reaction mixture was diluted with a mixed solvent of ethyl acetate/hexane (1:1), and then passed through a pad of Celite whereby the insoluble materials were filtered off, and eluted with the same mixed solvent. The eluted solution was washed with water and saturated saline, and the organic layer was concentrated under reduced pressure. The obtained residue was purified by silica gel column chromatography (ethyl acetate/hexane), and the fraction containing the target substance was concentrated under reduced pressure. To a solution of the obtained residue in dichloromethane (32 mL), trifluoroacetic acid (8 mL) was added at room temperature, and the mixture was stirred at room temperature for 4 hours. The reaction mixture was concentrated under reduced pressure, and the obtained residue was azeotropically concentrated twice by addition of dichloromethane. To the obtained residue, 60 mL of ethyl acetate was added, and the mixture was dissolved while being heated to reflux. To the reaction mixture, 120 mL of hexane was added, and the mixture was stirred for 20 minutes while being heated to reflux. The reaction mixture was cooled with stirring to room temperature. The solid was collected by filtration, and washed with a mixed solvent of ethyl acetate/hexane, and dried under reduced pressure to obtain the title compound (4.05 g, 14.0 mmol, yield 80.8%) .
$^1$H-NMR ($CDCl_3$) δ: 3.93 (3H, s), 6.71 (1H, dd, J = 11.6, 2.4 Hz), 6.78-6.84 (1H, m), 7.29-7.33 (1H, m), 7.52 (1H, t, J = 7.9 Hz), 7.75-7.77 (1H, m), 7.92-7.95 (1H, m), 8.01 (1H, t, J = 8.5 Hz).

[Reference Example C3] 2-Chloro-4-(3-ethynylphenoxy)-1-nitrobenzene

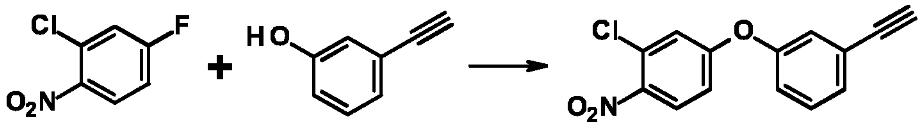

To a solution of 2-chloro-4-fluoronitrobenzene (2.23 g, 12.7 mmol) and 3-hydroxyphenylacetylene (1.50 g, 12.7 mmol) in N,N-dimethylformamide (13 mL), potassium carbonate (2.63 g, 19.0 mmol) was added, and the mixture was stirred at 55°C for 1.5 hours. After completion of the reaction, the reaction mixture was diluted with water, and then extracted with ethyl acetate. The organic layer was washed with water and saturated saline sequentially, dried over anhydrous sodium sulfate, filtered, and then concentrated under reduced pressure. The residue was purified by silica gel column chromatography (ethyl acetate/hexane) to obtain the title compound (2.19 g, 8.00 mmol, yield 63.1%). $^1$H-NMR ($CDCl_3$) δ: 3.15 (1H, s), 6.93 (1H, dd, J = 9.2, 2.4 Hz), 7.05-7.11 (2H, m), 7.20 (1H, s), 7.38-7.42 (2H, m), 7.98 (1H, d, J = 9.2 Hz).

[Reference Example C4] Ethyl 3-(4-amino-3-fluorophenoxy)benzoate

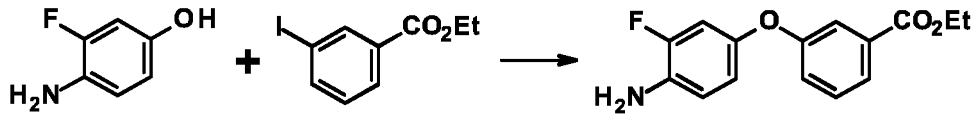

A suspension of ethyl 3-iodobenzoate (25.0g, 90.6 mmol), 4-amino-3-fluorophenol (23.1 g, 181 mmol), copper(I) iodide (0.89 g, 4.7 mmol), potassium carbonate (56.3 g, 407 mmol) and trans-N,N'-dimethylcyclohexane-1,2-diamine (2.9 mL, 18 mmol) in butyronitrile (226 mL) was stirred at 60°C under a nitrogen atmosphere for 86 hours. The reaction mixture was diluted with toluene, passed through a pad of Celite, and eluted with toluene. The eluted solution was concentrated under reduced pressure and the obtained residue was purified by amino silica gel column chromatography (ethyl acetate/hexane) to obtain the title compound (4.94 g, 18.0 mmol, yield 19.8%) .
$^1$H-NMR ($CDCl_3$) δ: 1.40 (3H, t, J = 7.3 Hz), 3.66 (2H, br s), 4.38 (2H, q, J = 7.3 Hz), 6.68-6.84 (3H, m), 7.16 (1H, dt, J = 8.9, 1.8 Hz), 7.39 (1H, t, J = 8.2 Hz), 7.61-7.63 (1H, m), 7.74-7.78 (1H, m).

[Reference Example C5] Methyl (3-{4-[(tert-Butoxycarbonyl)amino]-3-fluorophenoxy}phenyl) acetate

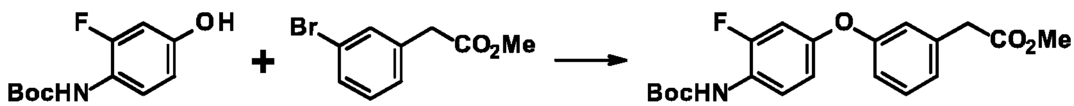

A suspension of methyl 2-(3-bromophenyl)acetate (3.67 mL, 21.3 mmol), copper(I) iodide (2.02g, 10.6 mmol), N,N-dimethylglycine (2.21 g, 21.4 mmol), tert-butyl (2-fluoro-4-hydroxyphenyl)carbamate (4.83 g, 21.3 mmol) and cesium carbonate (14.2 g, 43.5 mmol) in 1,4-dioxane (45 mL) was stirred at 90°C under a nitrogen atmosphere for 7.5 hours. The reaction solution was left to cool, and then filtered with Celite. The insoluble materials were washed with ethyl acetate, and the washing solution combined with the filtrate was concentrated under reduced pressure. The residue was purified by silica gel column chromatography (ethyl acetate/hexane) and dried under reduced pressure at 60°C to obtain the title compound (5.64 g, 15.0 mmol, yield 70.7%).
$^1$H-NMR ($CDCl_3$) δ: 1.53 (9H, s), 3.60 (2H, s), 3.70 (3H, s), 6.58 (1H, br s), 6.73-7.06 (5H, m), 7.25-7.32 (1H, m), 7.99 (1H, br s). MS m/z: 276 $[M-Boc+H]^+$.
By a similar method, the following compound was synthesized from the corresponding halobenzene.

[Table 3]

| Reference Example | Compound | Halobenzene |
|---|---|---|
| C6 | tert-Butyl [4-(3-bromophenoxy)-2-fluorophenyl]carbamate 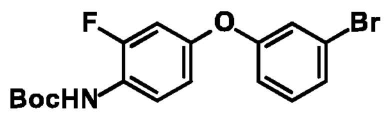 $^1$H-NMR ($CDCl_3$) δ: 1.53 (9H, s), 6.61 (1H, br s), 6.74-6.83 (2H, m), 6.89-6.94 (1H, m), 7.10-7.12 (1H, m), 7.16-7.25 (2H, m), 8.03 (1H, br s). | 1-Bromo-3-iodobenzene |

[Reference Example C7] 2-[3-(2-Chloro-5-fluoro-4-nitrophenoxy)phenyl]-5-ethyl-1,3,4-oxadiazole

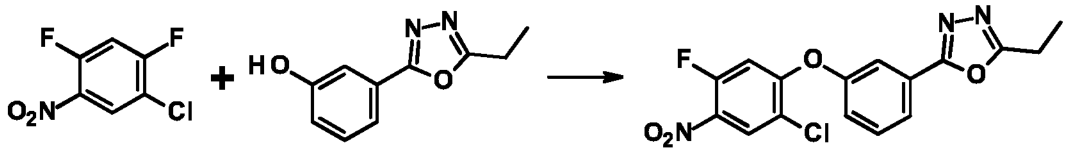

3-(5-Ethyl-1,3,4-oxadiazol-2-yl)phenol (250 mg, 1.31 mmol) and 5-chloro-2,4-difluoronitrobenzene (318 μL, 2.63 mmol) were dissolved in N,N-dimethylformamide (5 mL), and N,N-diisopropylethylamine (450 μL, 2.63 mmol) was added thereto. The mixture was stirred at room temperature for 7 hours, then diluted with ethyl acetate, and washed with water and saline. The organic layer was dried over anhydrous sodium sulfate and concentrated, and then purified by silica gel column chromatography (ethyl acetate/hexane) to obtain the title compound (441 mg, 1.21 mmol, yield 92.2%).
$^1$H-NMR ($CDCl_3$) δ: 1.45 (3H, t, J = 7.5 Hz), 2.97 (2H, q, J = 7.5 Hz), 6.70 (1H, d, J = 11.6 Hz), 7.27-7.30 (1H, m), 7.63 (1H, t, J = 7.9 Hz), 7.79-7.78 (1H, m), 8.00 (1H, d, J = 7.9 Hz), 8.31 (1H, d, J = 7.9 Hz). MS m/z: 364 $[M+H]^+$.

[Reference Example C8] tert-Butyl 3-(2-chloro-5-fluoro-4-nitrophenoxy)-1H-pyrazole-1-carboxylate

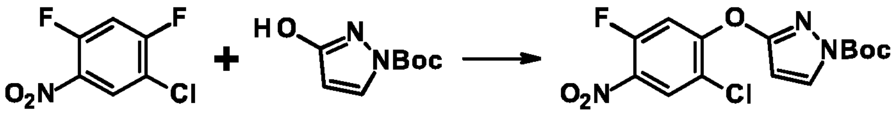

To a solution of 5-chloro-2,4-difluoronitrobenzene (6.30 g, 32.6 mmol) and tert-butyl 3-hydroxy-1H-pyrazole-1-carboxylate (6.00g, 32.6 mmol) in N,N-dimethylformamide (65 mL), potassium carbonate (9.00g, 65.1 mmol) was added at 0°C, and the mixture was stirred at 0°C for 2 hours. The reaction solution was diluted with ethyl acetate, and washed with water and saturated saline sequentially. The organic layer was dried over anhydrous sodium sulfate, filtered, and then the solvent was distilled off under reduced pressure. The residue was purified by silica gel column chromatography (ethyl acetate/hexane) and recrystallization (ethyl acetate/hexane) to obtain the title compound (5.05 g, 14.1 mmol, yield 43.3%) .
$^1$H-NMR ($CDCl_3$) δ: 1.65 (9H, s), 6.19 (1H, d, J = 3.0 Hz), 7.39 (1H, d, J = 11.5 Hz), 8.09 (1H, d, J = 3.0 Hz), 8.25 (1H, d, J = 7.9 Hz). MS m/z: 258 $[M-Boc+H]^+$

By a similar method, the following compounds were synthesized from the corresponding halobenzenes and intermediates.

[Table 4]

<table>
<tr><td rowspan="2">Reference Example</td><td rowspan="2">Compound</td><td>Halobenzene</td></tr>
<tr><td>Intermediate</td></tr>
<tr><td rowspan="2">C9</td><td rowspan="2">tert-Butyl 3-(2,5-difluoro-4-nitrophenoxy)-1 H-pyrazole-1-carboxylate<br>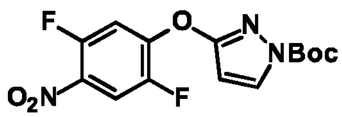<br>$^1$H-NMR ($CDCl_3$) δ: 1.64 (9H, s), 6.18 (1H, d, J = 2.4 Hz), 7.47-7.54 (1H, m), 7.93-8.01 (1H, m), 8.06 (1H, d, J = 2.4 Hz). MS m/z: 242 $[M-Boc+H]^+$.</td><td>2,4,5-Trifluoronitrobenzene</td></tr>
<tr><td>Reference Example B4</td></tr>
</table>

(continued)

| Reference Example | Compound | Halobenzene / Intermediate |
|---|---|---|
| C10 | 1-[3-(3-Chloro-4-nitrophenoxy)-1 H-pyrazol-1-yl]ethanone 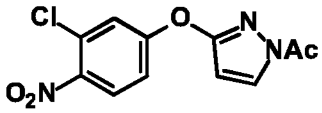 $^1$H-NMR ($CDCl_3$) δ: 2.64 (3H, s), 6.20 (1H, d, J = 3.0 Hz), 7.26-7.30 (1H, m), 7.45 (1H, d, J = 2.4 Hz), 8.02 (1H, d, J = 9.1 Hz), 8.24 (1H, d, J = 3.0 Hz). MS m/z: 282 [M+H]$^+$. | Halobenzene: 2-Chloro-4-fluoronitrobenzene<br>Intermediate: 1-(3-Hydroxypyrazol-1-yl) ethanone (WO 2015/041360 A1) |

[Reference Example C11] Ethyl 2-{4-[(tert-butoxycarbonyl)amino]-3-fluorophenoxy}-1,3-thiazole-4-carboxylate

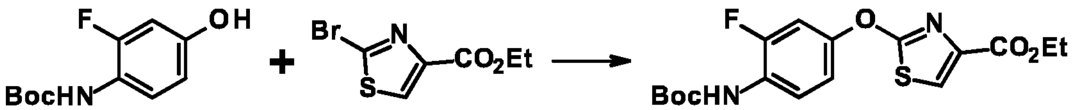

A suspension of tert-butyl (2-fluoro-4-hydroxyphenyl)carbamate (9.62 g, 42.3 mmol), ethyl 2-bromo-1,3-thiazole-4-carboxylate (10.0 g, 42.4 mmol) and cesium carbonate (20.0 g, 61.4 mmol) in tetrahydrofuran (200 mL) was stirred at 60°C for 8.5 hours. The reaction solution was diluted with water, and extracted with ethyl acetate (x 2). The organic layer was dried over anhydrous sodium sulfate, filtered, and then concentrated under reduced pressure, and the residue was purified by silica gel column chromatography (ethyl acetate/hexane). The roughly purified product was slurried with ethyl acetate/hexane (1:9) and the solid was collected by filtration and dried under reduced pressure at 50°C to obtain the title compound (11.2 g, 29.3 mmol, yield 69.1%) .
$^1$H-NMR ($CDCl_3$) δ: 1.39 (3H, t, J = 7.0 Hz), 1.53 (9H, s), 4.38 (2H, q, J = 7.0 Hz), 6.69 (1H, br s), 7.04-7.16 (2H, m), 7.72 (1H, s), 8.06-8.23 (1H, m). MS m/z: 383 [M+H]$^+$.

By a similar method, the following compounds were synthesized from the corresponding bromothiazole.

[Table 5]

| Reference Example | Compound | Bromothiazole |
|---|---|---|
| C12 | tert-Butyl {4-[(4-bromo-1,3-thiazol-2-yl)oxy]-2-fluorophenyl}carbamate 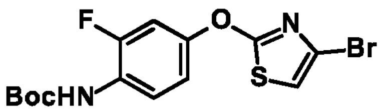 $^1$H-NMR ($CDCl_3$) δ: 1.53 (9H, s), 6.69 (1H, s), 6.74 (1H, s), 7.01-7.14 (2H, m), 8.15 (1H, s). MS m/z: 389 [M($^{79}$Br)+H]$^+$, 391 [M($^{81}$ Br)+H]$^+$. | 2,4-Dibromo-1,3-thiazole |

[Reference Example D1] tert-Butyl 3-(4-amino-3-fluorophenoxy)-1H-pyrazole-1-carboxylate

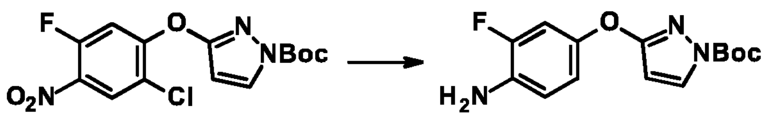

To a suspension of tert-butyl 3-(2-chloro-5-fluoro-4-nitrophenoxy)-1H-pyrazole-1-carboxylate (10.0 g, 28.0 mmol) in ethanol (200 mL), triethylamine (19.4 mL, 140 mmol) and 10% palladium on carbon (M) Wet (5.01 g) were

added, and the mixture was stirred at 50°C for 9.5 hours under a hydrogen atmosphere at normal pressure. After purging with nitrogen, the resultant was left at room temperature for 12.5 hours, then the catalyst was filtered off, and washed with ethanol. The filtrate was concentrated under reduced pressure and the residue was purified by silica gel column chromatography (ethyl acetate/hexane) to obtain the title compound (6.43 g, 21.9 mmol, yield 78.4%) .
$^1$H-NMR ($CDCl_3$) δ: 1.63 (9H, s), 3.63 (2H, br s), 5.89 (1H, d, J = 3.0 Hz), 6.74 (1H, t, J = 9.1 Hz), 6.80-6.85 (1H, m), 6.91 (1H, dd, J = 11.5, 2.4 Hz), 7.94 (1H, d, J = 3.0 Hz). MS m/z: 238 [M-tBu+H]$^+$.

[Reference Example D2] tert-Butyl 3-(4-amino-2-chloro-5-fluorophenoxy)-1H-pyrazole-1-carboxylate

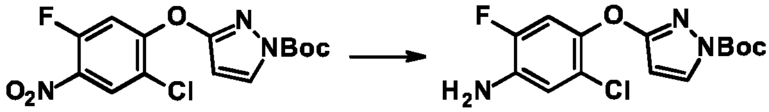

To a solution of tert-butyl 3-(2-chloro-5-fluoro-4-nitrophenoxy)-1H-pyrazole-1-carboxylate (6.00g, 16.8 mmol) in tetrahydrofuran (30 mL) and ethanol (30 mL), 5% platinum, sulfided, on carbon (1.0 g) was added, and the mixture was stirred at room temperature for 5.5 hours under a hydrogen atmosphere at normal pressure. The catalyst was filtered and then washed with ethanol, and the mother solution was concentrated to obtain the title compound (purity 98%, 5.63 g, 16.8 mmol, quantitative). $^1$H-NMR ($CDCl_3$) δ: 1.62 (9H, s), 5.89 (1H, d, J = 3.0 Hz), 6.84 (1H, d, J = 9.1 Hz), 7.01 (1H, d, J = 10.9 Hz), 7.93 (1H, d, J = 3.0 Hz). MS m/z: 272 [M-tBu+H]$^+$.

[Reference Example D3] tert-Butyl 3-(4-{[(benzyloxy)carbonyl]amino}-3-fluorophenoxy)-1H-pyrazole-1-carboxylate

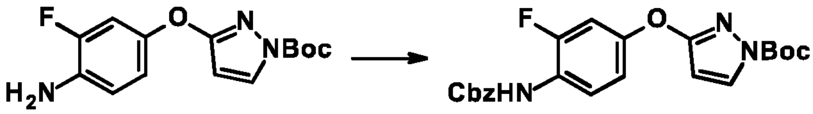

To a solution of tert-butyl 3-(4-amino-3-fluorophenoxy)-1H-pyrazole-1-carboxylate (6.14 g, 20.9 mmol) in tetrahydrofuran (50 mL), potassium carbonate (5.79 g, 41.9 mmol) and benzyl chloroformate (4.2 mL, 30 mmol) were added, and the mixture was stirred at 60°C for 4 hours. The reaction solution was diluted with ethyl acetate, passed through a pad of amino silica gel, and eluted with ethyl acetate. The eluted solution was concentrated under reduced pressure and the obtained residue was purified by silica gel column chromatography (ethyl acetate/hexane). The resultant was dried under reduced pressure at 60°C to obtain the title compound (8.15 g, 19.1 mmol, yield 91.1%).
$^1$H-NMR ($CDCl_3$) δ: 1.63 (9H, s), 5.22 (2H, s), 5.92-5.99 (1H, m), 6.80 (1H, s), 6.94-7.04 (2H, m), 7.31-7.48 (5H, m), 7.91-8.17 (2H, m). MS m/z: 450 [M+Na]$^+$.
By a similar method, the following compounds were synthesized from the corresponding intermediates.

[Table 6]

| Reference Example | Compound | Intermediate |
|---|---|---|
| D4 | tert-Butyl3-(4-{[(benzyloxy)carbonyl]amino}-2-chloro-5-fluorophenoxy)-1 H-pyrazole-1-carboxylate 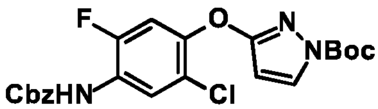 $^1$H-NMR ($CDCl_3$) δ: 1.62 (9H, s), 5.22 (2H, s), 5.97 (1H, d, J = 3.0 Hz), 6.87 (1H, br s), 7.14 (1H, d, J = 11.5 Hz), 7.35-7.43 (5H, m), 7.96 (1H, d, J = 3.0 Hz), 8.29 (1H, br s). | Reference Example D2 |

[Reference Example E1] 3-(3-Chloro-4-nitrophenoxy)-1H-pyrazole

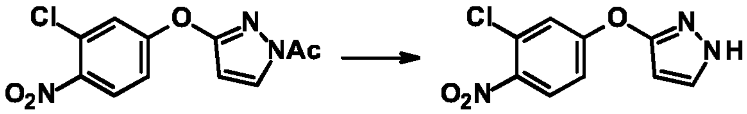

To a solution of 1-[3-(3-chloro-4-nitrophenoxy)-1H-pyrazol-1-yl]ethanone (970 mg, 3.44 mmol) in tetrahydrofuran (7 mL) and methanol (7 mL), 1 mol/L aqueous sodium hydroxide solution (6.9 mL, 6.9 mmol) was added dropwise, and the mixture was stirred at room temperature for 30 minutes. The reaction solution was diluted with water and extracted with dichloromethane. The organic layer was dried over anhydrous sodium sulfate, filtered, and then concentrated under reduced pressure. The concentrate was dried under reduced pressure to obtain the title compound (purity 95%, 871 mg, 3.44 mmol, quantitative).
$^1$H-NMR ($CDCl_3$) δ: 6.04 (1H, d, J = 2.4 Hz), 7.13 (1H, dd, J = 9.1, 3.0 Hz), 7.29 (1H, d, J = 3.0 Hz), 7.57 (1H, d, J = 2.4 Hz), 7.99 (1H, d, J = 9.1 Hz), 9.64 (1H, br s). MS m/z: 240 $[M+H]^+$.

[Reference Example E2] 3-(2-Chloro-5-fluoro-4-nitrophenoxy)-1H-pyrazole

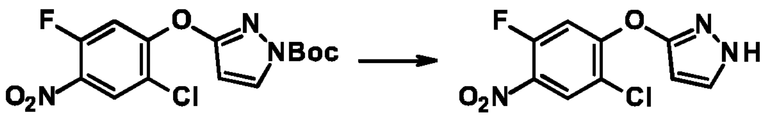

To a solution of tert-butyl 3-(2-chloro-5-fluoro-4-nitrophenoxy)-1H-pyrazole-1-carboxylate (9.0g, 25 mmol) in dichloromethane (18 mL), trifluoroacetic acid (12 mL) was added, and the mixture was stirred at room temperature for 3.5 hours. The reaction solution was concentrated under reduced pressure, and the residue was diluted with ethyl acetate, and washed with saturated aqueous sodium bicarbonate solution. The organic layer was dried over anhydrous sodium sulfate, filtered, and then the solvent was distilled off under reduced pressure, and the residue was purified by silica gel column chromatography (ethyl acetate/hexane) to obtain the title compound (6.36 g, 24.7 mmol, yield 98%).
$^1$H-NMR ($CDCl_3$) δ: 6.11 (1H, d, J = 2.4 Hz), 7.13 (1H, d, J = 12.1 Hz), 7.60 (1H, d, J = 2.4 Hz), 8.26 (1H, d, J = 7.9 Hz), 9.67 (1H, br s). MS m/z: 258 $[M+H]^+$.

By a similar method, the following compounds were synthesized from the corresponding intermediates.

[Table 7]

| Reference Example | Compound | Intermediate |
|---|---|---|
| E3 | 3-(2,5-Difluoro-4-nitrophenoxy)-1 H-pyrazole 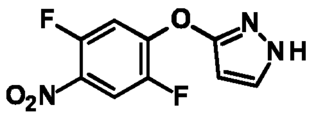 $^1$H-NMR ($CDCl_3$) δ: 6.09 (1H, d, J = 2.4 Hz), 7.20-7.27 (1H, m), 7.58 (1H, d, J = 2.4 Hz), 7.94-8.02 (1H, m), 9.70 (1H, br s). MS m/z: 242 [M+H]+. | Reference Example C9 |
| E4 | Benzyl [2-fluoro-4-(1 H-pyrazol-3-yloxy)phenyl]carbamate 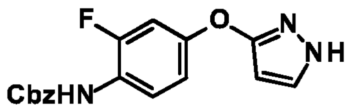 $^1$H-NMR ($CDCl_3$) δ: 5.21 (2H, s), 5.89 (1H, d, J = 2.5 Hz), 6.74-6.98 (3H, m), 7.30-7.50 (6H, m), 8.02 (1H, s), 9.55 (1H, s). MS m/z: 328 [M+H]+. | Reference Example D3 |

(continued)

| Reference Example | Compound | Intermediate |
|---|---|---|
| E5 | Benzyl {5-chloro-2-fluoro-4-[(1 H-pyrazol-3-yl)oxy]phenyl}carbamate 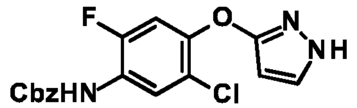 $^1$H-NMR ($CDCl_3$) δ: 5.22 (2H, s), 5.92 (1H, d, J = 2.4 Hz), 6.82 (1H, s), 7.01 (1H, d, J = 10.9 Hz), 7.33-7.44 (5H, m), 7.46 (1H, d, J = 2.4 Hz), 8.27 (1H, br s). | Reference Example D4 |

[Reference Example E6] tert-Butyl [2-fluoro-4-(1H-pyrazol-3-yloxy)phenyl]carbamate

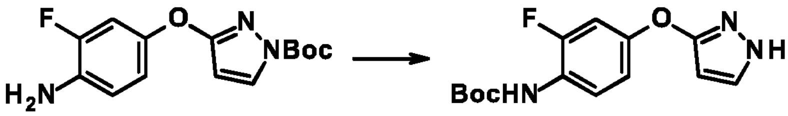

To tert-butyl 3-(4-amino-3-fluorophenoxy)-1H-pyrazole-1-carboxylate (23.9 g, 81.3 mmol), a solution of di-tert-butyl dicarbonate (39.0 g, 179 mmol) in tetrahydrofuran (100 mL) was added, and the mixture was heated to reflux for 15 hours. The reaction solution was cooled to room temperature, and 1-methylpiperazine (22.4 mL, 203 mmol) was added thereto. The mixture was stirred at room temperature for 2 hours, and then the reaction solution was concentrated under reduced pressure. The residue was diluted with ethyl acetate and washed with 1 mol/L hydrochloric acid (x 2), saturated aqueous sodium bicarbonate solution, and saturated saline. The organic layer was dried over anhydrous sodium sulfate, filtered, and then concentrated under reduced pressure. The residue was dissolved in methanol (410 mL), then potassium carbonate (1.13 g, 8.13 mmol) was added thereto, and the mixture was stirred at room temperature for 1 hour. The reaction solution was concentrated under reduced pressure, and the residue was diluted with ethyl acetate, and washed with water and saturated saline. The organic layer was dried over anhydrous sodium sulfate, filtered, and then concentrated under reduced pressure. The residue was slurried with a mixed solvent of ethyl acetate/hexane (1:9). The solid was collected by filtration, and dried under reduced pressure at 50°C to obtain the title compound (20.3 g, 69.3 mmol, yield 85.2%) .
$^1$H-NMR ($CDCl_3$) δ: 1.52 (9H, s), 5.87 (1H, d, J = 2.4 Hz), 6.59 (1H, br s), 6.88-6.96 (2H, m), 7.43 (1H, d, J = 2.4 Hz), 7.98 (1H, br s), 9.79 (1H, br s). MS m/z: 294 $[M+H]^+$.

[Reference Example F1] N-tert-Butyl-3-(2-chloro-5-fluoro-4-nitrophenoxy)-1H-pyrazole-1-carboxamide

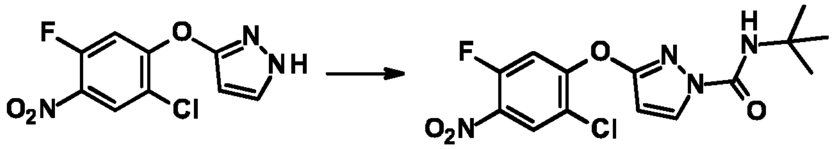

To a solution of 3-(2-chloro-5-fluoro-4-nitrophenoxy)-IH-pyrazole (1.93 g, 7.49 mmol) in 1,2-dichloroethane (37 mL), triethylamine (4.15 mL, 29.9 mmol) and tert-butyl isocyanate (2.57 mL, 22.5 mmol) were added, and the mixture was stirred at room temperature for 30 minutes and at 50°C for 2 hours, and then left at room temperature overnight. The solvent was distilled off under reduced pressure, and the residue was purified by silica gel column chromatography (dichloromethane/hexane) to obtain the title compound (2.39 g, 6.70 mmol, yield 89.5%).
$^1$H-NMR ($CDCl_3$) δ: 1.46 (9H, s), 6.15 (1H, d, J = 3.0 Hz), 6.70 (1H, s), 7.10 (1H, d, J = 11.5 Hz), 8.23 (1H, d, J = 3.0 Hz), 8.28 (1H, d, J = 7.3 Hz). MS m/z: 258 $[M-CONHtBu+H]^+$.
By a similar method, the following compounds were synthesized from the corresponding intermediates and isocyanic acid esters.

[Table 8]

| Reference Example | Compound | Intermediate |
|---|---|---|
| | | Isocyanic acid ester |
| F2 | 3-(2-Chloro-5-fluoro-4-nitrophenoxy)-N-(propan-2-yl)-1 H-pyrazole-1-carboxamide<br>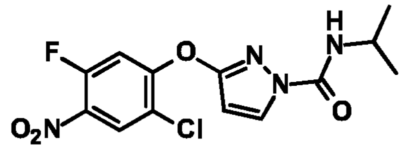<br>$^1$H-NMR ($CDCl_3$) δ: 1.28 (6H, d, J = 6.7 Hz), 4.05-4.17 (1H, m), 6.17 (1H, d, J = 2.4 Hz), 6.59 (1H, br s), 7.12 (1H, d, J = 10.9 Hz), 8.24 (1H, d, J = 2.4 Hz), 8.28 (1H, d, J = 7.3 Hz). | Reference Example E2 |
| | | Isopropyl isocyanate |
| F3 | N-tert-Butyl-3-(2,5-difluoro-4-nitrophenoxy)-1H-pyrazole-1-carboxamide<br>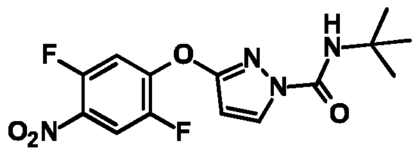<br>$^1$H-NMR ($CDCl_3$) δ: 1.48 (9H, s), 6.16 (1H, d, J = 2.4 Hz), 6.69 (1H, s), 7.19-7.27 (1H, m), 7.99-8.08 (1H, m), 8.23 (1H, d, J = 2.4 Hz). MS m/z: 242 [M-CONHtBu+H]$^+$. | Reference Example E3 |
| | | tert -Butyl isocyanate |

[Reference Example F4] 4-Nitrophenyl 3-(2-chloro-5-fluoro-4-nitrophenoxy)-1H-pyrazole-1-carboxylate

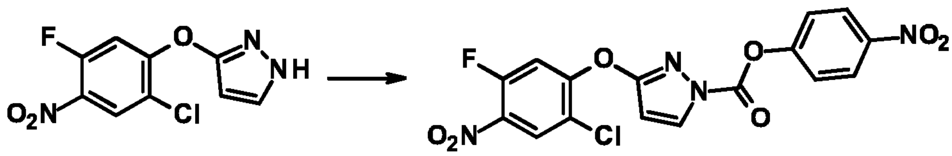

To a solution of 3-(2-chloro-5-fluoro-4-nitrophenoxy)-1H-pyrazole (1.33 g, 5.16 mmol) in dichloromethane (30 mL), 4-nitrophenyl chloroformate (1.14 g, 5.67 mmol) and triethylamine (1.00 mL, 7.22 mmol) were added under ice cooling, and the mixture was stirred at room temperature for 3 hours. The reaction solution was diluted with dichloromethane, and washed with water. The organic layer was dried over anhydrous sodium sulfate, filtered, and then the solvent was distilled off under reduced pressure to obtain the title compound (purity 97%, 2.26 g, 5.16 mmol, quantitative). The compound as roughly purified was used in the next reaction.
$^1$H-NMR ($CDCl_3$) δ: 6.39 (1H, d, J = 3.0 Hz), 7.46-7.53 (3H, m), 8.26-8.30 (2H, m), 8.34-8.39 (2H, m).

[Reference Example F5] 3-(2-Chloro-5-fluoro-4-nitrophenoxy)-N-(1-fluoro-2-methylpropan-2-yl)-1H-pyrazole-1-carboxamide

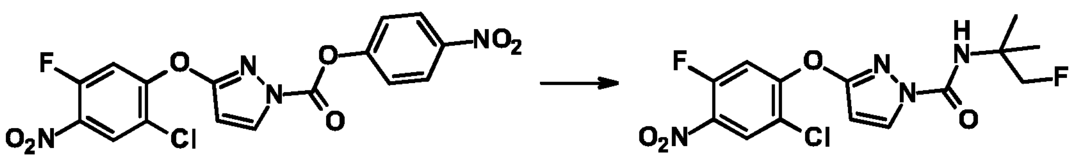

To a suspension of 4-nitrophenyl 3-(2-chloro-5-fluoro-4-nitrophenoxy)-1H-pyrazole-1-carboxylate (purity 97%, 333 mg, 0.761 mmol) in dichloromethane (12 mL), 1-fluoro-2-methylpropan-2-amine hydrochloride (106 mg, 0.831 mmol) and triethylamine (0.273 mL, 1.97 mmol) were added under ice cooling, and the mixture was stirred at 0°C for 4 hours.

The reaction solution was diluted with ethyl acetate, and washed with water and saturated saline sequentially. The organic layer was dried over anhydrous sodium sulfate, filtered, and then the solvent was distilled off under reduced pressure. The residue was purified by silica gel column chromatography (ethyl acetate/hexane) to obtain the title compound (126 mg, 0.336 mmol, yield 44.2%) .
$^1$H-NMR ($CDCl_3$) δ: 1.47 (6H, d, J = 1.8 Hz), 4.51 (2H, d, J = 47.4 Hz), 6.17 (1H, d, J = 3.0 Hz), 6.78 (1H, br s), 7.13 (1H, d, J = 11.5 Hz), 8.22 (1H, d, J = 3.0 Hz), 8.28 (1H, d, J = 7.9 Hz) .

By a similar method, the following compounds were synthesized from the corresponding amines.

[Table 9]

| Reference Example | Compound | Amine |
|---|---|---|
| F6 | 3-(2-Chloro-5-fluoro-4-nitrophenoxy)-N-(1-methylcyclopropyl)-1 H-pyrazole-1-carboxamide 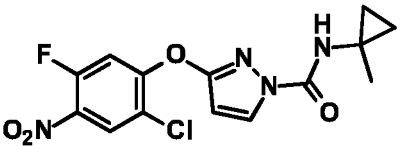 $^1$H-NMR ($CDCl_3$) δ: 0.71-0.78 (2H, m), 0.86-0.93 (2H, m), 1.47 (3H, s), 6.16 (1H, d, J = 3.1 Hz), 7.03 (1H, s), 7.15 (1H, d, J = 11.0 Hz), 8.21-8.32 (2H, m). | 1-Methylcyclopropylamine hydrochloride |

[Reference Example F7] 3-(2-Chloro-5-fluoro-4-nitrophenoxy) -N- [2-($^2H_3$) methyl ($^2H_6$) propan-2-yl]-1H-pyrazole-1-carboxamide

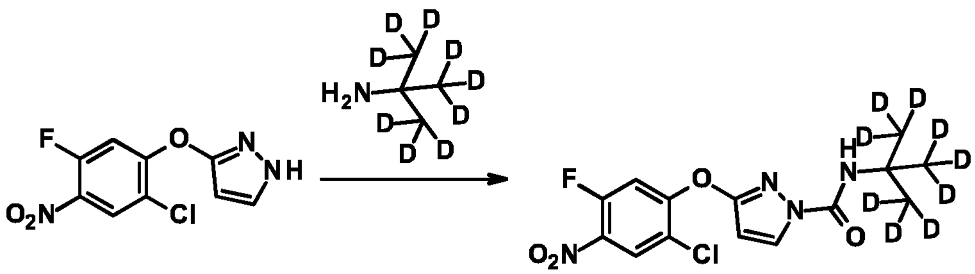

To a solution of 3-(2-chloro-5-fluoro-4-nitrophenoxy)-1H-pyrazole (438 mg, 1.70 mmol) in chloroform (34 mL), triethylamine (0.707 mL, 5.10 mmol) and 4-nitrophenyl chloroformate (377 mg, 1.87 mmol) were added under ice cooling, and the mixture was stirred at 0°C for 30 minutes and at room temperature for 1.5 hours, and then, 2-amino-2-methyl-$d_3$-propan-1,1,1,3,3,3-$d_6$ (175 mg, 2.13 mmol) was added thereto, and the mixture was stirred at room temperature for 4.5 hours. The solvent was then distilled off under reduced pressure, and the residue was purified by amino silica gel column chromatography (ethyl acetate/hexane) to obtain the title compound (148 mg, 0.405 mmol, yield 23.8%).
$^1$H-NMR ($CDCl_3$) δ: 6.15 (1H, d, J = 3.0 Hz), 6.69 (1H, br s), 7.10 (1H, d, J = 11.5 Hz), 8.23 (1H, d, J = 3.0 Hz), 8.28 (1H, d, J = 7.3 Hz).

[Reference Example G1] Methyl 3-{4-[(tert-butoxycarbonyl)amino]-3-fluorophenoxy}benzoate

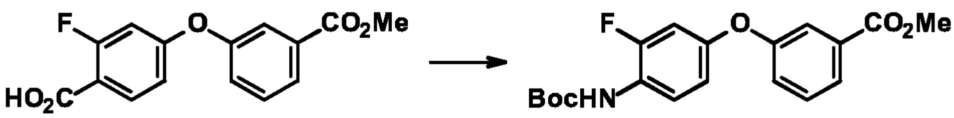

To a suspension of 2-fluoro-4-[3-(methoxycarbonyl)phenoxy]benzoic acid (4.73 g, 16.3 mmol) in toluene (81

mL), triethylamine (2.7 mL, 19.4 mmol) and diphenylphosphoryl azide (4.2 mL, 19 mmol) were added at 0°C, and the mixture was stirred at room temperature for 30 minutes and at 100°C for 45 minutes. To the reaction mixture, tert-butyl alcohol (7.64 mL, 81.4 mmol) was added, and the mixture was stirred at 100°C for 3 hours. The reaction mixture was concentrated under reduced pressure and the obtained residue was purified by silica gel column chromatography (ethyl acetate/hexane) to obtain the title compound (purity 93%, 6.36 g, 16.3 mmol, quantitative) as roughly purified.
$^1$H-NMR ($CDCl_3$) δ: 1.53 (9H, s), 3.90 (3H, s), 6.59 (1H, s), 6.73-6.82 (2H, m), 7.16-7.22 (1H, m), 7.36-7.43 (1H, m), 7.62 (1H, s), 7.78 (1H, d, J = 7.9 Hz), 8.02 (1H, s).

[Reference Example G2] 3-{4-[(tert-Butoxycarbonyl)amino]-3-fluorophenoxy}benzoic acid

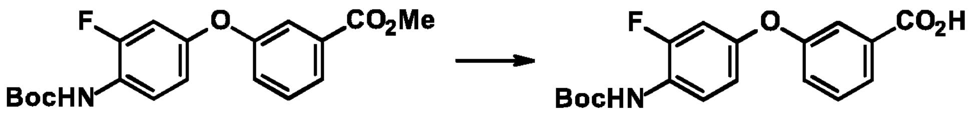

To a solution of methyl 3-{4-[(tert-butoxycarbonyl)amino]-3-fluorophenoxy}benzoate (purity 93%, 3.66 g, 9.39 mmol) in methanol (20 mL) and tetrahydrofuran (40 mL), an 1 mol/L aqueous sodium hydroxide solution (20.0 mL, 20.0 mmol) was added at room temperature, and the mixture was stirred at room temperature for 4 hours. The reaction mixture was neutralized by addition of 1 mol/L hydrochloric acid (20.0 mL, 20.0 mmol), and the mixture was concentrated under reduced pressure. The obtained residue was diluted by addition of water, and the mixture was extracted with ethyl acetate. The organic layer was washed with water and saturated saline, and dried over anhydrous sodium sulfate. The insoluble materials were filtered off and then the filtrate was concentrated under reduced pressure. The obtained solid was washed with water and hexane, and then dried under reduced pressure to obtain the title compound (2.97 g, 8.54 mmol, yield 90.9%).
$^1$H-NMR (DMSO-$D_6$) δ: 1.46 (9H, s), 6.83-6.89 (1H, m), 7.05 (1H, dd, J = 11.6, 2.4 Hz), 7.31 (1H, dd, J = 7.9, 2.4 Hz), 7.42-7.44 (1H, m), 7.49-7.60 (2H, m), 7.71 (1H, d, J = 7.3 Hz), 8.96 (1H, s), 13.18 (1H, s).
By a similar method, the following compounds were synthesized from the corresponding intermediates.

[Table 10]

| Reference Example | Compound | Intermediate |
|---|---|---|
| G3 | 2-{4-[(tert-Butoxycarbonyl)amino]-3-fluorophenoxy}-1,3-thiazol-4-carboxylic acid 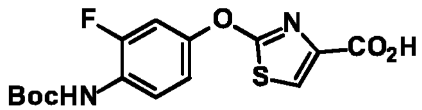 $^1$H-NMR (DMSO-$D_6$) δ: 1.47 (9H, s), 7.14-7.22 (1H, m), 7.43 (1H, dd, J = 11.0, 2.5 Hz), 7.63-7.70 (1H, m), 8.00 (1H, s), 9.10 (1H, s), 13.03 (1H, s). MS m/z: 355 [M+H]$^+$. | Reference Example C11 |

[Reference Example G4] tert-Butyl [3-(3-chloro-4-nitrophenoxy)-1H-pyrazol-1-yl]acetate

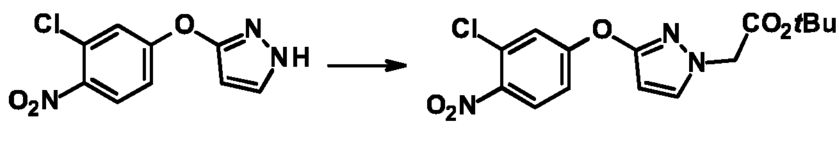

To a suspension of 3-(3-chloro-4-nitrophenoxy)-1H-pyrazole (purity 98%, 1.41 g, 5.76 mmol) and potassium carbonate (1.63 g, 11.8 mmol) in N,N-dimethylformamide (10 mL), tert-butyl chloroacetate (1.01 mL, 7.07 mmol) was added, and the mixture was stirred at 50°C for 2 hours. The reaction solution was diluted with ethyl acetate, and washed with water and saturated saline. The organic layer was dried over anhydrous sodium sulfate, filtered, and then the solvent was distilled off under reduced pressure. The residue was purified by silica gel column chromatography (ethyl acetate/hexane) to obtain the title compound (1.96 g, 5.53 mmol, yield 96.6%).

$^1H$-NMR ($CDCl_3$) δ: 1.49 (9H, s), 4.72 (2H, s), 5.96-6.03 (1H, m), 7.09-7.16 (1H, m), 7.27-7.30 (1H, m), 7.42-7.47 (1H, m), 7.95-8.00 (1H, m). MS m/z: 298 $[M-tBu+H]^+$.

[Reference Example G5] [3-(3-Chloro-4-nitrophenoxy)-1H-pyrazol-1-yl]acetic acid

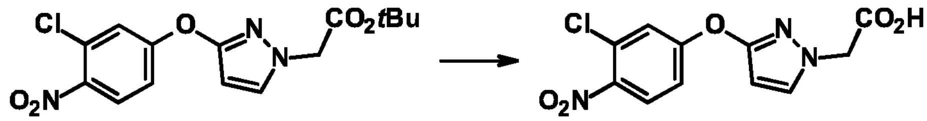

To a solution of tert-butyl [3-(3-chloro-4-nitrophenoxy)-1H-pyrazol-1-yl]acetate (500 mg, 1.41 mmol) in dichloromethane (2.0 mL), trifluoroacetic acid (3.0 mL, 39 mmol) was added dropwise at room temperature, and the mixture was stirred at the same temperature for 1.5 hours. The solvent was distilled off under reduced pressure, then the residue was diluted with ethyl acetate, and washed with water. The organic layer was dried over anhydrous sodium sulfate and filtered, and then the solvent was distilled off under reduced pressure to obtain the title compound (477 mg) as roughly purified. The compound was used in the next reaction without further purification.
$^1H$-NMR (DMSO-$D_6$) δ: 4.92 (2H, s), 6.12 (1H, d, J = 2.4 Hz), 7.23 (1H, dd, J = 9.1, 2.4 Hz), 7.42 (1H, t, J = 2.4 Hz), 7.81 (1H, d, J = 2.4 Hz), 8.17 (1H, d, J = 9.1 Hz), 13.15 (1H, br s). MS m/z: 298 $[M+H]^+$.

[Reference Example G6] tert-Butyl (2-fluoro-4-{[4-(hydroxymethyl)-1,3-thiazol-2-yl]oxy}phenyl) carbamate

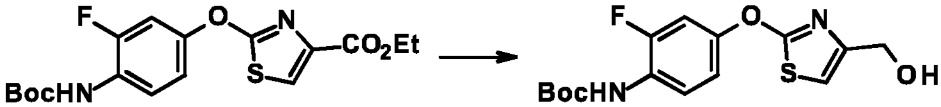

To a solution of ethyl 2-{4-[(tert-butoxycarbonyl)amino]-3-fluorophenoxy}-1,3-thiazole-4-carboxylate (919 mg, 2.40 mmol) in dichloromethane (12 mL), diisobutylaluminium hydride (1 mol/L toluene solution, 9.5 mL, 9.5 mmol) was added dropwise over 5 minutes under ice cooling, and the mixture was stirred at the same temperature for 10 minutes. The reaction solution was warmed to room temperature, stirred for 40 minutes, and then cooled to 0°C. A saturated aqueous Rochelle salt solution and water were sequentially added thereto, and the mixture was stirred at room temperature for 3 hours. The reaction solution was extracted with ethyl acetate, and the organic layer was dried over anhydrous sodium sulfate, filtered, and then concentrated under reduced pressure. The residue was purified by silica gel column chromatography (ethyl acetate/hexane) and dried under reduced pressure at 60°C to obtain the title compound (595 mg, 1.75 mmol, yield 72.7%). $^1H$-NMR ($CDCl_3$) δ: 1.53 (9H, s), 2.20 (1H, t, J = 5.5 Hz), 4.58 (2H, d, J = 5.5 Hz), 6.65-6.79 (2H, m), 7.01-7.14 (2H, m), 8.13 (1H, s). MS m/z: 341 $[M+H]^+$.

[Reference Example G7] tert-Butyl (4-{[4-(cyanomethyl)-1,3-thiazol-2-yl]oxy}-2-fluorophenyl)carbamate

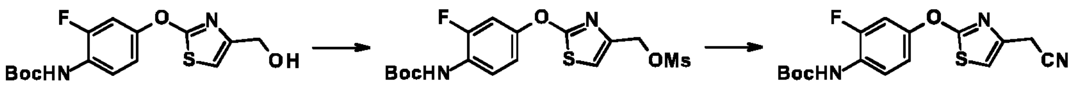

To a solution of tert-butyl(2-fluoro-4-{[4-(hydroxymethyl)-1,3-thiazol-2-yl]oxy}phenyl) carbamate (595 mg, 1.75 mmol) in dichloromethane (17 mL), triethylamine (0.315 mL, 2.27 mmol) and methanesulfonyl chloride (0.260 mg, 2.27 mmol) were added, and the mixture was stirred at room temperature for 16 hours. To the reaction solution, triethylamine (0.0484 mL, 0.349 mmol) and methanesulfonyl chloride (0.040 mg, 0.35 mmol) were added, and the mixture was stirred at room temperature for 30 minutes. The reaction solution was concentrated under reduced pressure. To the residue, a saturated aqueous ammonium chloride solution was added, and the mixture was extracted with ethyl acetate. The organic layer was washed with saturated saline, dried over anhydrous sodium sulfate, filtered, and then concentrated under reduced pressure. The concentrate was used in the next step as it was. To a solution of the obtained residue (805 mg) in dimethyl sulfoxide (17 mL), potassium cyanide (350 mg, 5.38 mmol) was added, and the mixture was stirred at

room temperature for 1.5 hours and at 50°C for 2 hours. The reaction solution was left to cool, and water was added. The mixture was extracted with ethyl acetate (x 2), and the obtained organic layer was washed with water and saturated saline sequentially, dried over anhydrous sodium sulfate, filtered, and then concentrated under reduced pressure. The concentrate was purified by silica gel column chromatography (ethyl acetate/hexane). The resultant was dried under reduced pressure at 60°C to obtain the title compound (527 mg, 1.51 mmol, yield 86.3%).
$^1$H-NMR ($CDCl_3$) δ: 1.53 (9H, s), 3.73 (2H, s), 6.55-6.76 (1H, m), 6.83 (1H, s), 6.98-7.14 (2H, m), 8.07-8.23 (1H, m). MS m/z: 294 $[M-tBu+H]^+$.

[Reference Example G8] [2-(4-Amino-3-fluorophenoxy)-1,3-thiazol-4-yl]acetic acid hydrochloride

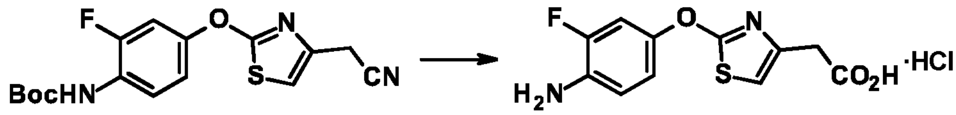

To a suspension of tert-butyl 4-{[4-(cyanomethyl)-1,3-thiazol-2-yl]oxyl-2-fluorophenyl)carbamate (398 mg, 1.14 mmol) in ethanol (5.68 mL) and water (1.42 mL), 4 mol/L aqueous potassium hydroxide solution (1.42 mL, 5.68 mmol) was added, and the mixture was stirred at 90°C for 2 hours. The reaction mixture was concentrated under reduced pressure, and the obtained residue was diluted with water, and washed with diethyl ether. Under ice cooling, the aqueous layer was neutralized with 1 mol/L hydrochloric acid (5.70 mL, 5.70 mmol), and the mixture was extracted with ethyl acetate. The organic layer was washed with saturated saline, dried over anhydrous sodium sulfate, filtered, and then concentrated under reduced pressure. To a solution of the obtained residue in tetrahydrofuran (4 mL), a 4 mol/L hydrogen chloride/1,4-dioxane solution (4 mL, 16 mmol) was added dropwise at room temperature, and the reaction mixture was stirred at 50°C for 2 hours. The reaction mixture was diluted with hexane, and the solid was collected by filtration. The obtained solid was washed with hexane and then dried under reduced pressure to obtain the title compound (320 mg, 1.05 mmol, yield 92.2%).
MS m/z: 269 $[M+H]^+$.

[Reference Example H1] tert-Butyl {4-[3-(tert-butylcarbamoyl)phenoxy]-2-fluorophenyl}carbamate

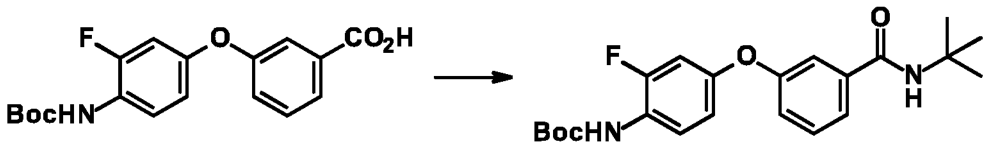

A solution of 3-{4-[(tert-butoxycarbonyl)amino]-3-fluorophenoxy}benzoic acid (600 mg, 1.73 mmol), 1-[bis(dimethylamino)methylene]-1H-1,2,3-triazolo[4,5-b]pyridinium 3-oxide hexafluorophosphate (852 mg, 2.24 mmol), N,N-diisopropylethylamine (0.44 mL, 2.6 mmol) and tert-butylamine (0.272 mL, 2.59 mmol) in N,N-dimethylformamide (6.9 mL) was stirred at room temperature overnight. The reaction mixture was diluted with ethyl acetate, washed with water and saturated saline, and the organic layer was dried over anhydrous sodium sulfate. The solvent was distilled off under reduced pressure, and the obtained residue was diluted with a mixed solvent of ethyl acetate/hexane (1:2), passed through a pad of silica gel, and eluted with the same solvent. The eluted solution was concentrated under reduced pressure to obtain the title compound (685 mg, 1.70 mmol, yield 98.5%).
$^1$H-NMR ($CDCl_3$) δ: 1.46 (9H, s), 1.53 (9H, s), 5.89 (1H, s), 6.59 (1H, s), 6.74-6.81 (2H, m), 7.06-7.11 (1H, m), 7.33-7.44 (3H, m), 8.01 (1H, s).
By a similar method, the following compounds were synthesized from the corresponding intermediates.

[Table 11]

| Reference Example | Compound | Intermediate |
|---|---|---|
| H2 | N-tert-Butyl-2-[3-(3-chloro-4-nitrophenoxy)-1H-pyrazol-1-yl]acetamide 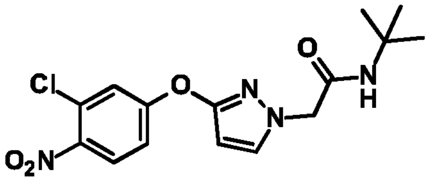 $^1$H-NMR ($CDCl_3$) δ: 1.32 (9H, s), 4.61 (2H, s), 6.02 (1H, d, J = 2.4 Hz), 7.12 (1H, dd, J = 9.1, 2.4 Hz), 7.47 (1H, d, J = 2.4 Hz), 7.99 (1H, d, J = 9.1 Hz). MS m/z: 353 [M+H]$^+$. | Reference Example G5 |
| H3 | tert-Butyl (4-{[4-(tert-Butylcarbamoyl)-1,3-thiazol-2-yl]oxy}-2-fluorophenyl)carbamate 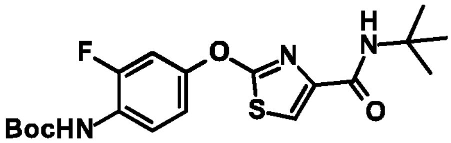 $^1$H-NMR ($CDCl_3$) δ: 1.44 (9H, s), 1.54 (9H, s), 6.70 (1H, s), 6.91 (1H, s), 7.03-7.09 (2H, m), 7.58 (1H, s), 8.10-8.22 (1H, m). | Reference Example G3 |
| H4 | 2-[2-(4-Amino-3-fluorophenoxy)-1,3-thiazol-4-yl]-N-tert-butylacetamide 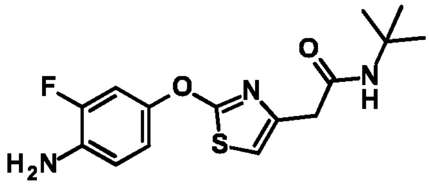 $^1$H-NMR ($CDCl_3$) δ: 1.29 (9H, s), 3.42 (2H, s), 3.76 (2H, s), 6.44-6.56 (2H, m), 6.74-6.82 (1H, m), 6.86-6.93 (1H, m), 6.98 (1H, dd, J = 11.0, 2.5 Hz). MS m/z: 324 [M+H]$^+$. | Reference Example G8 |

[Reference Example H5] Benzyl [4-({1-[2-(tert-butylamino)-2-oxoethyl]-1H-pyrazol-3-yl}oxy)-5-chloro-2-fluorophenyl]carbamate

[Chemical Formula 62]

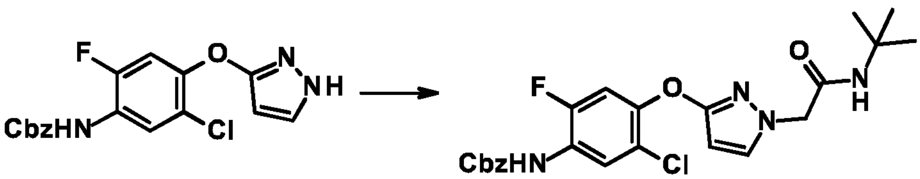

To a solution of benzyl {5-chloro-2-fluoro-4-[(1H-pyrazol-3-yl)oxy]phenyl}carbamate (5.94 g, 16.4 mmol) in N,N-dimethylformamide (32 mL), potassium carbonate (3.4 g, 25 mmol) and N-tert-butyl-2-chloroacetamide (2.95 g, 19.7 mmol) were added under ice cooling, and the mixture was stirred at room temperature for 1 hour, and at 50°C for 2 hours. N-tert-butyl-2-chloroacetamide (246 mg, 1.64 mmol) and potassium carbonate (340 mg, 2.46 mmol) were further added, and the mixture was stirred at 50°C for 1.5 hours. N-tert-butyl-2-chloroacetamide (120 mg, 0.802 mmol) and potassium carbonate (170 mg, 1.23 mmol) were added again, and the mixture was stirred at 50°C for 1.5 hours. The reaction solution was ice-cooled, diluted with ethyl acetate, and extracted by addition of water, and washed with saturated saline. The organic layer was dried over anhydrous sodium sulfate, filtered, and then the solvent was distilled off under reduced pressure. The residue was purified by silica gel column chromatography (ethyl acetate/hexane) to obtain the

title compound (3.06 g, 6.44 mmol, yield 39.2%). $^1$H-NMR ($CDCl_3$) δ: 1.28 (9H, s), 4.53 (2H, s), 5.23 (2H, s), 5.88 (1H, d, J = 2.4 Hz), 6.07 (1H, s), 6.84 (1H, s), 6.99 (1H, d, J = 10.9 Hz), 7.33 (1H, d, J = 2.4 Hz), 7.34-7.45 (5H, m), 8.28 (1H, br s). MS m/z: 475 $[M+H]^+$.

[Reference Example I1] N-tert-Butyl-2-[3-(3-chloro-4-nitrophenoxy)-4-fluoro-1H-pyrazol-1-yl]acetamide

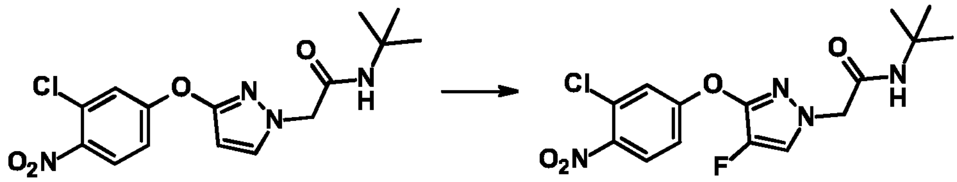

To a solution of N-tert-butyl-2-[3-(3-chloro-4-nitrophenoxy)-1H-pyrazol-1-yl]acetamide (260 mg, 0.737 mmol) in acetonitrile (15 mL), N-fluoro-N'-(chloromethyl)triethylenediamine bis(tetrafluoroborate) (784 mg, 2.21 mmol) was added, and the mixture was stirred at 60°C for 7.5 hours. The solvent was distilled off under reduced pressure, and then the residue was diluted with ethyl acetate, and washed with water and saturated aqueous sodium bicarbonate solution sequentially. The organic layer was dried over anhydrous sodium sulfate, filtered, and then the solvent was distilled off under reduced pressure. The residue was purified by silica gel column chromatography (ethyl acetate/hexane) to obtain the title compound (65 mg, 0.18 mmol, yield 24%).
$^1$H-NMR ($CDCl_3$) δ: 1.34 (9H, s), 4.53 (2H, s), 5.74 (1H, br s), 7.13 (1H, dd, J = 9.1, 2.4 Hz), 7.28 (1H, d, J = 2.4 Hz), 7.48 (1H, d, J = 4.3 Hz), 8.00 (1H, d, J = 9.1 Hz). MS m/z: 371 $[M+H]^+$.

By a similar method, the following compounds were synthesized from the corresponding intermediates.

[Table 12]

| Reference Example | Compound | Intermediate |
|---|---|---|
| I2 | tert-Butyl [3-(3-chloro-4-nitrophenoxy)-4-fluoro-1H-pyrazol-1-yl]acetate 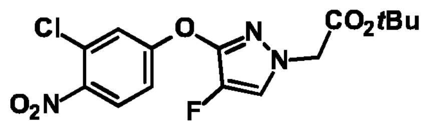 $^1$H-NMR ($CDCl_3$) δ: 1.49 (9H, s), 4.64 (2H, s), 7.10-7.16 (1H, m), 7.30 (1H, d, J = 2.5 Hz), 7.43 (1H, d, J = 4.3 Hz), 7.98 (1H, d, J = 9.2 Hz). | Reference Example G4 |
| I3 | Benzyl [4-({1-[2-(tert-butylamino)-2-oxoethyl]-4-fluoro-1 H-pyrazol-3-yl}oxy)-5-chloro-2-fluorophenyl]carbamate 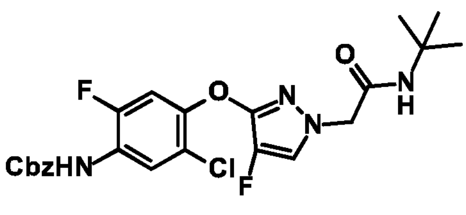 $^1$H-NMR ($CDCl_3$) δ: 1.29 (9H, s), 4.42-4.49 (2H, m), 5.23 (2H, s), 5.80-5.85 (1H, m), 6.82-6.86 (1H, m), 6.99 (1H, d, J = 10.9 Hz), 7.33-7.44 (6H, m), 8.31 (1H, br s). MS m/z: 493 $[M+H]^+$. | Reference Example H5 |

[Reference Example J1] 4-[3-(3-Chloro-4-nitrophenoxy)phenyl]-1-methyl-1H-1,2,3-triazole

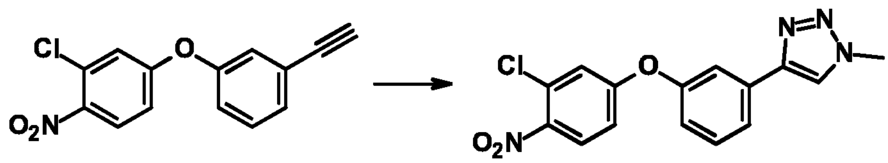

To a solution of 2-chloro-4-(3-ethynylphenoxy)-1-nitrobenzene (700 mg, 2.56 mmol) in acetonitrile (13 mL), sodium azide (333 mg, 5.12 mmol), iodomethane (0.287 mL, 4.60 mmol) and copper(I) iodide (487 mg, 2.56 mmol) were sequentially added, and the mixture was stirred at room temperature for 2 hours, and at 45°C for 4 hours, and then left at room temperature for 6 days. The reaction solution was diluted with ethyl acetate and water, filtered with Celite, and the filtrate was extracted with ethyl acetate. The organic layer was dried over anhydrous sodium sulfate, filtered, and then concentrated under reduced pressure. The residue was purified by silica gel column chromatography (dichloromethane/methanol) to obtain the title compound (547 mg, 1.65 mmol, yield 64.7%).
$^1$H-NMR ($CDCl_3$) δ: 4.17 (3H, s), 6.97 (1H, dd, J = 8.9, 2.7 Hz), 7.06 (1H, dd, J = 7.9, 2.4 Hz), 7.10 (1H, d, J = 2.4 Hz), 7.47-7.53 (1H, m), 7.59-7.61 (1H, m), 7.70 (1H, d, J = 7.9 Hz), 7.78 (1H, s), 7.99 (1H, d, J = 9.2 Hz). MS m/z: 331 $[M+H]^+$.

[Reference Example J2] tert-Butyl (2-fluoro-4-{3-[(trimethylsilyl)ethynyl]phenoxy}phenyl) carbamate

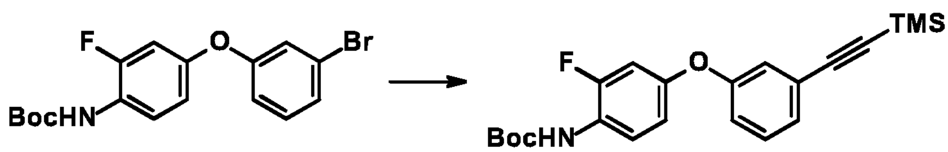

To a solution of tert-butyl [4-(3-bromophenoxy)-2-fluorophenyl]carbamate (1.16 g, 3.03 mmol) in triethylamine (6 mL), trimethylsilylacetylene (0.63 mL, 4.6 mmol), copper(I) iodide (58 mg, 0.30 mmol) and bis(triphenylphosphine)palladium(II) dichloride (213 mg, 0.303 mmol) were added, and the mixture was stirred at 90°C for 1.5 hours under a nitrogen atmosphere. The reaction mixture was cooled to room temperature and diluted with ethyl acetate, and the insoluble materials were filtered. The filtrate was concentrated under reduced pressure and the residue was purified by silica gel column chromatography (ethyl acetate/hexane) to obtain the title compound (649 mg, 1.62 mmol, yield 53.5%) .
$^1$H-NMR ($CDCl_3$) δ: 0.24 (9H, s), 1.53 (9H, s), 6.58 (1H, br s), 6.74-6.81 (2H, m), 6.94-6.98 (1H, m), 7.03-7.05 (1H, m), 7.18-7.27 (2H, m), 8.00 (1H, br s).

[Reference Example J3] tert-Butyl [4-(3-ethynylphenoxy)-2-fluorophenyl]carbamate

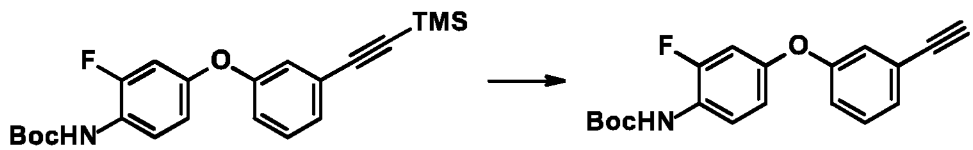

To a solution of tert-butyl (2-fluoro-4-{3-[(trimethylsilyl)ethynyl]phenoxy}phenyl)carbamate (648 mg, 1.62 mmol) in dichloromethane (5 mL) and methanol (5 mL), potassium carbonate (112 mg, 0.810 mmol) was added, and the mixture was stirred at room temperature for 1.5 hours. The reaction mixture was concentrated under reduced pressure, and extracted by addition of ethyl acetate and water. The organic layer was dried over anhydrous sodium sulfate, filtered, and then concentrated under reduced pressure. The residue was purified by silica gel column chromatography (ethyl acetate/hexane) to obtain the title compound (471 mg, 1.44 mmol, yield 88.7%) .
$^1$H-NMR ($CDCl_3$) δ: 1.53 (9H, d, J = 1.2 Hz), 6.59 (1H, br s), 6.74-6.83 (2H, m), 6.96-7.01 (1H, m), 7.06-7.09 (1H, m), 7.20-7.32 (3H, m), 8.01 (1H, br s).

[Reference Example J4] tert-Butyl {2-fluoro-4-[3-(1-methyl-1H-1,2,3-triazol-4-yl)phenoxy]phenyl}carbamate

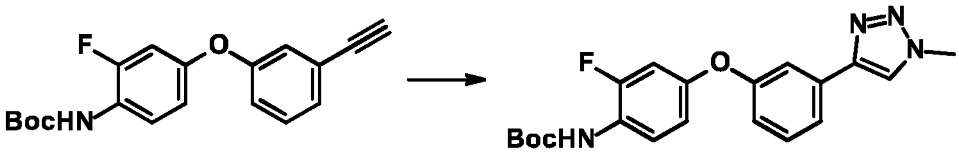

To a suspension of tert-butyl [4-(3-ethynylphenoxy)-2-fluorophenyl]carbamate (150 mg, 0.458 mmol) in methanol (6 mL) and water (2.5 mL), sodium azide (60 mg, 0.92 mmol), iodomethane (0.051 mL, 0.82 mmol), potassium carbonate (95 mg, 0.69 mmol), copper(II) sulfate pentahydrate (23 mg, 0.092 mmol), sodium L-ascorbate (36 mg, 0.18 mmol) and pyridine (0.018 mL, 0.22 mmol) were added, and the mixture was stirred at room temperature for 4 days. The reaction solution was diluted with ethyl acetate, and washed with water, saturated aqueous sodium bicarbonate solution, and saturated saline sequentially. The organic layer was dried over anhydrous sodium sulfate, filtered, and then concentrated under reduced pressure. The residue was purified by silica gel column chromatography (dichloromethane/methanol) to obtain the title compound (33 mg, 0.086 mmol, yield 19%).
$^1$H-NMR ($CDCl_3$) δ: 1.53 (9H, s), 4.14 (3H, s), 6.59 (1H, br s), 6.78-6.86 (2H, m), 6.94-6.98 (1H, m), 7.36-7.44 (2H, m), 7.56-7.60 (1H, m), 7.72 (1H, s), 8.00 (1H, br s) .

[Reference Example K1] Benzyl (2-fluoro-4-{[1-(2-fluoropyridin-4-yl)-1H-pyrazol-3-yl]oxy}phenyl)carbamate

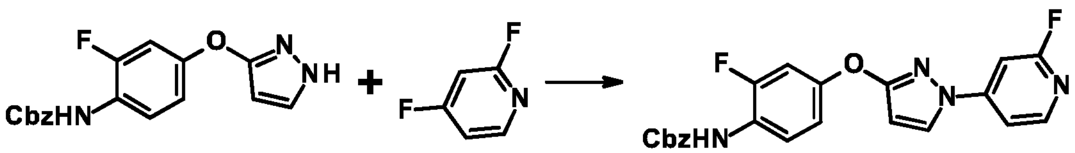

To a solution of benzyl [2-fluoro-4-(1H-pyrazol-3-yloxy)phenyl]carbamate (839 mg, 2.56 mmol) and 2,4-difluoropyridine (365 mg, 3.17 mmol) in dimethyl sulfoxide (13 mL), cesium carbonate (2.11 g, 6.48 mmol) was added at room temperature, and the mixture was stirred at room temperature overnight. The reaction mixture was diluted with ethyl acetate. The insoluble materials were filtered off and the reaction mixture was eluted with ethyl acetate. The eluted solution was washed with water and saturated saline, and the organic layer was dried over anhydrous sodium sulfate. The insoluble materials were filtered off, and the filtrate was concentrated under reduced pressure. The obtained residue was purified by silica gel column chromatography (ethyl acetate/hexane) to obtain the title compound (924 mg, 2.19 mmol, yield 85.4%).
$^1$H-NMR ($CDCl_3$) δ: 5.23 (2H, s), 6.12 (1H, d, J = 3.1 Hz), 6.85 (1H, br s), 7.00-7.07 (2H, m), 7.13-7.18 (1H, m), 7.34-7.44 (6H, m), 7.92 (1H, d, J = 3.1 Hz), 8.10 (1H, br s), 8.22 (1H, d, J = 6.1 Hz). MS m/z: 423 $[M+H]^+$.

[Reference Example K2] Benzyl (2-fluoro-4-{[1-(5-fluoro-6-methylpyridin-3-yl)-1H-pyrazol-3-yl]oxy}phenyl) carbamate

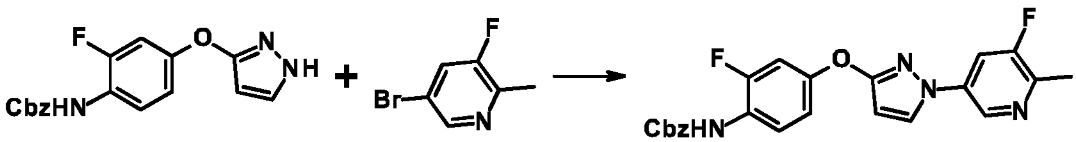

A suspension of benzyl [2-fluoro-4-(lH-pyrazol-3-yloxy)phenyl]carbamate (201 mg, 1.63 mmol), copper(I) iodide (62.8 mg, 0.330 mmol), 5-bromo-3-fluoro2-methylpyridine (178 mg, 0.936 mmol), trans-N,N'-dimethylcyclohexane-1,2-diamine (0.096 mL, 0.61 mmol) and potassium carbonate (230 mg, 2.21 mmol) in toluene (6.0 mL) was stirred overnight on an oil bath at 100°C. The reaction mixture was diluted with ethyl acetate, passed through a pad of silica gel and a pad of Celite, and eluted with ethyl acetate. The eluted solution was concentrated under reduced pressure and the obtained residue was purified by silica gel column chromatography (ethyl acetate/hexane) to obtain the title compound (86.0 mg, 0.197 mmol, yield 32.1%).
$^1$H-NMR ($CDCl_3$) δ: 2.55 (3H, d, J = 2.5 Hz), 5.23 (2H, s), 6.06 (1H, d, J = 3.1 Hz), 6.82 (1H, br s), 6.96-7.06 (2H, m), 7.32-7.46 (5H, m), 7.69 (1H, dd, J = 10.1, 2.1 Hz), 7.84 (1H, d, J = 3.1 Hz), 8.07 (1H, br s), 8.59 (1H, d, J = 1.8 Hz).
By a similar method, the following compounds were synthesized from the corresponding bromo compounds.

[Table 13]

| Reference Example | Compound | Bromo compound |
|---|---|---|
| K3 | Benzyl (2-fluoro-4-{[1-(6-methylpyridin-3-yl)-1 H-pyrazol-3-yl]oxy }phenyl)carbamate 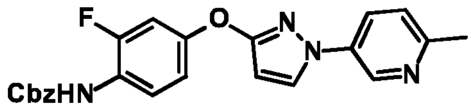 $^1$H-NMR ($CDCl_3$) δ: 2.59 (3H, s), 5.22 (2H, s), 6.04 (1H, d, J = 2.5 Hz), 6.81 (1H, br s), 6.94-7.05 (2H, m), 7.23 (1H, d, J = 8.6 Hz), 7.32-7.45 (5H, m), 7.80-7.90 (2H, m), 8.06 (1H, br s), 8.78 (1H, d, J = 2.5 Hz). MS m/z: 419 $[M+H]^+$. | 5-Bromo-2-methyl pyridine |

[Reference Example K4] tert-Butyl (2-fluoro-4-{[1-(2-methoxypyrimidin-5-yl)-1H-pyrazol-3-yl]oxy}phenyl) carbamate

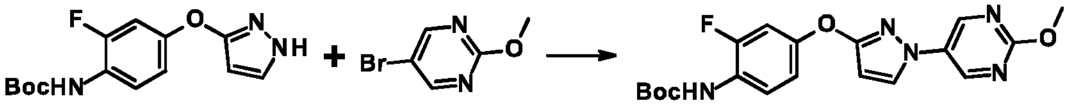

A solution of tert-butyl [2-fluoro-4-(1H-pyrazol-3-yloxy)phenyl]carbamate (193 mg, 0.657 mmol), 5-bromo-2-methoxypyrimidine (188 mg, 0.993 mmol), N,N-dimethylglycine (72.8 mg, 0.706 mmol), copper(I) iodide (64.4 mg, 0.338 mmol) and potassium carbonate (267 mg, 1.93 mmol) in dimethyl sulfoxide (3 mL) was stirred under a nitrogen atmosphere at 80°C for 6 hours. To the reaction solution, saturated aqueous sodium bicarbonate solution was added, and the mixture was extracted with ethyl acetate. The obtained organic layer was washed with saturated saline, dried over anhydrous sodium sulfate, filtered, and then concentrated under reduced pressure. The residue was purified by silica gel column chromatography (ethyl acetate/hexane) and dried under reduced pressure at 60°C to obtain the title compound (48.2 mg, 0.120 mmol, yield 18.3%) .
$^1$H-NMR ($CDCl_3$) δ: 1.54 (9H, s), 4.07 (3H, s), 6.02-6.07 (1H, m), 6.57-6.66 (1H, m), 6.94-7.06 (2H, m), 7.71-7.77 (1H, m), 8.04 (1H, br s), 8.80 (2H, s). MS m/z: 402 $[M+H]^+$.

By a similar method, the following compounds were synthesized from the corresponding bromo compounds.

[Table 14]

| Reference Example | Compound | Bromo compound |
|---|---|---|
| K5 | 4-{[1-(2-Cyclopropylpyrimidin-5-yl)-1 H-pyrazol-3-yl]oxy}-2-fluoroaniline 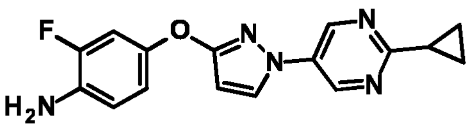 $^1$H-NMR ($CDCl_3$) δ: 1.03-1.21 (4H, m), 2.25-2.37 (1H, m), 3.65 (2H, br s), 6.00 (1H, d, J = 2.4 Hz), 6.74-6.80 (1H, m), 6.83-6.89 (1H, m), 6.96 (1H, dd, J = 11.6, 3.1 Hz), 7.76 (1H, d, J = 2.4 Hz), 8.85 (2H, s). MS m/z: 312 $[M+H]^+$. | 5-Bromo-2-cyclopropylpyrimidine |

[Reference Example K6] 2-Fluoro-4-{[1-(2-methoxypyridin-4-yl)-1H-pyrazol-3-yl]oxy}aniline

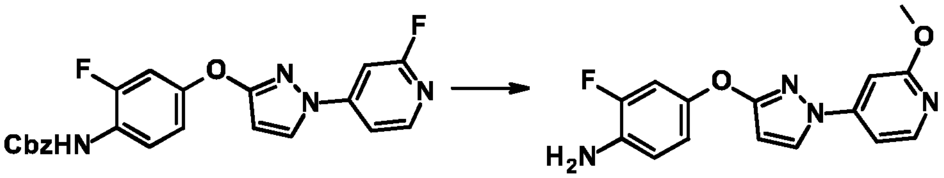

To a mixed solution of benzyl (2-fluoro-4-{[1-(2-fluoropyridin-4-yl)-1H-pyrazol-3-yl]oxy}phenyl)carbamate (304 mg, 0.719 mmol) in methanol (3 mL) and tetrahydrofuran (3 mL), a 28% sodium methoxide methanol solution (0.425 mL, 2.16 mmol) was added at room temperature, and the mixture was stirred at the same temperature overnight. To the reaction mixture, hydrazine monohydrate (0.4 mL, 8 mmol) was added at room temperature, and the mixture was stirred at the same temperature overnight. To the reaction mixture, a 1 mol/L aqueous sodium hydroxide solution (2.0 mL) was added at room temperature, and the mixture was stirred at 70°C for 9 hours. After the reaction mixture was returned to room temperature, the pH was adjusted to 8-9 by addition of 1 mol/L hydrochloric acid, and the mixture was diluted by addition of water, and extracted with ethyl acetate. The organic layer was washed with saturated saline, and dried over anhydrous sodium sulfate. The insoluble materials were filtered, and then the filtrate was concentrated under reduced pressure. The obtained residue was purified by silica gel column chromatography (ethyl acetate/hexane) to obtain the title compound (165 mg, 0.548 mmol, yield 76.3%).
$^1$H-NMR ($CDCl_3$) $\delta$: 3.65 (2H, s), 3.97 (3H, s), 5.99 (1H, d, J = 2.5 Hz), 6.74-6.80 (1H, m), 6.84-6.89 (1H, m), 6.93-6.99 (2H, m), 7.16 (1H, dd, J = 5.8, 2.1 Hz), 7.85 (1H, d, J = 2.5 Hz), 8.16 (1H, d, J = 5.5 Hz). MS m/z: 301 [M+H]$^+$.

[Reference Example L1] tert-Butyl (2-fluoro-4-{[4-(4,4,5,5-tetramethyl-1,3,2-dioxaborolan-2-yl)-1,3-thiazol-2-yl]oxy}phenyl) carbamate

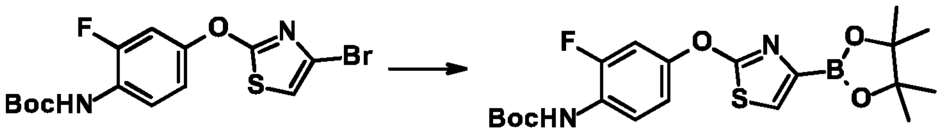

A suspension of tert-butyl {4-[(4-bromo-1,3-thiazol-2-yl)oxy]-2-fluorophenyl}carbamate (3.10, 7.96 mmol), [1,1'-bis(diphenylphosphino)ferrocene]palladium(II) dichloride dichloromethane adduct (650 mg, 0.796 mmol), potassium acetate (2.36 g, 24.1 mmol) and bis(pinacolato)diboron (2.44 g, 9.61 mmol) in 1,4-dioxane (60 mL) was stirred at 90°C for 6 hours. The reaction mixture was diluted by addition of ethyl acetate, filtered with Celite, and eluted with ethyl acetate. The eluted solution was concentrated under reduced pressure and the obtained residue was purified by silica gel column chromatography (ethyl acetate/hexane). To the roughly purified product, hexane was added to form a slurry. The slurry was left to stand still and then decanted to separate hexane, and the solid was dried under reduced pressure to obtain the title compound (2.47 g, 5.65 mmol, yield 71.0%).
$^1$H-NMR ($CDCl_3$) $\delta$: 1.34 (12H, s), 1.53 (9H, s), 6.65 (1H, s), 6.99-7.10 (2H, m), 7.50 (1H, s), 8.09 (1H, s).

[Reference Example L2] tert-Butyl (2-fluoro-4-{[4-(2-methoxypyrimidin-5-yl)-1,3-thiazol-2-yl]oxy}phenyl) carbamate

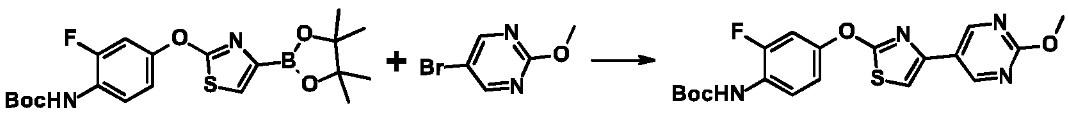

A suspension of tert-butyl (2-fluoro-4-{[4-(4,4,5,5-tetramethyl-1,3,2-dioxaborolan-2-yl)-1,3-thiazol-2-yl]oxy}phenyl)carbamate (400 mg, 0.917 mmol), 5-bromo-2-methoxypyrimidine (209 mg, 1.10 mmol), cesium carbonate (907 mg, 2.78 mmol) and [1,1'-bis(diphenylphosphino)ferrocene]palladium(II) dichloride dichloromethane adduct (75.4 mg, 0.0923 mmol) in water (0.50 mL) and 1,2-dimethoxyethane (10.0 mL) was stirred at 120°C under microwave irradiation for 1 hour. The reaction mixture was diluted with ethyl acetate, filtered with Celite, and eluted with ethyl acetate. The eluted solution was concentrated under reduced pressure and the obtained residue was purified by amino silica gel column chromatography (ethyl acetate/hexane) to obtain the title compound (314 mg, 0.750 mmol, yield 81.8%).

$^1$H-NMR ($CDCl_3$) δ: 1.54 (9H, s), 4.05 (3H, s), 6.70 (1H, br s), 7.00 (1H, s), 7.09-7.20 (2H, m), 8.09-8.23 (1H, m), 8.90 (2H, s). MS m/z: 419 $[M+H]^+$.

By a similar method, the following compounds were synthesized from the corresponding bromo compounds.

[Table 15]

| Reference Example | Compound | Bromo compound |
|---|---|---|
| L3 | tert-Butyl (2-fluoro-4-{[4-(5-fluoro-6-methylpyridin-3-yl)-1,3-thiazol-2-yl]oxy} phenyl)carbamate 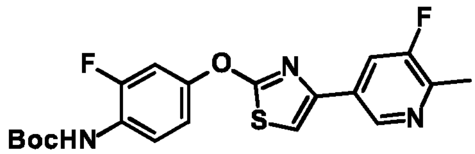 $^1$H-NMR ($CDCl_3$) δ: 1.54 (9H, s), 2.54 (3H, d, J = 3.1 Hz), 6.71 (1H, br s), 7.08 (1H, s), 7.09-7.20 (2H, m), 7.72 (1H, dd, J = 10.4, 1.8 Hz), 8.09-8.22 (1H, m), 8.71 (1H, s). MS m/z: 420 $[M+H]^+$. | 5-Bromo-3-fluoro-2-methyl pyridine |
| L4 | tert-Butyl (2 -fl uoro-4-{[4-(5-chloro-6-methylpyridin-3-yl)-1 , 3-thi azol-2-yl]oxy} phenyl)carbamate 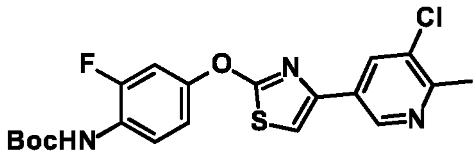 $^1$H-NMR ($CDCl_3$) δ: 1.54 (9H, s), 2.64 (3H, s), 6.72 (1H, br s), 7.08 (1H, s), 7.10-7.19 (2H, m), 8.04 (1H, d, J = 2.5 Hz), 8.10-8.23 (1H, m), 8.78 (1H, d, J = 2.5 Hz). | 5-Bromo-3-chloro-2-methyl pyridine |

[Reference Example M1] 3-(4-Amino-3-fluorophenoxy)-N-tert-butylbenzamide hydrochloride

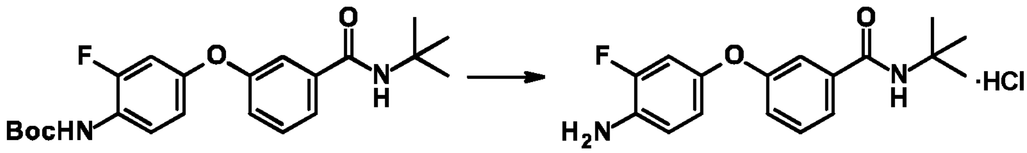

To a suspension of tert-butyl {4-[3-(tert-butylcarbamoyl)phenoxy]-2-fluorophenyl}carbamate (685 mg, 1.70 mmol) in ethanol (10 mL), a 4 mol/L hydrogen chloride/1,4-dioxane solution (10 mL, 40 mmol) was added at room temperature, and the reaction mixture was stirred at room temperature overnight. The reaction mixture was concentrated under reduced pressure, and toluene and ethanol were added to the obtained residue, and then the mixture was concentrated under reduced pressure to solidify. To the obtained residue, diethyl ether was added to form a slurry. The slurry was filtered and dried under reduced pressure to obtain the title compound (purity 86.0%, 619 mg, 1.57 mmol, yield 92.2%) as roughly purified.

$^1$H-NMR (DMSO-$D_6$) δ: 1.35 (9H, s), 6.74 (1H, dd, J = 8.5, 1.8 Hz), 6.90-7.29 (3H, m), 7.34-7.44 (2H, m), 7.55 (1H, d, J = 7.9 Hz), 7.79 (1H, s).

By a similar method, the following compounds were synthesized from the corresponding intermediates.

[Table 16]

| Reference Example | Compound | Intermediate |
|---|---|---|
| M2 | [3-(4-Amino-3-fluorophenoxy)phenyl]methyl acetate hydrochloride 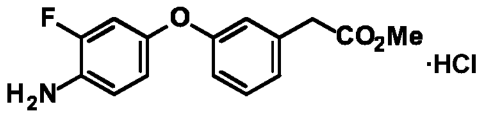 $^1$H-NMR (DMSO-$D_6$) δ: 3.61 (3H, s), 3.70 (2H, s), 6.77-6.85 (1H, m), 6.87-7.09 (4H, m), 7.16-7.40 (2H, m). MS m/z: 276 $[M+H]^+$. | Reference Example C5 |

[Reference Example M3] 2-Fluoro-4-[3-(1-methyl-1H-1,2,3-triazol-4-yl)phenoxy]aniline trifluoroacetate

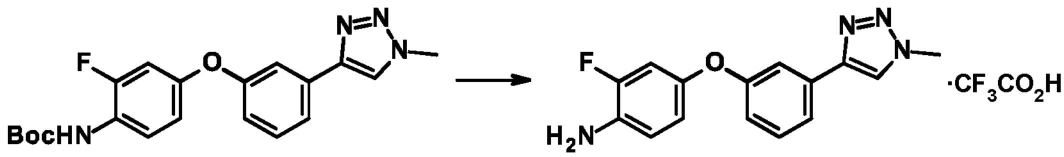

To a solution of tert-butyl {2-fluoro-4-[3-(1-methyl-1H-1,2,3-triazol-4-yl)phenoxy]phenyl}carbamate (33 mg, 0.086 mmol) in dichloromethane (1.5 mL), trifluoroacetic acid (0.5 mL, 7 mmol) was added dropwise, and the mixture was stirred at room temperature for 1.5 hours. The solvent was distilled off under reduced pressure to obtain the title compound (purity 92%, 37 mg, quantitative). The compound as roughly purified was used in the next reaction.
$^1$H-NMR ($CDCl_3$) δ: 4.17 (3H, s), 6.69-6.86 (3H, m), 6.94-6.99 (1H, m), 7.31-7.34 (1H, m), 7.36-7.41 (1H, m), 7.49 (1H, d, J = 7.9 Hz), 7.74 (1H, s).

By a similar method, the following compounds were synthesized from the corresponding intermediates.

[Table 17]

| Reference Example | Compound | Intermediate |
|---|---|---|
| M4 | Ethyl 2-(4-amino-3-fluorophenoxy)-1 ,3-thiazol-4-carboxylate 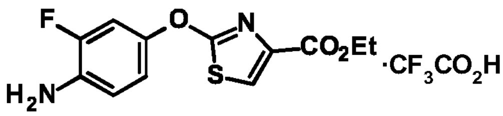 MS m/z: 283 $[M+H]^+$. | Reference Example C11 |

[Reference Example M5] 2-Fluoro-4-{[4-(5-fluoro-6-methylpyridin-3-yl)-1,3-thiazol-2-yl]oxy}aniline

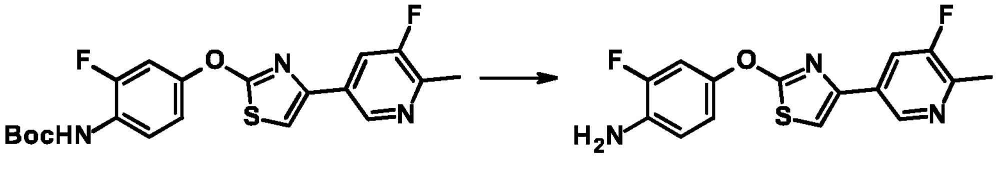

To a solution of tert-butyl (2-fluoro-4-{[4-(5-fluoro-6-methylpyridin-3-yl)-1,3-thiazol-2-yl]oxy}phenyl)carbamate (279 mg, 0.666 mmol) in dichloromethane (10 mL), trifluoroacetic acid (2 mL) was added at room temperature, and the mixture was stirred at room temperature overnight. The reaction mixture was concentrated under reduced pressure. To the obtained residue, saturated aqueous sodium bicarbonate solution was added to quench, and the mixture was extracted with dichloromethane. The organic layer was dried over anhydrous sodium sulfate, and the insoluble materials were filtered off and then the filtrate was concentrated under reduced pressure. The obtained residue was purified by

silica gel column chromatography (ethyl acetate/hexane) to obtain the title compound (189 mg, 0.591 mmol, yield 88.8%) . $^1$H-NMR ($CDCl_3$) δ: 2.54 (3H, d, J = 2.5 Hz), 3.78 (2H, br s), 6.81 (1H, t, J = 9.2 Hz), 6.95-6.99 (1H, m), 7.03-7.09 (2H, m), 7.71-7.77 (1H, m), 8.72 (1H, t, J = 1.5 Hz). MS m/z: 320 $[M+H]^+$.

By a similar method, the following compounds were synthesized from the corresponding intermediates.

[Table 18]

| Reference Example | Compound | Intermediate |
|---|---|---|
| M6 | 2-Fluoro-4-{[4-(5-chloro-6-methylpyridin-3-yl)-1,3-thiazol-2-yl]oxy}aniline 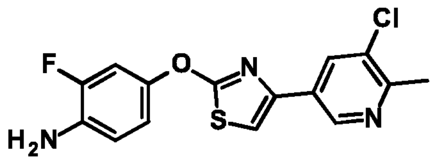 $^1$H-NMR ($CDCl_3$) δ: 2.64 (3H, s), 3.79 (2H, br s), 6.78-6.85 (1H, m), 6.95-6.98 (1H, m), 7.03-7.09 (2H, m), 8.06 (1H, d, J = 1.8 Hz), 8.79 (1H, d, J = 1.8 Hz). | Reference Example L4 |

[Reference Example N1] Methyl 3-(4-amino-3-chlorophenoxy)benzoate

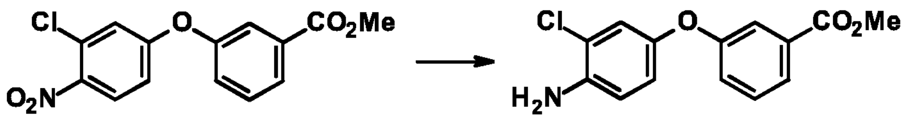

To a mixture of methyl 3-(3-chloro-4-nitrophenoxy)benzoate (6.59 g, 21.4 mmol) and iron powder (4.78 g, 85.7 mmol) in ethanol (36 mL), water (30 mL) and tetrahydrofuran (54 mL), ammonium chloride (119 mg, 2.23 mmol) was added while stirring at 90°C, and the mixture was stirred at 90°C for 3 hours. The reaction mixture was passed through a pad of Celite and a pad of amino silica gel, and eluted with ethanol. The eluted solution was concentrated under reduced pressure. The obtained residue was diluted by addition of ethyl acetate, passed through a pad of silica gel, and eluted with ethyl acetate. The eluted solution was concentrated under reduced pressure to obtain the title compound (purity 95%, 6.26 g, 21.4 mmol, quantitative) as roughly purified.
$^1$H-NMR ($CDCl_3$) δ: 3.89 (3H, s), 3.97 (2H, br s), 6.74-6.85 (2H, m), 7.00 (1H, d, J = 2.4 Hz), 7.13-7.16 (1H, m), 7.37 (1H, t, J = 7.9 Hz), 7.54-7.58 (1H, m), 7.71-7.74 (1H, m). MS m/z: 278 $[M+H]^+$.

By a similar method, the following compounds were synthesized from the corresponding intermediates.

[Table 19-1]

| Reference Examp le | Compound | Intermediate |
|---|---|---|
| N2 | 2-Chloro-4-[3-(1-methyl-1 H-1,2,3-triazol-4-yl)phenoxy]aniline 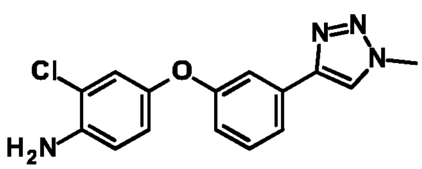 $^1$H-NMR ($CDCl_3$) δ: 3.95 (2H, br s), 4.14 (3H, s), 6.77 (1H, d, J = 8.5 Hz), 6.85 (1H, dd, J = 8.5, 2.4 Hz), 6.91 (1H, dd, J = 7.6, 2.1 Hz), 7.03 (1H, d, J = 2.4 Hz), 7.33-7.40 (2H, m), 7.52 (1H, d, J = 7.9 Hz), 7.71 (1H,s). MS m/z: 301 $[M+H]^+$. | Reference Example J1 |

(continued)

| Reference Examp le | Compound | Intermediate |
|---|---|---|
| N3 | 3-(4-Amino-2,5-difluorophenoxy)-N-tert-butyl-1 H-pyrazole-1-carboxamide 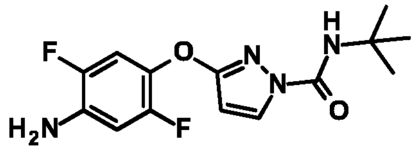 $^1$H-NMR ($CDCl_3$) δ: 1.46 (9H, s), 3.77 (2H, br s), 5.84 (1H, d, J = 2.4 Hz), 6.56-6.65 (1H, m), 6.75 (1H, br s), 6.91-7.01 (1H, m), 8.05 (1H, d, J = 2.4 Hz). MS m/z: 212 [M-CONHtBu+H]+. | Reference Example F3 |

[Table 19-2]

| Reference Example | Compound | Intermediate |
|---|---|---|
| N4 | 2-[3-(4-Amino-3-chlorophenoxy)-4-fluoro-1 H-pyrazol-1-yl]-N-tert-butyl acetamide 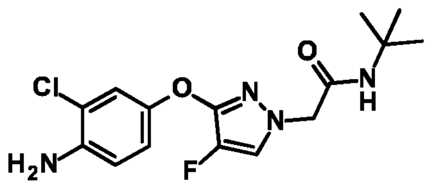 $^1$H-NMR ($CDCl_3$) δ: 1.30 (9H, s), 3.92 (2H, s), 4.45 (2H, br s), 5.93 (1H, br s), 6.69-6.76 (1H, m), 6.87-6.94 (1H, m), 7.04-7.10 (1H, m), 7.30-7.35 (1H, m). MS m/z: 341 $[M+H]^+$. | Reference Example 11 |
| N5 | tert-Butyl [3-(4-amino-3-chlorophenoxy)-4-fluoro-1 H-pyrazol-1-yl]acetate 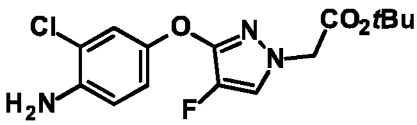 $^1$H-NMR ($CDCl_3$) δ: 1.47 (9H, s), 3.89 (2H, br s), 4.58 (2H, s), 6.69-6.75 (1H, m), 6.89-6.94 (1H, m), 7.09-7.13 (1H, m), 7.31-7.35 (1H, m). MS m/z: 342 $[M+H]^+$. | Reference Example 12 |

[Reference Example N6] 4-[3-(5-Ethyl-1,3,4-oxadiazol-2-yl)phenoxy]-2-fluoroaniline

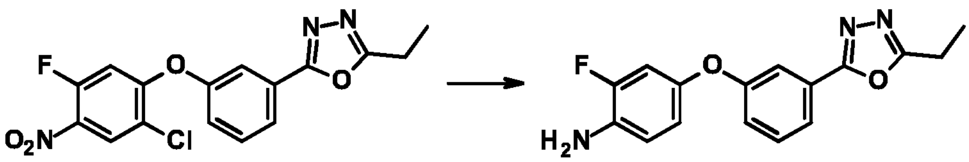

2-[3-(2-Chloro-5-fluoro-4-nitrophenoxy)phenyl]-5-ethyl-1,3,4-oxadiazole (441 mg, 1.21 mmol) was dissolved in ethanol (6 mL), and 10% palladium on carbon (M) Wet (100 mg, 1.88 mmol) and triethylamine (498 μL, 6.06 mmol) were added thereto. The mixture was stirred under a hydrogen atmosphere at normal pressure at 50°C for 5 hours, then the catalyst was removed by filtration with Celite. The filtrate was concentrated and then purified by silica gel column chromatography (ethyl acetate/hexane) to obtain the title compound (292 mg, 0.976 mmol, yield 80.5%).
$^1$H-NMR ($CDCl_3$) δ: 1.43 (3H, t, J = 7.7 Hz), 2.95 (2H, q, J = 7.7 Hz), 3.66 (2H, br s), 6.71 (1H, dd, J = 8.2, 2.1 Hz), 6.76-6.82 (2H, m), 7.09-7.12 (1H, m), 7.43 (1H, t, J = 8.2 Hz), 7.56-7.59 (1H, m), 7.72 (1H, d, J = 8.2 Hz). MS m/z: 300 $[M+H]^+$.

By a similar method, the following compounds were synthesized from the corresponding intermediates.

[Table 20-1]

| Reference Example | Compound | Intermediate |
|---|---|---|
| N7 | 3-(4-Amino-3-fluorophenoxy)-N-tert-butyl-1 H-pyrazole-1-carboxamide 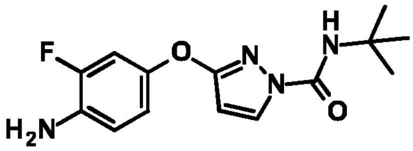 $^1$H-NMR ($CDCl_3$) δ: 1.45 (9H, s), 3.65 (2H, br s), 5.84 (1H, d, J = 3.0 Hz), 6.73-6.83 (3H, m), 6.88 (1H, dd, J = 11.5, 2.4 Hz), 8.06 (1H, d, J = 3.0 Hz). MS m/z: 293 $[M+H]^+$. | Reference Example F1 |
| N8 | 3-(4-Amino-3-fluorophenoxy)-N-(1-fluoro-2-methylpropan-2-yl)-1 H-pyrazole-1-carboxamide 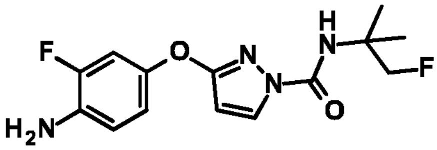 $^1$H-NMR ($CDCl_3$) δ: 1.45 (6H, d, J = 1.8 Hz), 3.66 (2H, br s), 4.51 (2H, d, J = 47.4 Hz), 5.86 (1H, d, J = 3.0 Hz), 6.73-6.82 (2H, m), 6.84-6.92 (2H, m), 8.04 (1H, d, J = 3.0 Hz). MS m/z: 311 $[M+H]^+$. | Reference Example F5 |
| N9 | 3-(4-Amino-3-fluorophenoxy)-N-(1-methylcyclopropyl)-1 H-pyrazole-1-carboxamide 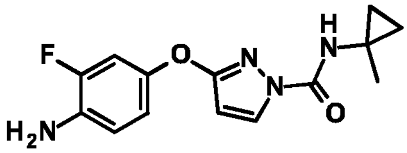 $^1$H-NMR ($CDCl_3$) δ: 0.68-0.77 (2H, m), 0.85-0.98 (2H, m), 1.47 (3H, s), 3.59-3.78 (2H, m), 5.89 (1H, d, J = 3.0 Hz), 6.74-6.85 (2H, m), 6.87-6.94 (1H, m), 7.13 (1H, br s), 8.09 (1H, d, J = 3.0 Hz). MS m/z: 194 $[M-C_5H_8NO+H]^+$. | Reference Example F6 |

[Table 20-2]

| Reference Example | Compound | Intermediate |
|---|---|---|
| N10 | 3-(4-Amino-3-fluorophenoxy)-N-[2-($^2H_3$)methyl ($^2H_6$)propan-2-yl]-1H-pyrazole-1-carboxamide 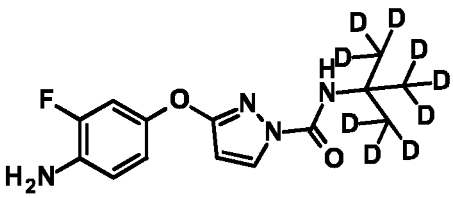 $^1$H-NMR ($CDCl_3$) δ: 3.66 (2H, br s), 5.84 (1H, d, J = 3.0 Hz), 6.73-6.84 (3H, m), 6.86-6.91 (1H, m), 8.06 (1H, d, J = 3.0 Hz). MS m/z: 302 [M+H]+. | Reference Example F7 |

(continued)

| Reference Example | Compound | Intermediate |
|---|---|---|
| N11 | 2-[3-(4-Amino-3-fluorophenoxy)-4-fluoro-1 H-pyrazol-1-yl]-N-tert-butylacetamide 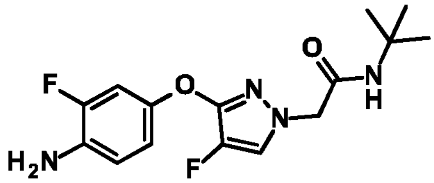 $^1$H-NMR ($CDCl_3$) δ: 1.30 (9H, s), 3.60 (2H, br s), 4.45 (2H, s), 5.94 (1H, br s), 6.70-6.80 (2H, m), 6.86 (1H, dd, J = 11.5, 2.4 Hz), 7.34 (1H, d, J = 4.3 Hz). MS m/z: 325 $[M+H]^+$. | Reference Example I3 |

[Reference Example N12] 3-(4-Amino-3-fluorophenoxy)-N-(propan-2-yl)-1H-pyrazole-1-carboxamide

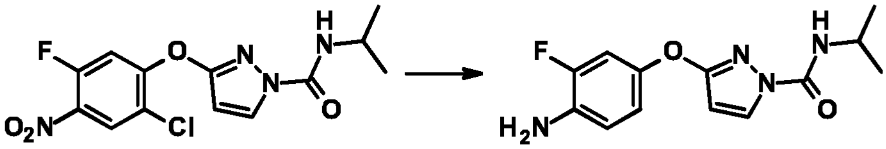

To a mixed solution of 3-(2-chloro-5-fluoro-4-nitrophenoxy)-N-(propan-2-yl)-1H-pyrazole-1-carboxamide (157 mg, 0.458 mmol) in ethanol (5 mL) and tetrahydrofuran (5 mL), 10% palladium on carbon powder (aqueous product) Type K (N.E. CHEMCAT CORPORATION) (60 mg) was added, and the mixture was stirred vigorously at room temperature for 1 hour under a hydrogen atmosphere at normal pressure. 10% palladium on carbon powder (aqueous product) Type K (N.E. CHEMCAT CORPORATION) (40 mg) was further added, and the mixture was stirred at 45°C for 10 hours under a hydrogen atmosphere at normal pressure. Furthermore, 10% palladium on carbon powder (aqueous product) Type K (N.E. CHEMCAT CORPORATION) (40 mg) was added, and the mixture was stirred at 45°C for 2.5 hours under a hydrogen atmosphere at normal pressure. The reaction solution was filtered, and concentrated under reduced pressure. To the concentrate, ethyl acetate was added, and the mixture was washed with saturated aqueous sodium bicarbonate solution. The organic layer was dried over anhydrous sodium sulfate, filtered, and then concentrated under reduced pressure. To the residue, ethanol (3 mL), tetrahydrofuran (3 mL), 10% palladium on carbon powder (aqueous product) Type K (N.E. CHEMCAT CORPORATION) (60 mg) was added, and the mixture was stirred at 45°C for 7 hours under a hydrogen atmosphere at normal pressure. Furthermore, 10% palladium on carbon powder (aqueous product) Type K (N.E. CHEMCAT CORPORATION) (40 mg) was added, and the mixture was stirred at 45°C for 3.5 hours under a hydrogen atmosphere at normal pressure. The reaction solution was filtered, and then washed with ethanol. The mother solution was concentrated under reduced pressure, and then diluted with dichloromethane, and washed with saturated aqueous sodium bicarbonate solution. The organic layer was dried over anhydrous sodium sulfate, filtered, and then concentrated under reduced pressure. The residue was purified by amino silica gel column chromatography (dichloromethane/hexane) to obtain the title compound (76 mg, 0.27 mmol, yield 60%). $^1$H-NMR ($CDCl_3$) δ: 1.27 (6H, d, J = 6.7 Hz), 3.66 (2H, br s), 4.04-4.14 (1H, m), 5.86 (1H, d, J = 3.0 Hz), 6.64-6.71 (1H, m), 6.73-6.82 (2H, m), 6.89 (1H, dd, J = 11.5, 2.4 Hz), 8.08 (1H, d, J = 3.0 Hz). MS m/z: 279 $[M+H]^+$.

[Reference Example O1] Methyl 3-{4-[(tert-butoxycarbonyl)amino]-3-chlorophenoxy}benzoate

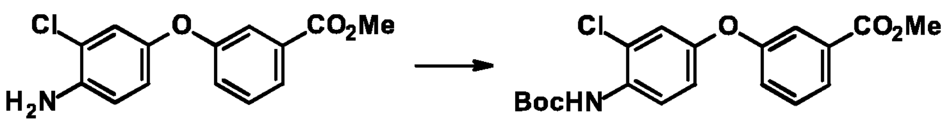

A mixture of methyl 3-(4-amino-3-chlorophenoxy)benzoate (purity 97%, 13.9 g, 48.7 mmol) and di-tert-butyl dicarbonate (22.0 g, 101 mmol) was stirred at 50°C for 5 hours, and at 90°C for 3 hours. The reaction solution was

purified by silica gel column chromatography (ethyl acetate/hexane) and amino silica gel column chromatography (ethyl acetate/hexane) to obtain the title compound (9.87 g, 26.1 mmol, yield 53.6%) .
$^1$H-NMR ($CDCl_3$) δ: 1.51 (9H, s), 3.88 (3H, s), 6.88-6.93 (2H, m), 7.02 (1H, d, J = 3.1 Hz), 7.14-7.17 (1H, m), 7.38 (1H, t, J = 7.9 Hz), 7.56-7.60 (1H, m), 7.73-7.79 (1H, m), 8.10 (1H, d, J = 9.2 Hz).

By a similar method, the following compounds were synthesized from the corresponding intermediates.

[Table 21]

| Reference Example | Compound | Intermediate |
|---|---|---|
| O2 | Ethyl 3-{4-[(tert-butoxycarbonyl)amino]-3-fluorophenoxy}benzoate 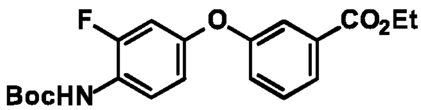 $^1$H-NMR ($CDCl_3$) δ: 1.38 (3H, t, J = 7.3 Hz), 1.53 (9H, s), 4.37 (2H, q, J = 7.3 Hz), 6.60 (1H, br s), 6.73-6.83 (2H, m), 7.15-7.20 (1H, m), 7.40 (1H, t, J = 7.9 Hz), 7.62-7.66 (1H, m), 7.79 (1H, d, J = 7.9 Hz), 7.94-8.11 (1H, m). MS m/z 320 [M-tBu+H]+. | Reference Example C4 |

[Reference Example O3] tert-Butyl {2-Chloro-4-[3-(hydrazinylcarbonyl)phenoxy]phenyl}carbamate

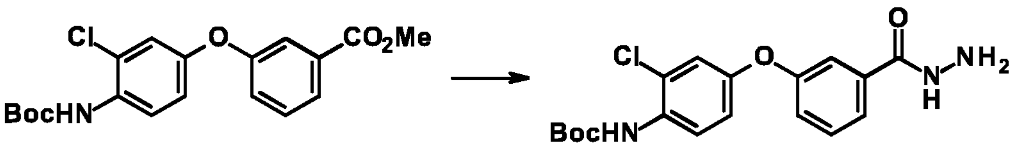

To a suspension of methyl 3-{4-[(tert-butoxycarbonyl)amino]-3-chlorophenoxy}benzoate (2.81 g, 7.44 mmol) in ethanol (24.8 mL), hydrazine monohydrate (1.8 mL, 37 mmol) was added at room temperature, and the mixture was stirred while being heated to reflux for 14 hours. The reaction mixture was concentrated under reduced pressure and the obtained residue was purified by silica gel column chromatography (ethyl acetate/hexane) to obtain the title compound (2.53 g, 6.69 mmol, yield 90.0%).
$^1$H-NMR ($CDCl_3$) δ: 1.54 (9H, s), 4.08 (2H, s), 6.85-7.16 (4H, m), 7.30-7.49 (4H, m), 8.13 (1H, d, J = 9.1 Hz). MS m/z: 378 $[M+H]^+$.

By a similar method, the following compounds were synthesized from the corresponding intermediates.

[Table 22]

| Reference Example | Compound | Intermediate |
|---|---|---|
| O4 | tert-Butyl{2-fluoro-4-[3-(hydrazinylcarbonyl)phenoxy]phenyl}carbamate 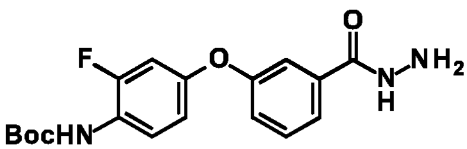 $^1$H-NMR ($CDCl_3$) δ: 1.53 (9H, s), 4.08 (2H, br s), 6.61 (1H, br s), 6.75-6.84 (2H, m), 7.08-7.48 (5H, m), 7.94-8.11 (1H, m). MS m/z 306 [M-tBu+H]+. | Reference Example O2 |

[Reference Example O5] tert-Butyl {2-chloro-4-[3-(5-methyl-1,3,4-oxadiazol-2-yl)phenoxy]phenyl}carbamate

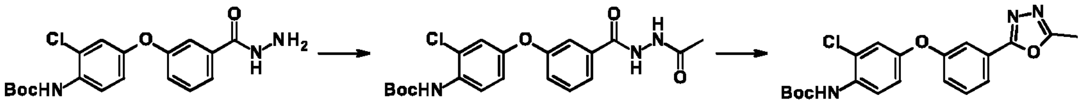

To a solution of tert-butyl {2-chloro-4-[3-(hydrazinylcarbonyl)phenoxy]phenyl}carbamate (506 mg, 1.34 mmol) and triethylamine (0.28 mL, 2.0 mmol) in dichloromethane (14 mL), acetyl chloride (0.128 mL, 1.81 mmol) was added dropwise at 0°C, and the mixture was stirred at 0°C for 1 hour. To the reaction mixture, saturated aqueous sodium bicarbonate solution was added to quench the reaction, and the mixture was diluted with water, and then extracted with dichloromethane. The organic layer was dried over anhydrous sodium sulfate, and the insoluble materials were filtered, and then the filtrate was concentrated under reduced pressure. To the obtained residue, p-toluenesulfonyl chloride (0.33 g, 1.7 mmol), triethylamine (0.28 mL, 2.0 mmol) and dichloromethane (6.7 mL) were added, and the mixture was stirred at room temperature overnight. The reaction mixture was concentrated under reduced pressure and the obtained residue was purified by silica gel column chromatography (ethyl acetate/hexane) to obtain the title compound (289 mg, 0.718 mmol, yield 53.6%).
$^1$H-NMR ($CDCl_3$) δ: 1.54 (9H, s), 2.61 (3H, s), 6.87-7.01 (2H, m), 7.05-7.17 (2H, m), 7.46 (1H, t, J = 8.2 Hz), 7.56-7.60 (1H, m), 7.75-7.81 (1H, m), 8.15 (1H, d, J = 9.1 Hz). MS m/z: 402 $[M+H]^+$.

[Reference Example O6] tert-Butyl {2-fluoro-4-[3-(5-methyl-1,3,4-oxadiazol-2-yl)phenoxy]phenyl}carbamate

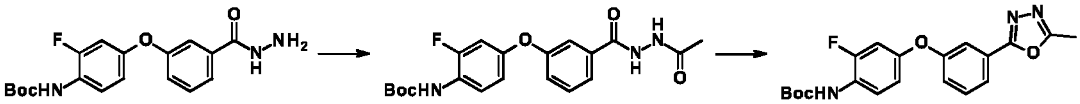

To a suspension of tert-butyl {2-fluoro-4-[3-(hydrazinylcarbonyl)phenoxy]phenyl}carbamate (15.8 g, 43.6 mmol) in dichloromethane (145 mL), acetic anhydride (4.33 mL, 45.8 mmol) was added dropwise at room temperature, and the reaction mixture was stirred at room temperature for 5 hours. The reaction mixture was diluted with water, and the organic layer and the aqueous layer were separated. The aqueous layer was then extracted with a mixed solvent of dichloromethane/methanol (9:1). The organic layers were combined, dried over anhydrous sodium sulfate, then passed through a pad of amino silica gel, and eluted with the same mixed solvent. The eluted solution was concentrated under reduced pressure. To the obtained tert-butyl (4-{3-[(2-acetylhydrazinyl)carbonyl]phenoxy}-2-fluorophenyl)carbamate (17.1 g) as roughly purified, triethylamine (12 mL, 87 mmol), p-toluenesulfonyl chloride (12.5 g, 65.4 mmol) and dichloromethane (220 mL) were added, and the reaction solution was stirred at room temperature overnight. The reaction mixture was diluted with water, and the organic layer and the aqueous layer were separated. The aqueous layer was then extracted with dichloromethane. The organic layer was dried over anhydrous sodium sulfate, then passed through a pad of amino silica gel, and eluted with dichloromethane. The eluted solution was concentrated under reduced pressure and the obtained residue was purified by silica gel column chromatography (ethyl acetate/hexane) to obtain the title compound (14.9 g, 38.8 mmol, yield 88.9%). $^1$H-NMR ($CDCl_3$) δ: 1.54 (9H, s), 2.61 (3H, s), 6.62 (1H, br s), 6.78-6.87 (2H, m), 7.15 (1H, dd, J = 8.2, 2.7 Hz), 7.46 (1H, t, J = 7.9 Hz), 7.57-7.61 (1H, m), 7.76-7.80 (1H, m), 7.96-8.13 (1H, m). MS m/s 386 $[M+H]^+$.

[Reference Example O7] tert-Butyl (2-fluoro-4-{3-[5-(propan-2-yl)-1,3,4-oxadiazol-2-yl]phenoxy}phenyl) carbamate

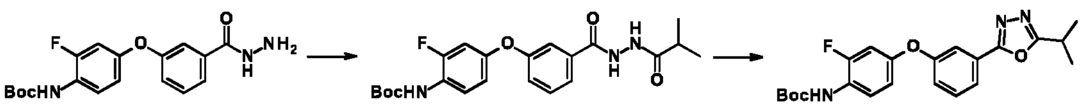

To a solution of tert-butyl {2-fluoro-4-[3-(hydrazinylcarbonyl)phenoxy]phenyl}carbamate (318 mg, 0.881 mmol) and triethylamine (0.244 mL, 1.76 mmol) in dichloromethane (9 mL), isobutyl chloride (0.139 mL, 1.32 mmol) was added under ice cooling, and the mixture was stirred at the same temperature for 1.5 hours. To the reaction solution, saturated aqueous sodium bicarbonate solution was added, and the mixture was extracted with dichloromethane. The organic layer was dried over anhydrous sodium sulfate, filtered, and then concentrated under reduced pressure. The residue

was dissolved in dichloromethane, triethylamine (0.366 mL, 2.64 mmol) and p-toluenesulfonyl chloride (250 mg, 1.31 mmol) were added thereto, and the mixture was stirred at room temperature for 17 hours. The reaction solution was concentrated under reduced pressure and purified by silica gel column chromatography (ethyl acetate/hexane) and amino silica gel column chromatography (ethyl acetate/hexane). The resultant was dried under reduced pressure at 60°C to obtain the title compound (196 mg, 0.474 mmol, yield 53.8%).
$^1$H-NMR ($CDCl_3$) δ: 1.45 (6H, d, J = 7.3 Hz), 1.53 (9H, s), 3.21-3.33 (1H, m), 6.61 (1H, br s), 6.77-6.87 (2H, m), 7.10-7.16 (1H, m), 7.42-7.49 (1H, m), 7.62-7.66 (1H, m), 7.76-7.82 (1H, m), 7.97-8.11 (1H, m). MS m/z: 414 $[M+H]^+$.

[Reference Example O8] tert-Butyl [4-(3-{[2-(cyclopropylcarbonyl)hydrazinyl]carbonyl}phenoxy)-2-fluorophenyl]carbamate

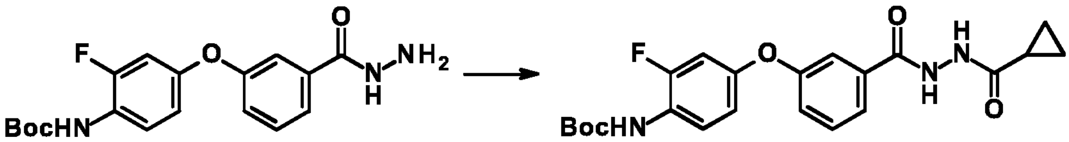

tert-Butyl {2-fluoro-4-[3-(hydrazinylcarbonyl)phenoxy]phenyl}carbamate (800 mg, 2.21 mmol) was dissolved in dichloromethane (20 mL), and triethylamine (614 μL, 4.43 mmol) and cyclopropanecarbonyl chloride (239 μL, 2.66 mmol) were added thereto at 0°C. The mixture was stirred at room temperature for 2 hours, then washed with saline. The organic layer was dried over anhydrous sodium sulfate, concentrated, and then purified by silica gel column chromatography (ethyl acetate/hexane) to obtain the title compound (452 mg, 1.05 mmol, yield 47.5%).
$^1$H-NMR ($CDCl_3$) δ: 0.86-0.91 (2H, m), 1.05-1.09 (2H, m), 1.50-1.55 (1H, m), 1.53 (9H, s), 6.60 (1H, br s), 6.76-6.81 (2H, m), 7.15 (1H, dd, J = 7.3, 2.4 Hz), 7.38-7.43 (2H, m), 7.51 (1H, d, J = 9.2 Hz), 8.02 (1H, br s), 8.57-8.61 (1H, m), 8.85-8.81 (1H, m). MS m/z: 374 $[M-tBu+H]^+$.

[Reference Example O9] tert-Butyl {4-[3-(5-cyclopropyl-1,3,4-oxadiazol-2-yl)phenoxy]-2-fluorophenyl}carbamate

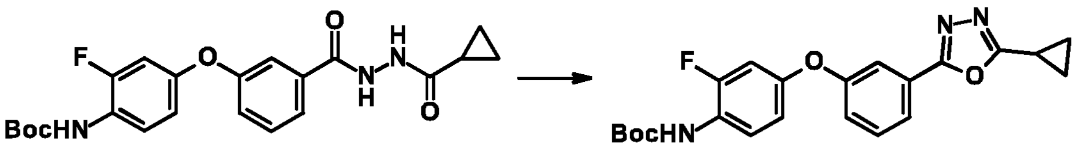

tert-Butyl [4-(3-{[2-(cyclopropylcarbonyl)hydrazinyl]carbonyl}phenoxy)-2-fluorophenyl]carbamate (452 mg, 1.05 mmol) was dissolved in dichloromethane (10 mL), and triethylamine (293 μL, 2.11 mmol) and p-toluenesulfonyl chloride (301 mg, 1.58 mmol) were added thereto at 0°C. The mixture was stirred at room temperature for 18 hours. The reaction solution was diluted with dichloromethane, washed with saline, and the organic layer was dried over anhydrous sodium sulfate. After concentration, the mixture was purified by silica gel column chromatography (ethyl acetate/hexane) to obtain the title compound (382 mg, 0.928 mmol, yield 88.2%) .
MS m/z: 412 $[M+H]^+$.

[Reference Example O10] tert-Butyl {2-fluoro-4-[3-(5-methyl-1,3,4-thiadiazol-2-yl)phenoxy]phenyl}carbamate

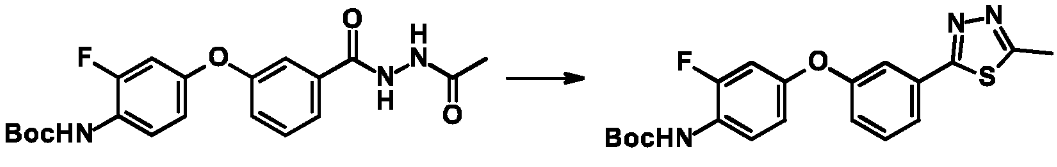

To a solution of tert-butyl (4-{3-[(2-acetylhydrazinyl)carbonyl]phenoxy}-2-fluorophenyl)carbamate (624 mg, 1.55 mmol) obtained in Reference Example O6 in toluene (6.0 mL), Lawesson's reagent (770 mg, 1.90 mmol) was added at

room temperature, and the reaction mixture was stirred at 100°C for 2 hours. The reaction mixture was diluted by addition of ethyl acetate, and the organic layer was washed with saturated aqueous sodium bicarbonate solution and saturated saline, and dried over anhydrous sodium sulfate. The insoluble materials were filtered off and the solvent was distilled off under reduced pressure. The obtained residue was purified by silica gel column chromatography (ethyl acetate/hexane) to obtain the title compound (103 mg, 0.257 mmol, yield 16.6%).
$^1$H-NMR ($CDCl_3$) δ: 1.53 (9H, s), 2.82 (3H, s), 6.61 (1H, br s), 6.76-6.89 (2H, m), 7.04-7.12 (1H, m), 7.42 (1H, t, J = 7.9 Hz), 7.56-7.58 (1H, m), 7.62-7.66 (1H, m), 8.04 (1H, br s). MS m/z: 402 $[M+H]^+$.

[Reference Example O11] tert-Butyl (4-{[4-(5-cyclopropyl-1,3,4-oxadiazol-2-yl)-1,3-thiazol-2-yl]oxyl-2-fluorophenyl) carbamate

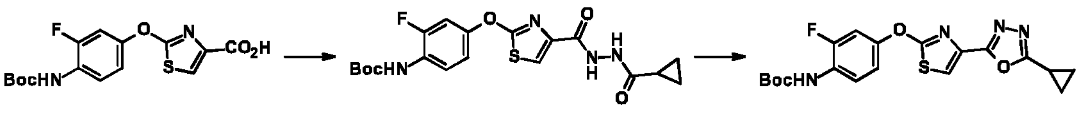

To a solution of 2-{4-[(tert-butoxycarbonyl)amino]-3-fluorophenoxy}-1,3-thiazole-4-carboxylic acid (902 mg, 2.55 mmol), cyclopropanecarboxylic acid hydrazide (386 mg, 3.86 mmol) and triethylamine (1.1 mL, 7.6 mmol) in N,N-dimethylformamide (10 mL), 1-[bis(dimethylamino)methylene]-1H-1,2,3-triazolo[4,5-b]pyridinium 3-oxide hexafluorophosphate (1.45 g, 3.82 mmol) was added at room temperature. The reaction mixture was stirred at room temperature overnight. The reaction mixture was diluted by addition of water, and extracted with ethyl acetate. The organic layer was washed with water and saturated saline, and dried over anhydrous sodium sulfate. The insoluble materials were then filtered, and the filtrate was concentrated under reduced pressure. A suspension of the obtained residue (1.31 g), triethylamine (1.8 mL, 13 mmol) and p-toluenesulfonyl chloride (983 mg, 5.15 mmol) in chloroform (50 mL) was stirred at room temperature for 6 hours. To the reaction mixture, triethylamine (1.1 mL, 7.6 mmol) and p-toluenesulfonyl chloride (973 mg, 5.10 mmol) were added at room temperature, and the mixture was stirred at room temperature overnight. The reaction solution was concentrated under reduced pressure, and the residue was purified by silica gel column chromatography (ethyl acetate/hexane). The resultant was dried under reduced pressure at 60°C to obtain the title compound (950 mg, 2.27 mmol, yield 89.2%).
$^1$H-NMR ($CDCl_3$) δ: 1.07-1.31 (4H, m), 1.54 (9H, s), 2.13-2.28 (1H, m), 6.65-6.78 (1H, m), 7.03-7.19 (2H, m), 7.55-7.69 (1H, m), 8.09-8.22 (1H, m).

[Reference Example P1] 2-Fluoro-4-[3-(5-methyl-1,3,4-oxadiazol-2-yl)phenoxy]aniline

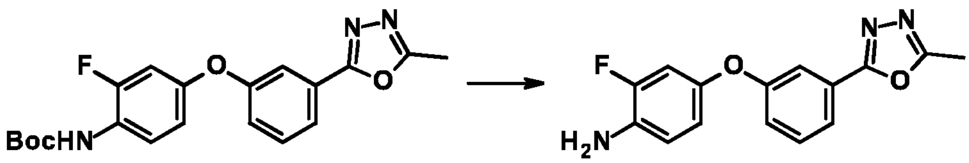

To a solution of tert-butyl {2-fluoro-4-[3-(5-methyl-1,3,4-oxadiazol-2-yl)phenoxy]phenyl}carbamate (6.71 g, 17.4 mmol) in dichloromethane (80 mL) placed in a water bath, trifluoroacetic acid (20 mL) was added, and the mixture was stirred at room temperature for 3 hours. The reaction mixture was concentrated under reduced pressure and azeotropically concentrated with dichloromethane to obtain a residue (15.0 g). A portion (7.90 g) of the obtained residue was diluted with dichloromethane and neutralized with 0.2 mol/L aqueous sodium hydroxide solution under ice cooling. The organic layer and the aqueous layer were separated, and the aqueous layer was then extracted with dichloromethane. The organic layers were combined, and dried over anhydrous sodium sulfate. The insoluble materials were filtered, and then the filtrate was concentrated under reduced pressure. The obtained residue was purified by amino silica gel column chromatography (dichloromethane), and the obtained product as roughly purified was solidified with a mixed solvent of hexane/diethyl ether and dried under reduced pressure to obtain the title compound (2.18 g, 7.63 mmol, yield 43.8%).
$^1$H-NMR ($CDCl_3$) δ: 2.61 (3H, s), 3.66 (2H, br s), 6.68-6.84 (3H, m), 7.10-7.13 (1H, m), 7.43 (1H, t, J = 7.9 Hz), 7.53-7.57 (1H, m), 7.69-7.74 (1H, m). MS m/s 286 $[M+H]^+$.

By a method similar to that in the first half step of Reference Example P1, the following compounds were

synthesized from the corresponding intermediates.

[Table 23]

| Reference Example | Compound | Intermediate |
|---|---|---|
| P2 | 2-Fluoro-4-{3-[5-(propan-2-yl)-1,3,4-oxadiazol-2-yl]phenoxy}aniline trifluoroacetate 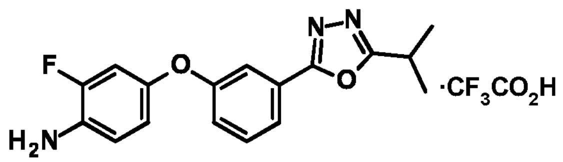 | Reference Example O7 |
| P3 | 4-[3-(5-Cyclopropyl-1,3,4-oxadiazol-2-yl)phenoxy]-2-fluoroaniline trifluoroacetate 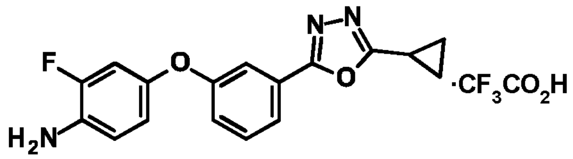 $^1$H-NMR ($CDCl_3$) $\delta$: 1.24-1.27 (4H, m), 2.28-2.35 (1H, m), 6.71-6.75 (1H, m), 6.80 (1H, dd, J = 11.6, 2.4 Hz), 6.88 (1H, t, J = 9.2 Hz), 7.13 (1H, dd, J = 7.3, 2.4 Hz), 7.45 (1H, t, J = 7.9 Hz), 7.53-7.54 (1H, m), 7.70-7.67 (1H, m). | Reference Example O9 |
| P4 | 2-Fluoro-4-[3-(5-methyl-1,3,4-thiadiazol-2-yl)phenoxy]aniline trifluoroacetate 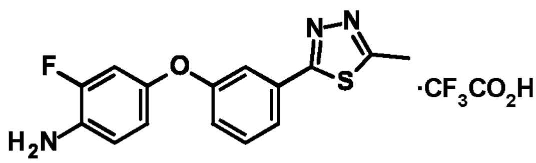 | Reference Example O10 |

[Reference Example Q1] Methyl 3-{3-fluoro-4-[(7-methoxy-6-{[1-(prop-2-enoyl)piperidin-4-yl]oxy}quinazolin-4-yl)amino]phenoxy}benzoate

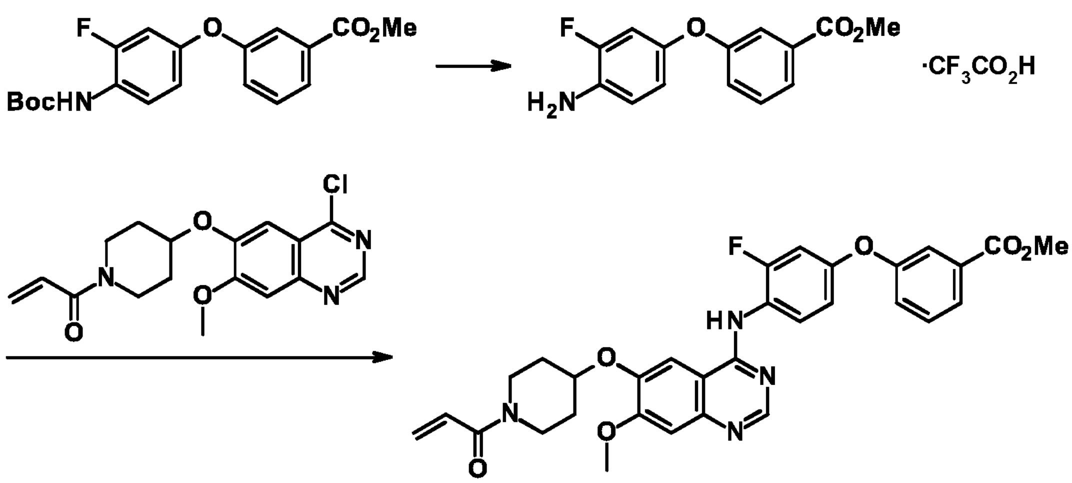

To a solution of methyl 3-{4-[(tert-butoxycarbonyl)amino]-3-fluorophenoxy}benzoate (purity 93%, 2.50 g, 6.40 mmol) in dichloromethane (15 mL), trifluoroacetic acid (5 mL) was added at room temperature, and the mixture was stirred at room temperature for 5 hours. The reaction mixture was concentrated under reduced pressure. To the obtained residue, toluene was added, and the mixture was azeotropically concentrated. To the obtained residue, 2-propanol (40 mL) and 1-{4-[(4-chloro-7-methoxyquinazolin-6-yl)oxy]piperidin-1-yl}prop-2-en-1-one (2.11 g, 6.07 mmol) were added,

and the mixture was stirred at 50°C for 3 hours. The reaction mixture was diluted with chloroform, and an amino silica gel was added thereto. The insoluble materials were filtered, and then the reaction mixture was eluted with a mixed solvent of chloroform/methanol (9:1). The eluted solution was concentrated under reduced pressure and the obtained residue was purified by silica gel column chromatography (dichloromethane/methanol) to obtain the title compound (3.36 g, 5.86 mmol, yield 96.6%).

$^1$H-NMR ($CDCl_3$) δ: 1.88-2.11 (4H, m), 3.49-3.62 (1H, m), 3.67-3.77 (1H, m), 3.82-4.04 (2H, m), 3.91 (3H, s), 4.02 (3H, s), 4.67-4.76 (1H, m), 5.72 (1H, dd, J = 10.7, 1.8 Hz), 6.31 (1H, dd, J = 17.1, 1.8 Hz), 6.63 (1H, dd, J = 17.1, 10.7 Hz), 6.84-6.94 (2H, m), 7.15-7.19 (1H, m), 7.21-7.33 (3H, m), 7.45 (1H, t, J = 8.2 Hz), 7.68-7.71 (1H, m), 7.83 (1H, d, J = 7.3 Hz), 8.33-8.41 (1H, m), 8.68 (1H, s). MS m/z: 573 $[M+H]^+$.

By a similar method, the following compounds were synthesized from the corresponding intermediates.

[Table 24-1]

| Reference Example | Compound | Intermediate |
|---|---|---|
| Q2 | 1-[4-({4-[2-Fluoro-4-(1 H-pyrazol-3-yloxy)anilino]-7-methoxyquinazolin-6-yl}oxy) piperidin-1-yl]prop-2-en-1-one 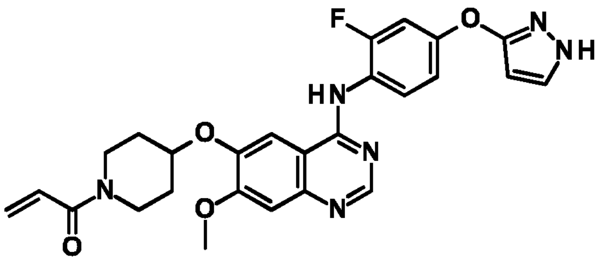 $^1$H-NMR (DMSO-$D_6$) δ: 1.67-1.79 (2H, m), 1.95-2.06 (2H, m), 3.44-3.54 (2H, m), 3.81-3.91 (2H, m), 3.94 (3H, s), 4.69-4.78 (1H, m), 5.65 (1H, dd, J = 10.6, 2.4 Hz), 5.91-5.96 (1H, m), 6.08 (1H, dd, J = 16.7, 2.4 Hz), 6.77 (1H, dd, J = 16.7, 10.6 Hz), 6.93-6.99 (1H, m), 6.99-7.06 (1H, m), 7.20 (1H, s), 7.47 (1H, t, J = 9.1 Hz), 7.66 (1H, s), 7.89 (1H, s), 8.32 (1H, s), 9.17 (1H, s), 12.26 (1H, br s). MS m/z: 505 $[M+H]^+$. | Reference Example D1 |
| Q3 | Ethyl 2-{3-fluoro-4-[(7-methoxy-6-{[1-(prop-2-enoyl)piperidin-4-yl]oxy}quinazolin-4-yl) amino]phenoxy}-1,3-thiazol-4-carboxylate 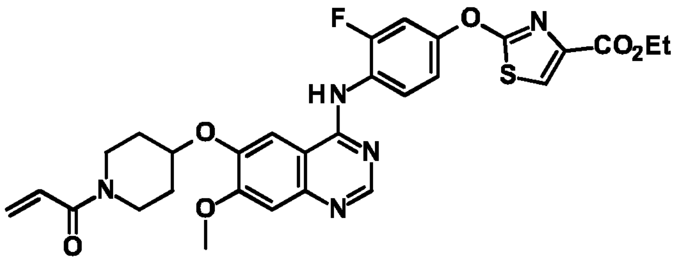 $^1$H-NMR ($CDCl_3$) δ: 1.39 (3H, t, J = 7.2 Hz), 1.91-2.09 (4H, m), 3.51-3.62 (1H, m), 3.68-3.78 (1H, m), 3.83-3.98 (2H, m), 4.02 (3H, s), 4.39 (2H, q, J = 7.2 Hz), 4.69-4.77 (1H, m), 5.72 (1H, dd, J = 10.4, 1.8 Hz), 6.31 (1H, dd, J = 16.6, 1.8 Hz), 6.63 (1H, dd, J = 16.6, 10.4 Hz), 7.17-7.29 (4H, m), 7.32 (1H, s), 7.76 (1H, s), 8.59 (1H, t, J = 9.2 Hz), 8.70 (1H, s). MS m/z: 594 $[M+H]^+$. | Reference Example C11 |

[Table 24-2]

| Reference Example | Compound | Intermediate |
|---|---|---|
| Q4 | 1-{4-[(4-{4-[(4-Bromo-1,3-thiazol-2-yl)oxy]-2-fluoroanilino}-7-methoxyquinazolin-6-yl)oxy]piperidin-1-yl}prop-2-en-1-one 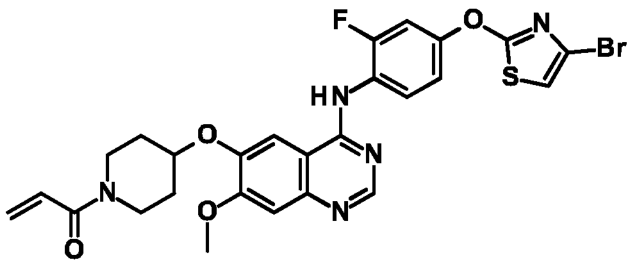 $^1$H-NMR (DMSO-$D_6$) δ: 1.61-1.77 (2H, m), 1.98-2.12 (2H, m), 3.37-3.57 (2H, m), 3.83-3.99 (2H, m), 3.94 (3H, s), 4.72-4.83 (1H, m), 5.69 (1H, dd, J = 10.4, 2.5 Hz), 6.12 (1H, dd, J = 16.9, 2.5 Hz), 6.86 (1H, dd, J = 16.9, 10.4 Hz), 7.23 (1H, s), 7.30-7.35 (1H, m), 7.40 (1H, s), 7.57 (1H, dd, J = 10.4, 2.5 Hz), 7.66 (1H, t, J = 8.6 Hz), 7.89 (1H, s), 8.39 (1H, s), 9.52 (1H, s). MS m/z: 600 [M($^{79}$Br)+H]$^+$, 602 [M($^{81}$Br)+H]$^+$. | Reference Example C12 |

[Reference Example Q5] tert-Butyl 4-[(7-ethoxy-4-{2-fluoro-4-[3-(5-methyl-1,3,4-oxadiazol-2-yl)phenoxy]anilino}quinazolin-6-yl)oxy]piperidine-1-carboxylate

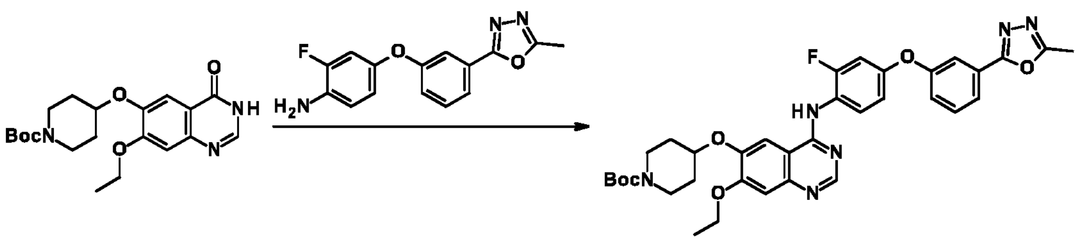

To a suspension of tert-butyl 4-[(7-ethoxy-4-oxo-3,4-dihydroquinazolin-6-yl)oxy]piperidine-1-carboxylate (145 mg, 0.372 mmol) in acetonitrile (5 mL), 1H-benzotriazol-1-yloxy-tris(dimethylamino)phosphonium hexafluorophosphate (214 mg, 0.484 mmol) and 1,8-diazabicyclo[5,4,0]-7-undecene (0.083 mL, 0.56 mmol) were added, and the mixture was stirred at room temperature for 1 hour. The reaction solution was concentrated under reduced pressure. To a suspension of the obtained residue in 2-propanol (5 mL), 2-fluoro-4-[3-(5-methyl-1,3,4-oxadiazol-2-yl)phenoxy]aniline (106 mg, 0.372 mmol) and trifluoroacetic acid (0.128 mL, 1.68 mmol) were added, and the mixture was stirred at room temperature for 16 hours. The reaction solution was concentrated under reduced pressure. To the obtained residue, saturated aqueous sodium bicarbonate solution was added, and the mixture was extracted with ethyl acetate. The organic layer was washed with saturated saline and then dried over anhydrous sodium sulfate. The insoluble materials were filtered off and then the filtrate was concentrated under reduced pressure and purified by silica gel column chromatography (dichloromethane/methanol) to obtain the title compound (240 mg, 0.365 mmol, yield 98.2%).
$^1$H-NMR ($CDCl_3$) δ: 1.48 (9H, s), 1.54 (3H, t, J = 7.0 Hz), 1.85-1.88 (2H, m), 1.95-1.98 (2H, m), 2.62 (3H, s), 3.34-3.40 (2H, m), 3.74-3.78 (2H, m), 4.23 (2H, q, J = 7.0 Hz), 4.62-4.63 (1H, m), 6.89-6.94 (2H, m), 7.19-7.95 (5H, m), 7.51 (1H, J = 7.9 Hz), 7.81 (1H, d, J = 7.9 Hz), 8.28 (1H, t, J = 8.8 Hz), 8.63 (1H, s). MS m/z: 657 [M+H]$^+$.

By a similar method, the following compounds were synthesized from the corresponding intermediates.

[Table 25-1]

| Reference Example | Compound | Intermediate |
|---|---|---|
| Q6 | tert-Butyl 4-{[7-(fluoromethoxy)-4-{2-fluoro-4-[3-(5-methyl-1,3,4-oxadiazol-2-yl) phenoxy]anilino}quinazolin-6-yl]oxy}piperidine-1-carboxylate<br>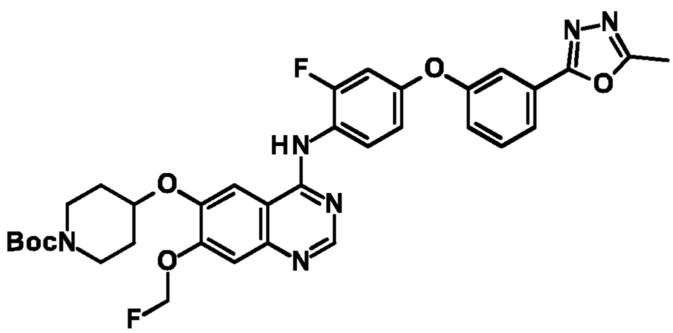<br>$^1$H-NMR ($CDCl_3$) $\delta$: 1.48 (9H, s), 1.89-1.90 (2H, m), 1.99-2.01 (2H, m), 2.62 (3H, s), 3.36-3.42 (2H, m), 3.76-3.78 (2H, m), 4.66-4.67 (1H, m), 5.91 (2H, d, J = 53.1 Hz), 6.92-6.95 (2H, m), 7.22-7.28 (3H, m), 7.51 (1H, t, J = 7.9 Hz), 7.62 (1H, s), 7.69 (1H, s), 7.81 (1H, d, J = 7.9 Hz), 8.41 (1H, t, J = 9.2 Hz), 8.70 (1H, s). MS m/z: 661 $[M+H]^+$. | Reference Example A10<br>Reference Example P1 |
| Q7 | tert-Butyl 4-{[7-(benzyloxy)-4-(4-{[1-(tert-butylcarbamoyl)-1H-pyrazol-3-yl]oxy}-2-fluoroanilino)quinazolin-6-yl]oxy}piperidine-1-carboxylate<br>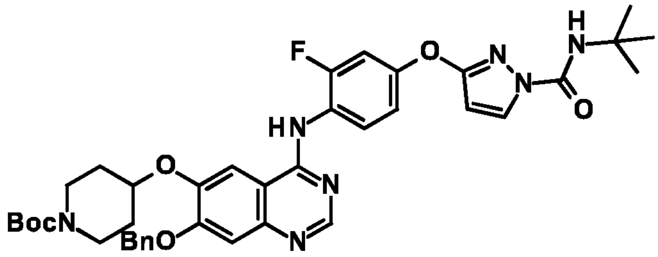<br>MS m/z: 726 $[M+H]^+$. | Reference Example A11<br>Reference Example N7 |

[Table 25-2]

| Reference Example | Compound | Intermediate |
|---|---|---|
| Q8 | tert-Butyl 4-[(4-{2-fluoro-4-[(1-{[2-($^2H_3$)methyl($^2H_6$)propan-2-yl]carbamoyl}-1H-pyrazol-3-yl)oxy]anilino}-7-[($^2H_3$)methyloxy]quinazolin-6-yl)oxy]piperidine-1-carboxylate<br>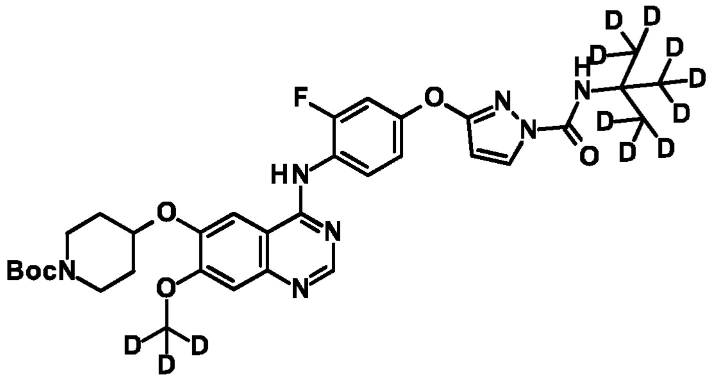<br>$^1$H-NMR($CDCl_3$) δ: 1.48 (9H, s), 1.86-1.90 (2H, m), 1.99-2.00 (2H, m), 3.30-3.37 (2H, m), 3.80-3.82 (2H, m), 4.59-4.63 (1H, m), 5.99 (1H, d, J = 3.1 Hz), 6.80 (1H, br s), 7.05-7.06 (2H, m), 7.15 (1H, br s), 7.20 (1H, s), 7.29 (1H, s), 8.13 (1H, d, J = 3.1 Hz), 8.44 (1H, t, J = 9.2 Hz), 8.68 (1H, s). MS m/z: 662 [M+H]+. | Reference Example A12<br>Reference Example N10 |

[Reference Example R1] 3-{3-Fluoro-4-[(7-methoxy-6-{[1-(prop-2-enoyl)piperidin-4-yl]oxy}quinazolin-4-yl)amino]phenoxy}benzoic acid

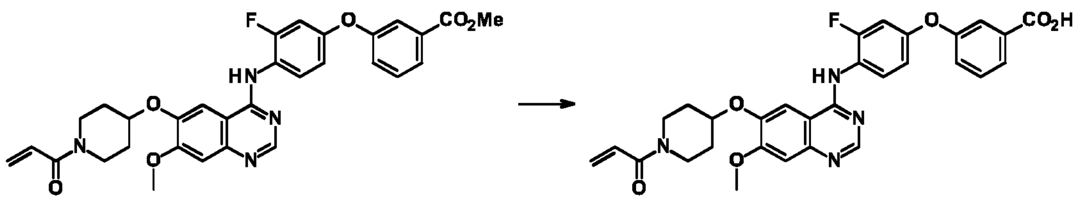

To a mixed solution of methyl 3-{3-fluoro-4-[(7-methoxy-6-{[1-(prop-2-enoyl)piperidin-4-yl]oxy}quinazolin-4-yl)amino]phenoxy}benzoate (3.36 g, 5.86 mmol) in 1,4-dioxane (10 mL) and tetrahydrofuran (20 mL), a 4 mol/L aqueous lithium hydroxide solution (3.0 mL) and water (7 mL) were added at room temperature, and the mixture was stirred at 50°C for 3 hours. To the reaction mixture, 1 mol/L hydrochloric acid (12 mL) was added at room temperature, and the mixture was concentrated under reduced pressure. Water was added to the obtained residue to form a slurry, and the solid was collected by filtration and washed with water, hexane and diethyl ether, and then dried under reduced pressure to obtain the title compound (3.21 g, 5.75 mmol, yield 98.2%).
$^1$H-NMR (DMSO-$D_6$) δ: 1.59-1.78 (2H, m), 1.98-2.11 (2H, m), 3.24-3.54 (2H, m), 3.84-3.99 (2H, m), 3.94 (3H, s), 4.72-4.82 (1H, m), 5.69 (1H, dd, J = 10.3, 2.4 Hz), 6.12 (1H, dd, J = 17.0, 2.4 Hz), 6.86 (1H, dd, J = 17.0, 10.3 Hz), 6.97 (1H, dd, J = 8.8, 2.1 Hz), 7.16 (1H, dd, J = 11.2, 2.7 Hz), 7.22 (1H, s), 7.41 (1H, dd, J = 8.8, 2.1 Hz), 7.48-7.64 (3H, m), 7.76 (1H, d, J = 7.9 Hz), 7.89 (1H, s), 8.36 (1H, s), 9.45 (1H, br, s), 13.15 (1H, br, s). MS m/z: 559 [M+H]$^+$.

By a similar method, the following compounds were synthesized from the corresponding intermediates.

[Table 26]

| Reference Example | Compound | Intermediate |
|---|---|---|
| R2 | 2-{3-Fluoro-4-[(7-methoxy-6-{[1-(prop-2-enoyl)piperidin-4-yl]oxy}quinazolin-4-yl)amino]phenoxy}-1,3-thiazole-4-carboxylic acid 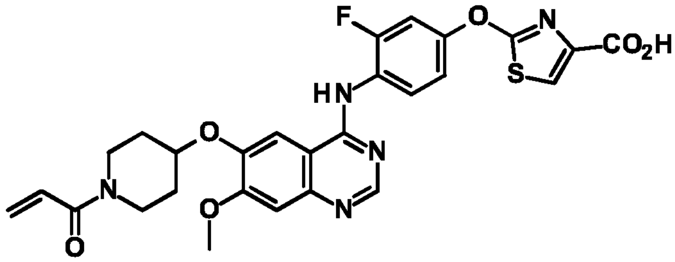 $^1$H-NMR (DMSO-$D_6$) δ: 1.60-1.77 (2H, m), 1.98-2.14 (2H, m), 3.40-3.55 (2H, m), 3.84-4.02 (2H, m), 3.96 (3H, s), 4.77-4.91 (1H, m), 5.69 (1H, dd, J = 10.4, 2.1 Hz), 6.12 (1H, dd, J = 16.6, 2.1 Hz), 6.86 (1H, dd, J = 16.6, 10.4 Hz), 7.25 (1H, s), 7.31-7.38 (1H, m), 7.52-7.70 (2H, m), 7.99-8.09 (2H, m), 8.52 (1H, s), 10.10 (1H, br s), 13.09 (1H, br s). MS m/z: 566 [M+H]$^+$. | Reference Example Q3 |

[Reference Example R3] N'-Acetyl-3-{3-fluoro-4-[(7-methoxy-6-{[1-(prop-2-enoyl)piperidin-4-yl]oxy}quinazolin-4-yl)amino]phenoxy}benzohydrazide

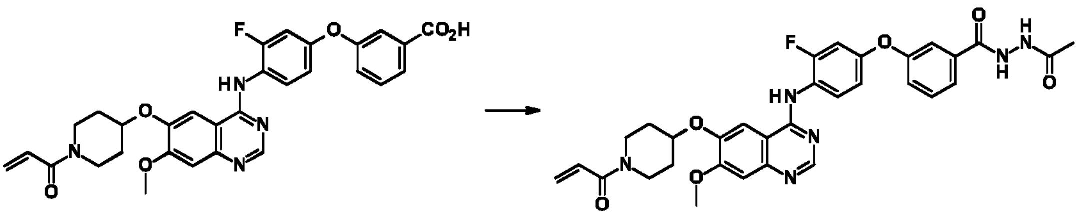

To a mixture of 3-{3-fluoro-4-[(7-methoxy-6-{[1-(prop-2-enoyl)piperidin-4-yl]oxy}quinazolin-4-yl)amino]phenoxy}benzoic acid (303 mg, 0.542 mmol), N,N-diisopropylethylamine (0.12 mL, 0.71 mmol) and acetohydrazide (55.2 mg, 0.745 mmol) in N,N-dimethylformamide (2.8 mL), 1-[bis(dimethylamino)methylene]-1H-1,2,3-triazolo[4,5-b]pyridinium 3-oxide hexafluorophosphate (267 mg, 0.703 mmol) was added, and the mixture was stirred at room temperature for 4 hours. To the reaction mixture, N,N-diisopropylethylamine (0.092 mL, 0.54 mmol), acetohydrazide (20 mg, 0.27 mmol), 1-[bis(dimethylamino)methylene]-1H-1,2,3-triazolo[4,5-b]pyridinium 3-oxide hexafluorophosphate (103 mg, 0.271 mmol) and N,N-dimethylformamide (0.5 mL) were added, and the mixture was stirred at room temperature for 3 hours. The reaction mixture was added dropwise to saturated aqueous sodium bicarbonate solution (40 mL) while stirring. The resulting solid was collected by filtration, and washed with water, hexane and diethyl ether, and then dried under reduced pressure to obtain the title compound (305 mg, 0.497 mmol, yield 91.7%).
$^1$H-NMR (DMSO-$D_6$) δ: 1.60-1.78 (2H, m), 1.91 (3H, s), 1.99-2.11 (2H, m), 3.19-3.56 (2H, m), 3.84-3.99 (2H, m), 3.94 (3H, s), 4.72-4.81 (1H, m), 5.69 (1H, dd, J = 10.6, 2.4 Hz), 6.12 (1H, dd, J = 16.7, 2.4 Hz), 6.86 (1H, dd, J = 16.7, 10.6 Hz), 6.91-6.97 (1H, m), 7.13 (1H, dd, J = 11.2, 2.7 Hz), 7.21 (1H, s), 7.35 (1H, dd, J = 7.6, 2.1 Hz), 7.49-7.62 (3H, m), 7.70 (1H, d, J = 7.9 Hz), 7.89 (1H, s), 8.36 (1H, s), 9.44 (1H, s), 9.91 (1H, s), 10.38 (1H, s).

[Reference Example S1] tert-Butyl 4-{[4-(4-{[1-(tert-butylcarbamoyl)-1H-pyrazol-3-yl]oxy}-2-fluoroanilino)-7-hydroxyquinazolin-6-yl]oxy}piperidine-1-carboxylate

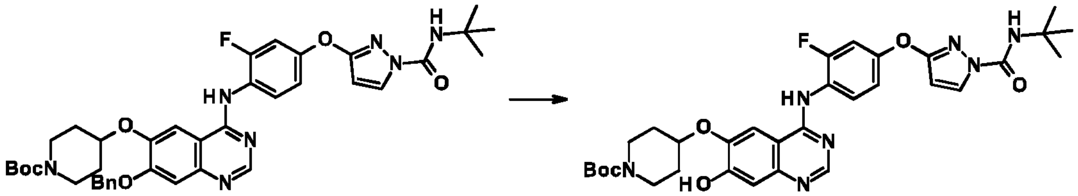

To a solution of tert-butyl 4-{[7-(benzyloxy)-4-(4-{[1-(tert-butylcarbamoyl)-1H-pyrazol-3-yl]oxy}-2-fluoroanilino)quinazolin-6-yl]oxy}piperidine-1-carboxylate (1.80 g, 2.50 mmol) in ethanol (50 mL), 20% palladium hydroxide on carbon (360 mg) was added, and the mixture was stirred at room temperature for 3.5 hours under a hydrogen atmosphere. The insoluble materials were filtered off, then the filtrate was concentrated under reduced pressure, and purified by silica gel column chromatography (dichloromethane/methanol) to obtain the title compound (1.48 g, 2.33 mmol, yield 93.2%). $^1$H-NMR ($CD_3OD$) δ: 1.45 (9H, s), 1.48 (9H, s), 1.72-1.90 (2H, m), 2.03-2.17 (2H, m), 3.21-3.40 (2H, m), 3.85-3.97 (2H, m), 4.73-4.86 (1H, m), 6.17 (1H, d, J = 3.1 Hz), 7.06-7.24 (2H, m), 7.13 (1H, s), 7.19 (1H, br s), 7.56 (1H, t, J = 8.6 Hz), 7.85 (1H, s), 7.90 (1H, s), 8.19 (1H, d, J = 3.1 Hz), 8.36 (1H, s). MS m/z: 636 [M+H]$^+$.

[Reference Example S2] tert-Butyl 4-({4-(4-{[1-(tert-butylcarbamoyl)-1H-pyrazol-3-yl]oxyl-2-fluoroanilino)-7-[($^2H_3$)methyloxy]quinazolin-6-yl}oxy)piperidine-1-carboxylate

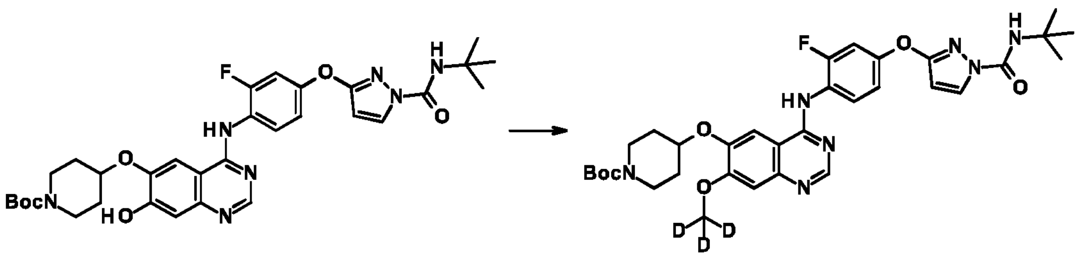

To a solution of tert-butyl 4-{[4-(4-{[1-(tert-butylcarbamoyl)-1H-pyrazol-3-yl]oxy}-2-fluoroanilino)-7-hydroxyquinazolin-6-yl]oxy}piperidine-1-carboxylate (64 mg, 0.10 mmol) in tetrahydrofuran (5 mL), methanol-$d_4$ (0.75 mL), triphenylphosphine (79 mg, 0.30 mmol) and bis(2-methoxyethyl) azodicarboxylate (70 mg, 0.30 mmol) were added, and the mixture was stirred at room temperature for 40 minutes. The reaction solution was concentrated and then purified by amino silica gel column chromatography (ethyl acetate/hexane) to obtain the title compound as a crude product.

[Reference Example T1] 7-Ethoxy-N-{2-fluoro-4-[3-(5-methyl-1,3,4-oxadiazol-2-yl)phenoxy]phenyl}-6-[(piperidin-4-yl)oxy]quinazolin-4-amine

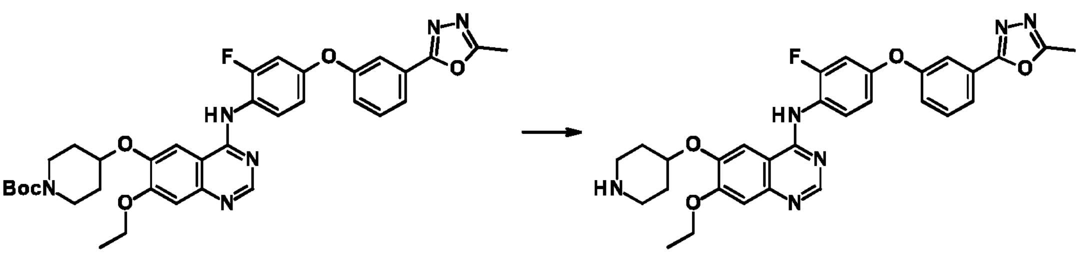

To a solution of tert-butyl 4-[(7-ethoxy-4-{2-fluoro-4-[3-(5-methyl-1,3,4-oxadiazol-2-yl)phenoxy]anilino}quinazolin-6-yl)oxy]piperidine-1-carboxylate (240 mg, 0.365 mmol) in dichloromethane (6 mL), trifluoroacetic acid (1.5 mL) was added, and the mixture was stirred at room temperature for 2 hours. The reaction solution was concentrated under reduced pressure. To the obtained residue, saturated aqueous sodium bicarbonate solution was added, and the mixture was extracted with ethyl acetate. The organic layer was washed with saturated saline and then dried over anhydrous sodium sulfate. The insoluble materials were filtered off and then the filtrate was concentrated under reduced pressure

to obtain the title compound as a crude product.

By a similar method, the following compounds were synthesized from the corresponding intermediates.

[Table 27-1]

| Reference Example | Compound | Intermediate |
|---|---|---|
| T2 | 7-(Fluoromethoxy)-N-{2-fluoro-4-[3-(5-methyl-1,3,4-oxadiazol-2-yl)phenoxy]phenyl}-6-[(piperidin-4-yl)oxy]quinazolin-4-amine 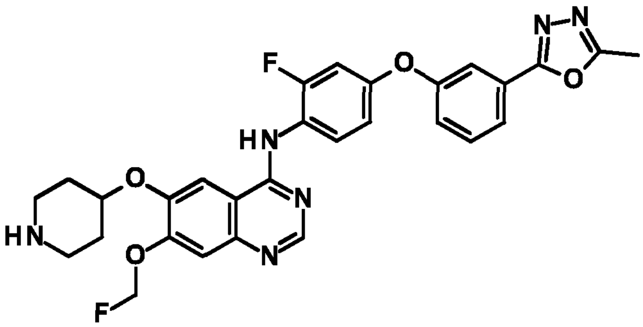 MS m/z: 561 [M+H]+. | Reference Example Q6 |
| T3 | N-tert-Butyl-3-[3-fluoro-4-({7-[($^2H_3$)methyloxy]-6-(piperidin-4-yloxy)quinazolin-4-yl} amino)phenoxy]-1 H-pyrazole-1-carboxamide 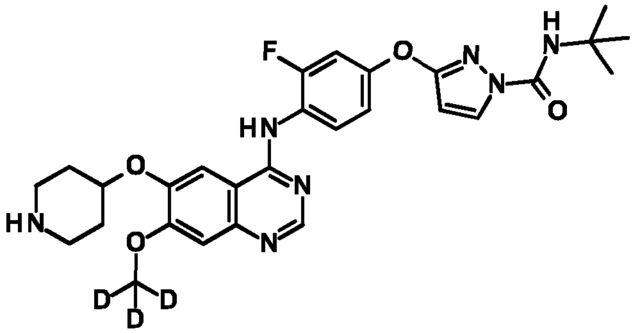 MS m/z: 553 [M+H]+. | Reference Example S2 |

[Table 27-2]

| Reference Example | Compound | Intermediate |
|---|---|---|
| T4 | 3-[3-Fluoro-4-({7-[($^2H_3$)methyloxy]-6-(piperidin-4-yloxy) quinazolin-4-yl}amino) phenoxy]-N-[2-($^2H_3$)methyl($^2H_6$)propan-2-yl]-1 H-pyrazole-1-carboxamide 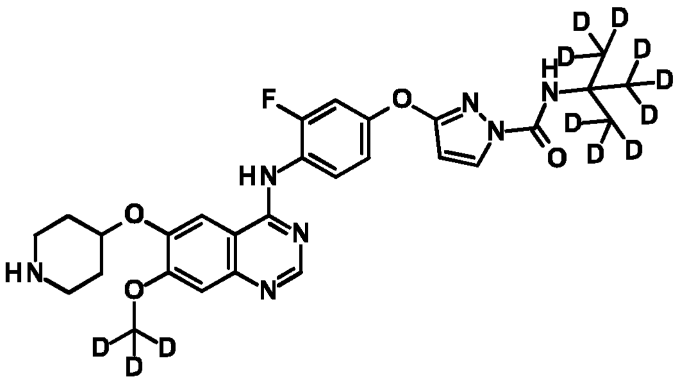 MS m/z: 562 [M+H]+. | Reference Example Q8 |

[Reference Example U1] Methyl (3-{3-Fluoro-4-[(7-methoxy-6-nitroquinazolin-4-yl)amino]phenoxy}phenyl)acetate hydrochloride

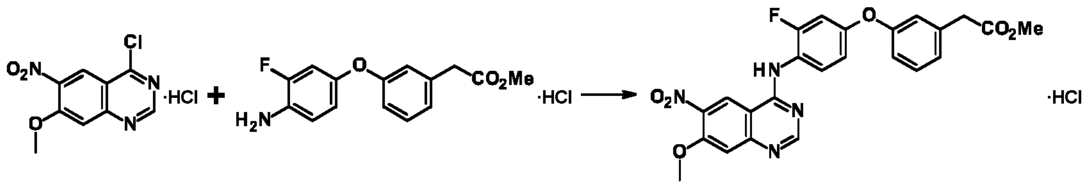

A suspension of methyl [3-(4-amino-3-fluorophenoxy)phenyl]acetate hydrochloride (1.68 g, 5.39 mmol) and 4-chloro-7-methoxy-6-nitroquinazoline hydrochloride (1.66 g, 6.00 mmol) in 2-propanol (30 mL) was stirred at 50°C for 1.5 hours. To the reaction solution, 4-chloro-7-methoxy-6-nitroquinazoline hydrochloride (188 mg, 0.680 mmol) was added, and the mixture was stirred at 50°C for 30 minutes. The reaction solution was left to cool, and then concentrated under reduced pressure. To the residue, ethyl acetate was added, and the insoluble materials were collected by filtration. The obtained solid was dried under reduced pressure at 60°C to obtain the title compound (2.77 g, 5.38 mmol, yield 99.8%).
$^1$H-NMR (DMSO-$D_6$) δ: 3.63 (3H, s), 3.75 (2H, s), 4.10 (3H, s), 6.85-7.20 (5H, m), 7.32-7.59 (3H, m), 8.79 (1H, s), 9.37 (1H, s), 11.17 (1H, br s). MS m/z: 479 $[M+H]^+$.

[Reference Example U2] N-{2-Fluoro-4-[3-(5-methyl-1,3,4-oxadiazol-2-yl)phenoxy]phenyl}-7-methoxy-6-nitroquinazolin-4-amine

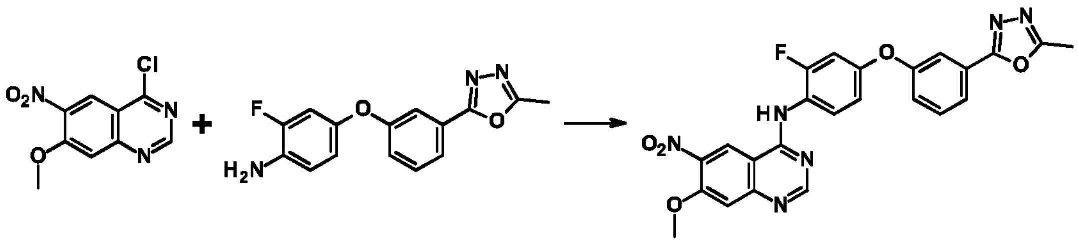

To a suspension of 2-fluoro-4-[3-(5-methyl-1,3,4-oxadiazol-2-yl)phenoxy]aniline (250 mg, 0.876 mmol), 4-chloro-7-methoxy-6-nitroquinazoline (J. Med. Chem. 1996, 39, 267-276) (210 mg, 0.876 mmol) in 2-propanol (10 mL), trifluoroacetic acid (201 μL, 2.63 mmol) was added, and the mixture was stirred at room temperature for 3 hours. The reaction solution was diluted with dichloromethane, and washed with aqueous potassium carbonate solution. The organic layer was dried over anhydrous sodium sulfate and then concentrated to obtain the title compound (428 mg, 0.876 mmol, quantitative) as roughly purified.
$^1$H-NMR (DMSO-$D_6$) δ: 2.58 (3H, s), 4.07 (3H, s), 7.01 (1H, d, J = 9.1 Hz), 7.20 (1H, d, J = 10.9 Hz), 7.39 (1H, d, J = 7.3 Hz), 7.49 (1H, s), 7.60-7.54 (2H, m), 7.67 (1H, t, J = 7.6 Hz), 7.80 (1H, d, J = 7.9 Hz), 8.56 (1H, s), 9.19 (1H, s), 10.18 (1H, s). MS m/z: 489 $[M+H]^+$.

By a similar method, the following compounds were synthesized from the corresponding intermediates.

[Table 28-1]

| Reference Example | Compound | Intermediate |
|---|---|---|
| U3 | N-{4-[3-(5-Ethyl-1,3,4-oxadiazol-2-yl)phenoxy]-2-fluorophenyl}-7-methoxy-6-nitroquinazolin-4-amine 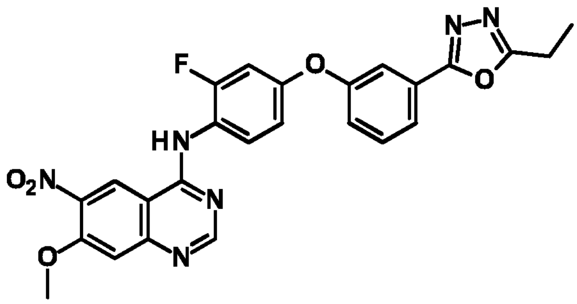 | Reference Example N6 |
| U4 | N-(2-Fluoro-4-{3-[5-(propan-2-yl)-1,3,4-oxadiazol-2-yl]phenoxy}phenyl)-7-methoxy-6-nitroquinazolin-4-amine 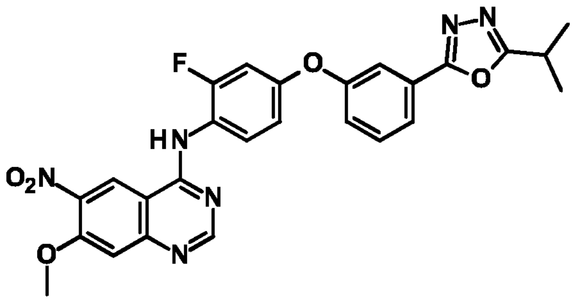 $^1$H-NMR ($CDCl_3$) δ: 1.46 (6H, d, J = 8.6 Hz), 3.19-3.36 (1H, m), 4.11 (3H, s), 6.86-6.99 (2H, m), 7.19-7.27 (1H, m), 7.44 (1H, s), 7.49-7.57 (1H, m), 7.61-7.91 (3H, m), 8.18-8.31 (1H, m), 8.55 (1H, s), 8.77 (1H, s). MS m/z: 517 $[M+H]^+$. | Reference Example P2 |

[Table 28-2]

| Reference Example | Compound | Intermediate |
|---|---|---|
| U5 | N-{2-Fluoro-4-[3-(5-methyl-1,3,4-thiadiazol-2-yl)phenoxy]phenyl}-7-methoxy-6-nitroquinazolin-4-amine 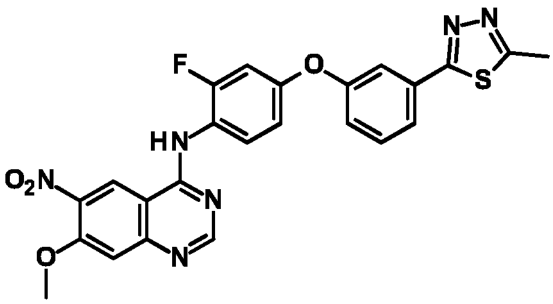 $^1$H-NMR ($CDCl_3$) δ: 2.83 (3H, s), 4.11 (3H, s), 6.86-7.01 (2H, m), 7.15-7.21 (1H, m), 7.41-7.54 (3H, m), 7.64-7.74 (2H, m), 8.25-8.35 (1H, m), 8.51 (1H, s), 8.77 (1H, s). MS m/z: 505 $[M+H]^+$. | Reference Example P4 |

(continued)

| Reference Example | Compound | Intermediate |
|---|---|---|
| U6 | Ethyl 2-{3-fluoro-4-[(7-methoxy-6-nitroquinazolin-4-yl)amino]phenoxy}-1,3-thiazole-4-carboxylate 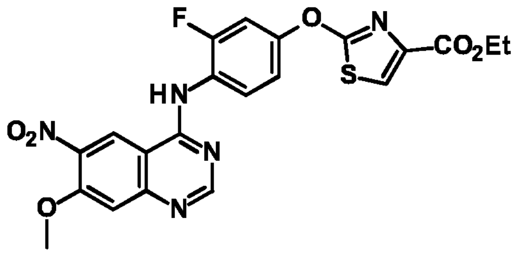 MS m/z: 486 [M+H]+. | Reference Example M4 |

[Table 28-3]

| Reference Example | Compound | Intermediate |
|---|---|---|
| U7 | N-tert-Butyl-2-(3-{3-chloro-4-[(7-methoxy-6-nitroquinazolin-4-yl)amino] phenoxy}-4-fluoro-1 H-pyrazol-1-yl)acetamide 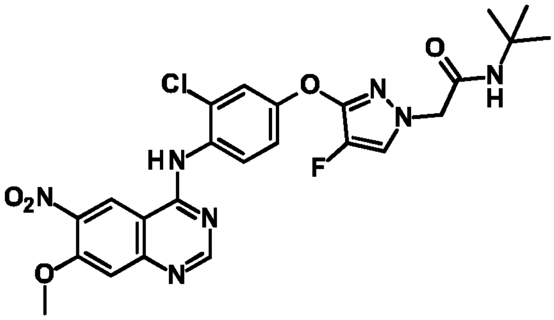 $^1$H-NMR ($CDCl_3$) δ: 1.33 (9H, s), 4.11 (3H, s), 4.51 (2H, s), 5.88 (1H, s), 7.13-7.20 (1H, m), 7.27-7.30 (1H, m), 7.39-7.52 (2H, m), 7.88-8.00 (1H, m), 8.36-8.58 (2H, m), 8.75 (1H, s). MS m/z: 544 [M+H]+. | Reference Example N4 |
| U8 | tert-Butyl (3-{3-chloro-4-[(7-methoxy-6-nitroquinazolin-4-yl)amino] phenoxy}-4-fluoro-1H-pyrazol-1-yl)acetate 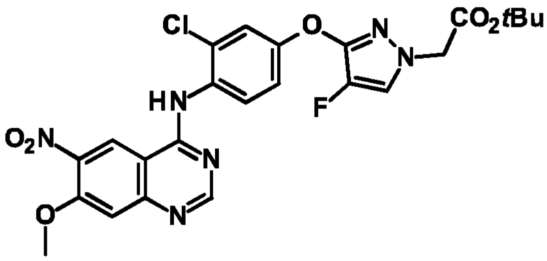 $^1$H-NMR ($CDCl_3$) δ: 1.49 (9H, s), 4.10 (3H, s), 4.63 (2H, s), 7.12-7.20 (1H, m), 7.28-7.32 (1H, m), 7.37-7.45 (2H, m), 7.79 (1H, s), 8.38-8.51 (2H, m), 8.76 (1H, s). MS m/z: 545 [M+H]+. | Reference Example N5 (carried out without addition of trifluoroacetic acid) |

[Table 28-4]

| Reference Example | Compound | Intermediate |
|---|---|---|
| U9 | N-tert-Butyl-2-(4-fluoro-3-{3-fluoro-4-[(7-methoxy-6-nitroquinazolin-4-yl)amino] phenoxy}-1 H-pyrazol-1-yl)acetamide 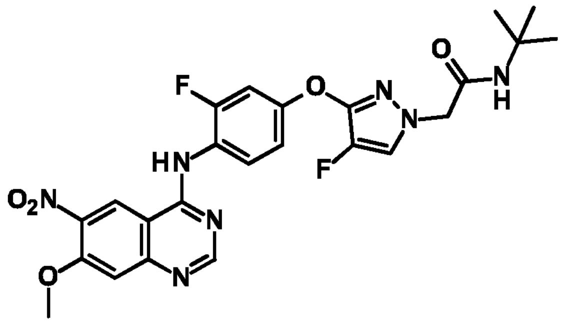 $^1$H-NMR ($CDCl_3$) δ: 1.33 (9H, s), 4.10 (3H, s), 4.51 (2H, s), 5.88 (1H, br s), 6.99-7.07 (2H, m), 7.39-7.45 (3H, m), 8.27-8.33 (1H, m), 8.49 (1H, s), 8.76 (1H, s). MS m/z: 528 $[M+H]^+$. | Reference Example N11 |

[Reference Example U10] N-(4-{[4-(5-Cyclopropyl-1,3,4-oxadiazol-2-yl)-1,3-thiazol-2-yl]oxyl-2-fluorophenyl)-7-methoxy-6-nitroquinazolin-4-amine hydrochloride

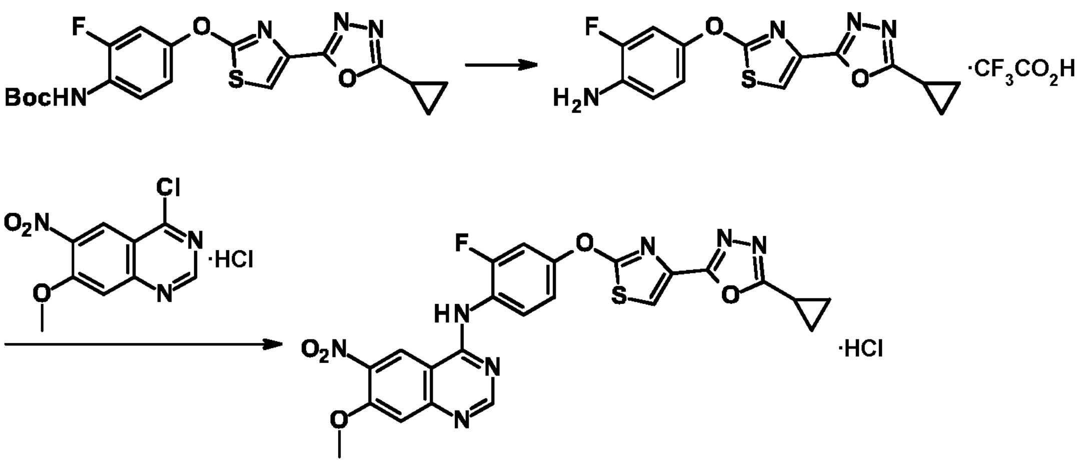

To a solution of tert-butyl (4-{[4-(5-cyclopropyl-1,3,4-oxadiazol-2-yl)-1,3-thiazol-2-yl]oxy}-2-fluorophenyl)carbamate (824 mg, 1.97 mmol) in dichloromethane (10 mL), trifluoroacetic acid (10 mL) was added, and the mixture was stirred at room temperature for 3 hours. The reaction solution was concentrated under reduced pressure, and the residue was dissolved in 2-propanol (20 mL). Then, 4-chloro-7-methoxy-6-nitroquinazoline hydrochloride (566 mg, 2.05 mmol) was added thereto, and the mixture was stirred at 50°C for 3 hours. The reaction solution was left to cool, and the insoluble materials were collected by filtration. The obtained solid was washed with 2-propanol and dried under reduced pressure at 60°C to obtain the title compound (1.06 g, 1.89 mmol, yield 96.0%).
$^1$H-NMR (DMSO-$D_6$) δ: 1.00-1.23 (4H, m), 2.23-2.36 (1H, m), 4.11 (3H, s), 7.38-7.46 (1H, m), 7.54 (1H, s), 7.63-7.74 (2H, m), 8.06-8.10 (1H, m), 8.79 (1H, s), 9.35 (1H, s). MS m/z: 522 $[M+H]^+$.

[Reference Example U11] tert-Butyl 3-{3-fluoro-4-[(7-methoxy-6-nitroquinazolin-4-yl)amino]phenoxy}-1H-pyrazole-1-carboxylate

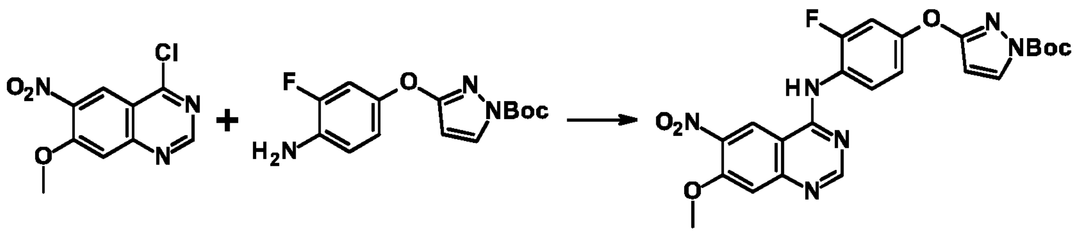

To a suspension of tert-butyl 3-(4-amino-3-fluorophenoxy)-1H-pyrazole-1-carboxylate (924 mg, 3.15 mmol) and 4-chloro-7-methoxy-6-nitroquinazoline (775 mg, 3.24 mmol) in 2-propanol (15 mL), pyridinium p-toluenesulfonate (1.20 g, 4.76 mmol) was added, and the mixture was stirred at room temperature for 4 hours. To the reaction solution, 4-chloro-7-methoxy-6-nitroquinazoline (142 mg, 0.591 mmol) was added, and the mixture was stirred at room temperature for 1 hour. The reaction solution was concentrated under reduced pressure. To the concentrate, dichloromethane and saturated aqueous sodium bicarbonate solution were added. The two layers were separated, and the aqueous layer was extracted with dichloromethane. The obtained organic layers were combined, washed with saturated saline, dried over anhydrous sodium sulfate and filtered, and then the filtrate was concentrated under reduced pressure. The residue was purified by silica gel column chromatography (ethyl acetate/hexane, using an amino silica gel column as a pre-column). The resultant was dried under reduced pressure at 60°C to obtain the title compound (1.12 g, 2.25 mmol, yield 71.6%).
$^1$H-NMR ($CDCl_3$) δ: 1.63 (9H, s), 4.09 (3H, s), 6.07 (1H, d, J = 3.0 Hz), 7.01-7.11 (2H, m), 7.41 (1H, s), 7.68-7.75 (1H, m), 8.03 (1H, d, J = 3.0 Hz), 8.20 (1H, t, J = 8.8 Hz), 8.56 (1H, s), 8.74 (1H, s). MS m/z: 497 $[M+H]^+$.

[Reference Example U12] 7-Fluoro-N-{2-fluoro-4-[3-(5-methyl-1,3,4-oxadiazol-2-yl)phenoxy]phenyl}-6-nitroquinazolin-4-amine

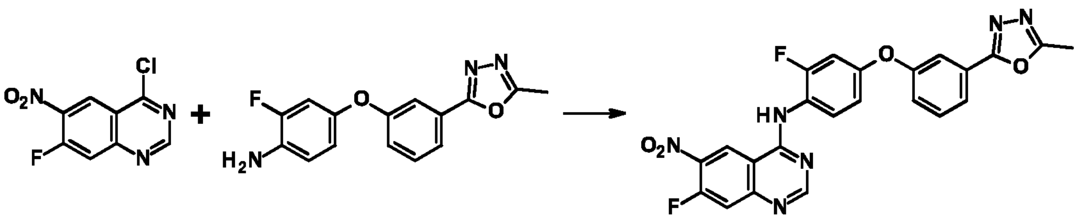

2-Fluoro-4-[3-(5-methyl-1,3,4-oxadiazol-2-yl)phenoxy]aniline (500 mg, 1.75 mmol) and 4-chloro-7-fluoro-6-nitroquinazoline (Bioorg. Med. Chem. 2009, 17, 3152-3161) (419 mg, 1.84 mmol) were dissolved in 2-propanol (15 mL), and trifluoroacetic acid (402 μL, 5.26 mmol) was added thereto. The mixture was stirred at room temperature for 3 hours, then diluted with dichloromethane, and washed with an aqueous potassium carbonate solution. The organic layer was concentrated, and the obtained solid was washed with a mixed solvent of ethyl acetate/hexane (1:1) to obtain the title compound (737 mg, 1.55 mmol, yield 88.3%).
$^1$H-NMR (DMSO-$D_6$) δ: 2.58 (3H, s), 7.01 (1H, dd, J = 9.2, 2.4 Hz), 7.21 (1H, dd, J = 11.6, 2.4 Hz), 7.38-7.41 (1H, m), 7.54-7.59 (2H, m), 7.67 (1H, t, J = 7.9 Hz), 7.79-7.85 (2H, m), 8.60 (1H, br s), 9.50-9.54 (1H, m), 10.58 (1H, br s). MS m/z: 477 $[M+H]^+$.
By a similar method, the following compounds were synthesized from the corresponding intermediates.

[Table 29]

| Reference Example | Compound | Intermediate |
|---|---|---|
| U13 | N-{4-[3-(5-Ethyl-1,3,4-oxadiazol-2-yl)phenoxy]-2-fluorophenyl}-7-fluoro-6-nitroquinazolin-4-amine 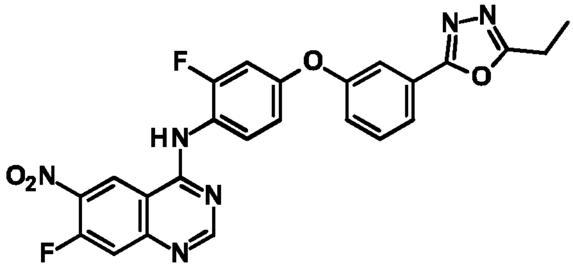 $^1$H-NMR (DMSO-$D_6$) δ: 1.32 (3H, t, J = 7.3 Hz), 2.95 (2H, q, J = 7.3 Hz), 7.03 (1H, dd, J = 8.2, 2.7 Hz), 7.23 (1H, dd, J = 11.3, 2.7 Hz), 7.38-7.43 (1H, m), 7.58 (1H, t, J = 8.5 Hz), 7.61-7.64 (1H, m), 7.68 (1H, t, J = 7.9 Hz), 7.80-7.85 (1H, m), 7.88 (1H, d, J = 12.2 Hz), 8.68 (1H, s), 9.59 (1H, d, J = 7.9 Hz), 10.78 (1H, br s). | Reference Example N6 |
| U14 | N-{4-[3-(5-Cyclopropyl-1,3,4-oxadiazol-2-yl)phenoxy]-2-fluorophenyl}-7-fluoro-6-nitroquinazolin-4-amine 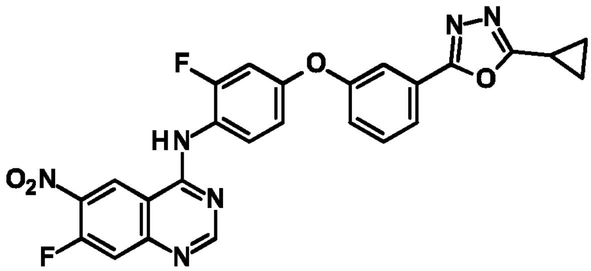 $^1$H-NMR (DMSO-$D_6$) δ: 1.10-1.21 (4H, m), 2.28-2.35 (1H, m), 7.00-7.03 (1H, m), 7.19-7.23 (1H, m), 7.37-7.40 (1H, m), 7.57 (1H, t, J = 8.5 Hz), 7.63-7.69 (2H, m), 7.79-7.89 (2H, m), 8.34 (1H, s), 8.67 (1H, s), 9.58 (1H, d, J = 7.9 Hz). MS m/z: 503 $[M+H]^+$. | Reference Example P3 |

[Reference Example U15] 2-(2-{4-[(6-Amino-7-methoxyquinazolin-4-yl)amino]-3-fluorophenoxy}-1,3-thiazol-4-yl)-N-tert-butylacetamide

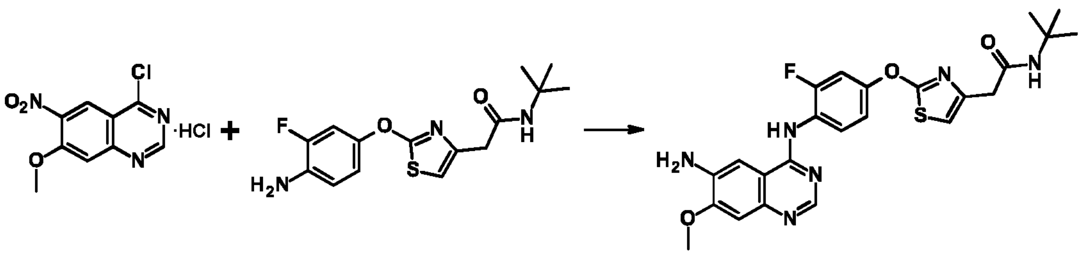

A suspension of 2-[2-(4-amino-3-fluorophenoxy)-1,3-thiazol-4-yl]-N-tert-butyl acetamide (249 mg, 0.771 mmol) and 4-chloro-7-methoxy-6-nitroquinazoline hydrochloride (254 mg, 0.924 mmol) in 2-propanol (11 mL) was stirred at room temperature overnight. The reaction solution was concentrated under reduced pressure. To the residue, diethyl ether was added. The insoluble materials were collected by filtration and dried under reduced pressure at 60°C. To a solution of the obtained solid (430 mg) in ethanol (4 mL), water (4 mL) and tetrahydrofuran (8 mL), iron powder (251 mg, 4.49 mmol) was added, and the mixture was stirred at 90°C for 2 hours. The reaction mixture was cooled to room temperature and triethylamine (0.32 mL, 2.3 mmol) was added. The reaction mixture was sequentially filtered with a

pad of amino silica gel and a pad of Celite, and eluted with ethanol. The eluted solution was concentrated under reduced pressure and the obtained residue was purified by amino silica gel column chromatography (chloroform) to obtain the title compound (355 mg, 0.714 mmol, yield 92.6%).
$^1$H-NMR ($CDCl_3$) δ: 1.30 (9H, s), 3.44 (2H, s), 4.04 (3H, s), 4.33 (2H, br s), 6.43 (1H, br s), 6.61 (1H, s), 6.95 (1H, s), 7.11-7.36 (4H, m), 8.62 (1H, s), 8.75 (1H, t, J = 8.9 Hz). MS m/z: 497 $[M+H]^+$.

[Reference Example V1] (3-{3-Fluoro-4-[(7-methoxy-6-nitroquinazolin-4-yl)amino]phenoxy}phenyl)acetic acid

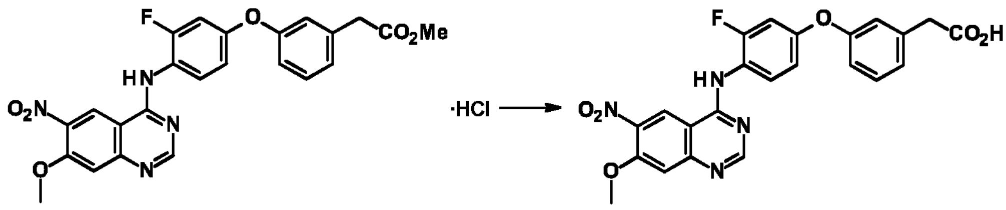

To a suspension of methyl (3-{3-fluoro-4-[(7-methoxy-6-nitroquinazolin-4-yl)amino]phenoxy}phenyl)acetate hydrochloride (2.77 g, 5.38 mmol) in tetrahydrofuran (20 mL) and methanol (20 mL), 1 mol/L aqueous sodium hydroxide solution (20 mL, 20 mmol) was added, and the mixture was stirred at room temperature for 1 hour. To the reaction solution, 1 mol/L hydrochloric acid (16 mL, 16 mmol) was added, and the precipitated solid was collected by filtration. The obtained solid was washed with water and then dried under reduced pressure at 60°C to obtain the title compound (2.47 g, 5.32 mmol, yield 98.9%).
$^1$H-NMR (DMSO-$D_6$) δ: 3.63 (2H, s), 4.07 (3H, s), 6.81-7.18 (5H, m), 7.31-7.60 (3H, m), 8.55 (1H, s), 9.19 (1H, s), 10.15 (1H, s), 12.38 (1H, br s). MS m/z: 465 $[M+H]^+$.

[Reference Example V2] N-tert-Butyl-2-(3-{3-fluoro-4-(7-methoxy-6-nitroquinazolin-4-yl)amino]phenoxy}phenyl)acetamide

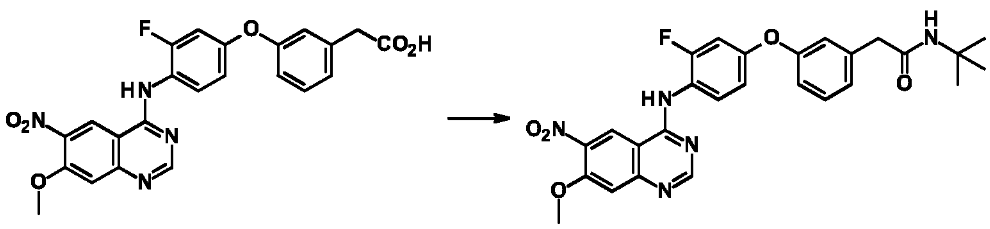

To a solution of (3-{3-fluoro-4-[(7-methoxy-6-nitroquinazolin-4-yl)amino]phenoxy}phenyl)acetic acid (595 mg, 1.28 mmol), tert-butylamine (0.162 mL, 1.54 mmol) and N,N-diisopropylethylamine (0.658 mL, 3.84 mmol) in N,N-dimethylformamide (6 mL), 1-[bis(dimethylamino)methylene]-1H-1,2,3-triazolo[4,5-b] pyridinium 3-oxide hexafluorophosphate (728 mg, 1.91 mmol) was added, and the mixture was stirred at room temperature for 1.5 hours. The reaction solution was diluted with ethyl acetate, and washed with saturated aqueous sodium bicarbonate solution, water, and saturated saline sequentially. The organic layer was dried over anhydrous sodium sulfate, filtered, and then concentrated under reduced pressure. The residue was purified by silica gel column chromatography (dichloromethane/ethyl acetate) to obtain the title compound (548 mg, 1.01 mmol, yield 82.4%).
$^1$H-NMR ($CDCl_3$) δ: 1.30 (9H, s), 3.46 (2H, s), 4.09 (3H, s), 5.29 (1H, s), 6.76-7.09 (5H, m), 7.28-7.36 (1H, m), 7.41 (1H, s), 7.98-8.13 (1H, m), 8.58 (1H, s), 8.71 (1H, s). MS m/z: 520 $[M+H]^+$.

[Reference Example V3] N-tert-Butyl-2-{3-fluoro-4-[(7-methoxy-6-nitroquinazolin-4-yl)amino]phenoxy}-1,3-thiazole-4-carboxamide

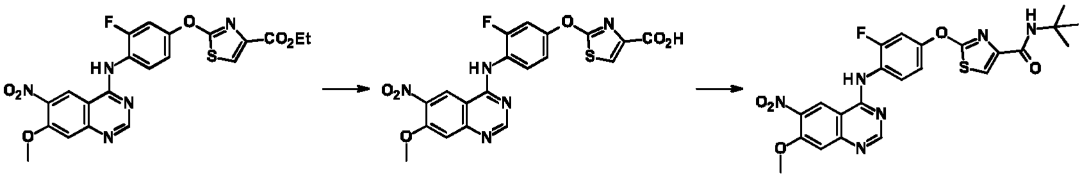

To a suspension of ethyl 2-{3-fluoro-4-[(7-methoxy-6-nitroquinazolin-4-yl)amino]phenoxy}-1,3-thiazole-4-carboxylate (667 mg, 1.37 mmol) in ethanol (16 mL), 1 mol/L aqueous sodium hydroxide solution (4.0 mL, 4.0 mmol) was added, and the mixture was stirred at 60°C for 4 hours. After the reaction solution was left to cool, 1 mol/L hydrochloric acid (6.0 mL, 6.0 mmol) was added thereto, and the precipitated solid was collected by filtration to obtain 2-{3-fluoro-4-[(7-methoxy-6-nitroquinazolin-4-yl)amino]phenoxy}-1,3-thiazole-4-carboxylic acid as a crude product. The crude product was used in the next step without further purification. To a solution of 2-{3-fluoro-4-[(7-methoxy-6-nitroquinazolin-4-yl)amino]phenoxyl-1,3-thiazole-4-carboxylic acid in N,N-dimethylformamide (5 mL), tert-butylamine (0.289 mL, 2.75 mmol), N,N-diisopropylethylamine (0.470 mL, 2.75 mmol) and 4-(4,6-dimethoxy-1,3,5-triazin-2-yl)-4-methylmorpholinium chloride (607 mg, 2.06 mmol) were added, and the mixture was stirred at room temperature for 2.5 hours. To the reaction solution, saturated aqueous sodium bicarbonate solution was added, and the mixture was extracted with ethyl acetate. The organic layer was washed with saturated saline and then dried over anhydrous sodium sulfate. The insoluble materials were filtered off, and then the filtrate was concentrated under reduced pressure and purified by silica gel column chromatography (ethyl acetate/hexane) to obtain the title compound (423 mg, 0.825 mmol, yield 60.1%).
$^1$H-NMR ($CDCl_3$) $\delta$: 1.45 (9H, s), 4.11 (3H, s), 6.91 (1H, br s), 7.21-7.23 (2H, m), 7.46 (1H, s), 7.57 (1H, br s), 7.64 (1H, s), 8.50 (1H, s), 8.55 (1H, t, J = 8.9 Hz), 8.81 (1H, s). MS m/z: 513 [M+H]$^+$.

[Reference Example W1] 7-Ethoxy-N-{2-fluoro-4-[3-(5-methyl-1,3,4-oxadiazol-2-yl)phenoxy]phenyl}-6-nitroquinazolin-4-amine

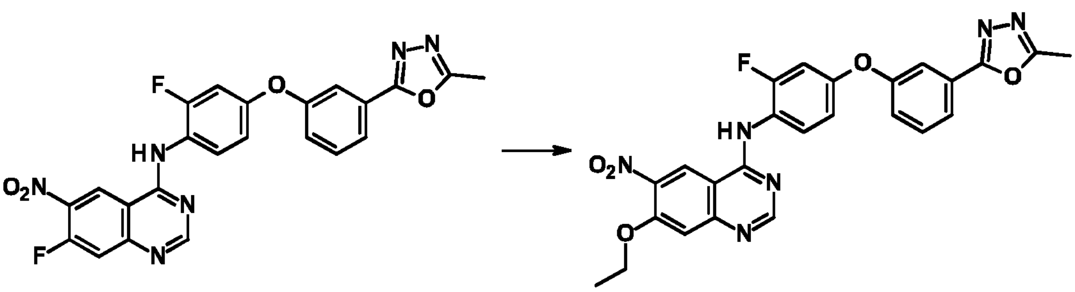

Sodium hydride (63% in oil, 256 mg, 6.19 mmol) was washed with hexane, and suspended in N,N-dimethylformamide (5 mL). To the suspension, ethanol (271 $\mu$L, 4.64 mmol) was added at 0°C. After 5 minutes, a solution of 7-fluoro-N-{2-fluoro-4-[3-(5-methyl-1,3,4-oxadiazol-2-yl)phenoxy]phenyl}-6-nitroquinazolin-4-amine (737 mg, 1.55 mmol) in N,N-dimethylformamide (5 mL) was added. The mixture was stirred at room temperature for 2 hours, then diluted with ethyl acetate, and washed with water and saline. The organic layer was dried over anhydrous sodium sulfate, and concentrated, and then purified by silica gel column chromatography (dichloromethane/methanol) to obtain the title compound (371 mg, 0.738 mmol, yield 47.7%).
$^1$H-NMR ($CDCl_3$) $\delta$: 1.56 (3H, t, J = 6.9 Hz), 2.62 (3H, s), 4.33 (2H, q, J = 6.9 Hz), 6.91-6.95 (2H, m), 7.24 (1H, dd, J = 8.5, 2.4 Hz), 7.40 (1H, s), 7.52 (1H, t, J = 7.9 Hz), 7.64 (1H, br s), 7.71 (1H, s), 7.83 (1H, d, J = 7.9 Hz), 8.28 (1H, q, J = 9.8 Hz), 8.51 (1H, d, J = 2.4 Hz), 8.76 (1H, s). MS m/z: 503 [M+H]$^+$.

By a similar method, the following compounds were synthesized from the corresponding intermediates.

[Table 30]

| Reference Example | Compound | Intermediate |
|---|---|---|
| W2 | 7-Ethoxy-N-{4-[3-(5-Ethyl-1,3,4-oxadiazol-2-yl)phenoxy]-2-fluorophenyl}-6-nitroquinazolin-4-amine 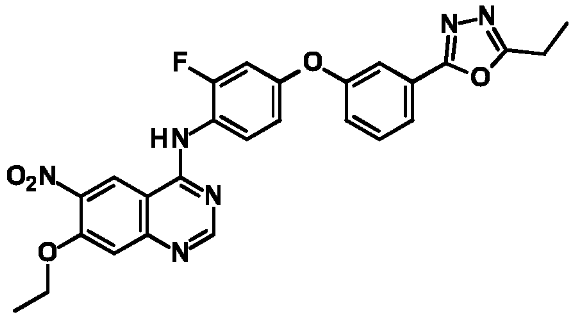 $^1$H-NMR (DMSO-$D_6$) δ: 1.32 (3H, t, J = 7.6 Hz), 1.41 (3H, t, J = 7.0 Hz), 2.95 (2H, q, J = 7.6 Hz), 4.36 (2H, q, J = 7.0 Hz), 7.00 (1H, dd, J = 8.5, 2.4 Hz), 7.20 (1H, dd, J = 11.3, 2.4 Hz), 7.38-7.40 (1H, m), 7.47 (1H, s), 7.57 (1H, t, J = 8.9 Hz), 7.60-7.63 (1H, m), 7.67 (1H, t, J = 7.9 Hz), 7.81 (1H, d, J = 7.9 Hz), 8.55 (1H, s), 9.17 (1H, s), 10.16 (1H, s). MS m/z: 517 [M+H]$^+$. | Reference Example U13 |
| W3 | N-{4-[3-(5-Cyclopropyl-1,3,4-oxadiazol-2-yl)phenoxy]-2-fluorophenyl}-7-ethoxy-6-nitroquinazolin-4-amine 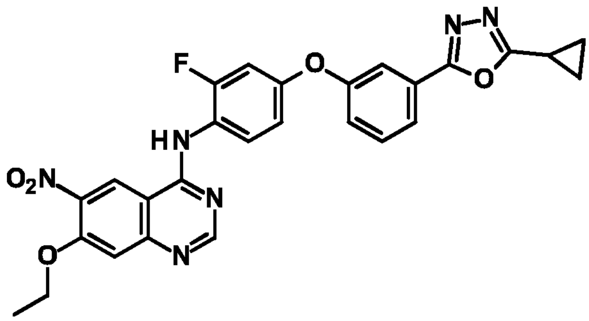 $^1$H-NMR (DMSO-$D_6$) δ: 1.09-1.21 (4H, m), 1.41 (3H, t, J = 7.1 Hz), 2.28-2.35 (1H, m), 4.36 (2H, q, J = 7.1 Hz), 6.96-7.01 (1H, m), 7.18 (1H, dd, J = 11.3, 2.7 Hz), 7.35-7.39 (1H, m), 7.47 (1H, s), 7.57 (1H, t, J = 8.9 Hz), 7.60-7.63 (1H, m), 7.66 (1H, t, J = 7.9 Hz), 7.77-7.81 (1H, m), 8.55 (1H, s), 9.17 (1H, s), 10.16 (1H, s). MS m/z: 529 [M+H]$^+$. | Reference Example U14 |

[Reference Example X1] 2-(3-{4-[(6-Amino-7-methoxyquinazolin-4-yl)amino]-3-fluorophenoxy}phenyl)-N-tert-butylacetamide

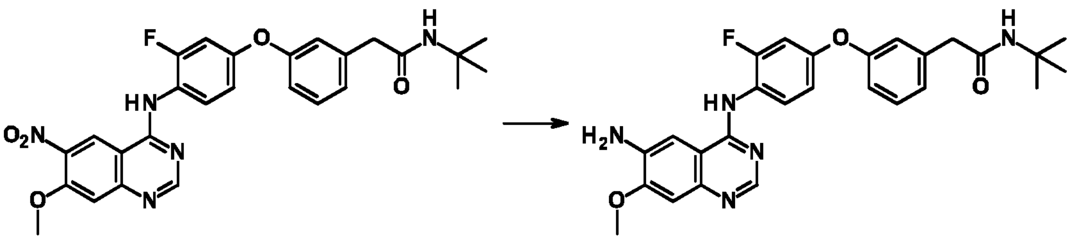

A suspension of N-tert-butyl-2-(3-{3-fluoro-4-[(7-methoxy-6-nitroquinazolin-4-yl)amino]phenoxy}phenyl)acetamide (548 mg, 1.06 mmol), iron powder (231 mg, 4.14 mmol) and ammonium chloride (59.4 mg, 1.11 mmol) in ethanol

(8 mL) and water (2 mL) was stirred at 65°C for 2.5 hours. The reaction solution was left to cool, and then filtered with Celite. The insoluble materials were washed with methanol. The washing solution combined with the filtrate was concentrated under reduced pressure. The residue was purified by silica gel column chromatography (ethyl acetate/methanol) and dried under reduced pressure at 60°C to obtain the title compound (387 mg, 0.791 mmol, yield 75.0%).
$^1$H-NMR ($CDCl_3$) δ: 1.31 (9H, s), 3.46 (2H, s), 4.03 (3H, s), 4.31 (2H, br s), 5.22 (1H, br s), 6.82-7.05 (6H, m), 7.13 (1H, br s), 7.20 (1H, s), 7.33 (1H, t, J = 8.0 Hz), 8.50 (1H, t, J = 8.9 Hz), 8.59 (1H, s). MS m/z: 490 [M+H]$^+$.

By a similar method, the following compounds were synthesized from the corresponding intermediates.

[Table 31-1]

| Reference Example | Compound | Intermediate |
|---|---|---|
| X2 | N$^4$-{2-Fluoro-4-[3-(5-methyl-1,3,4-oxadiazol-2-yl)phenoxy]phenyl}-7-methoxyquinazoline-4,6-diamine 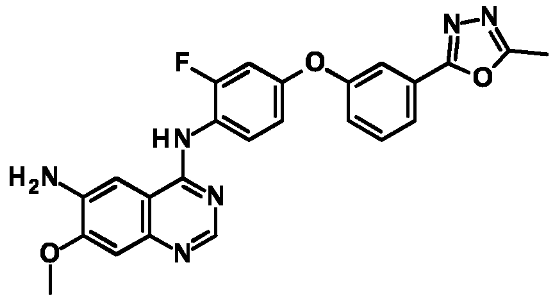 $^1$H-NMR (DMSO-$D_6$) δ: 2.58 (3H, s), 3.96 (3H, s), 5.38 (2H, br s), 6.98 (1H, dd, J = 8.9, 2.1 Hz), 7.08 (1H, s), 7.15 (1H, dd, J = 11.3, 2.7 Hz), 7.32 (1H, s), 7.37 (1H, dd, J = 8.5, 2.4 Hz), 7.55-7.61 (2H, m), 7.66 (1H, t, J = 7.9 Hz), 7.79-7.76 (1H, m), 8.23 (1H, s), 9.17 (1H, s). MS m/z: 459 [M+H]$^+$. | Reference Example U2 |
| X3 | 7-Ethoxy-N$^4$-{2-fluoro-4-[3-(5-methyl-1,3,4-oxadiazol-2-yl)phenoxy]phenyl} quinazoline-4,6-diamine 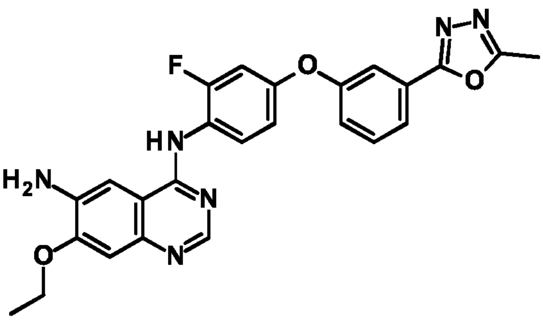 $^1$H-NMR (DMSO-$D_6$) δ: 1.45 (3H, t, J = 6.9 Hz), 2.58 (3H, s), 4.21 (2H, q, J = 6.9 Hz), 5.33 (2H, br s), 6.98 (1H, dd, J = 8.5, 2.4 Hz), 7.05 (1H, s), 7.15 (1H, dd, J = 11.3, 2.7 Hz), 7.32 (1H, s), 7.37 (1H, dd, J = 7.3, 2.4 Hz), 7.54-7.61 (2H, m), 7.66 (1H, t, J = 7.9 Hz), 7.79-7.76 (1H, m), 8.22 (1H, s), 9.15 (1H, s). MS m/z: 473 [M+H]$^+$. | Reference Example W1 |

[Table 31-2]

| Reference Example | Compound | Intermediate |
|---|---|---|
| X4 | $N^4$-{4-[3-(5-Ethyl-1,3,4-oxadiazol-2-yl)phenoxy]-2-fluorophenyl}-7-methoxyquinazoline-4,6-diamine 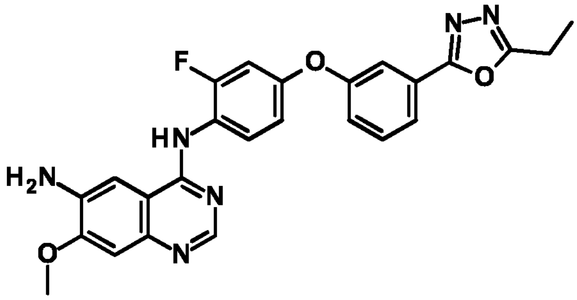 $^1$H-NMR (DMSO-$D_6$) δ: 1.32 (3H, t, J = 7.5 Hz), 2.95 (2H, q, J = 7.5 Hz), 3.96 (3H, s), 5.37 (2H, br s), 6.97 (1H, dd, J = 8.5, 2.4 Hz), 7.08 (1H, s), 7.15 (1H, dd, J = 11.0, 3.1 Hz), 7.31 (1H, s), 7.36 (1H, dd, J = 7.6, 2.1 Hz), 7.60-7.56 (2H, m), 7.66 (1H, t, J = 7.9 Hz), 7.79 (1H, d, J = 7.3 Hz), 8.22 (1H, s), 9.16 (1H, s). MS m/z: 473 [M+H]+. | Reference Example U3 |
| X5 | 7-Ethoxy-$N^4$-{4-[3-(5-ethyl-1,3,4-oxadiazol-2-yl)phenoxy]-2-fluorophenyl} quinazoline-4,6-diamine 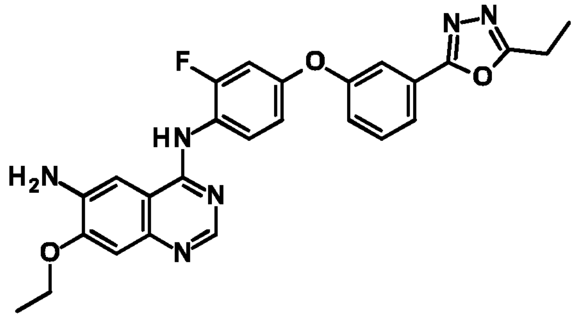 $^1$H-NMR (DMSO-$D_6$) δ: 1.32 (3H, t, J = 7.5 Hz), 1.45 (3H, t, J = 6.9 Hz), 2.94 (2H, q, J = 7.5 Hz), 4.21 (2H, q, J = 6.9 Hz), 5.33 (2H, br s), 6.97 (1H, dd, J = 8.5, 2.4 Hz), 7.05 (1H, s), 7.15 (1H, dd, J = 11.0, 2.4 Hz), 7.32 (1H, s), 7.36 (1H, dd, J = 8.2, 2.1 Hz), 7.60-7.56 (2H, m), 7.66 (1H, t, J = 7.9 Hz), 7.79 (1H, d, J = 7.9 Hz), 8.21 (1H, s), 9.15 (1H, s). MS m/z: 487 [M+H]+. | Reference Example W2 |

[Table 31-3]

| Reference Example | Compound | Intermediate |
|---|---|---|
| X6 | $N^4$-(2-Fluoro-4-{3-[5-(propan-2-yl)-1,3,4-oxadiazol-2-yl]phenoxy}phenyl)-7-methoxyquinazoline-4,6-diamine 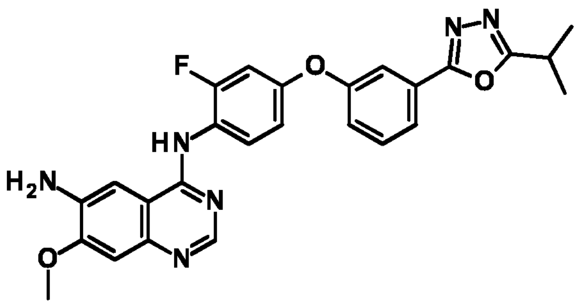 MS m/z:487 [M+H]+. | Reference Example U4 |

(continued)

| Reference Example | Compound | Intermediate |
|---|---|---|
| X7 | $N^4$-{2-Fluoro-4-[3-(5-methyl1,3,4-thiadiazol-2-yl)phenoxy]phenyl}-7-methoxyquinazolin-4,6-diamine 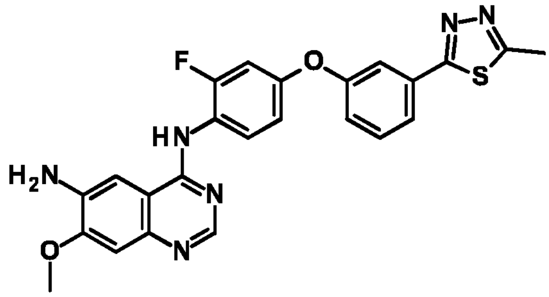 $^1$H-NMR ($CDCl_3$) δ: 2.82 (3H, s), 4.03 (3H, s), 4.32 (2H, br s), 6.88-6.97 (3H, m), 7.12-7.18 (2H, m), 7.21 (1H, s), 7.45 (1H, t, J = 7.9 Hz), 7.62-7.69 (2H, m), 8.52-8.62 (2H, m). MS m/z: 475 [M+H]+. | Reference Example U5 |

[Table 31-4]

| Reference Example | Compound | Intermediate |
|---|---|---|
| X8 | 2-(3-{4-[(6-Amino-7-methoxyquinazolin-4-yl)amino]-3-chlorophenoxy}-4-fluoro-1 H-pyrazol-1-yl)-N-tert-butylacetamide 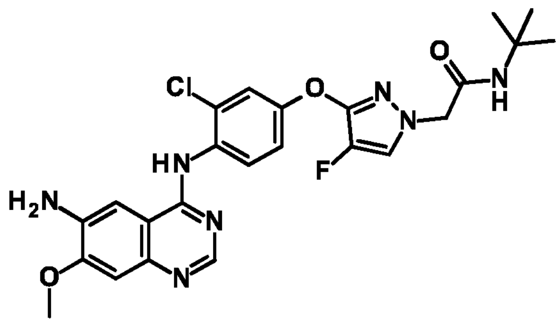 $^1$H-NMR ($CDCl_3$) δ: 1.33 (9H, s), 4.03 (3H, s), 4.35 (2H, brs), 4.49 (2H, s), 5.92 (1H, brs), 6.96 (1H, s), 7.11-7.19 (1H, m), 7.21-7.29 (2H, m), 7.37-7.42 (1H, m), 7.53-7.62 (1H, m), 8.60 (1H, s), 8.65-8.73 (1H, m). MS m/z: 514 $[M+H]^+$. | Reference Example U7 |
| X9 | tert-Butyl (3-{4-[(6-Amino-7-methoxyquinazolin-4-yl)amino]-3-chlorophenoxy}-4-fluoro-1H-pyrazol-1-yl)acetate 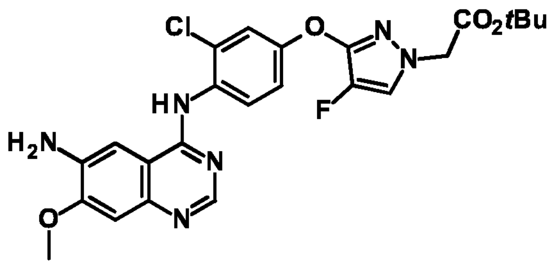 $^1$H-NMR ($CDCl_3$) δ: 1.49 (9H, s), 4.03 (3H, s), 4.33 (2H, brs), 4.61 (2H, s), 6.94-6.97 (1H, m), 7.12-7.24 (2H, m), 7.26-7.31 (1H, m), 7.36-7.41 (1H, m), 7.51-7.56 (1H, m), 8.56-8.75 (2H, m). MS m/z: 515 [M+H]+. | Reference Example U8 |

[Table 31-5]

| Reference Example | Compound | Intermediate |
|---|---|---|
| X10 | 2-(3-{4-[(6-Amino-7-methoxyquinazolin-4-yl)amino]-3-fluorophenoxy}-4-fluoro-1 H-pyrazol-1-yl)-N-tert-butylacetamide 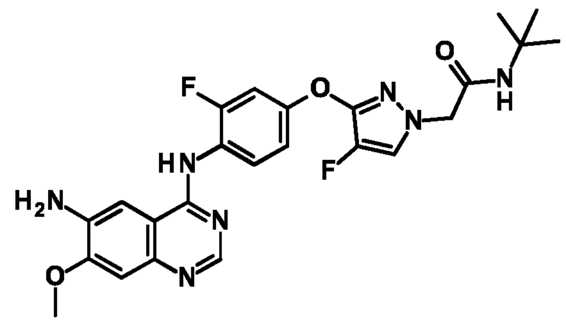 $^1$H-NMR ($CDCl_3$) δ: 1.33 (9H, s), 4.03 (3H, s), 4.34 (2H, brs), 4.50 (2H, s), 5.89-5.97 (1H, m), 6.96 (1H, s), 6.97-7.04 (2H, m), 7.18-7.28 (2H, m), 7.40 (1H, d, J = 4.3 Hz), 8.48-8.54 (1H, m), 8.58 (1H, s). MS m/z: 498 [M+H]$^+$. | Reference Example U9 |

[Reference Example X11] 2-{4-[(6-Amino-7-methoxyquinazolin-4-yl)amino]-3-fluorophenoxy}-N-tert-butyl-1,3-thiazole-4-carboxamide

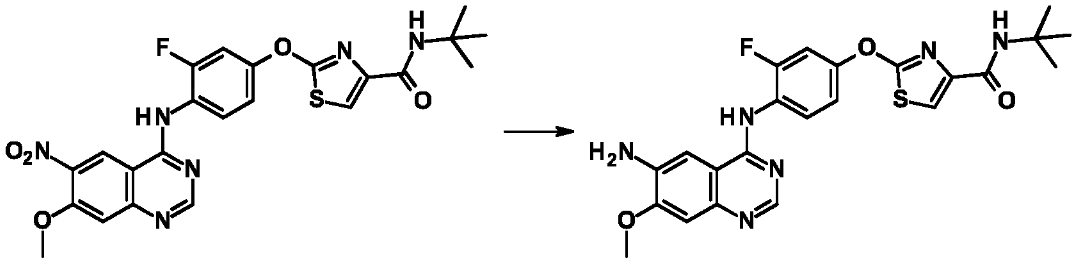

To a mixed solution of N-tert-butyl-2-{3-fluoro-4-[(7-methoxy-6-nitroquinazolin-4-yl)amino]phenoxy}-1,3-thiazole-4-carboxamide (423 mg, 0.825 mmol) in ethanol (10 mL) and tetrahydrofuran (10 mL), acetic acid (2 mL) and iron powder (277 mg, 4.95 mmol) were added, and the mixture was stirred at 75°C for 2 hours. After the reaction solution was left to cool, saturated aqueous sodium bicarbonate solution was added thereto. The insoluble materials were filtered off, and the filtrate was extracted with ethyl acetate. The organic layer was washed with saturated saline and then dried over anhydrous sodium sulfate. The insoluble materials were filtered off and then the filtrate was concentrated under reduced pressure and purified by amino silica gel column chromatography (dichloromethane/methanol) to obtain the title compound (230 mg, 0.477 mmol, yield 57.8%). $^1$H-NMR ($CDCl_3$) δ: 1.45 (9H, s), 4.04 (3H, s), 4.34 (2H, br s), 6.95 (2H, s), 7.16-7.18 (2H, m), 7.22 (1H, s), 7.25 (1H, d, J = 3.7 Hz), 7.61 (1H, s), 8.63 (1H, s), 8.80 (1H, t, J = 9.2 Hz). MS m/z: 483 [M+H]$^+$.

By a similar method, the following compounds were synthesized from the corresponding intermediates.

[Table 32]

| Reference Example | Compound | Intermediate |
|---|---|---|
| X12 | N4-{4-[3-(5-Cyclopropyl-1,3,4-oxadiazol-2-yl)phenoxy]-2-fluorophenyl}-7-ethoxyquinazoline-4,6-diamine 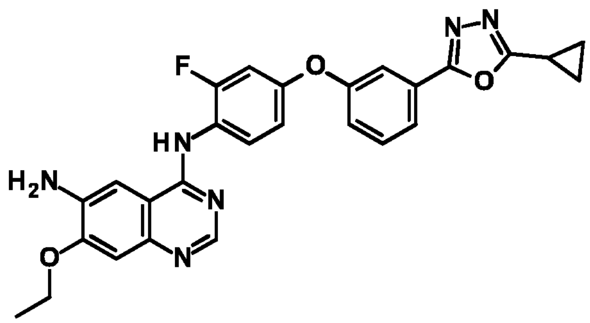 $^1$H-NMR (DMSO-D$_6$) δ: 1.10-1.20 (4H, m), 1.45 (3H, t, J = 6.9 Hz), 2.28-2.33 (1H, m), 4.21 (2H, q, J = 6.9 Hz), 5.32 (2H, br s), 6.96 (1H, dd, J = 8.9, 2.1 Hz), 7.05 (1H, s), 7.13 (1H, dd, J = 11.3, 2.7 Hz), 7.35-7.32 (2H, m), 7.55-7.60 (2H, m), 7.64 (1H, t, J = 7.9 Hz), 7.76 (1H, d, J = 7.9 Hz), 8.21 (1H, s), 9.14 (1H, s). MS m/z: 499 [M+H]+. | Reference Example W3 |

[Reference Example X13] N4-(4-{[4-(5-cyclopropyl-1,3,4-oxadiazol-2-yl)-1,3-thiazol-2-yl]oxy}-2-fluorophenyl)-7-methoxyquinazoline-4,6-diamine

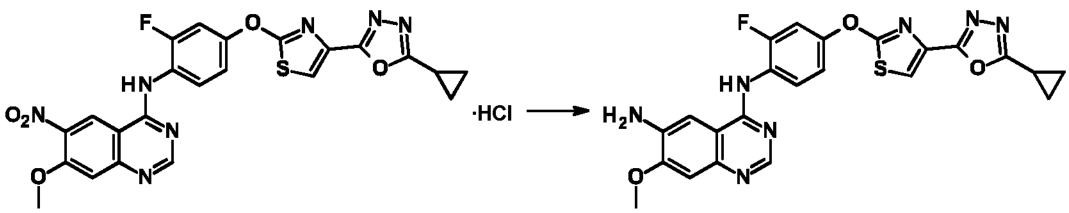

To a suspension of N-(4-{ [4-(5-cyclopropyl-1,3,4-oxadiazol-2-yl)-1,3-thiazol-2-yl]oxy}-2-fluorophenyl)-7-methoxy-6-nitroquinazolin-4-amine hydrochloride (1.06 g, 1.89 mmol) in ethanol (16 mL) and water (4 mL), iron powder (438 mg, 7.83 mmol) was added, and the mixture was stirred at 70°C for 1 hour. The reaction solution was left to cool, and then dichloromethane and methanol were added thereto. The insoluble materials were filtered off and the filtrate was washed with methanol. The filtrate and the washing solution were combined, and concentrated under reduced pressure, then azeotropically concentrated with ethanol. To the residue, a mixed solvent of dichloromethane/methanol (9:1) and saturated aqueous sodium bicarbonate solution were added, and the insoluble materials were filtered off. The two layers were separated, and the aqueous layer was then extracted with a mixed solvent of dichloromethane/methanol (9:1). The obtained organic layers were combined, dried over anhydrous sodium sulfate, filtered, and then concentrated under reduced pressure. The residue was purified by silica gel column chromatography (ethyl acetate/methanol) and dried under reduced pressure at 60°C to obtain the title compound (663 mg, 1.35 mmol, yield 71.4%).
$^1$H-NMR (DMSO-D$_6$) δ: 1.03-1.22 (4H, m), 2.22-2.37 (1H, m), 3.97 (3H, s), 5.41 (2H, s), 7.09 (1H, s), 7.27-7.35 (2H, m), 7.51-7.59 (1H, m), 7.65-7.74 (1H, m), 8.04-8.07 (1H, m), 8.24-8.28 (1H, m), 9.24 (1H, s). MS m/z: 492 [M+H]$^+$.

[Reference Example X14] tert-Butyl 3-{4-[(6-amino-7-methoxyquinazolin-4-yl)amino]-3-fluorophenoxy}-1H-pyrazole-1-carboxylate

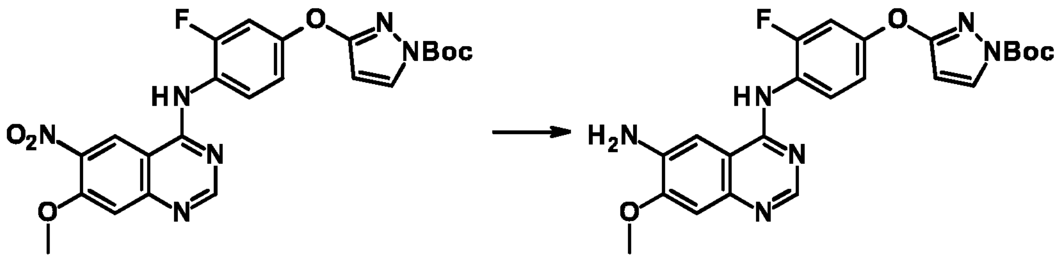

To a suspension of tert-butyl 3-{3-fluoro-4-[(7-methoxy-6-nitroquinazolin-4-yl)amino]phenoxy}-1H-pyrazole-1-carboxylate (529 mg, 1.07 mmol) in ethanol (5 mL) and ethyl acetate (5 mL), 5% palladium on carbon (M) Wet (466 mg) was added, and the mixture was stirred at 50°C for 6.5 hours under a hydrogen atmosphere. The reaction solution was left to cool, and then filtered with Celite. The insoluble materials were washed with ethyl acetate, and the washing solution combined with the filtrate was concentrated under reduced pressure. The resultant was dried under reduced pressure at 60°C to obtain the title compound (434 mg, 0.929 mmol, yield 87.2%) .
$^1$H-NMR ($CDCl_3$) δ: 1.65 (9H, s), 4.03 (3H, s), 4.31 (2H, br s), 5.98-6.04 (1H, m), 6.92-7.23 (5H, m), 7.99-8.03 (1H, m), 8.53-8.64 (2H, m). MS m/z: 467 $[M+H]^+$.

[Reference Example Y1] (2E)-N-[4-(4-{3-[2-(tert-Butylamino)-2-oxoethyl]phenoxy}-2-fluoroanilino)-7-methoxyquinazolin-6-yl]-4-chlorobut-2-enamide

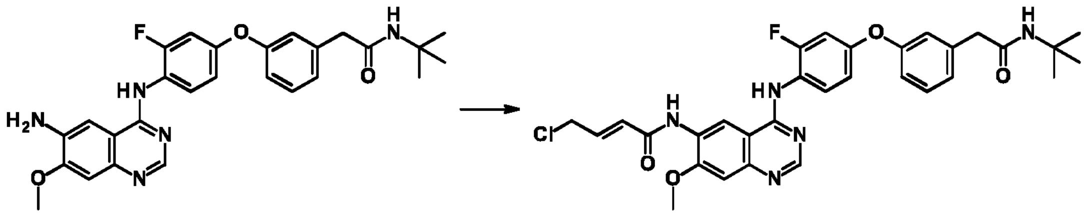

To a solution of 4-bromocrotonic acid (85.9 mg, 0.521 mmol) in acetonitrile (2 mL), oxalyl chloride (0.0670 mL, 0.792 mmol) and N,N-dimethylformamide (0.002 mL) were sequentially added, and the mixture was stirred at 50°C for 30 minutes. After the reaction solution was cooled to be at 0°C, a solution of 2-(3-{4-[(6-amino-7-methoxyquinazolin-4-yl)amino]-3-fluorophenoxy}phenyl)-N-tert-butylacetamide (194 mg, 0.396 mmol) in N,N-dimethylacetamide (2 mL) was added to the reaction solution, and the mixture was stirred at 0°C for 10 minutes, and at room temperature for 3 hours. The reaction solution was diluted with ethyl acetate, and washed with saturated aqueous sodium bicarbonate solution and saturated saline sequentially. The organic layer was dried over anhydrous sodium sulfate, filtered, and then concentrated under reduced pressure. The residue was purified by silica gel column chromatography (ethyl acetate/methanol). The resultant was dried under reduced pressure at 60°C to obtain the title compound (161 mg, 0.271 mmol, yield 68.4%).
$^1$H-NMR ($CDCl_3$) δ: 1.31 (9H, s), 3.47 (2H, s), 4.09 (3H, s), 4.24-4.34 (2H, m), 5.23 (1H, br s), 6.30-6.45 (1H, m), 6.75-7.20 (6H, m), 7.30-7.40 (2H, m), 7.51 (1H, br s), 8.13-8.26 (2H, m), 8.66 (1H, s), 9.15 (1H, s). MS m/z: 592 $[M+H]^+$.

By a similar method, the following compounds were synthesized from the corresponding intermediates.

[Table 33-1]

| Reference Example | Compound | Intermediate |
| --- | --- | --- |
| Y2 | (2E)-4-Chloro-N-(4-{2-fluoro-4-[3-(5-methyl-1,3,4-oxadiazol-2-yl)phenoxy]anilino}-7-methoxyquinazolin-6-yl)but-2-enamide 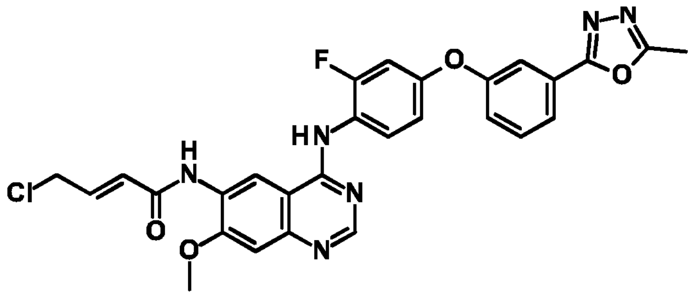 | Reference Example X2 |
| Y3 | (2E)-4-Chloro-N-(7-ethoxy-4-{2-fluoro-4-[3-(5-methyl-1,3,4-oxadiazol-2-yl) phenoxy]anilino}quinazolin-6-yl)but-2-enamide 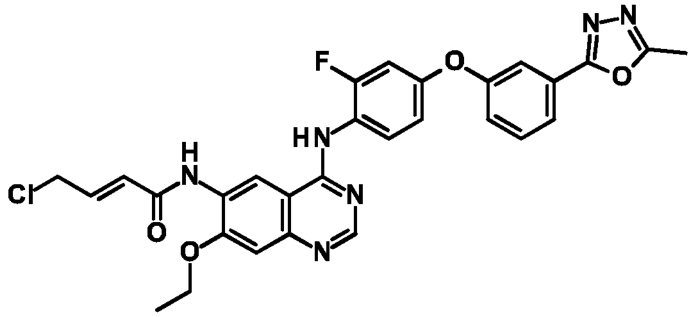 | Reference Example X3 |

[Table 33-2]

| Reference Example | Compound | Intermediate |
| --- | --- | --- |
| Y4 | (2E)-4-Chloro-N-(4-{4-[3-(5-cyclopropyl-1,3,4-oxadiazol-2-yl)phenoxy]-2-fluoroanilino}-7-ethoxyquinazolin-6-yl)but-2-enamide 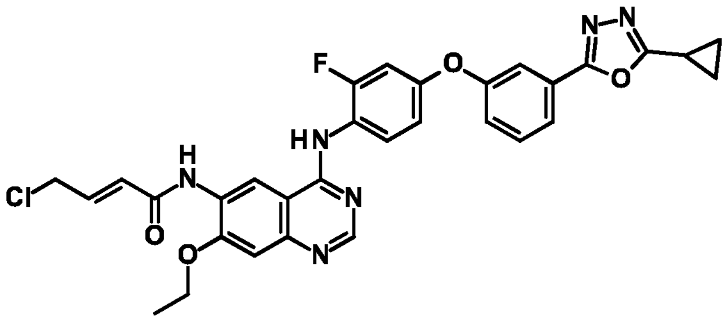 $^1$H-NMR (DMSO-$D_6$) δ: 1.10-1.20 (4H, m), 1.46 (3H, t, J = 7.0 Hz), 2.28-2.34 (1H, m), 4.27-4.32 (2H, m), 4.47 (2H, d, J = 4.3 Hz), 6.75 (1H, d, J = 15.3 Hz), 6.87-6.98 (2H, m), 7.14 (1H, dd, J = 11.0, 2.5 Hz), 7.26 (1H, s), 7.36 (1H, dd, J = 9.2, 2.5 Hz), 7.51 (1H, t, J = 8.6 Hz), 7.60-7.61 (1H, m), 7.65 (1H, t, J = 8.0 Hz), 7.78 (1H, d, J = 8.0 Hz), 8.41 (1H, s), 8.90 (1H, s), 9.71 (1H, s), 9.77 (1H, br s). MS m/z: 601 [M+H]+. | Reference Example X12 |

(continued)

| Reference Example | Compound | Intermediate |
|---|---|---|
| Y5 | (2E)-N-{4-[4-({1-[2-(tert-butylamino)-2-oxoethyl]-4-fluoro-1 H-pyrazol-3-yl}oxy)-2-chloroanilino]-7-methoxyquinazolin-6-yl}-4-chlorobut-2-enamide 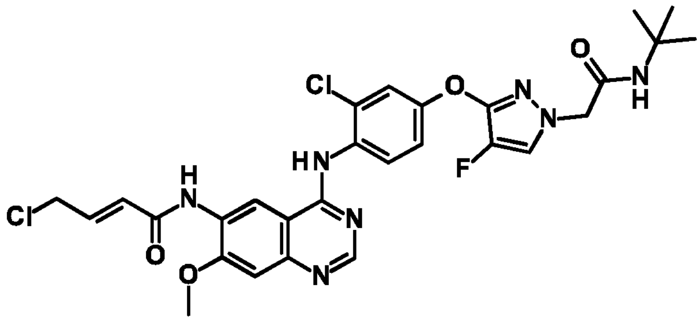 $^1$H-NMR ($CDCl_3$) δ: 1.40 (9H, s), 4.09 (3H, s), 4.24-4.33 (2H, m), 4.50 (2H, s), 5.96 (1H, s), 6.34-6.41 (1H, m), 7.07-7.20 (2H, m), 7.25-7.29 (1H, m), 7.31 (1H, s), 7.38-7.43 (1H, m), 7.89 (1H, s), 8.19 (1H, s), 8.51-8.58 (1H, m), 8.64-8.71 (1H, m), 9.23 (1H, s). MS m/z: 616 $[M+H]^+$. | Reference Example X8 |

[Table 33-3]

| Reference Example | Compound | Intermediate |
|---|---|---|
| Y6 | (2E)-N-{4-[4-({1-[2-(tert-Butylamino)-2-oxoethyl]-4-fluoro-1 H-pyrazol-3-yl}oxy)-2-fluoroanilino]-7-methoxyquinazolin-6-yl}-4-chlorobut-2-enamide 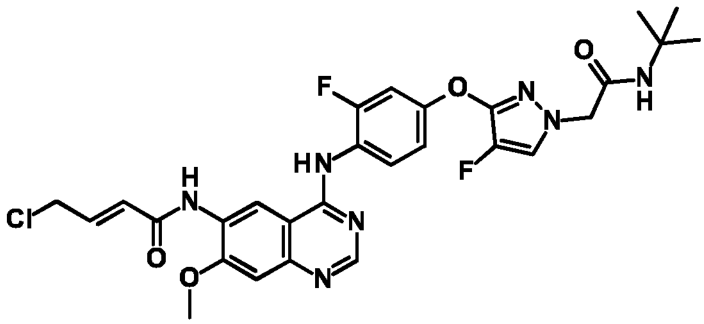 $^1$H-NMR ($CDCl_3$) δ: 1.33 (9H, s), 4.09 (3H, s), 4.28-4.31 (2H, m), 4.50 (2H, s), 5.95 (1H, br s), 6.34-6.41 (1H, m), 6.97-7.05 (2H, m), 7.10-7.18 (1H, m), 7.30 (1H, s), 7.39-7.43 (1H, m), 7.48-7.53 (1H, m), 8.18 (1H, br s), 8.22-8.30 (1H, m), 8.65 (1H, s), 9.14 (1H, s). MS m/z: 600 [M+H]+. | Reference Example X10 |

(continued)

| Reference Example | Compound | Intermediate |
|---|---|---|
| Y7 | (2E)-N-{4-[4-({4-[2-(tert-Butylamino)-2-oxoethyl]-1,3-thiazol-2-yl}oxy)-2-fluoroanilino]-7-methoxyquinazolin-6-yl}-4-chlorobut-2-enamide 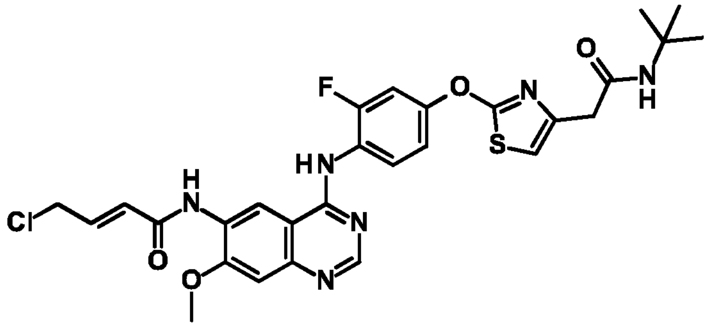 $^1$H-NMR ($CDCl_3$) δ: 1.31 (9H, s), 3.44 (2H, s), 4.10 (3H, s), 4.27-4.33 (2H, m), 6.32-6.55 (2H, m), 6.61-6.68 (1H, m), 7.07-7.23 (3H, m), 7.30-7.34 (1H, m), 7.72 (1H, s), 8.21 (1H, s), 8.36-8.50 (1H, m), 8.66-8.69 (1H, m), 9.09-9.21 (1H, m). MS m/z: 599 [M+H]+. | Reference Example U15 |

[Table 33-4]

| Reference Example | Compound | Intermediate |
|---|---|---|
| Y8 | (2E)-4-Chloro-N-[4-(4-{[4-(5-cyclopropyl-1,3,4-oxadiazol-2-yl)-1,3-thiazol-2-yl]oxy}-2-fluoroanilino)-7-methoxyquinazolin-6-yl]but-2-enamide 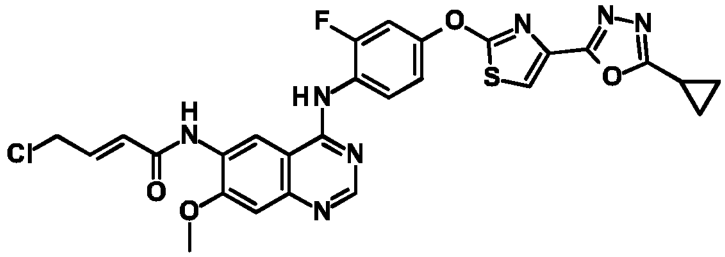 $^1$H-NMR (DMSO-$D_6$) δ: 1.02-1.26 (4H, m), 2.23-2.37 (1H, m), 4.03 (3H, s), 4.39-4.53 (2H, m), 6.71-6.82 (1H, m), 6.85-6.98 (1H, m), 7.26-7.38 (2H, m), 7.51-7.69 (2H, m), 8.04-8.09 (1H, m), 8.42-8.48 (1H, m), 8.94 (1H, s), 9.79-9.91 (2H, m). MS m/z: 594 [M+H]+. | Reference Example X13 |

[Reference Example Z1] tert-Butyl 3-{4-[(6-{[(2E)-4-(dimethylamino)but-2-enoyl]amino}-7-methoxyquinazolin-4-yl)amino]-3-fluorophenoxy}-1H-pyrazole-1-carboxylate

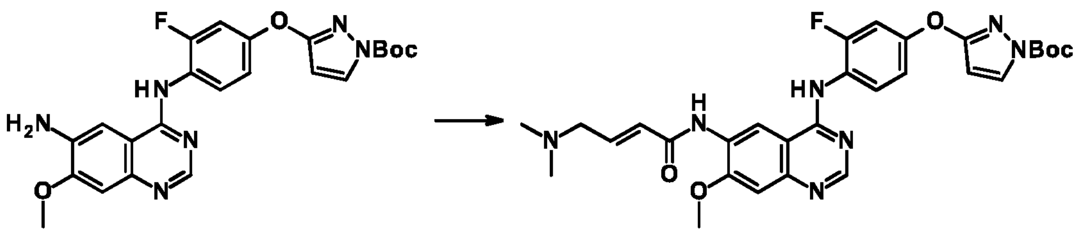

To a suspension of (2E)-4-(dimethylamino)but-2-enoic acid hydrochloride (230 mg, 1.39 mmol) in acetonitrile

(5 mL), oxalyl chloride (0.118 mL, 1.39 mmol) and N,N-dimethylformamide (0.004 mL, 0.05 mmol) were sequentially added, and the mixture was stirred at 50°C for 30 minutes. The reaction solution was cooled to be at 0°C and a solution of tert-butyl 3-{4-[(6-amino-7-methoxyquinazolin-4-yl)amino]-3-fluorophenoxyl-1H-pyrazole-1-carboxylate (434 mg, 0.929 mmol) in N,N-dimethylacetamide (5 mL) was added thereto. The mixture was stirred at the same temperature for 30 minutes, and the reaction solution was warmed to room temperature. The reaction solution was stirred for 2.5 hours, then saturated aqueous sodium bicarbonate solution was added thereto, and the mixture was extracted with dichloromethane. The organic layer was dried over anhydrous sodium sulfate, filtered, and then concentrated under reduced pressure. The residue was purified by silica gel column chromatography (dichloromethane/methanol) and the obtained solid was dried under reduced pressure at 60°C to obtain the title compound (322 mg, 0.557 mmol, yield 59.9%).
$^1$H-NMR ($CDCl_3$) δ: 1.65 (9H, s), 2.32 (6H, s), 3.14-3.20 (2H, m), 4.06 (3H, s), 6.03 (1H, m), 6.18-6.29 (1H, m), 6.97-7.12 (3H, m), 7.23-7.29 (1H, m), 7.53 (1H, s), 7.99-8.03 (1H, m), 8.12-8.31 (2H, m), 8.65 (1H, s), 9.16 (1H, s). MS m/z: 578 $[M+H]^+$.

By a similar method, the following compounds were synthesized from the corresponding intermediates.

[Table 34]

| Reference Example | Compound | Intermediate |
|---|---|---|
| Z2 | tert-Butyl (3-{3-chloro-4-[(6-{[(2E)-4-(dimethylamino)but-2-enoyl]amino}-7-methoxyquinazolin-4-yl)amino]phenoxy}-4-fluoro-1 H-pyrazol-1-yl)acetate 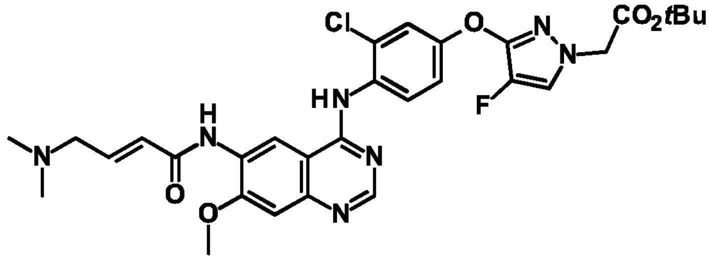 $^1$H-NMR ($CDCl_3$) δ: 1.49 (9H, s), 2.34 (6H, s), 3.18-3.22 (2H, m), 4.07 (3H, s), 4.62 (2H, s), 6.26 (1H, d, J = 15.8 Hz), 6.99-7.09 (1H, m), 7.13-7.19 (1H, m), 7.26-7.32 (2H, m), 7.39 (1H, d, J = 4.3 Hz), 7.85 (1H, s), 8.18 (1H, s), 8.49 (1H, d, J = 9.1 Hz), 8.66 (1H, s), 9.23 (1H, s). MS m/z: 626 [M+H]+. | Reference Example X9 |

[Reference Example Z3] (2E)-4-(Dimethylamino)-N-{4-[2-fluoro-4-(lH-pyrazol-3-yloxy)anilino]-7-methoxyquinazolin-6-yl}but-2-enamide

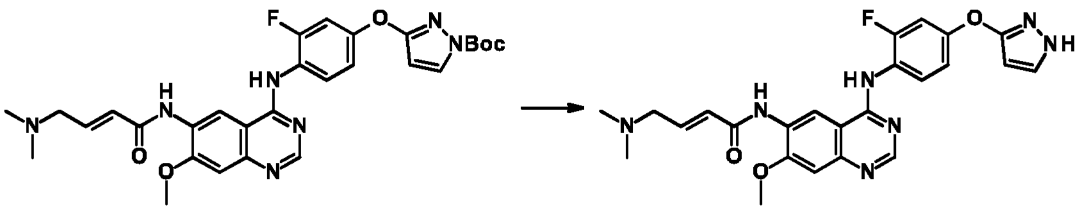

To a solution of tert-butyl 3-{4-[(6-{[(2E)-4-(dimethylamino)but-2-enoyl]amino}-7-methoxyquinazolin-4-yl)amino]-3-fluorophenoxy}-1H-pyrazole-1-carboxylate (1.13 g, 1.95 mmol) in dichloromethane (20 mL), trifluoroacetic acid (4 mL) was added, and the mixture was stirred at room temperature for 2 hours. The reaction solution was concentrated under reduced pressure, and saturated aqueous sodium bicarbonate solution was added thereto. The mixture was stirred for 5 minutes, and then the precipitated solid was collected by filtration. The obtained solid was washed with water, and dissolved in a mixed solvent of dichloromethane/methanol (9:1). The solution was dried over anhydrous sodium sulfate, filtered, and then concentrated under reduced pressure. The concentrate was dried under reduced pressure at 60°C to obtain the title compound (purity 96%, 968 mg, 1.95 mmol, quantitative).
$^1$H-NMR (DMSO-$D_6$) δ: 2.43 (6H, s), 3.36-3.47 (2H, m), 4.01 (3H, s), 5.97-6.02 (1H, m), 6.56-7.11 (4H, m), 7.22-7.50 (2H, m), 7.71-7.78 (1H, m), 8.37 (1H, s), 8.90 (1H, s), 9.57-9.89 (2H, m), 12.48 (1H, s). MS m/z: 478 $[M+H]^+$.

[Reference Example Z4] (3-{3-Chloro-4-[(6-{[(2E)-4-(dimethylamino)but-2-enoyl]amino}-7-methoxyquinazolin-4-yl)aminolphenoxyl-4-fluoro-1H-pyrazol-1-yl)acetic acid

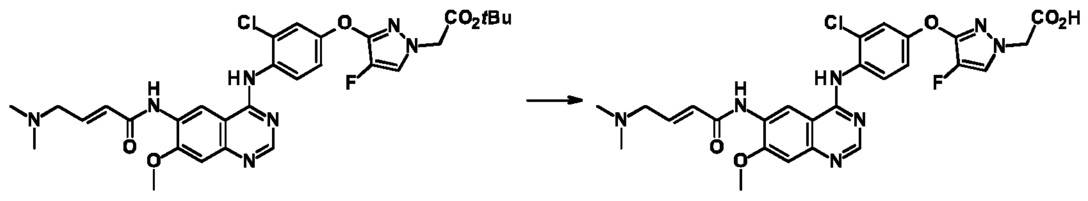

To a solution of tert-butyl (3-{3-chloro-4-[(6-{[(2E)-4-(dimethylamino)but-2-enoyl]amino}-7-methoxyquinazolin-4-yl)amino]phenoxy}-4-fluoro-1H-pyrazol-1-yl)acetate (227 mg, 0.362 mmol) in dichloromethane (2 mL), trifluoroacetic acid (2 mL) was added, and the mixture was stirred at room temperature for 2 hours. The reaction solution was concentrated under reduced pressure. To the residue, saturated aqueous sodium bicarbonate solution and 10% aqueous citric acid solution were added. The precipitated solid was collected by filtration and dried under reduced pressure at 60°C to obtain the title compound (188 mg, 0.330 mmol, yield 91.2%).
$^1$H-NMR ($CD_3OD$) δ: 2.93 (6H, s), 3.95-4.03 (2H, m), 4.10 (3H, s), 4.76 (2H, s), 6.73-6.85 (1H, m), 6.88-7.01 (1H, m), 7.11-7.19 (1H, m), 7.24 (1H, s), 7.32 (1H, s), 7.58-7.64 (1H, m), 7.71-7.77 (1H, m), 8.41 (1H, s), 9.07 (1H, s). MS m/z: 570 $[M+H]^+$.

[Example 1] N-tert-Butyl-3-{3-fluoro-4-[(7-methoxy-6-{[1-(prop-2-enoyl)piperidin-4-yl]oxy}quinazolin-4-yl)amino]phenoxy}benzamide

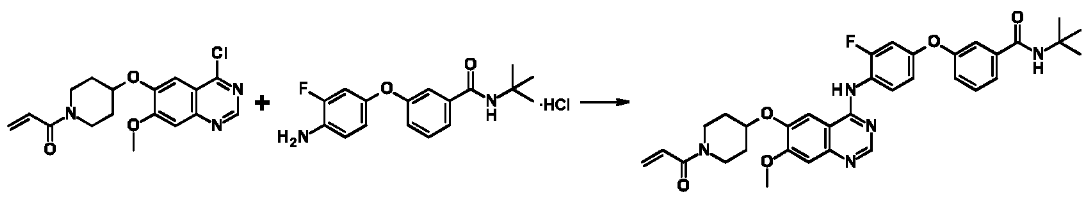

A solution of 1-{4-[(4-chloro-7-methoxyquinazolin-6-yl)oxy]piperidin-1-yl}prop-2-en-1-one (30.2 mg, 0.0868 mmol) and 3-(4-amino-3-fluorophenoxy)-N-tert-butylbenzamide hydrochloride (purity 86.0%, 36.8 mg, 0.0934 mmol) in 2-propanol (2.9 mL) was stirred at 50°C for 3.5 hours. The reaction mixture was diluted with chloroform. Then, an amino silica gel was added, and the insoluble materials were filtered. The reaction mixture was eluted with a mixed solvent of chloroform/methanol (9:1), and the eluted solution was concentrated under reduced pressure. The obtained residue was purified by preparative thin layer chromatography (dichloromethane/methanol). A solution of the obtained product as roughly purified in ethanol was added dropwise to water with stirring, and the mixture was stirred at room temperature for 30 minutes. The solid was collected by filtration and dried under reduced pressure to obtain the title compound (36.8 mg, 0.0600 mmol, yield 69.1%). $^1$H-NMR ($CDCl_3$) δ: 1.47 (9H, s), 1.89-2.09 (4H, m), 3.50-3.79 (2H, m), 3.82-4.00 (2H, m), 4.01 (3H, s), 4.68-4.77 (1H, m), 5.72 (1H, dd, J = 10.4, 1.8 Hz), 5.93 (1H, s), 6.31 (1H, dd, J = 17.1, 1.8 Hz), 6.63 (1H, dd, J = 17.1, 10.4 Hz), 6.84-6.92 (2H, m), 7.11-7.20 (2H, m), 7.22-7.31 (2H, m), 7.38-7.48 (3H, m), 8.30-8.37 (1H, m), 8.67 (1H, s). MS m/z: 614 $[M+H]^+$.

By a similar method, the following compounds were synthesized from the corresponding intermediates.

[Table 35]

| Example | Compound | Intermediate |
|---|---|---|
| 2 | 1-{4-[(4-{2-Fluoro-4-[3-(1-methyl-1 H-1,2,3-triazol-4-yl)phenoxy]anilino}-7-methoxyquinazolin-6-yl)oxy]piperidin-1-yl}prop-2-en-1-one 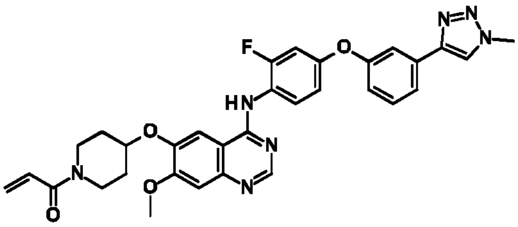 $^1$H-NMR ($CDCl_3$) δ: 1.90-2.08 (4H, m), 3.50-3.61 (1H, m), 3.69-3.78 (1H, m), 3.83-3.99 (2H, m), 4.01 (3H, s), 4.15 (3H, s), 4.68-4.76 (1H, m), 5.72 (1H, dd, J = 10.9, 1.8 Hz), 6.31 (1H, dd, J = 16.7, 1.8 Hz), 6.62 (1H, dd, J = 16.7, 10.9 Hz), 6.87-6.96 (2H, m), 7.04 (1H, dd, J = 8.2, 1.5 Hz), 7.18 (1H, br s), 7.23-7.27 (1H, m), 7.30 (1H, s), 7.39-7.46 (1H, m), 7.51-7.56 (1H, m), 7.60 (1H, d, J = 7.9 Hz), 7.76 (1H, s), 8.25-8.32 (1H, m), 8.67 (1H, s). MS m/z: 596 [M+H]+. | Reference Example M3 |

[Example 3] 1-{4-[(4-{2-Chloro-4-[3-(1-methyl-1H-1,2,3-triazol-4-yl)phenoxy]anilino}-7-methoxyquinazolin-6-yl)oxy]piperidin-1-yl}prop-2-en-1-one

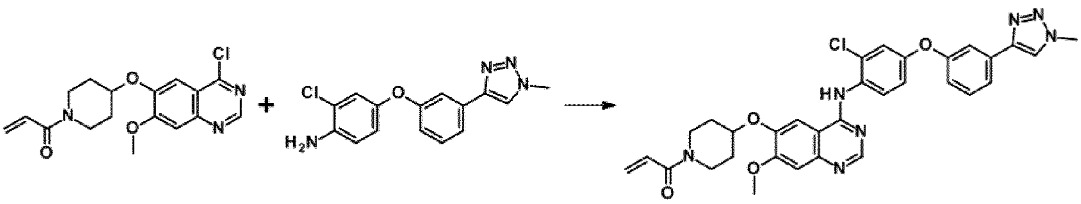

To a solution of 1-{4-[(4-chloro-7-methoxyquinazolin-6-yl)oxy]piperidin-1-yl}prop-2-en-1-one (50 mg, 0.14 mmol), 2-chloro-4-[3-(1-methyl-1H-1,2,3-triazol-4-yl)phenoxy]aniline (43 mg, 0.14 mmol) in 2-propanol (2.5 mL), trifluoroacetic acid (0.016 mL, 0.21 mmol) was added, and the mixture was stirred at 50°C for 3 hours. The reaction mixture was cooled to room temperature, and water and saturated aqueous sodium bicarbonate solution were added thereto, and the mixture was extracted with dichloromethane. The organic layer was dried over anhydrous sodium sulfate, filtered, and then concentrated under reduced pressure. The residue was purified by silica gel column chromatography (dichloromethane/methanol) to obtain the title compound (71 mg, 0.12 mmol, yield 81%).
$^1$H-NMR ($CDCl_3$) δ: 1.92-2.10 (4H, m), 3.50-3.61 (1H, m), 3.66-3.77 (1H, m), 3.84-3.99 (2H, m), 4.02 (3H, s), 4.15 (3H, s), 4.67-4.75 (1H, m), 5.72 (1H, dd, J = 10.4, 1.8 Hz), 6.31 (1H, dd, J = 17.1, 1.8 Hz), 6.63 (1H, dd, J = 17.1, 10.4 Hz), 7.02 (1H, dd, J = 7.9, 2.4 Hz), 7.09 (1H, dd, J = 8.9, 2.7 Hz), 7.18 (1H, d, J = 2.4 Hz), 7.24 (1H, s), 7.31 (1H, s), 7.40-7.45 (1H, m), 7.51-7.53 (1H, m), 7.55 (1H, s), 7.60 (1H, d, J = 7.9 Hz), 7.76 (1H, s), 8.55 (1H, d, J = 9.2 Hz), 8.69 (1H, s). MS m/z: 612 $[M+H]^+$.

[Example 4] N-tert-Butyl-3-{3-fluoro-4-[(7-methoxy-6-{[1-(prop-2-enoyl)piperidin-4-yl]oxy}quinazolin-4-yl)amino]phenoxy}-1H-pyrazole-1-carboxamide

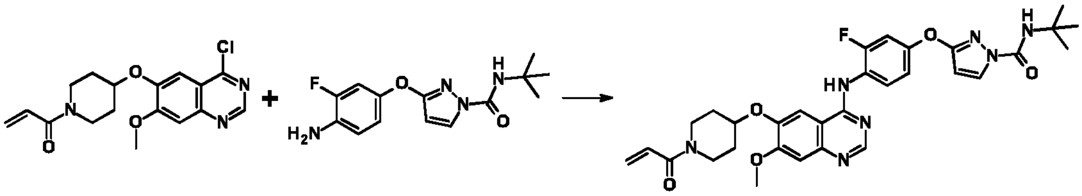

To a solution of 1-{4-[(4-chloro-7-methoxyquinazolin-6-yl)oxy]piperidin-1-yl}prop-2-en-1-one (55 mg, 0.16 mmol) and 3-(4-amino-3-fluorophenoxy)-N-tert-butyl-1H-pyrazole-1-carboxamide (46 mg, 0.16 mmol) in 2-propanol (2.5 mL), trifluoroacetic acid (0.018 mL, 0.24 mmol) was added, and the mixture was stirred at 50°C for 3 hours. After the reaction mixture was left to cool, water and saturated aqueous sodium bicarbonate solution were added thereto, and the mixture was extracted with dichloromethane (x 3). The organic layer was dried over anhydrous sodium sulfate, filtered, and then the solvent was distilled off under reduced pressure. The residue was purified by silica gel column chromatography (dichloromethane/methanol) to obtain the title compound (68.5 mg, 0.113 mmol, yield 72%).
$^1$H-NMR ($CDCl_3$) $\delta$: 1.46 (9H, s), 1.90-2.07 (4H, m), 3.49-3.60 (1H, m), 3.68-3.79 (1H, m), 3.84-3.97 (2H, m), 4.02 (3H, s), 4.67-4.75 (1H, m), 5.72 (1H, dd, J = 10.4, 1.8 Hz), 5.99 (1H, d, J = 3.1 Hz), 6.31 (1H, dd, J = 17.1, 1.8 Hz), 6.63 (1H, dd, J = 17.1, 10.4 Hz), 6.81 (1H, s), 7.01-7.08 (2H, m), 7.17 (1H, s), 7.22 (1H, s), 7.31 (1H, s), 8.13 (1H, d, J = 2.4 Hz), 8.39-8.46 (1H, m), 8.69 (1H, s). MS m/z: 604 $[M+H]^+$.

By a similar method, the following compounds were synthesized from the corresponding intermediates.

[Table 36-1]

| Example | Compound | Intermediate |
|---|---|---|
| 5 | 3-{3-Fluoro-4-[(7-methoxy-6-{[1-(prop-2-enoyl)piperidin-4-yl]oxy}quinazolin-4-yl) amino]phenoxy}-N-(1-fluoro-2-methylpropan-2-yl)-1 H-pyrazole-1-carboxamide 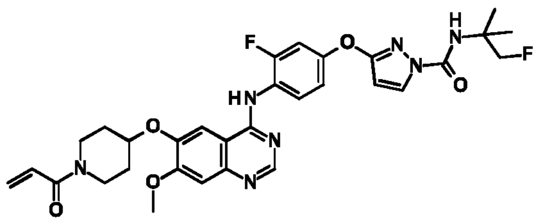 $^1$H-NMR ($CDCl_3$) $\delta$: 1.47 (6H, d, J = 1.8 Hz), 1.91-2.07 (4H, m), 3.51-3.61 (1H, m), 3.70-3.79 (1H, m), 3.83-3.98 (2H, m), 4.02 (3H, s), 4.52 (2H, d, J = 47.4 Hz), 4.68-4.75 (1H, m), 5.72 (1H, dd, J = 10.6, 2.1 Hz), 6.01 (1H, d, J = 3.0 Hz), 6.31 (1H, dd, J = 16.7, 2.1 Hz), 6.62 (1H, dd, J = 16.7, 10.6 Hz), 6.87 (1H, br s), 7.03-7.09 (2H, m), 7.18-7.25 (2H, m), 7.31 (1H, s), 8.12 (1H, d, J = 3.0 Hz), 8.40-8.47 (1H, m), 8.69 (1H, s). MS m/z: 622 [M+H]+. | Reference Example N8 |
| 6 | 3-{3-Fluoro-4-[(7-methoxy -6-{[1-(prop-2-enoyl)piperidin-4-yl]oxy}quinazolin-4-yl) amino]phenoxy}-N-(propan-2-yl)-1 H-pyrazole-1-carboxamide 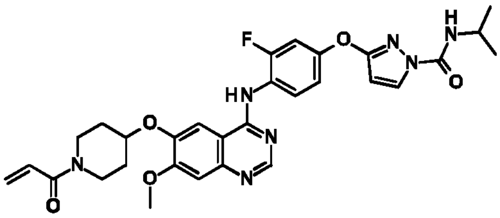 $^1$H-NMR ($CDCl_3$) $\delta$: 1.28 (6H, d, J = 6.7 Hz), 1.89-2.08 (4H, m), 3.50-3.62 (1H, m), 3.69-3.79 (1H, m), 3.83-3.97 (2H, m), 4.02 (3H, s), 4.06-4.17 (1H, m), 4.67-4.75 (1H, m), 5.72 (1H, dd, J = 10.9,1.8 Hz), 6.01 (1H, d, J = 3.0 Hz), 6.31 (1H, dd, J = 17.0, 1.8 Hz), 6.62 (1H, dd, J = 17.0, 10.9 Hz), 6.67-6.72 (1H, m), 7.03-7.08 (2H, m), 7.19-7.24 (2H, m), 7.31 (1H, s), 8.15 (1H, d, J = 3.0 Hz), 8.40-8.46 (1H, m), 8.69 (1H, s). MS m/z: 590 [M+H]+. | Reference Example N12 |

[Table 36-2]

| Example | Compound | Intermediate |
|---|---|---|
| 7 | N-tert-Butyl-3-{2,5-difluoro-4-[(7-methoxy-6-{[1-(prop-2-enoyl)piperidin-4-yl]oxy} quinazolin-4-yl)amino]phenoxy}-1 H-pyrazole-1-carboxamide<br>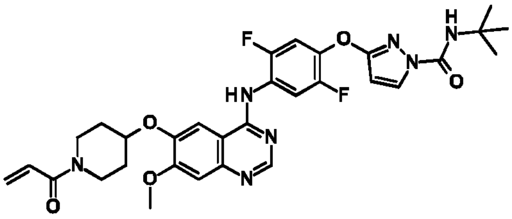<br>$^{1}$H-NMR ($CDCl_3$) δ: 1.45 (9H, s), 1.77-2.19 (4H, m), 3.39-3.79 (2H, m), 3.79-4.10 (5H, m), 4.66-4.79 (1H, m), 5.71 (1H, d, J = 10.3 Hz), 5.97 (1H, d, J = 2.4 Hz), 6.29 (1H, d, J = 17.0 Hz), 6.62 (1H, dd, J = 17.0, 10.3 Hz), 6.75 (1H, s), 7.14 (1H, dd, J = 10.9, 7.3 Hz), 7.30-7.42 (2H, m), 7.70 (1H, s), 8.11 (1H, d, J = 2.4 Hz), 8.50 (1H, dd, J = 11.8, 7.6 Hz), 8.72 (1H, s). MS m/z: 622 [M+H]+. | Reference Example N3 |
| 8 | 3-{3-Fluoro-4-[(7-methoxy -6-{[1-(prop-2-enoyl)piperidin-4-yl]oxy}quinazolin-4-yl) amino]phenoxy}-N-(1-methylcyclopropyl)-1 H-pyrazole-1-carboxamide<br>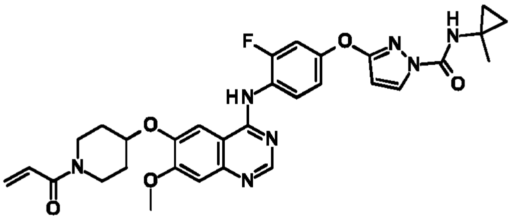<br>$^{1}$H-NMR ($CDCl_3$) δ: 0.71-0.76 (2H, m), 0.85-0.93 (2H, m), 1.47 (3H, s), 1.88-2.08 (4H, m), 3.49-3.63 (1H, m), 3.67-3.79 (1H, m), 3.81-3.99 (2H, m), 4.02 (3H, s), 4.68-4.75 (1H, m), 5.72 (1H, dd, J = 10.3, 1.8 Hz), 6.01 (1H, d, J = 2.4 Hz), 6.31 (1H, dd, J = 17.0, 1.8 Hz), 6.62 (1H, dd, J = 17.0, 10.3 Hz), 7.02-7.08 (2H, m), 7.14 (1H, s), 7.19-7.25 (2H, m), 7.32 (1H, s), 8.14 (1H, d, J = 2.4 Hz), 8.39-8.46 (1H, m), 8.69 (1H, s). MS m/z: 602 [M+H]+. | Reference Example N9 |

[Example 9] 1-(4-{ [4-(2-Fluoro-4-{ [1-(2-methoxypyridin-4-yl)-1H-pyrazol-3-yl]oxy}anilino)-7-methoxyquinazolin-6-yl]oxy}piperidin-1-yl)prop-2-en-1-one

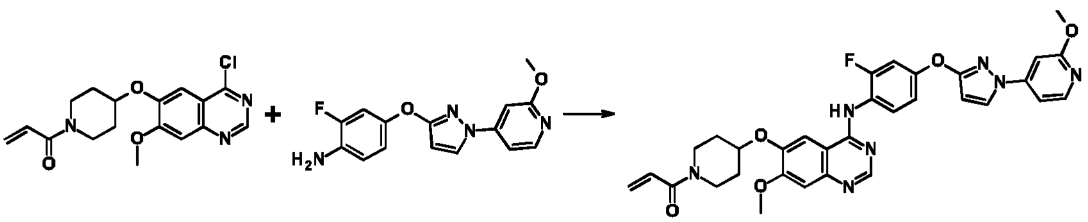

To a suspension of 1-{4-[ (4-chloro-7-methoxyquinazolin-6-yl)oxy]piperidin-1-yl}prop-2-en-1-one (199 mg, 0.571 mmol) and 2-fluoro-4-f[1-(2-methoxypyridin-4-yl)-1H-pyrazol-3-yl]oxy}aniline (165 mg, 0.548 mmol) in 2-propanol (15 mL), a mixed solution of trifluoroacetic acid/2-propanol (1:4) (0.210 mL, 0.548 mmol) was added, and the mixture was stirred at room temperature overnight. To the reaction mixture, saturated aqueous sodium bicarbonate solution was added to quench, and the reaction mixture was extracted with chloroform. The organic layer was washed with saturated saline, then dried over anhydrous sodium sulfate. The insoluble materials were filtered and then the filtrate was concentrated under reduced pressure. The obtained residue was purified by silica gel column chromatography (chloroform/methanol) to obtain the title compound (275 mg, 0.450 mmol, yield 82.0%).
$^{1}$H-NMR (DMSO-$D_6$) δ: 1.66-1.78 (2H, m), 1.98-2.09 (2H, m), 3.44-3.56 (2H, m), 3.83-3.90 (2H, m), 3.90 (3H, s), 3.94

(3H, s), 4.71-4.81 (1H, m), 5.66 (1H, dd, J = 10.4, 2.1 Hz), 6.09 (1H, dd, J = 16.6, 2.1 Hz), 6.36 (1H, d, J = 2.8 Hz), 6.80 (1H, dd, J = 16.6, 10.4 Hz), 7.10-7.17 (2H, m), 7. 22 (1H, s), 7.26 (1H, dd, J = 11.3, 2. 8 Hz), 7.37-7.45 (1H, m), 7.56 (1H, t, J = 8.9 Hz), 7.90 (1H, s), 8.21 (1H, d, J = 6.1 Hz), 8.35 (1H, s), 8.65 (1H, d, J = 2.8 Hz), 9.31 (1H, s). MS m/z: 612 [M+H]+.

By a similar method, the following compounds were synthesized from the corresponding intermediates.

[Table 37-1]

| Example | Compound | Intermediate |
|---|---|---|
| 10 | 1-(4-{[4-(4-{[1-(2-cyclopropylpyrimidin-5-yl)-1H-pyrazol-3-yl]oxy}-2-fluoroanilino)-7-methoxyquinazolin-6-yl]oxy}piperidin-1-yl)prop-2-en-1-one<br>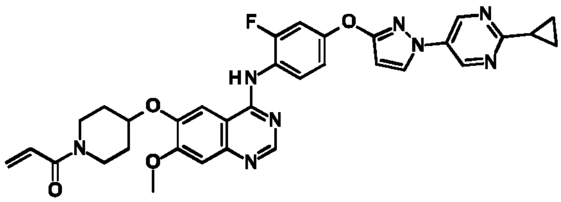<br>$^1$H-NMR ($CDCl_3$) δ: 1.06-1.22 (4H, m), 1.86-2.11 (4H, m), 2.24-2.40 (1H, m), 3.47-3.62 (1H, m), 3.66-3.79 (1H, m), 3.82-3.99 (2H, m), 4.02 (3H, s), 4.67-4.78 (1H, m), 5.72 (1H, dd, J = 11.0, 1.8 Hz), 6.14 (1H, d, J = 2.4 Hz), 6.31 (1H, dd, J = 16.5, 1.8 Hz), 6.62 (1H, dd, J = 16.5, 11.0 Hz), 7.08-7.25 (4H, m), 7.31 (1H, s), 7.83 (1H, d, J = 2.4 Hz), 8.36-8.47 (1H, m), 8.69 (1H, s), 8.88 (2H, s). MS m/z: 623 [M+H]+. | Reference Example K5 |
| 11 | 1-(4-{[4-(2-Fluoro-4-{[4-(5-fluoro-6-methylpyridin-3-yl)-1,3-thiazol-2-yl]oxy}anilino)-7-methoxyquinazolin-6-yl]oxy}piperidin-1-yl)prop-2-en-1-one<br>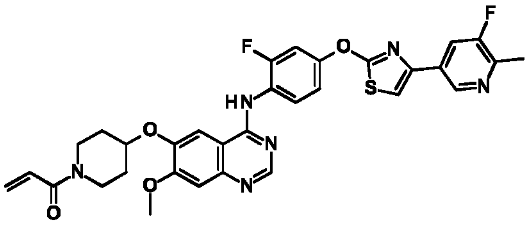<br>$^1$H-NMR (DMSO-$D_6$) δ: 1.67-1.78 (2H, m), 1.98-2.08 (2H, m), 2.47 (3H, d, J = 3.1 Hz), 3.44-3.55 (2H, m), 3.83-3.92 (2H, m), 3.95 (3H, s), 4.71-4.81 (1H, m), 5.63-5.69 (1H, m), 6.05-6.14 (1H, m), 6.74-6.85 (1H, m), 7.23 (1H, s), 7.34-7.40 (1H, m), 7.54 (1H, dd, J = 11.0, 2.4 Hz), 7.68 (1H, t, J = 8.9 Hz), 7.84-8.01 (3H, m), 8.38 (1H, s), 8.79 (1H, s), 9.37 (1H, s). MS m/z: 631 [M+H]+. | Reference Example M5 |

[Table 37-2]

| Example | Compound | Intermediate |
|---|---|---|
| 12 | 1-(4-{[4-(4-{[4-(5-Chloro-6-methylpyridin-3-yl)-1,3-thiazol-2-yl]oxy}-2-fluoroanilino)-7-methoxyquinazolin-6-yl]oxy}piperidin-1-yl)prop-2-en-1-one 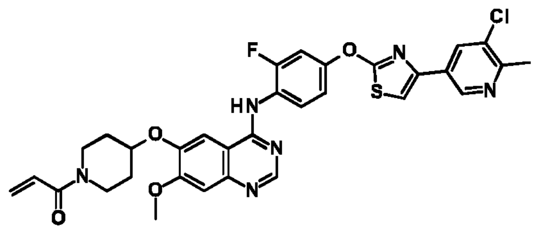 $^1$H-NMR (DMSO-$D_6$) δ: 1.65-1.80 (2H, m), 1.98-2.09 (2H, m), 2.57 (3H, s), 3.44-3.55 (2H, m), 3.82-3.92 (2H, m), 3.95 (3H, s), 4.71-4.82 (1H, m), 5.66 (1H, dd, J = 10.4, 2.5 Hz), 6.10 (1H, dd, J = 16.6, 2.5 Hz), 6.80 (1H, dd, J = 16.6, 10.4 Hz), 7.23 (1H, s), 7.33-7.40 (1H, m), 7.50-7.59 (1H, m), 7.69 (1H, t, J = 8.9 Hz), 7.86-7.94 (2H, m), 8.21 (1H, d, J = 1.8 Hz), 8.38 (1H, s), 8.88 (1H, d, J = 1.8 Hz), 9.38 (1H, s). MS m/z: 647 [M+H]+ | Reference Example M6 |

[Example 13] 1-{4-[(4-{2-Chloro-4-[3-(5-methyl-1,3,4-oxadiazol-2-yl)phenoxy]anilino}-7-methoxyquinazolin-6-yl)oxy]piperidin-1-yl}prop-2-en-1-one

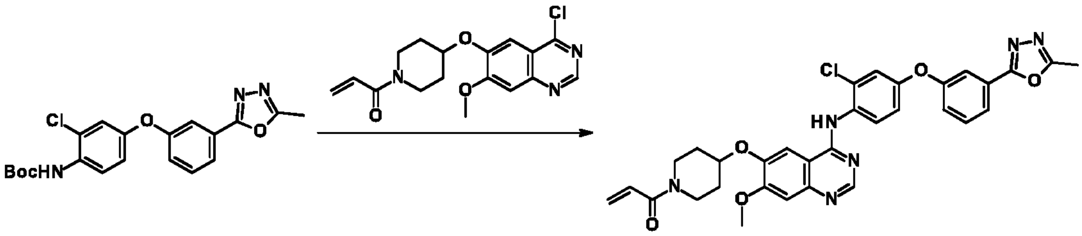

To a solution of tert-butyl {2-chloro-4-[3-(5-methyl-1,3,4-oxadiazol-2-yl)phenoxy]phenyl}carbamate (72.5 mg, 0.180 mmol) in dichloromethane (3 mL), trifluoroacetic acid (1 mL) was added at room temperature, and the mixture was stirred at room temperature for 1 hour. The reaction mixture was concentrated under reduced pressure, and the obtained residue was diluted by addition of dichloromethane, and then the mixture was concentrated under reduced pressure. To the obtained residue, 2-propanol (10 mL) and 1-{4-[(4-chloro-7-methoxyquinazolin-6-yl)oxy]piperidin-1-yl}prop-2-en-1-one (50.4 mg, 0.145 mmol) were added, and the mixture was stirred at room temperature for 3 hours. To the reaction mixture, 1-{4-[ (4-chloro-7-methoxyquinazolin-6-yl)oxy]piperidin-1-yl}prop-2-en-1-one (15.2 mg, 0.0437 mmol) and 2-propanol (5 mL) were added at room temperature, and the mixture was stirred at room temperature for 4 days. The reaction mixture was passed through a pad of amino silica gel, and eluted with 2-propanol. The eluted solution was concentrated under reduced pressure and the obtained residue was purified by silica gel column chromatography (dichloromethane/methanol). The obtained product as roughly purified was purified by preparative thin layer chromatography (dichloromethane/methanol) to obtain the title compound (42.8 mg, 0.0698 mmol, yield 38.7%).
$^1$H-NMR ($CDCl_3$) δ: 1.92-2.11 (4H, m), 2.62 (3H, s), 3.52-3.61 (1H, m), 3.67-3.78 (1H, m), 3.85-4.00 (2H, m), 4.03 (3H, s), 4.67-4.75 (1H, m), 5.72 (1H, dd, J = 10.9, 1.8 Hz), 6.31 (1H, dd, J = 17.0, 1.8 Hz), 6.63 (1H, dd, J = 17.0, 10.9 Hz), 7.10 (1H, dd, J = 9.2, 3.1 Hz), 7.17-7.25 (3H, m), 7.32 (1H, s), 7.50 (1H, t, J = 7.9 Hz), 7.58 (1H, s), 7.64-7.69 (1H, m), 7.81 (1H, d, J = 7.9 Hz), 8.62-8.73 (2H, m). MS m/z: 613 $[M+H]^+$.

By a similar method, the following compounds were synthesized from the corresponding intermediates.

[Table 38-1]

| Example | Compound | Intermediate |
|---|---|---|
| 14 | 1-(4-{[4-(2-Fluoro-4-{[1-(2-methoxypyrimidin-5-yl)-1 H-pyrazol-3-yl]oxy}anilino)-7-methoxyquinazolin-6-yl]oxy}piperidin-1-yl)prop-2-en-1-one 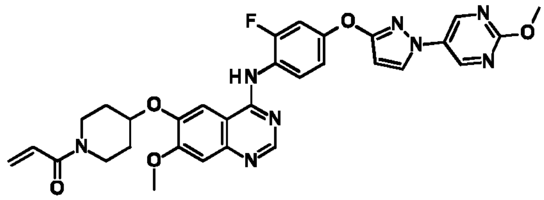 $^1$H-NMR ($CDCl_3$) $\delta$: 1.88-2.13 (4H, m), 3.50-3.63 (1H, m), 3.67-3.80 (1H, m), 3.82-3.99 (2H, m), 4.02 (3H, s), 4.07 (3H, s), 4.66-4.79 (1H, m), 5.72 (1H, dd, J = 10.7, 1.8 Hz), 6.14 (1H, d, J = 3.0 Hz), 6.31 (1H, dd, J = 16.8, 1.8 Hz), 6.62 (1H, dd, J = 16.8, 10.7 Hz), 7.08-7.29 (4H, m), 7.31 (1H, s), 7.77 (1H, d, J = 3.0 Hz), 8.35-8.45 (1H, m), 8.69 (1H, s), 8.83 (2H, s). MS m/z: 613 [M+H]+. | Reference Example K4 |
| 15 | N-tert-Butyl-2-{3-fluoro-4-[(7-methoxy-6-{[1-(prop-2-enoyl)piperidin-4-yl]oxy}quinazolin-4-yl)amino]phenoxy}-1,3-thiazole-4-carboxamide 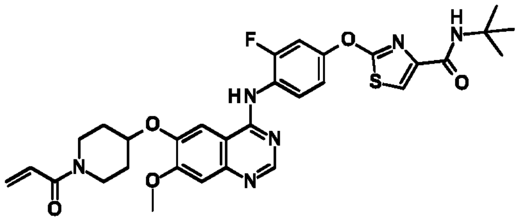 $^1$H-NMR (DMSO-$D_6$) $\delta$: 1.37 (9H, s), 1.61-1.77 (2H, m), 1.98-2.12 (2H, m), 3.37-3.58 (2H, m), 3.83-4.00 (5H, m), 4.73-4.83 (1H, m), 5.69 (1H, dd, J = 10.7, 2.5 Hz), 6.12 (1H, dd, J = 16.6, 2.5 Hz), 6.86 (1H, dd, J = 16.9, 10.7 Hz), 7.23 (1H, s), 7.27 (1H, s), 7.34 (1H, dd, J = 8.6, 1.8 Hz), 7.59 (1H, dd, J = 10.7, 2.8 Hz), 7.67 (1H, t, J = 8.9 Hz), 7.80 (1H, s), 7.90 (1H, br s), 8.38 (1H, s), 9.53 (1H, br s). MS m/z: 621 [M+H]+. | Reference Example H3 |

[Table 38-2]

| Example | Compound | Intermediate |
|---|---|---|
| 16 | 1-(4-{[4-(2-Fluoro-4-{[4-(2-methoxypyrimidin-5-yl)-1,3-thiazol-2-yl]oxy}anilino)-7-methoxyquinazolin-6-yl]oxy}piperidin-1-yl)prop-2-en-1-one 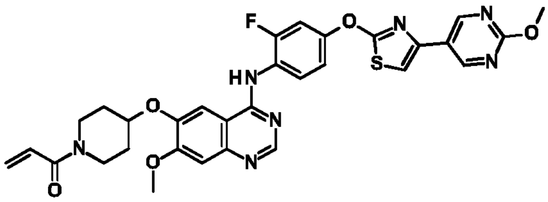 $^1$H-NMR (DMSO-$D_6$) $\delta$: 1.66-1.79 (2H, m), 1.98-2.08 (2H, m), 3.44-3.55 (2H, m), 3.83-3.92 (2H, m), 3.95 (3H, s), 3.97 (3H, s), 4.73-4.80 (1H, m), 5.66 (1H, dd, J = 10.4, 2.5 Hz), 6.09 (1H, dd, J = 16.9, 2.5 Hz), 6.80 (1H, dd, J = 16.9, 10.4 Hz), 7.23 (1H, s), 7.33-7.39 (1H, m), 7.54 (1H, dd, J = 11.0, 2.5 Hz), 7.68 (1H, t, J = 8.6 Hz), 7.76 (1H, s), 7.91 (1H, s), 8.38 (1H, s), 9.01 (2H, s), 9.37 (1H, br s). MS m/z: 630 [M+H]+ | Reference Example L2 |

[Example 17] 1-{4-[(4-{2-Fluoro-4-[3-(5-methyl-1, 3, 4-oxadiazol-2-yl)phenoxy] anilino}-7-methoxyquinazolin-6-yl)oxy]piperidin-1-yl}prop-2-en-1-one

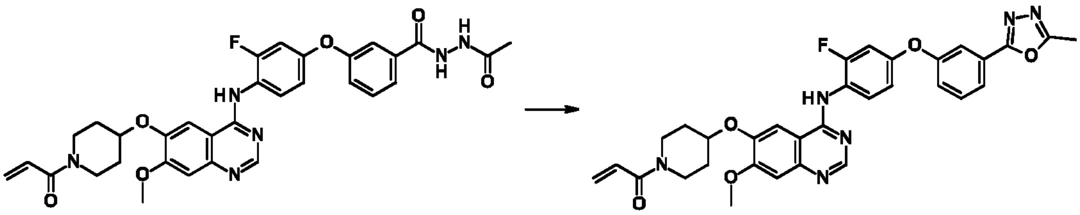

To a suspension of N'-acetyl-3-{3-fluoro-4-[(7-methoxy-6-{[1-(prop-2-enoyl)piperidin-4-yl]oxy}quinazolin-4-yl)amino]phenoxy}benzohydrazide (130 mg, 0.212 mmol), triphenylphosphine (170 mg, 0.648 mmol) and triethylamine (147 μL, 1.06 mmol) in dichloromethane (2.5 mL), hexachloroethane (154 mg, 0.651 mmol) was added at room temperature, and the mixture was stirred at room temperature for 1.5 hours. To the reaction mixture, hexachloroethane (6.2 mg, 0.322 mmol), triphenylphosphine (83.7 mg, 0.319 mmol) and triethylamine (88.2 μL, 0.633 mmol) were added, and the mixture was stirred at room temperature for 2 hours. The reaction mixture was purified by silica gel column chromatography (dichloromethane/ethyl acetate and dichloromethane/methanol). To the roughly purified product, a mixed solvent of hexane/2-propanol (1:1) was added to form a slurry. The solid was collected by filtration, and then washed with the same mixed solvent and dried under reduced pressure to obtain the title compound (48.9 mg, 0.0820 mmol, yield 38.6%).
$^1$H-NMR ($CDCl_3$) δ: 1.89-2.09 (4H, m), 2.62 (3H, s), 3.51-3.61 (1H, m), 3.68-3.79 (1H, m), 3.82-3.99 (2H, m), 4.02 (3H, s), 4.68-4.76 (1H, m), 5.68-5.75 (1H, m), 6.26-6.35 (1H, m), 6.63 (1H, dd, J = 16.8, 10.7 Hz), 6.89-6.97 (2H, m), 7.17-7.33 (4H, m), 7.51 (1H, t, J = 7.9 Hz), 7.67-7.70 (1H, m), 7.81 (1H, d, J = 7.9 Hz), 8.40 (1H, t, J = 9.5 Hz), 8.69 (1H, s). MS m/z: 597 [M+H]$^+$.

[Example 18] 1-{4-[(7-Ethoxy-4-{2-fluoro-4-[3-(5-methyl-1,3,4-oxadiazol-2-yl)phenoxy]anilino}quinazolin-6-yl)oxy]piperidin-1-yl}prop-2-en-1-one

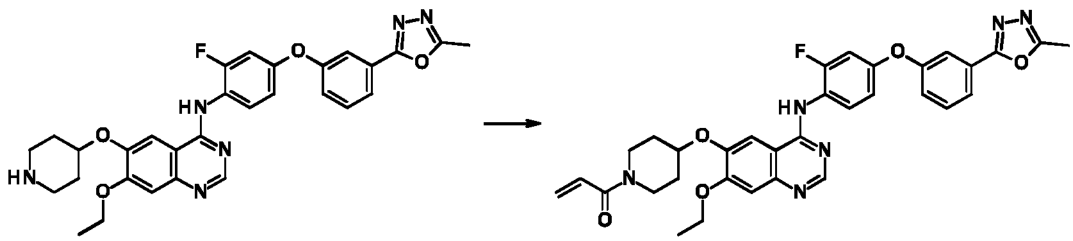

To a solution of 7-ethoxy-N-{2-fluoro-4-[3-(5-methyl-1,3,4-oxadiazol-2-yl)phenoxylphenyl}-6-[(piperidin-4-yl)oxy]quinazolin-4-amine in N,N-dimethylacetamide (4 mL), acryloyl chloride (0.035 mL, 0.44 mmol) was added, and the mixture was stirred at room temperature for 15 minutes. To the reaction solution, saturated aqueous sodium bicarbonate solution was added, and the mixture was extracted with ethyl acetate. The organic layer was washed with saturated saline and then dried over anhydrous sodium sulfate. The insoluble materials were filtered off and then the filtrate was concentrated under reduced pressure and purified by silica gel column chromatography (dichloromethane/methanol). To the obtained product as roughly purified, ethyl acetate and hexane were added, and the precipitated solid was collected by filtration to obtain the title compound (33 mg, 0.054 mmol, yield 15%). $^1$H-NMR ($CDCl_3$) δ: 1.54 (3H, t, J = 7.0 Hz), 1.98-2.01 (4H, m), 2.62 (3H, s), 3.59-3.61 (1H, m), 3.86-3.88 (3H, m), 4.24 (2H, q, J = 7.0 Hz), 4.68-4.71 (1H, m), 5.72 (1H, dd, J = 10.3, 1.8 Hz), 6.31 (1H, dd, J = 16.7, 1.8 Hz), 6.63 (1H, dd, J = 16.7, 10.3 Hz), 6.91-6.94 (2H, m), 7.21-7.29 (4H, m), 7.50 (1H, t, J = 8.2 Hz), 7.68 (1H, t, J = 2.1 Hz), 7.81 (1H, d, J = 7.9 Hz), 8.40-8.47 (1H, m), 8.68 (1H, s). MS m/z: 611 [M+H]$^+$.

By a similar method, the following compounds were synthesized from the corresponding intermediates.

[Table 39-1]

| Example | Compound | Intermediate |
|---|---|---|
| 19 | 1-(4-{[7-(Fluoromethoxy)-4-{2-fluoro-4-[3-(5-methyl-1,3,4-oxadiazol-2-yl)phenoxy] anilino}quinazolin-6-yl]oxy}piperidin-1-yl)prop-2-en-1-one 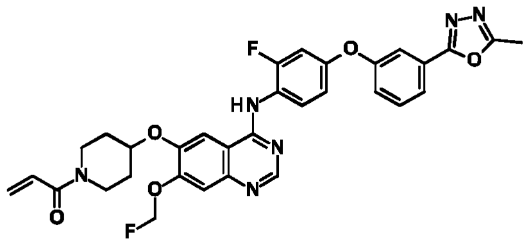 $^1$H-NMR ($CDCl_3$) δ: 2.01-2.03 (4H, m), 2.62 (3H, s), 3.60 (1H, br s), 3.80-3.87 (3H, br m), 4.76-4.76 (1H, m), 5.73 (1H, dd, J = 10.4, 1.8 Hz), 5.91 (2H, d, J = 53.1 Hz), 6.32 (1H, dd, J = 16.5, 1.8 Hz), 6.63 (1H, dd, J = 16.5, 10.4 Hz), 6.92-6.95 (2H, m), 7.22-7.24 (2H, m), 7.29 (1H, d, J = 2.4 Hz), 7.51 (1H, t, J = 7.9 Hz), 7.63 (1H, s), 7.69 (1H, t, J = 1.8 Hz), 7.81 (1H, d, J = 7.9 Hz), 8.40 (1H, t, J = 9.2 Hz), 8.71 (1H, s). MS m/z: 615 [M+H]+. | Reference Example T2 |
| 20 | N-tert-Butyl-3-{3-fl uoro-4-[(7-[($^2H_3$)methyloxy]-6-{[1-(prop-2-enoyl)piperidin-4-yl]oxy} quinazolin-4-yl)amino]phenoxy}-1 H-pyrazole-1-carboxamide 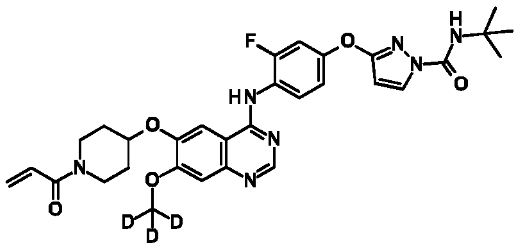 1H-NMR($CDCl_3$) δ: 1.46 (9H, s), 1.99-2.06 (4H, m), 3.57 (1H, br s), 3.75 (1H, br s), 3.91 (2H, br s), 4.70-4.72 (1H, m), 5.72 (1H, dd, J = 10.4, 1.8 Hz), 5.99 (1H, d, J = 3.1 Hz), 6.31 (1H, dd, J = 16.6, 1.8 Hz), 6.62 (1H, dd, J = 16.6, 10.4 Hz), 6.81 (1H, br s), 7.03-7.07 (2H, m), 7.16 (1H, br s), 7.22 (1H, s), 7.31 (1H, s), 8.13 (1H, d, J = 3.1 Hz), 8.43 (1H, t, J = 9.2 Hz), 8.69 (1H, s). MS m/z: 607 [M+H]+. | Reference Example T3 |

[Table 39-2]

| Example | Compound | Intermediate |
|---|---|---|
| 21 | 3-{3-Fluoro-4-[(7-[($^2H_3$)methyloxy]-6-{[1-(prop-2-enoyl)piperidin-4-yl]oxy}quinazolin-4-yl)amino]phenoxy}-N-[2-($^2H_3$)methyl($^2H_6$)propan-2-yl]-1 H-pyrazole-1-carboxamide 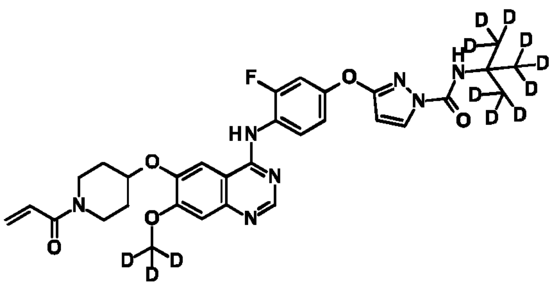 $^1$H-NMR($CDCl_3$) δ: 1.99-2.06 (4H, m), 3.58 (1H, br s), 3.75 (1H, br s), 3.92 (2H, br s), 4.70-4.72 (1H, m), 5.72 (1H, dd, J = 10.4, 1.8 Hz), 5.99 (1H, d, J = 3.1 Hz), 6.31 (1H, dd, J = 17.1, 1.8 Hz), 6.62 (1H, dd, J = 17.1, 10.4 Hz), 6.80 (1H, s), 7.01-7.06 (2H, m), 7.16 (1H, s), 7.22 (1H, s), 7.31 (1H, s), 8.13 (1H, d, J = 3.1 Hz), 8.43 (1H, t, J = 8.9 Hz), 8.69 (1H, s). MS m/z: 616 [M+H]+. | Reference Example T4 |

[Example 22] 3-{3-Fluoro-4-[(7-methoxy-6-{[1-(prop-2-enoyl)piperidin-4-yl]oxy}quinazolin-4-yl)amino]phenoxy}-N-[1-(trifluoromethyl)cyclopropyl]-1H-pyrazole-1-carboxamide

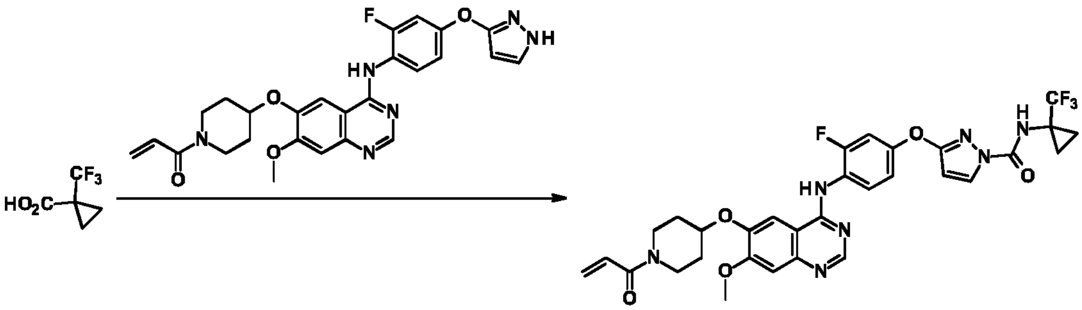

To a solution of trifluoromethylcyclopropane-1-carboxylic acid (33 mg, 0.21 mmol) in toluene (3 mL), triethylamine (0.124 mL, 0.895 mmol) and diphenylphosphoryl azide (0.062 mL, 0.29 mmol) were added, and the mixture was stirred at 85°C for 1 hour. After the reaction mixture was left to cool, a suspension of 1-{4-[(4-{2-fluoro-4-[(1H-pyrazol-3-yl)oxy]anilino}-7-methoxyquinazolin-6-yl)oxy]piperidin-1-yl}prop-2-en-1-one (90 mg, 0.18 mmol) in 1,2-dichloroethane (6 mL) was added thereto, and the mixture was stirred at room temperature for 20 minutes, at 40°C for 30 minutes, at 50°C for 2 hours, at 60°C for 2 hours, at 70°C for 1 hour, and at 85°C for 6 hours. After the reaction mixture was left to cool, it was concentrated under reduced pressure, and the residue was purified by silica gel column chromatography (dichloromethane/methanol) to obtain the title compound (62 mg, 0.095 mmol, yield 53%).
$^1$H-NMR ($CDCl_3$) δ: 1.24-1.30 (2H, m), 1.40-1.45 (2H, m), 1.91-2.08 (4H, m), 3.52-3.62 (1H, m), 3.69-3.80 (1H, m), 3.84-3.98 (2H, m), 4.02 (3H, s), 4.66-4.75 (1H, m), 5.72 (1H, dd, J = 10.9, 1.8 Hz), 6.06 (1H, d, J = 3.0 Hz), 6.31 (1H, dd, J = 16.4, 1.8 Hz), 6.62 (1H, dd, J = 16.4, 10.9 Hz), 7.04-7.10 (2H, m), 7.18-7.23 (2H, m), 7.32 (1H, s), 7.40 (1H, br s), 8.14 (1H, d, J = 3.0 Hz), 8.42-8.49 (1H, m), 8.70 (1H, s). MS m/z: 656 $[M+H]^+$.

By a similar method, the following compounds were synthesized from the corresponding carboxylic acid.

[Table 40]

| Example | Compound | Carboxylic acid |
|---|---|---|
| 23 | N-(1,3-Difluoro-2-methylpropan-2-yl)-3-{3-fluoro-4-[(7-methoxy-6-{[1-(prop-2-enoyl)piperidin-4-yl]oxy}quinazolin-4-yl)amino]phenoxy}-1H-pyrazole-1-carboxamide 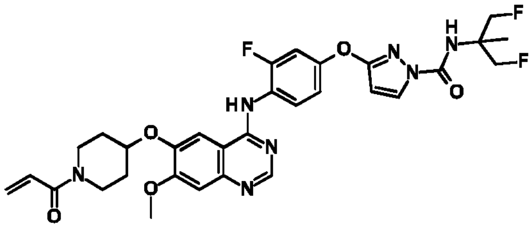 $^1$H-NMR ($CDCl_3$) δ: 1.50-1.57 (3H, m), 1.89-2.10 (4H, m), 3.51-3.63 (1H, m), 3.68-3.79 (1H, m), 3.81-3.99 (2H, m), 4.02 (3H, s), 4.50-4.80 (5H, m), 5.72 (1H, dd, J = 10.3, 1.5 Hz), 6.03 (1H, d, J = 3.0 Hz), 6.31 (1H, dd, J = 16.7, 1.5 Hz), 6.62 (1H, dd, J = 16.7, 10.3 Hz), 7.01-7.09 (3H, m), 7.24-7.37 (3H, m), 8.11 (1H, d, J = 3.0 Hz), 8.34-8.50 (1H, m), 8.68 (1H, s). MS m/z: 640 [M+H]+. | 3-Fluoro-2-(fluoromethyl)-2-methyl propanoic acid |

[Example 24] 3-{3-Fluoro-4-[(7-methoxy-6-{[1-(prop-2-enoyl)piperidin-4-yl]oxy}quinazolin-4-yl)amino]phenoxy}-N-[(2R)-1,1,1-trifluoropropan-2-yl]-1H-pyrazole-1-carboxamide

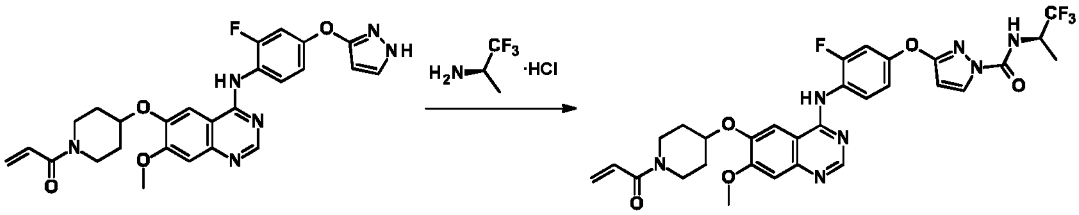

To a solution of 1-f4-[(4-f2-fluoro-4-[(1H-pyrazol-3-yl)oxy]anilino}-7-methoxyquinazolin-6-yl)oxy]piperidin-1-yl}prop-2-en-1-one (75 mg, 0.15 mmol) in dichloromethane (3 mL), 4-nitrophenyl chloroformate (35 mg, 0.17 mmol) and triethylamine (0.082 mL, 0.59 mmol) were added under ice cooling, and the mixture was stirred at 0°C for 40 minutes and at room temperature for 1.5 hours. (R)-2-Amino-1,1,1-trifluoropropane hydrochloride (27 mg, 0.18 mmol) was added, and the mixture was stirred at room temperature overnight. The reaction solution was diluted with water, and extracted with dichloromethane. The organic layer was dried over anhydrous sodium sulfate, filtered, and then concentrated. The residue was purified by silica gel column chromatography (dichloromethane/methanol) to obtain the title compound (63.5 mg, 0.0987 mmol, yield 66%).

$^1$H-NMR ($CDCl_3$) δ: 1.46 (3H, d, J = 7.3 Hz), 1.91-2.08 (4H, m), 3.52-3.61 (1H, m), 3.69-3.80 (1H, m), 3.84-3.98 (2H, m), 4.02 (3H, s), 4.63-4.75 (2H, m), 5.72 (1H, dd, J = 10.5, 1.8 Hz), 6.07 (1H, d, J = 3.0 Hz), 6.31 (1H, dd, J = 16.7, 1.8 Hz), 6.63 (1H, dd, J = 16.7, 10.5 Hz), 6.94-7.01 (1H, m), 7.05-7.10 (2H, m), 7.17-7.23 (2H, m), 7.32 (1H, s), 8.15 (1H, d, J = 3.0 Hz), 8.43-8.51 (1H, m), 8.69 (1H, s) . MS m/z: 644 $[M+H]^+$.

By a similar method, the following compounds were synthesized from the corresponding intermediates and amines.

[Table 41]

| Example | Compound | Intermediate / Amine |
|---|---|---|
| 25 | 3-{3-Fluoro-4-[(7-methoxy-6-{[1-(prop-2-enoyl)piperidin-4-yl]oxy}quinazolin-4-yl) amino]phenoxy}-N-[2-($^2H_3$)methyl($^2H_6$)propan-2-yl]-1H-pyrazole-1-carboxamide 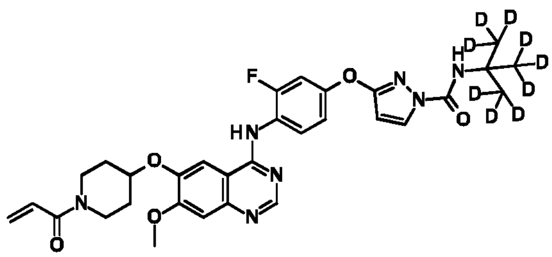 $^1$H-NMR ($CDCl_3$) δ: 1.90-2.10 (4H, m), 3.50-3.63 (1H, m), 3.70-3.80 (1H, m), 3.83-4.00 (2H, m), 4.02 (3H, s), 4.68-4.75 (1H, m), 5.72 (1H, dd, J = 10.3, 1.8 Hz), 5.99 (1H, d, J = 3.0 Hz), 6.31 (1H, dd, J = 17.0, 1.8 Hz), 6.62 (1H, dd, J = 17.0, 10.3 Hz), 6.80 (1H, br s), 7.02-7.08 (2H, m), 7.15-7.25 (2H, m), 7.31 (1H, s), 8.13 (1H, d, J = 3.0 Hz), 8.39-8.46 (1H, m), 8.69 (1H, s). MS m/z: 613 [M+H]+. | Reference Example Q2 / 2-Amino-2-methyl-$d_3$-propane-1,1,1,3,3,3-$d_6$ |

(continued)

| Example | Compound | Intermediate / Amine |
|---|---|---|
| 26 | (2E)-4-(Dimethylamino)-N-[4-(2-fluoro-4-{[1-(pyrrolidin-1-ylcarbonyl)-1 H-pyrazol-3-yl] oxy}anilino)-7-methoxyquinazolin-6-yl]but-2-enamide 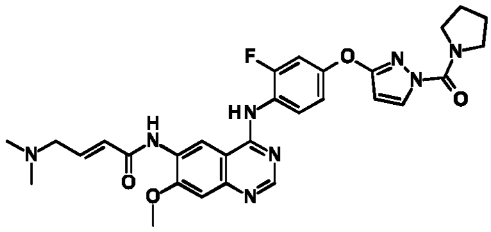 $^1$H-NMR ($CDCl_3$) δ: 1.84-2.05 (4H, m), 2.34 (6H, s), 3.14-3.24 (2H, m), 3.57-3.76 (2H, m), 3.83-4.02 (2H, m), 4.08 (3H, s), 6.03 (1H, d, J = 3.0 Hz), 6.26 (1H, d, J = 15.2 Hz), 6.98-7.15 (3H, m), 7.29 (1H, s), 7.45-7.53 (1H, m), 8.17 (1H, br s), 8.21 (1H, d, J = 3.0 Hz), 8.27 (1H, t, J = 8.8 Hz), 8.66 (1H, s), 9.17 (1H, s). MS m/z: 575 [M+H]+. | Reference Example Z3 / Pyrrolidine |

[Example 27] 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one

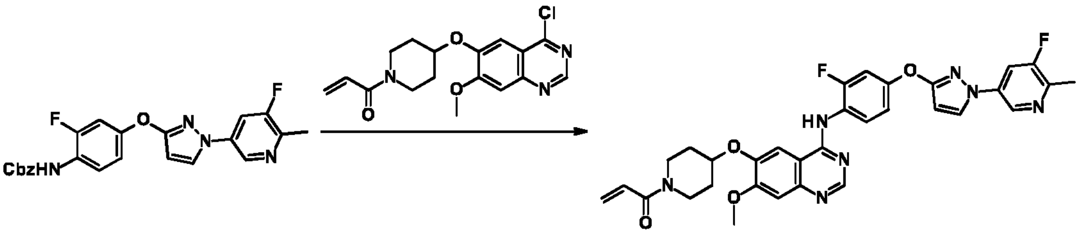

To a suspension of benzyl (2-fluoro-4-{[1-(5-fluoro-6-methylpyridin-3-yl)-1H-pyrazol-3-yl]oxy}phenyl)carbamate (86.0 mg, 0.197 mmol) in ethanol (4 mL), 10% palladium on carbon (M) Wet (43.0 mg) was added, and the mixture was stirred at room temperature for 2 hours under a hydrogen atmosphere at normal pressure. The insoluble materials were filtered off, and the reaction mixture was eluted with ethanol. The eluted solution was concentrated under reduced pressure. To the obtained residue, 1-{4-[(4-chloro-7-methoxyquinazolin-6-yl)oxy]piperidin-1-yl}prop-2-en-1-one (74.0 mg, 0.213 mmol) and 2-propanol (8.0 mL) were added. To the reaction mixture, a mixed solution (0.0754 mL, 0.197 mmol) of trifluoroacetic acid/2-propanol (1:4) was added, and the mixture was stirred at room temperature overnight. The reaction mixture was diluted with saturated aqueous sodium bicarbonate solution and extracted with chloroform. The organic layer was washed with saturated saline, and dried over anhydrous sodium sulfate. The insoluble materials were filtered off and then the filtrate was concentrated under reduced pressure. The obtained residue was purified by silica gel column chromatography (chloroform/methanol) to obtain the title compound (102 mg, 0.167 mmol, yield 84.6%). $^1$H-NMR (DMSO-$D_6$) δ: 1.66-1.79 (2H, m), 1.96-2.08 (2H, m), 2.46-2.48 (3H, m), 3.43-3.54 (2H, m), 3.82-3.92 (2H, m), 3.94 (3H, s), 4.71-4.80 (1H, m), 5.66 (1H, dd, J = 10.4, 2.4 Hz), 6.09 (1H, dd, J = 17.1, 2.4 Hz), 6.34 (1H, d, J = 2.4 Hz), 6.80 (1H, dd, J = 17.1, 10.4 Hz), 7.09-7.15 (1H, m), 7.19-7.27 (2H, m), 7.55 (1H, t, J = 8.9 Hz), 7.90 (1H, s), 8.00-8.08 (1H, m), 8.34 (1H, s), 8.56 (1H, d, J = 2.4 Hz), 8.79 (1H, d, J = 1.8 Hz), 9.29 (1H, s). MS m/z: 614 $[M+H]^+$.

By a similar method, the following compounds were synthesized from the corresponding intermediate.

[Table 42]

| Example | Compound | Intermediate |
|---|---|---|
| 28 | 1-(4-{[4-(2-Fluoro-4-{[1-(6-methylpyridin-3-yl)-1 H-pyrazol-3-yl]oxy}anilino)-7-methoxyquinazolin-6-yl]oxy}piperidin-1-yl)prop-2-en-1-one 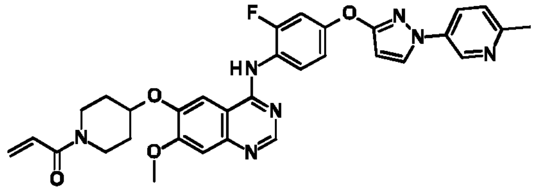 $^1$H-NMR (DMSO-D$_6$) δ: 1.65-1.79 (2H, m), 1.97-2.09 (2H, m), 2.46-2.52 (3H, m), 3.43-3.55 (2H, m), 3.82-3.91 (2H, m), 3.94 (3H, s), 4.70-4.80 (1H, m), 5.63-5.69 (1H, m), 6.09 (1H, dd, J = 16.6, 2.5 Hz), 6.29-6.32 (1H, m), 6.75-6.85 (1H, m), 7.07-7.13 (1H, m), 7.19-7.25 (2H, m), 7.37 (1H, d, J = 8.0 Hz), 7.54 (1H, t, J = 8.9 Hz), 7.90 (1H, s), 8.03 (1H, dd, J = 8.6, 2.5 Hz), 8.34 (1H, s), 8.47-8.52 (1H, m), 8.88 (1H, d, J = 3.1 Hz), 9.29 (1H, s). MS m/z: 596 [M+H]+. | Reference Example K3 |

[Example 29] 1-(4-{[4-(4-{[1-(2-Chloropyridin-4-yl)-1H-pyrazol-3-yl]oxy}-2-fluoroanilino)-7-methoxyquinazolin-6-yl]oxy}piperidin-1-yl)prop-2-en-1-one

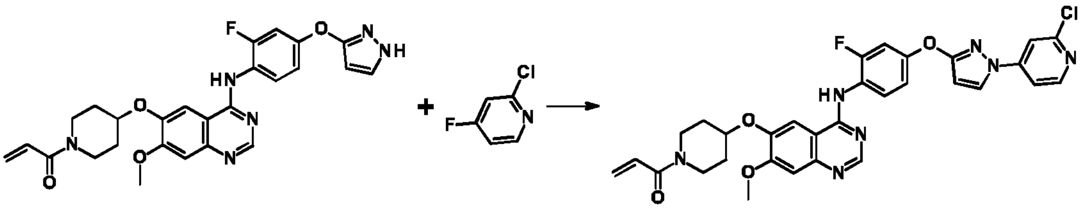

To a solution of 1-[4-({4-[2-fluoro-4-(1H-pyrazol-3-yloxy)anilino]-7-methoxyquinazolin-6-yl}oxy)piperidin-1-yl]prop-2-en-1-one (120 mg, 0.238 mmol) and 2-chloro-4-fluoropyridine (0.0282 mL, 0.285 mmol) in dimethyl sulfoxide (2 mL), cesium carbonate (234 mg, 0.719 mmol) was added at room temperature, and the reaction mixture was stirred at room temperature for 4 hours. The reaction mixture was diluted by addition of ethyl acetate, and the organic layer was washed with water and saturated saline, and dried over anhydrous sodium sulfate. The insoluble materials were filtered off and then the filtrate was concentrated under reduced pressure. The obtained residue was purified by silica gel column chromatography (chloroform/methanol) to obtain the title compound (93.3 mg, 0.152 mmol, yield 63.6%). $^1$H-NMR (DMSO-D$_6$) δ: 1.66-1.79 (2H, m), 1.96-2.08 (2H, m), 3.43-3.55 (2H, m), 3.82-3.92 (2H, m), 3.95 (3H, s), 4.71-4.80 (1H, m), 5.61-5.71 (1H, m), 6.03-6.13 (1H, m), 6.41-6.44 (1H, m), 6.80 (1H, dd, J = 16.6, 10.4 Hz), 7.11-7.32 (3H, m), 7.57 (1H, t, J = 8.6 Hz), 7.76-7.81 (1H, m), 7.86-7.93 (2H, m), 8.35 (1H, s), 8.44 (1H, d, J = 5.5 Hz), 8.70-8.75 (1H, m), 9.31 (1H, s). MS m/z: 616 $[M+H]^+$.

[Example 30] 1-(4-{[4-(2-Fluoro-4-{[1-(2-fluoropyridin-4-yl)-1H-pyrazol-3-yl]oxy}anilino)-7-methoxyquinazolin-6-yl]oxy}piperidin-1-yl)prop-2-en-1-one

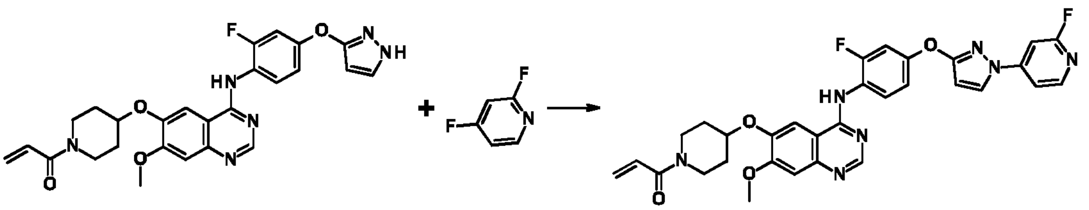

To a solution of 1-[4-(f4-[2-fluoro-4-(1H-pyrazol-3-yloxy)anilino]-7-methoxyquinazolin-6-yl}oxy)piperidin-1-yl]prop-2-en-1-one (200 mg, 0.396 mmol) and 2,4-difluoropyridine (0.054 mL, 0.60 mmol) in N,N-dimethylformamide (4.0 mL), cesium carbonate (324 mg, 0.995 mmol) was added at room temperature, and the mixture was stirred at 0°C for 2 hours. After the reaction mixture was warmed to room temperature, it was stirred overnight. The reaction mixture was diluted by addition of ethyl acetate. The insoluble materials were filtered off, and the reaction mixture was eluted with ethyl acetate. The eluted solution was washed with water and saturated saline, and the organic layer was dried over anhydrous sodium sulfate. The insoluble materials were filtered off and then the filtrate was concentrated under reduced pressure. The obtained residue was purified by silica gel column chromatography (chloroform/methanol). The roughly purified product was dissolved in ethyl acetate, diluted with hexane and solidified. The suspension was concentrated under reduced pressure and then dried under reduced pressure to obtain the title compound (121 mg, 0.201 mmol, yield 50.8%).
$^1$H-NMR (DMSO-D$_6$) δ: 1.64-1.77 (2H, m), 1.98-2.08 (2H, m), 3.42-3.55 (2H, m), 3.82-3.92 (2H, m), 3.94 (3H, s), 4.72-4.81 (1H, m), 5.67 (1H, dd, J = 10.4, 2.5 Hz), 6.10 (1H, dd, J = 16.6, 2.5 Hz), 6.45 (1H, d, J = 2.5 Hz), 6.82 (1H, dd, J = 16.6, 10.4 Hz), 7.16 (1H, dd, J = 8.6, 2.5 Hz), 7.22 (1H, s), 7.29 (1H, dd, J = 11.0, 2.5 Hz), 7.49-7.60 (2H, m), 7.74 (1H, d, J = 5.5 Hz), 7.90 (1H, s), 8.29 (1H, d, J = 6.1 Hz), 8.35 (1H, s), 8.74 (1H, d, J = 2.5 Hz), 9.35 (1H, br s). MS m/z: 600 [M+H]$^+$.

[Example 31] 1-{[4-{[4-(4-{[1-(6-Cyclopropylpyridin-3-yl)-1H-pyrazol-3-yl]oxy}-2-fluoroanilino)-7-methoxyquinazolin-6-yl]oxy}piperidin-1-yl)prop-2-en-1-one

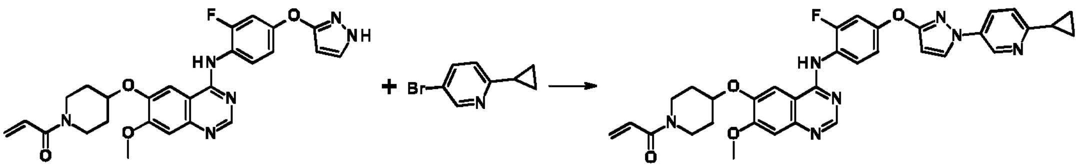

A suspension of 1-[4-({4-[2-fluoro-4-(1H-pyrazol-3-yloxy)anilino]-7-methoxyquinazolin-6-yl}oxy)piperidin-1-yl]prop-2-en-1-one (200 mg, 0.392 mmol), copper(I) iodide (37.7 mg, 0.198 mmol), 3-bromo-6-(cyclopropyl)pyridine (122 mg, 0.616 mmol), N,N-dimethylglycine (46.1 mg, 0.447 mmol) and potassium carbonate (115 mg, 0.835 mmol) in dimethyl sulfoxide (2.7 mL) was stirred at 130°C under microwave irradiation for 1 hour. The reaction mixture was diluted by addition of ethyl acetate, passed through a pad of Celite, and eluted with ethyl acetate. The eluted solution was washed with water (x 2) and saturated saline, and the organic layer was dried over anhydrous sodium sulfate. The insoluble materials were filtered off and then the filtrate was concentrated under reduced pressure. The obtained residue was purified by amino silica gel column chromatography (ethyl acetate/methanol and chloroform/methanol) to obtain the title compound (65.2 mg, 0.105 mmol, yield 26.5%).
$^1$H-NMR (DMSO-D$_6$) δ: 0.92-1.00 (4H, m), 1.64-1.79 (2H, m), 1.97-2.08 (2H, m), 2.11-2.20 (1H, m), 3.43-3.54 (2H, m), 3.81-3.91 (2H, m), 3.94 (3H, s), 4.71-4.79 (1H, m), 5.66 (1H, dd, J = 10.4, 2.5 Hz), 6.09 (1H, dd, J = 16.6, 2.5 Hz), 6.29 (1H, d, J = 2.5 Hz), 6.80 (1H, dd, J = 16.6, 10.4 Hz), 7.10 (1H, dd, J = 9.2, 2.5 Hz), 7.18-7.24 (2H, m), 7.39 (1H, d, J = 8.6 Hz), 7.53 (1H, t, J = 8.6 Hz), 7.90 (1H, s), 7.99 (1H, dd, J = 8.3, 2.8 Hz), 8.34 (1H, s), 8.44-8.48 (1H, m), 8.82 (1H, d, J = 2.5 Hz), 9.29 (1H, s). MS m/z: 622 [M+H]$^+$.

By a similar method, the following compounds were synthesized from the corresponding bromo compounds.

[Table 43]

| Example | Compound | Bromo compoun d |
|---|---|---|
| 32 | 1-(4-{[4-(2-Fluoro-4-{[1-(6-fluoropyridin-3-yl)-1 H-pyrazol-3-yl]oxy}anilino)-7-methoxyquinazolin-6-yl]oxy}piperidin-1-yl)prop-2-en-1-one 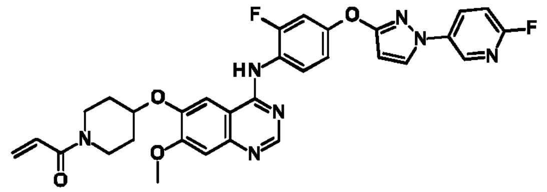 $^1$H-NMR (DMSO-$D_6$) δ: 1.65-1.79 (2H, m), 1.97-2.08 (2H, m), 3.42-3.54 (2H, m), 3.81-3.92 (2H, m), 3.94 (3H, s), 4.71-4.79 (1H, m), 5.66 (1H, dd, J = 10.4, 2.5 Hz), 6.09 (1H, dd, J = 16.6, 2.5 Hz), 6.34 (1H, d, J = 2.5 Hz), 6.80 (1H, dd, J = 16.6, 10.4 Hz), 7.09-7.14 (1H, m), 7.20-7.26 (2H, m), 7.32 (1H, dd, J = 8.6, 3.1 Hz), 7.54 (1H, t, J = 8.9 Hz), 7.90 (1H, s), 8.31-8.40 (2H, m), 8.53 (1H, d, J = 2.5 Hz), 8.63-8.68 (1H, m), 9.30 (1H, s). MS m/z: 600 [M+H]+. | 5-Bromo-2-fluoropyridine |

[Example 33] 1-[4-({4-[2-Fluoro-4-({4-[5-(propan-2-yl)-1,3,4-oxadiazol-2-yl]-1,3-thiazol-2-yl}oxy)anilino]-7-methoxyquinazolin-6-yl}oxy)piperidin-1-yl]prop-2-en-1-one

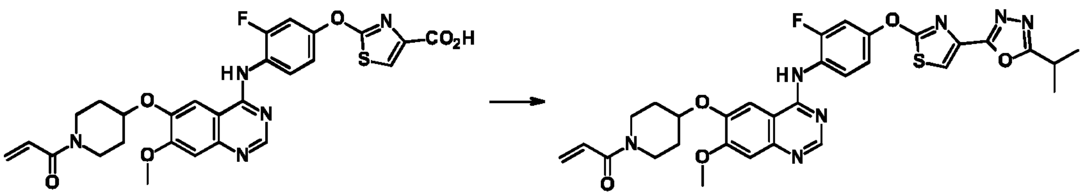

To a solution of 2-{3-fluoro-4-[(7-methoxy-6-{[1-(prop-2-enoyl)piperidin-4-yl]oxy}quinazolin-4-yl)amino]phenoxy}-1,3-thiazole-4-carboxylic acid (151 mg, 0.266 mmol), isobutyrohydrazide (57.1 mg, 0.559 mmol) and triethylamine (0.11 mL, 0.79 mmol) in N,N-dimethylformamide (1.0 mL), 1-[bis(dimethylamino)methylene]-1H-1,2,3-triazolo[4,5-b]pyridinium 3-oxide hexafluorophosphate (157 mg, 0.413 mmol) was added at room temperature, and the mixture was stirred at the same temperature overnight. The reaction mixture was diluted by addition of saturated saline, and sonicated to form a slurry, and then the slurry was filtered. The obtained solid was washed with water and hexane, and dried under reduced pressure. To the obtained solid, triethylamine (0.11 mL, 1.3 mmol), p-toluenesulfonyl chloride (102 mg, 0.535 mmol) and chloroform (10 mL) were added, and the mixture was stirred at room temperature overnight. The reaction solution was purified by silica gel column chromatography (chloroform/methanol) and amino silica gel column chromatography (chloroform/methanol) to obtain the title compound (47.0 mg, 0.0744 mmol, yield 27.9%).
$^1$H-NMR (DMSO-$D_6$) δ: 1.35 (6H, d, J = 7.4 Hz), 1.65-1.79 (2H, m), 1.95-2.09 (2H, m), 3.22-3.33 (1H, m), 3.44-3.55 (2H, m), 3.82-3.92 (2H, m), 3.95 (3H, s), 4.72-4.80 (1H, m), 5.66 (1H, dd, J = 10.4, 2.5 Hz), 6.09 (1H, dd, J = 16.9, 2.5 Hz), 6.80 (1H, dd, J = 16.9, 10.4 Hz), 7.23 (1H, s), 7.31-7.37 (1H, m), 7.50-7.58 (1H, m), 7.69 (1H, t, J = 8.6 Hz), 7.91 (1H, s), 8.06 (1H, s), 8.38 (1H, s), 9.38 (1H, s). MS m/z: 632 $[M+H]^+$.

[Example 34] 1-(4-{[4-(4-{[4-(6-Ethylpyridin-3-yl)-1,3-thiazol-2-yl]oxy}-2-fluoroanilino)-7-methoxyquinazolin-6-yl]oxy}piperidin-1-yl)prop-2-en-1-one

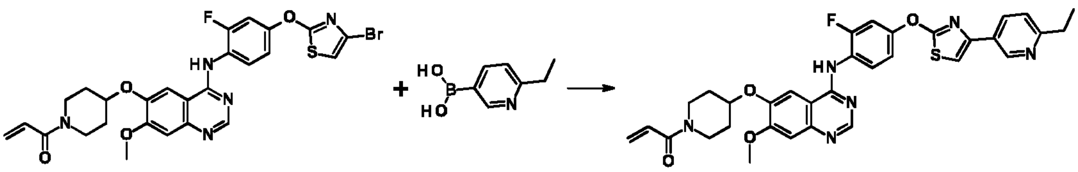

A suspension of 1-{4-[(4-{4-[(4-bromo-1,3-thiazol-2-yl)oxy]-2-fluoroanilino}-7-methoxyquinazolin-6-yl)oxy]piperidin-1-yl}prop-2-en-1-one (99.9 mg, 0.166 mmol), 2-ethylpyridine-5-boronic acid (50.9 mg, 0.337 mmol), cesium carbonate (170 mg, 0.523 mmol) and [1,1'-bis(diphenylphosphino)ferrocene]palladium(II) dichloride dichloromethane adduct (15.2 mg, 0.0186 mmol) in water (0.150 mL) and 1,2-dimethoxyethane (3.0 mL) was stirred at 120°C under microwave irradiation for 1 hour. The reaction mixture was diluted with ethyl acetate, the insoluble materials were filtered, and the reaction mixture was eluted with ethyl acetate. The eluted solution was concentrated under reduced pressure. The obtained residue was purified by amino silica gel column chromatography (ethyl acetate/methanol) to obtain the title compound (52.5 mg, 0.0838 mmol, yield 50.4%).

$^1$H-NMR (DMSO-D$_6$) δ: 1.25 (3H, t, J = 7.4 Hz), 1.67-1.79 (2H, m), 1.97-2.08 (2H, m), 2.78 (2H, q, J = 7.4 Hz), 3.42-3.55 (2H, m), 3.82-3.92 (2H, m), 3.95 (3H, s), 4.72-4.81 (1H, m), 5.66 (1H, dd, J = 10.7, 2.5 Hz), 6.09 (1H, dd, J = 16.6, 2.5 Hz), 6.80 (1H, dd, J = 16.6, 10.7 Hz), 7.23 (1H, s), 7.28-7.40 (2H, m), 7.50-7.57 (1H, m), 7.63-7.75 (2H, m), 7.91 (1H, s), 8.08 (1H, dd, J = 8.0, 2.5 Hz), 8.38 (1H, s), 8.94 (1H, d, J = 2.5 Hz), 9.37 (1H, s). MS m/z: 627 [M+H]$^+$.

By a similar method, the following compounds were synthesized from the corresponding boronic acids.

[Table 44]

| Example | Compound | Boronic acid |
|---|---|---|
| 35 | 1-(4-{[4-(2-Fluoro-4-{[4-(6-methoxypyridin-3-yl)-1,3-thiazol-2-yl]oxy}anilino)-7-methoxyquinazolin-6-yl]oxy}piperidin-1-yl)prop-2-en-1-one 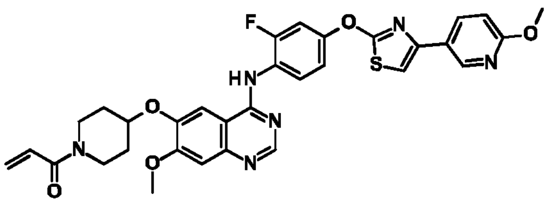 $^1$H-NMR (DMSO-D$_6$) δ: 1.67-1.79 (2H, m), 1.96-2.10 (2H, m), 3.43-3.55 (2H, m), 3.82-3.92 (5H, m), 3.95 (3H, s), 4.71-4.81 (1H, m), 5.66 (1H, dd, J = 10.4, 2.5 Hz), 6.09 (1H, dd, J = 16.6, 2.5 Hz), 6.80 (1H, dd, J = 16.6, 10.4 Hz), 6.87 (1H, d, J = 8.6 Hz), 7.23 (1H, s), 7.33-7.38 (1H, m), 7.49-7.56 (1H, m), 7.61 (1H, s), 7.67 (1H, t, J = 8.9 Hz), 7.91 (1H, s), 8.10 (1H, dd, J = 9.2, 2.5 Hz), 8.38 (1H, s), 8.64 (1H, d, J = 1.8 Hz), 9.37 (1H, s). MS m/z: 629 [M+H]+. | 2-Methoxypyridine-5-boronic acid |
| 36 | 1-(4-{[4-(2-Fluoro-4-{[4-(2-methylpyridin-5-yl)-1,3-thiazol-2-yl]oxy}anilino)-7-methoxyquinazolin-6-yl]oxy}piperidin-1-yl)prop-2-en-1-one 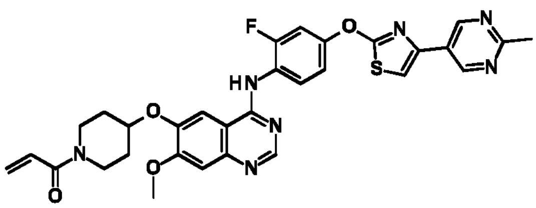 $^1$H-NMR (DMSO-D$_6$) δ: 1.63-1.81 (2H, m), 1.96-2.11 (2H, m), 2.65 (3H, s), 3.41-3.56 (2H, m), 3.81-4.01 (5H, m), 4.71-4.82 (1H, m), 5.62-5.73 (1H, m), 6.05-6.17 (1H, m), 6.80 (1H, dd, J = 16.9, 10.7 Hz), 7.23 (1H, s), 7.33-7.41 (1H, m), 7.50-7.60 (1H, m), 7.63-7.75 (1H, m), 7.85-7.97 (2H, m), 8.38 (1H, s), 9.03-9.14 (2H, m), 9.37 (1H, s). MS m/z: 614 [M+H]+. | (2-Methylpyrimidin-5-yl)boronic acid |

[Example 37] 1-(4-{[4-(2-Fluoro-4-{[4-(6-methylpyridin-3-yl)-1,3-thiazol-2-yl]oxy}anilino)-7-methoxyquinazolin-6-yl]oxy}piperidin-1-yl)prop-2-en-1-one

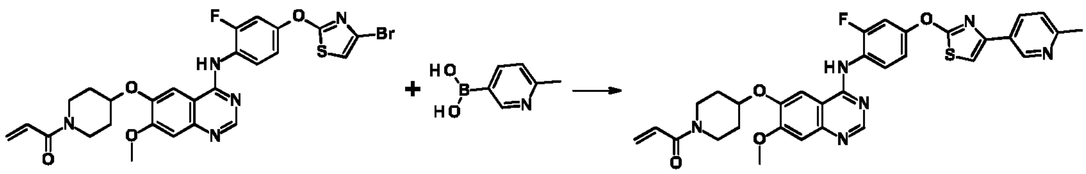

A suspension of 1-{4-[(4-{4-[(4-bromo-1,3-thiazol-2-yl)oxy]-2-fluoroanilino}-7-methoxyquinazolin-6-yl)oxy]piperidin-1-yl}prop-2-en-1-one (101 mg, 0.168 mmol), 2-methyl-5-pyridinylboronic acid (49.9 mg, 0.364 mmol), cesium carbonate (115 mg, 0.354 mmol) and tetrakis(triphenylphosphine)palladium(0) (12.4 mg, 0.0107 mmol) in water (0.050 mL) and 1,2-dimethoxyethane (1.7 mL) was stirred at 160°C under microwave irradiation for 30 minutes. The reaction mixture was diluted with tetrahydrofuran. The insoluble materials were filtered, and the reaction mixture was eluted with tetrahydrofuran. The eluted solution was concentrated under reduced pressure, and the obtained residue was purified by silica gel column chromatography (chloroform/methanol). The roughly purified product was purified by preparative thin layer chromatography (chloroform/methanol) to obtain the title compound (54.1 mg, 0.0883 mmol, yield 52.7%).
$^1$H-NMR (DMSO-$D_6$) δ: 1.67-1.79 (2H, m), 1.97-2.08 (2H, m), 2.44-2.56 (3H, m), 3.44-3.55 (2H, m), 3.82-3.92 (2H, m), 3.95 (3H, s), 4.72-4.81 (1H, m), 5.66 (1H, dd, J = 10.4, 2.1 Hz), 6.09 (1H, dd, J = 16.6, 2.1 Hz), 6.80 (1H, dd, J = 16.6, 10.4 Hz), 7.23 (1H, s), 7.27-7.39 (2H, m), 7.50-7.57 (1H, m), 7.68 (1H, t, J = 8.6 Hz), 7.73 (1H, s), 7.91 (1H, s), 8.06 (1H, dd, J = 8.0, 2.5 Hz), 8.38 (1H, s), 8.91 (1H, d, J = 1.8 Hz), 9.37 (1H, s). MS m/z: 613 $[M+H]^+$.

By a similar method, the following compounds were synthesized from the corresponding boronic acid.

[Table 45]

| Example | Compound | Boronic acid |
|---|---|---|
| 38 | 1-(4-{[4-(2-Fluoro-4-{[4-(5-fluoropyridin-3-yl)-1,3-thiazol-2-yl]oxy}anilino)-7-methoxyquinazolin-6-yl]oxy}piperidin-1-yl)prop-2-en-1-one 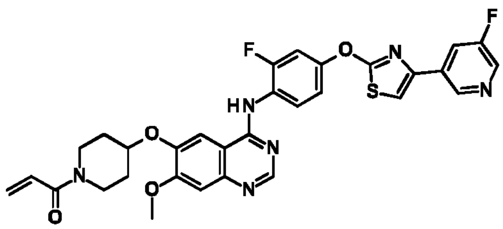 $^1$H-NMR (DMSO-$D_6$) δ: 1.66-1.78 (2H, m), 1.98-2.08 (2H, m), 3.44-3.55 (2H, m), 3.82-3.91 (2H, m), 3.95 (3H, s), 4.73-4.81 (1H, m), 5.62-5.70 (1H, m), 6.05-6.13 (1H, m), 6.80 (1H, dd, J = 16.6, 10.4 Hz), 7.23 (1H, s), 7.37 (1H, d, J = 7.4 Hz), 7.55 (1H, d, J = 11.0 Hz), 7.69 (1H, t, J = 8.6 Hz), 7.87-7.97 (2H, m), 8.07 (1H, d, J = 11.7 Hz), 8.38 (1H, s), 8.51-8.54 (1H, m), 8.94 (1H, s), 9.38 (1H, s). MS m/z: 617 [M+H]+. | (5-Fluoropyridin-3-yl)boronic acid |

[Example 39] (2E)-4-(Dimethylamino)-N-(4-{2-fluoro-4-[3-(5-methyl-1,3,4-oxadiazol-2-yl)phenoxy]anilino}-7-methoxyquinazolin-6-yl)but-2-enamide

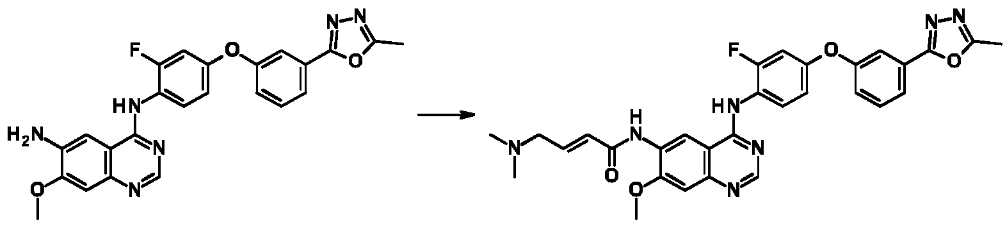

(2E)-4-(Dimethylamino)but-2-enoic acid hydrochloride (101 mg, 0.611 mmol) was dissolved in acetonitrile (2 mL), and oxalyl chloride (52 μL, 0.61 mmol) and N,N-dimethylformamide (1 drop) were added thereto. The mixture was stirred at 50°C for 20 minutes, and a solution of $N^{4-}${2-fluoro-4-[3-(5-methyl-1,3,4-oxadiazol-2-yl)phenoxy]phenyl}-7-methoxyquinazolin-4,6-diamine (140 mg, 0.305 mmol) in N,N-dimethylacetamide (3 mL) was added at 0°C. The mixture was stirred at room temperature for 3 hours, then diluted with dichloromethane, and washed with an aqueous potassium carbonate solution. The organic layer was dried over anhydrous sodium sulfate, and concentrated, and then purified by silica gel column chromatography (dichloromethane/methanol) to obtain the title compound (89 mg, 0.16 mmol, yield 51%).

$^1$H-NMR (DMSO-$D_6$) δ: 2.19 (6H, s), 2.58 (3H, s), 3.08 (2H, d, J = 5.5 Hz), 4.02 (3H, s), 6.59 (1H, d, J = 15.3 Hz), 6.79 (1H, dt, J = 15.3, 5.5 Hz), 6.97-7.00 (1H, m), 7.15-7.18 (1H, m), 7.27 (1H, s), 7.37-7.40 (1H, m), 7.53 (1H, t, J = 8.9 Hz), 7.58 (1H, s), 7.67 (1H, t, J = 7.9 Hz), 7.79 (1H, d, J = 7.9 Hz), 8.40 (1H, s), 8.93 (1H, s), 9.68 (1H, s), 9.71 (1H, s). MS m/z: 570 $[M+H]^+$.

By a similar method, the following compounds were synthesized from the corresponding intermediates.

[Table 46-1]

| Example | Compound | Intermediate |
|---|---|---|
| 40 | (2E)-4-(Dimethylamino)-N-(7-ethoxy-4-{2-fluoro-4-[3-(5-methyl-1,3,4-oxadiazol-2-yl) phenoxy]anilino}quinazolin-6-yl)but-2-enamide 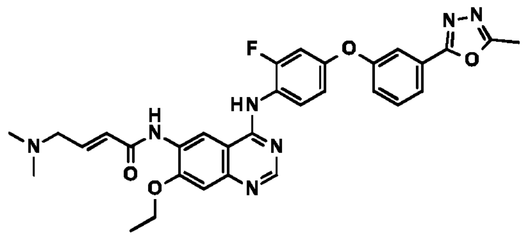 $^1$H-NMR (DMSO-$D_6$) δ: 1.47 (3H, t, J = 6.9 Hz), 2.19 (6H, s), 2.58 (3H, s), 3.09 (2H, d, J = 6.1 Hz), 4.29 (2H, q, J = 6.9 Hz), 6.60 (1H, d, J = 15.3 Hz), 6.78-6.81 (1H, m), 7.00-6.97 (1H, m), 7.16 (1H, dd, J = 11.3, 2.7 Hz), 7.25 (1H, s), 7.38 (1H, dd, J = 7.6, 2.1 Hz), 7.52 (1H, t, J = 8.5 Hz), 7.56-7.59 (1H, m), 7.67 (1H, t, J = 7.9 Hz), 7.79 (1H, d, J = 7.9 Hz), 8.39 (1H, s), 8.92 (1H, s), 9.51 (1H, s), 9.70 (1H, s). MS m/z: 584 [M+H]+. | Reference Example X3 |
| 41 | (2E)-4-(Dimethylamino)-N-(4-{4-[3-(5-ethyl-1,3,4-oxadiazol-2-yl)phenoxy]-2-fluoroanilino}-7-methoxyquinazolin-6-yl)but-2-enamide 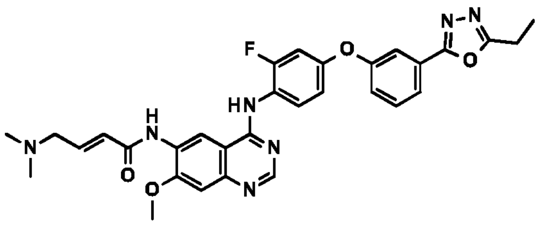 $^1$H-NMR (DMSO-$D_6$) δ: 1.32 (3H, t, J = 7.5 Hz), 2.18 (6H, s), 2.95 (2H, q, J = 7.5 Hz), 3.08 (2H, d, J = 6.1 Hz), 4.02 (3H, s), 6.60 (1H, d, J = 15.3 Hz), 6.79 (1H, dt, J = 15.3, 6.1 Hz), 6.98 (1H, dd, J = 8.5, 2.4 Hz), 7.16 (1H, dd, J = 11.0, 2.4 Hz), 7.27 (1H, s), 7.36-7.39 (1H, m), 7.52 (1H, t, J = 8.5 Hz), 7.60-7.61 (1H, m), 7.67 (1H, t, J = 7.9 Hz), 7.79-7.81 (1H, m), 8.40 (1H, s), 8.93 (1H, s), 9.68 (1H, s), 9.71 (1H, s). MS m/z: 584 [M+H]+. | Reference Example X4 |

[Table 46-2]

| Example | Compound | Intermediate |
|---|---|---|
| 42 | (2E)-4-(Dimethylamino)-N-(7-ethoxy-4-{4-[3-(5-ethyl-1,3,4-oxadiazol-2-yl)phenoxy]-2-fluoroanilino}quinazolin-6-yl)but-2-enamide 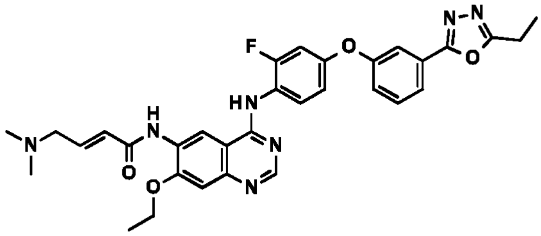 $^1$H-NMR (DMSO-$D_6$) δ: 1.32 (3H, t, J = 7.5 Hz), 1.47 (3H, t, J = 6.7 Hz), 2.19 (6H, s), 2.95 (2H, q, J = 7.5 Hz), 3.09 (2H, d, J = 6.1 Hz), 4.29 (2H, q, J = 6.7 Hz), 6.60 (1H, d, J = 15.3 Hz), 6.80 (1H, dt, J = 15.3, 6.1 Hz), 6.97 (1H, dd, J = 8.5, 1.8 Hz), 7.16 (1H, dd, J = 11.0, 2.4 Hz), 7.25 (1H, s), 7.38 (1H, dd, J = 7.3, 2.4 Hz), 7.52 (1H, t, J = 8.5 Hz), 7.61-7.60 (1H, m), 7.67 (1H, t, J = 8.2 Hz), 7.80 (1H, d, J = 7.9 Hz), 8.39 (1H, s), 8.92 (1H, s), 9.51 (1H, s), 9.70 (1H, s). MS m/z: 598 [M+H]+. | Reference Example X5 |
| 43 | (2E)-4-(Dimethylamino)-N-[4-(2-fluoro-4-{3-[5-(propan-2-yl)-1,3,4-oxadiazol-2-yl] phenoxy}anilino)-7-methoxyquinazolin-6-yl]but-2-enamide 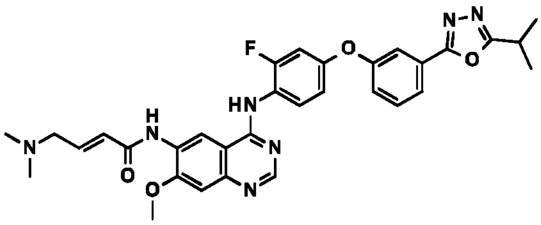 $^1$H-NMR ($CDCl_3$) δ: 1.46 (6H, d, J = 6.7 Hz), 2.35 (6H, s), 3.16-3.35 (3H, m), 4.07 (3H, s), 6.27 (1H, d, J = 15.8 Hz), 6.86-6.96 (2H, m), 6.98-7.09 (1H, m), 7.19-7.24 (1H, m), 7.28 (1H, s), 7.42-7.55 (2H, m), 7.70-7.75 (1H, m), 7.86 (1H, d, J = 7.3 Hz), 8.13-8.34 (2H, m), 8.66 (1H, s), 9.17 (1H, s). MS m/z: 598 [M+H]+. | Reference Example X6 |

[Table 46-3]

| Example | Compound | Intermediate |
|---|---|---|
| 44 | (2E)-4-(Dimethylamino)-N-(4-{2-fluoro-4-[3-(5-methyl-1,3,4-thiadiazol-2-yl)phenoxy] anilino}-7-methoxyquinazolin-6-yl)but-2-enamide 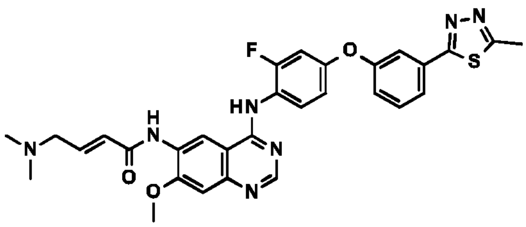 $^1$H-NMR ($CDCl_3$) δ: 2.48 (6H, s), 2.83 (3H, s), 3.31-3.43 (2H, m), 4.07 (3H, s), 6.35-6.46 (1H, m), 6.88-6.95 (2H, m), 6.98-7.09 (1H, m), 7.15-7.20 (1H, m), 7.29 (1H, s), 7.42-7.54 (2H, m), 7.63-7.66 (1H, m), 7.68-7.73 (1H, m), 8.21-8.31 (2H, m), 8.67 (1H, s), 9.16 (1H, s). MS m/z: 586 [M+H]+. | Reference Example X7 |

(continued)

| Example | Compound | Intermediate |
|---|---|---|
| 45 | (2E)-N-{4-[4-({1-[2-(tert-Butylamino)-2-oxoethyl]-4-fluoro-1 H-pyrazol-3-yl}oxy)-2-chloroanilino]-7-methoxyquinazolin-6-yl}-4-(dimethylamino)but-2-enamide 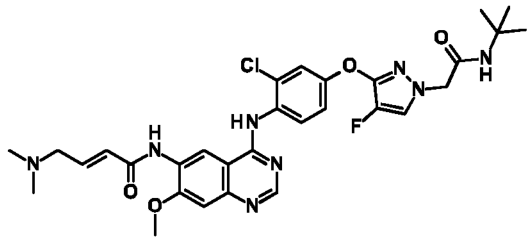 $^1H$-NMR (DMSO-$D_6$) δ: 1.27 (9H, s), 2.79 (6H, s), 3.89-4.11 (5H, m), 4.60 (2H, s), 6.71-6.96 (2H, m), 7.13-7.21 (1H, m), 7.31-7.38 (2H, m), 7.50-7.57 (1H, m), 7.82-7.87 (1H, m), 7.96-8.02 (1H, m), 8.62 (1H, s), 9.04-9.10 (1H, m), 10.06-10.15 (1H, m), 10.40-10.59 (1H, m). MS m/z: 625 [M+H]+. | Reference Example X8 |

[Table 46-4]

| Example | Compound | Intermediate |
|---|---|---|
| 46 | (2E)-N-{4-[4-({1-[2-(tert-Butylamino)-2-oxoethyl]-4-fluoro-1 H-pyrazol-3-yl}oxy)-2-fluoroanilino]-7-methoxyquinazolin-6-yl}-4-(dimethylamino)but-2-enamide 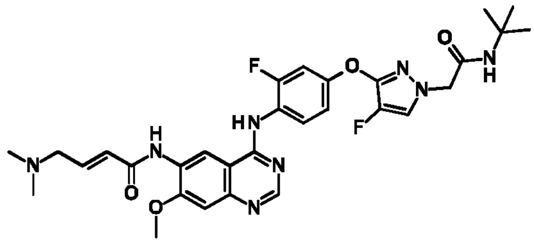 $^1H$-NMR ($CDCl_3$) δ: 1.33 (9H, s), 2.33 (6H, s), 3.16-3.20 (2H, m), 4.07 (3H, s), 4.50 (2H, s), 5.96 (1H, br s), 6.25 (1H, d, J = 15.2 Hz), 6.97-7.08 (3H, m), 7.28 (1H, s), 7.40 (1H, d, J = 4.3 Hz), 7.48 (1H, br s), 8.17 (1H, br s), 8.21-8.28 (1H, m), 8.64 (1H, s), 9.15 (1H, s). MS m/z: 609 [M+H]+. | Reference Example X10 |
| 47 | N-tert-Butyl-2-{4-[(6-{[(2E)-4-(dimethylamino)but-2-enoyl]amino}-7-methoxyquinazolin-4-yl)amino]-3-fluorophenoxy}-1,3-thiazole-4-carboxamide 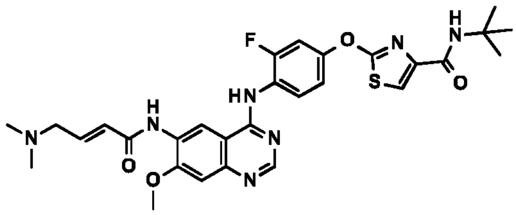 $^1H$-NMR ($CDCl_3$) δ: 1.45 (9H, s), 2.32 (6H, s), 3.18 (2H, dd, J = 5.5, 1.2 Hz), 4.08 (3H, s), 6.25 (1H, d, J = 15.3 Hz), 6.95 (1H, s), 7.00-7.08 (1H, m), 7.15-7.23 (2H, m), 7.30 (1H, s), 7.62 (1H, s), 7.65 (1H, s), 8.17 (1H, s), 8.49 (1H, t, J = 8.9 Hz), 8.69 (1H, s), 9.19 (1H, s). MS m/z: 594 [M+H]+. | Reference Example X11 |

[Example 48] (2E)-N-(4-{2-Chloro-4-[(4-fluoro-1-{2-[(1-fluoro-2-methylpropan-2-yl)amino]-2-oxoethyl}-1H-pyrazol-3-yl)oxy]anilino}-7-methoxyquinazolin-6-yl)-4-(dimethylamino)but-2-enamide

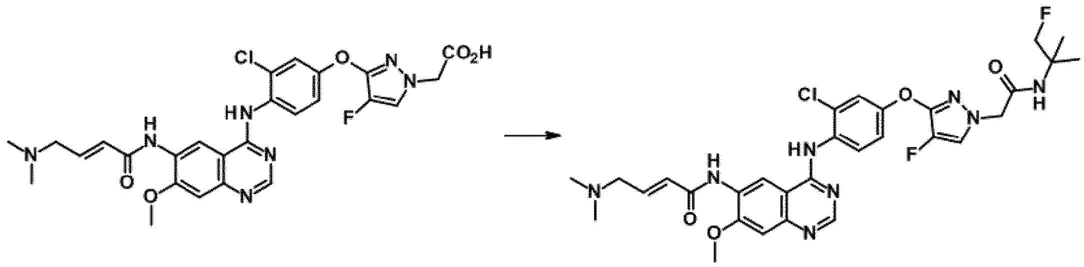

To a solution of (3-{3-chloro-4-[(6-{[(2E)-4-(dimethylamino)but-2-enoyl]amino}-7-methoxyquinazolin-4-yl)amino]phenoxy}-4-fluoro-1H-pyrazol-1-yl)acetic acid (45.8 mg, 0.0804 mmol), N,N-diisopropylethylamine (0.055 mL, 0.32 mmol) and 1-fluoro-2-methylpropan-2-amine hydrochloride (12.9 mg, 0.101 mmol) in N,N-dimethylformamide (0.877 mL), 1-[bis(dimethylamino)methylene]-1H-1,2,3-triazolo[4,5-b]pyridinium 3-oxide hexafluorophosphate (50.1 mg, 0.132 mmol) was added, and the mixture was stirred at room temperature for 3 hours. The reaction solution was purified by silica gel column chromatography (methanol dichloromethane/methanol) and amine-modified silica gel column chromatography (ethyl acetate/methanol). The obtained solid was washed with hexane and then dried under reduced pressure at 60°C to obtain the title compound (22.7 mg, 0.0353 mmol, yield 43.9%).
$^1$H-NMR (DMSO-$D_6$) δ: 1.21-1.28 (6H, m), 2.20 (6H, s), 3.05-3.15 (2H, m), 4.01 (3H, s), 4.44 (2H, d, J = 47.8 Hz), 4.65 (2H, s), 6.52-6.64 (1H, m), 6.71-6.86 (1), 7.06-7.16 (1H, m), 7.23-7.32 (2H, m), 7.44-7.52 (1H, m), 7.93-8.07 (2H, m), 8.33 (1H, s), 8.91 (1H, s), 9.62-9.76 (2H, m). MS m/z: 643 $[M+H]^+$.

[Example 49] (2E)-N-[4-(4-{3-[2-(tert-Butylamino)-2-oxoethyl]phenoxy}-2-fluoroanilino)-7-methoxyquinazolin-6-yl]-4-(morpholin-4-yl)but-2-enamide

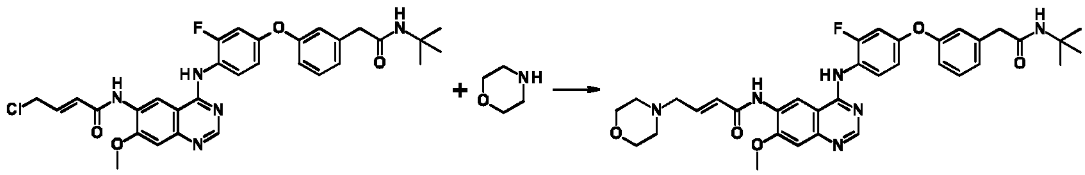

To a solution of (2E)-N-[4-(4-{3-[2-(tert-butylamino)-2-oxoethyl]phenoxy}-2-fluoroanilino)-7-methoxyquinazolin-6-yl]-4-chlorobut-2-enamide (47.5 mg, 0.0802 mmol) in N,N-dimethylformamide (1 mL), morpholine (0.0076 mL, 0.088 mmol) was added, and the mixture was stirred at room temperature for 3 hours. To the reaction solution, morpholine (0.0076 mL, 0.088 mmol) was added, and the mixture was stirred at room temperature for 19 hours. The reaction solution was diluted with ethyl acetate, and washed with water and saturated saline sequentially. The organic layer was dried over anhydrous sodium sulfate, filtered, and then concentrated under reduced pressure. The residue was purified by amino silica gel column chromatography (ethyl acetate/methanol). The roughly purified product was dissolved in ethyl acetate, and hexane was added thereto. The precipitated solid was collected by filtration and dried under reduced pressure at 60°C to obtain the title compound (41.4 mg, 0.0644 mmol, yield 80.3%).
$^1$H-NMR (DMSO-$D_6$) δ: 1.23 (9H, s), 2.36-2.44 (4H, m), 3.09-3.20 (2H, m), 3.34-3.44 (2H, m), 3.55-3.68 (4H, m), 4.01 (3H, s), 6.53-6.65 (1H, m), 6.72-6.89 (2H, m), 6.93-7.11 (4H, m), 7.26 (1H, s), 7.31-7.53 (2H, m), 7.71 (1H, s), 8.37 (1H, s), 8.90 (1H, s), 9.60-9.74 (2H, m). MS m/z: 643 $[M+H]^+$.

By a similar method, the following compounds were synthesized from the corresponding intermediates and amines.

[Table 47-1]

| Example | Compound | Intermediate / Amine |
|---|---|---|
| 50 | (2E)-N-(4-{2-Fluoro-4-[3-(5-methyl-1,3,4-oxadiazol-2-yl)phenoxy]anilino}-7-methoxyquinazolin-6-yl)-4-(pyrrolidin-1-yl)but-2-enamide 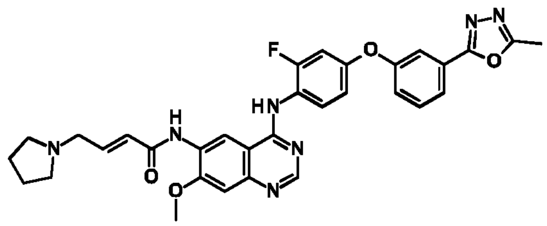 | Reference Example Y2 |
| | $^1$H-NMR (DMSO-D$_6$) δ: 1.69-1.74 (4H, m), 2.44-2.53 (4H, m), 2.58 (3H, s), 3.25 (2H, d, J = 5.5 Hz), 4.02 (3H, s), 6.60 (1H, d, J = 15.9 Hz), 6.80-6.87 (1H, m), 6.96-7.00 (1H, m), 7.14-7.19 (1H, m), 7.27 (1H, s), 7.36-7.40 (1H, m), 7.52 (1H, t, J = 8.9 Hz), 7.56-7.59 (1H, m), 7.67 (1H, t, J = 7.9 Hz), 7.79 (1H, d, J = 7.9 Hz), 8.40 (1H, s), 8.93 (1H, s), 9.67 (1H, s), 9.71 (1H, s). MS m/z: 596 [M+H]+. | Pyrrolidine |
| 51 | (2E)-N-(4-{4-[3-(5-Cyclopropyl-1,3,4-oxadiazol-2-yl)phenoxy]-2-fluoroanilino}-7-ethoxyquinazolin-6-yl)-4-(dimethylamino)but-2-enamide 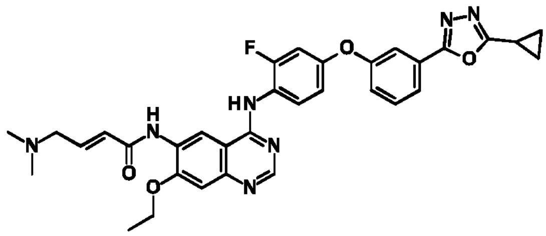 | Reference Example Y4 |
| | $^1$H-NMR (DMSO-D$_6$) δ: 1.10-1.20 (4H, m), 1.47 (3H, t, J = 7.0 Hz), 2.19 (6H, s), 2.28-2.35 (1H, m), 3.09 (2H, d, J = 6.1 Hz), 4.29 (2H, q, J = 7.0 Hz), 6.60 (1H, d, J = 15.3 Hz), 6.80 (1H, dt, J = 15.3, 6.1 Hz), 6.95-6.98 (1H, m), 7.14 (1H, dd, J = 11.0, 2.5 Hz), 7.25 (1H, s), 7.34-7.37 (1H, m), 7.51 (1H, t, J = 8.9 Hz), 7.59-7.62 (1H, m), 7.65 (1H, t, J = 8.0 Hz), 7.78 (1H, d, J = 8.0 Hz), 8.39 (1H, s), 8.92 (1H, s), 9.51 (1H, s), 9.70 (1H, s). MS m/z: 610 [M+H]+. | Dimethylamine (tetrahydrofuran solution) |

[Table 47-2]

| Example | Compound | Intermediate |
|---|---|---|
| | | Amine |
| 52 | (2E)-N-(7-Ethoxy-4-{2-fluoro-4-[3-(5-methyl-1,3,4-oxadiazol-2-yl)phenoxy] anilino}quinazolin-6-yl)-4-[(3S)-tetrahydrofuran-3-ylamino]but-2-enamide 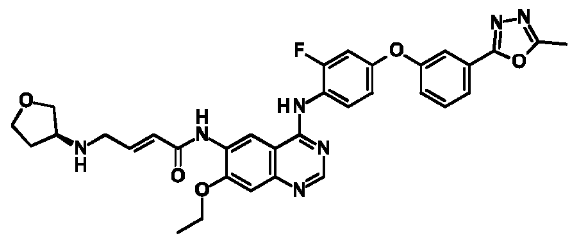 | Reference Example Y3 |
| | $^1$H-NMR (DMSO-$D_6$) $\delta$: 1.47 (3H, t, J = 6.9 Hz), 1.64-1.71 (1H, m), 1.92-2.01 (1H, m), 2.58 (3H, s), 3.29-3.37 (4H, m), 3.29-3.37 (4H, m), 3.41-3.44 (1H, m), 3.63-3.69 (1H, m), 3.71-3.80 (2H, m), 4.29 (2H, q, J = 6.9 Hz), 6.57 (1H, d, J = 15.3 Hz), 6.88 (1H, dt, J = 15,3, 5.1 Hz), 6.98 (1H, dd, J = 8.6, 1.8 Hz), 7.16 (1H, dd, J = 11.3, 2.8 Hz), 7.25 (1H, s), 7.37-7.40 (1H, m), 7.53 (1H, t, J = 8.9 Hz), 7.56-7.59 (1H, m), 7.67 (1H, t, J = 8.0 Hz), 7.79 (1H, d, J = 8.0 Hz), 8.39 (1H, s), 8.91 (1H, s), 9.47 (1H, s), 9.70 (1H, s). MS m/z: 626 [M+H]+. | (3S)-3-Aminotetrahydrofuran |
| 53 | (2E)-N-(7-Ethoxy-4-{2-fluoro-4-[3-(5-methyl-1,3,4-oxadiazol-2-yl)phenoxy] anilino}quinazolin-6-yl)-4-[(3R)-tetrahydrofuran-3-ylamino]but-2-enamide 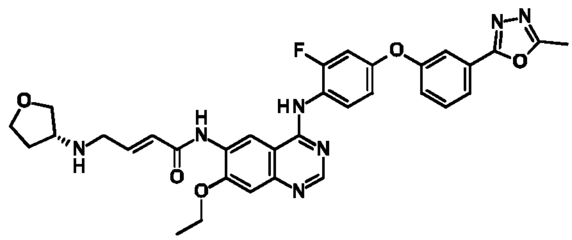 | Reference Example Y3 |
| | $^1$H-NMR (DMSO-$D_6$) $\delta$: 1.47 (3H, t, J = 6.9 Hz), 1.64-1.71 (1H, m), 1.92-2.01 (1H, m), 2.58 (3H, s), 3.29-3.37 (4H, m), 3.29-3.37 (4H, m), 3.41-3.44 (1H, m), 3.63-3.69 (1H, m), 3.71-3.80 (2H, m), 4.29 (2H, q, J = 6.9 Hz), 6.57 (1H, d, J = 15.3 Hz), 6.88 (1H, dt, J = 15,3, 5.1 Hz), 6.98 (1H, dd, J = 8.6, 1.8 Hz), 7.16 (1H, dd, J = 11.3, 2.8 Hz), 7.25 (1H, s), 7.37-7.40 (1H, m), 7.53 (1H, t, J = 8.9 Hz), 7.56-7.59 (1H, m), 7.67 (1H, t, J = 8.0 Hz), 7.79 (1H, d, J = 8.0 Hz), 8.39 (1H, s), 8.91 (1H, s), 9.47 (1H, s), 9.70 (1H, s). MS m/z: 626 [M+H]+. | (3R)-3-Aminotetrahydrofuran |

[Table 47-3]

| Example | Compound | Intermediate / Amine |
|---|---|---|
| 54 | (2E)-N-{4-[4-({1-[2-(tert-Butylamino)-2-oxoethyl]-4-fluoro-1 H-pyrazol-3-yl}oxy)-2-chloroanilino]-7-methoxyquinazolin-6-yl}-4-(morpholin-4-yl)but-2-enamide 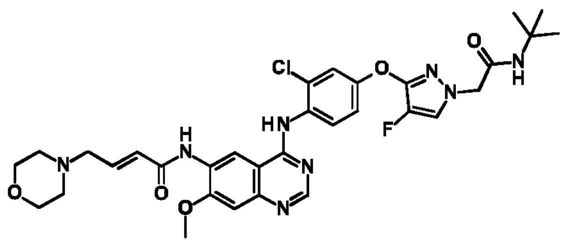 $^1$H-NMR (DMSO-$D_6$) δ: 1.27 (9H, s), 2.26-2.52 (4H, m), 3.05-3.22 (2H, m), 3.50-3.72 (4H, m), 4.01 (3H, s), 4.59 (2H, s), 6.52-6.87 (2H, m), 7.06-7.16 (1H, m), 7.21-7.33 (2H, m), 7.41-7.53 (1H, m), 7.82 (1H, s), 7.95-8.00 (1H, m), 8.35 (1H, s), 8.91 (1H, s), 9.53-9.90 (2H, m). MS m/z: 667 [M+H]+. | Reference Example Y5 / Morpholine |

[Example 55] rac-(2E)-N-{4-[4-({1-[2-(tert-Butylamino)-2-oxoethyl]-4-fluoro-1H-pyrazol-3-yl}oxy)-2-chloroanilino]-7-methoxyquinazolin-6-yl}-4-(3-fluoropyrrolidin-1-yl)but-2-enamide

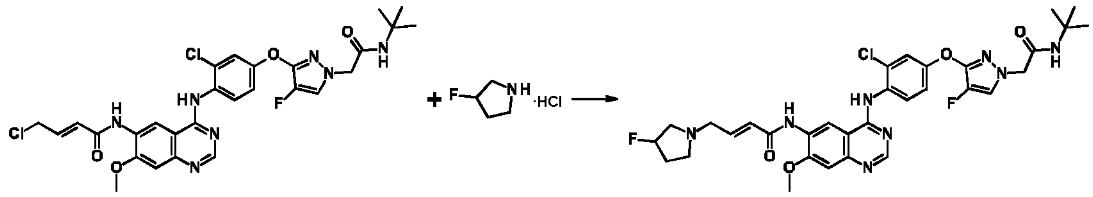

To a solution of (2E)-N-{4-[4-({1-[2-(tert-butylamino)-2-oxoethyl]-4-fluoro-1H-pyrazol-3-yl}oxy)-2-chloroanilino]-7-methoxyquinazolin-6-yl}-4-chlorobut-2-enamide (48.2 mg, 0.0782 mmol) and N,N-diisopropylethylamine (0.0244 mL, 0.143 mmol) in N,N-dimethylformamide (1 mL), 3-fluoropyrrolidine hydrochloride (20.9 mg, 0.166 mmol) was added, and the mixture was stirred at room temperature for 15 hours. The reaction solution was warmed to be at 60°C and stirred for 4 hours. The reaction solution was diluted with ethyl acetate, and washed with water and saturated saline sequentially. The organic layer was dried over anhydrous sodium sulfate, filtered, and then concentrated under reduced pressure. The residue was purified by amino silica gel column chromatography (ethyl acetate/methanol) and silica gel column chromatography (dichloromethane/methanol). The roughly purified product was dissolved in a small amount of ethyl acetate, then hexane was added thereto, and the precipitated solid was collected by filtration and dried under reduced pressure at 60°C to obtain the title compound (30.2 mg, 0.0451 mmol, yield 57.7%).
$^1$H-NMR ($CD_3OD$) δ: 1.35 (9H, s), 1.95-2.35 (2H, m), 2.50-2.62 (1H, m), 2.68-2.87 (1H, m), 2.95-3.16 (2H, m), 3.39-3.48 (2H, m), 4.09 (3H, s), 4.62 (2H, s), 5.10-5.36 (1H, m), 6.50-6.65 (1H, m), 6.97-7.07 (1H, m), 7.12-7.20 (1H, m), 7.24 (1H, s), 7.31-7.36 (1H, m), 7.57-7.89 (3H, m), 8.33 (1H, s), 9.01 (1H, s). MS m/z: 669 [M+H]$^+$.
By a similar method, the following compounds were synthesized from the corresponding intermediates and amines.

[Table 48-1]

| Example | Compound | Intermediate / Amine |
|---|---|---|
| 56 | (2E)-N-{4-[4-({1-[2-(tert-Butylamino)-2-oxoethyl]-4-fluoro-1 H-pyrazol-3-yl}oxy)-2-chloroanilino]-7-methoxyquinazolin-6-yl}-4-[(3R)-3-fluoropyrrolidin-1-yl]but-2-enamide<br>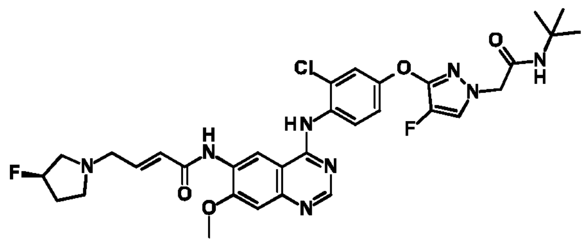<br>$^1$H-NMR (DMSO-D$_6$) δ: 1.27 (9H, s), 1.75-2.39 (2H, m), 2.60-3.49 (6H, m), 4.02 (3H, s), 4.59 (2H, s), 5.12-5.46 (1H, m), 6.53-6.70 (1H, m), 6.74-6.92 (1H, m), 7.05-7.15 (1H, m), 7.24-7.33 (2H, m), 7.44-7.54 (1H, m), 7.81 (1H, s), 7.93-8.00 (1H, m), 8.34 (1H, s), 8.92 (1H, s), 9.64-9.77 (2H, m). MS m/z: 669 [M+H]+. | Reference Example Y5<br>(3R)-3-Fluoropyrrolidine hydrochloride |
| 57 | (2E)-N-{4-[4-({1-[2-(tert-Butylamino)-2-oxoethyl]-4-fluoro-1 H-pyrazol-3-yl}oxy)-2-chloroanilino]-7-methoxyquinazolin-6-yl}-4-[(3S)-3-fluoropyrrolidin-1-yl]but-2-enamide<br>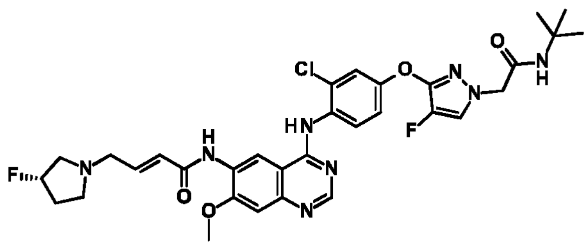<br>$^1$H-NMR (DMSO-D$_6$) δ: 1.28 (9H, s), 1.82-2.36 (2H, m), 2.60-3.49 (6H, m), 4.03 (3H, s), 4.60 (2H, s), 5.15-5.44 (1H, m), 6.56-6.73 (1H, m), 6.77-6.97 (1H, m), 7.09-7.19 (1H, m), 7.24-7.35 (2H, m), 7.45-7.57 (1H, m), 7.82 (1H, s), 7.98 (1H, s), 8.35 (1H, s), 8.93 (1H, s), 9.58-9.82 (2H, m). MS m/z: 669 [M+H]+. | Reference Example Y5<br>(3S)-3-Fluoropyrrolidine hydrochloride |

[Table 48-2]

| Example | Compound | Intermediate |
|---|---|---|
| | | Amine |
| 58 | rac-(2E)-N-{4-[4-({1-[2-(tert-Butylamino)-2-oxoethyl]-4-fluoro-1H-pyrazol-3-yl}oxy)-2-fluoroanilino]-7-methoxyquinazolin-6-yl}-4-(3-fluoropyrrolidin-1-yl)but-2-enamide | Reference Example Y6 |
| | 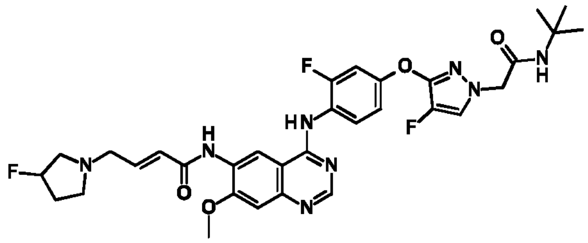<br>$^1$H-NMR ($CDCl_3$) δ: 1.33 (9H, s), 2.04-2.29 (2H, m), 2.46-2.54 (1H, m), 2.67-2.82 (1H, m), 2.94-3.08 (2H, m), 3.37-3.41 (2H, m), 4.08 (3H, s), 4.50 (2H, s), 5.13-5.31 (1H, m), 5.96 (1H, br s), 6.28 (1H, dd, J = 14.0, 1.8 Hz), 6.97-7.12 (3H, m), 7.28 (1H, s), 7.40 (1H, d, J = 4.9 Hz), 7.50 (1H, br s), 8.16 (1H, br s), 8.20-8.27 (1H, m), 8.64 (1H, s), 9.15 (1H, s). MS m/z: 653 [M+H]+. | 3-Fluoropyrrolidine hydrochloride |
| 59 | (2E)-N-{4-[4-({1-[2-(tert-Butylamino)-2-oxoethyl]-4-fluoro-1 H-pyrazol-3-yl}oxy)-2-fluoroanilino]-7-methoxyquinazolin-6-yl}-4-(3-fluoroazetidin-1-yl)but-2-enamide | Reference Example Y6 |
| | 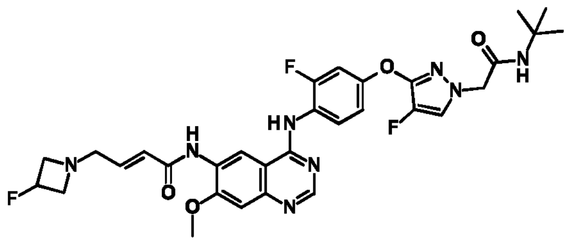<br>$^1$H-NMR ($CDCl_3$) δ: 1.33 (9H, s), 3.21-3.34 (2H, m), 3.37-3.41 (2H, m), 3.71-3.81 (2H, m), 4.08 (3H, s), 4.50 (2H, s), 5.09-5.29 (1H, m), 5.96 (1H, br s), 6.16-6.23 (1H, m), 6.91-7.04 (3H, m), 7.28 (1H, s), 7.40 (1H, d, J = 4.9 Hz), 7.50 (1H, br s), 8.14 (1H, br s), 8.20-8.27 (1H, m), 8.64 (1H, s), 9.14 (1H, s). MS m/z: 639 [M+H]+. | 3-Fluoroazetidine hydrochloride |

[Table 48-3]

| Example | Compound | Intermediate / Amine |
|---|---|---|
| 60 | (2E)-N-{4-[4-({1-[2-(tert-Butylamino)-2-oxoethyl]-4-fluoro-1 H-pyrazol-3-yl}oxy)-2-fluoroanilino]-7-methoxyquinazolin-6-yl}-4-(3-methoxyazetidin-1-yl)but-2-enamide 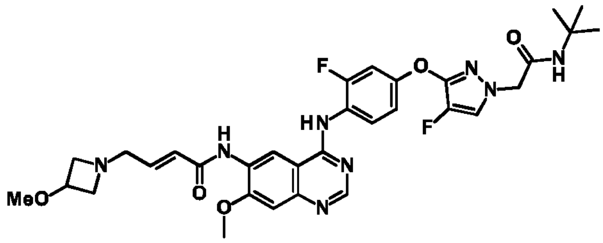 $^1$H-NMR ($CDCl_3$) δ: 1.33 (9H, s), 3.01-3.08 (2H, m), 3.29 (3H, s), 3.33-3.39 (2H, m), 3.66-3.73 (2H, m), 4.07 (3H, s), 4.08-4.13 (1H, m), 4.50 (2H, s), 5.96 (1H, br s), 6.16-6.23 (1H, m), 6.90-7.04 (3H, m), 7.28 (1H, s), 7.40 (1H, d, J = 4.3 Hz), 7.49 (1H, br s), 8.14 (1H, br s), 8.20-8.27 (1H, m), 8.64 (1H, s), 9.14 (1H, s). MS m/z: 651 [M+H]+. | Reference Example Y6 / 3-Methoxyazetidine |
| 61 | (2E)-N-{4-[4-({4-[2-(tert-Butylamino)-2-oxoethyl]-1,3-thiazol-2-yl}oxy)-2-fluoroanilino]-7-methoxyquinazolin-6-yl}-4-[(3R)-3-methoxypyrrolidin-1-yl]but-2-enamide 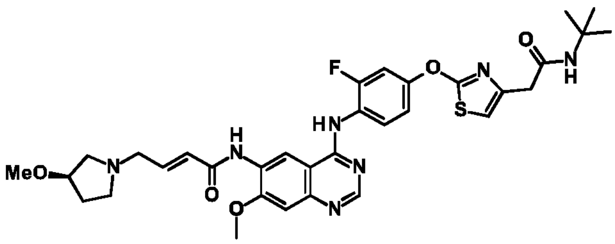 $^1$H-NMR ($CDCl_3$) δ: 1.33 (9H, s), 1.86-1.99 (1H, m), 2.10-2.22 (1 H, m), 2.55-2.66 (1H, m), 2.74-2.84 (2H, m), 2.85-2.96 (1H, m), 3.34 (3H, s), 3.37-3.44 (2H, m), 3.47 (2H, s), 3.94-4.04 (1H, m), 4.11 (3H, s), 6.33 (1H, d, J = 15.2 Hz), 6.50 (1H, br s), 6.65 (1H, s), 7.05-7.25 (3H, m), 7.32 (1H, s), 7.68 (1H, br s), 8.23 (1H, br s), 8.36-8.52 (1H, m), 8.69 (1H, s), 9.19 (1H, s). MS m/z: 664 $[M+H]^+$. | Reference Example Y7 / (3R)-3-Methoxypyrrolidine hydrochloride |

[Table 48-4]

| Example | Compound | Intermediate / Amine |
|---|---|---|
| 62 | (2E)-N-[4-(4-{[4-(5-Cyclopropyl-1,3,4-oxadiazol-2-yl)-1,3-thiazol-2-yl]oxy}-2-fluoroanilino)-7-methoxyquinazolin-6-yl]-4-[(3R)-3-fluoropyrrolidin-1-yl]but-2-enamide 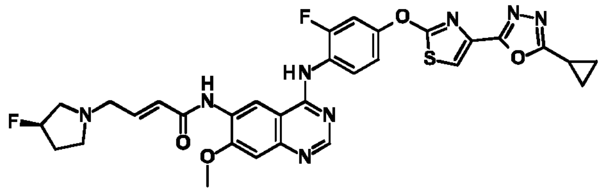 $^1$H-NMR ($CDCl_3$) $\delta$: 1.11-1.32 (4H, m), 1.99-2.35 (3H, m), 2.45-2.57 (1H, m), 2.68-2.84 (1H, m), 2.92-3.10 (2H, m), 3.37-3.45 (2H, m), 4.08 (3H, s), 5.11-5.35 (1H, m), 6.25-6.36 (1H, m), 7.04-7.12 (1H, m), 7.20-7.28 (2H, m), 7.30 (1H, s), 7.62-7.74 (2H, m), 8.19 (1H, br s), 8.47-8.56 (1H, m), 8.69 (1H, s), 9.18 (1H, s). MS m/z: 647 [M+H]$^+$. | Reference Example Y8 / (3R)-3-Fluoropyrrolidine hydrochloride |
| 63 | (2E)-N-[4-(4-{[4-(5-Cyclopropyl-1,3,4-oxadiazol-2-yl)-1,3-thiazol-2-yl]oxy}-2-fluoroanilino)-7-methoxyquinazolin-6-yl]-4-[(3R)-3-methoxypyrrolidin-1-yl]but-2-enamide 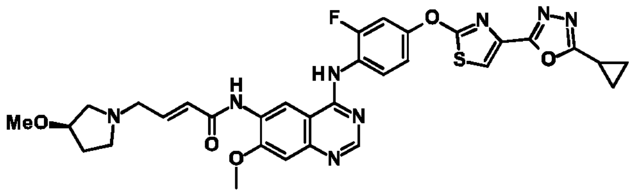 $^1$H-NMR ($CDCl_3$) $\delta$: 1.11-1.36 (4H, m), 1.82-1.95 (1H, m), 2.06-2.29 (2H, m), 2.45-2.56 (1H, m), 2.65-2.92 (3H, m), 3.32 (3H, s), 3.33-3.39 (2H, m), 3.92-4.00 (1H, m), 4.08 (3H, s), 6.29 (1H, d, J = 15.2 Hz), 7.02-7.13 (1H, m), 7.19-7.31 (3H, m), 7.66 (1H, s), 7.73 (1H, br s), 8.20 (1H, br s), 8.43-8.54 (1H, m), 8.68 (1H, s), 9.17 (1H, s). MS m/z: 659 [M+H]+. | Reference Example Y8 / (3R)-3-Methoxypyrrolidine hydrochloride |

[Example 64] Crystal of 3-{3-fluoro-4-[(7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide

To the compound (7.95 g, 12.8 mmol) described in Example 5, ethyl acetate (64 mL) and hexane (32 mL) were added at room temperature. The precipitated crystal was collected by filtration, washed with a mixed solvent of hexane/ethyl acetate = 2:1, and then dried under reduced pressure at 60°C for 5 hours to obtain the title crystal. For this crystal, powder X-ray diffraction measurements and elemental analysis were performed.

Elemental analysis: found; C: 59.61%, H: 5.42%, N: 15.60%, F: 5.93%

Diffraction angle (2θ), lattice spacing (d value), and relative intensity of the powder X-ray diffraction spectrum are described in Table 49.

[Table 49]

| Peak number | 2θ | d Value | Relative intensity | Peak number | 2θ | d Value | Relative intensity |
|---|---|---|---|---|---|---|---|
| 1 | 5.78 | 15.28 | 99 | 6 | 19.90 | 4.46 | 69 |
| 2 | 15.48 | 5.72 | 28 | 7 | 20.42 | 4.35 | 17 |
| 3 | 16.38 | 5.41 | 100 | 8 | 20.82 | 4.26 | 30 |
| 4 | 17.24 | 5.14 | 45 | 9 | 22.04 | 4.03 | 29 |
| 5 | 19.28 | 4.60 | 15 | 10 | 24.50 | 3.63 | 30 |

[Example 65] Crystal of 1-{4-[(4-{2-chloro-4-[3-(5-methyl-1,3,4-oxadiazol-2-yl)phenoxy]anilino}-7-methoxyquinazolin-6-yl)oxy]piperidin-1-yl}prop-2-en-1-one

To the compound described in Example 13 (18.0 g, 29.4 mmol), 2-propanol (120 mL) was added, and the mixture was stirred at 50°C for 30 minutes. Then, water (120 mL) was added, and the mixture was stirred at 50°C for 15 minutes and at room temperature for 1 hour. The precipitated crystal was collected by filtration and dried under reduced pressure at 60°C to obtain the title crystal. For this crystal, powder X-ray diffraction measurements and elemental analysis were performed.
Elemental analysis: found; C: 61.01%, H: 5.12%, N: 13.21%, Cl: 5.76%
Diffraction angle (2θ), lattice spacing (d value), and relative intensity of the powder X-ray diffraction spectrum are described in Table 50.

[Table 50]

| Peak number | 2θ | d Value | Relative intensity | Peak number | 2θ | d Value | Relative intensity |
|---|---|---|---|---|---|---|---|
| 1 | 4.26 | 20.72 | 51 | 6 | 17.60 | 5.04 | 23 |
| 2 | 8.66 | 10.20 | 55 | 7 | 19.08 | 4.65 | 16 |
| 3 | 13.64 | 6.49 | 45 | 8 | 22.10 | 4.02 | 77 |
| 4 | 14.34 | 6.17 | 43 | 9 | 23.02 | 3.86 | 100 |
| 5 | 14.98 | 5.91 | 99 | 10 | 25.88 | 3.44 | 56 |

[Example 66] Crystal of 1-(4-1[4-(2-fluoro-4-1[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one

To the compound (143 g, 233 mmol) described in Example 27, 2-propanol (1 L) was added and the mixture was stirred, and then 2-propanol was distilled off under reduced pressure. This procedure was further repeated 3 times, then 2-propanol (1 L) was added to the residue, and the mixture was stirred. The precipitated crystal was collected by filtration and washed with 2-propanol to obtain the title crystal. For this crystal, powder X-ray diffraction measurements and elemental analysis were performed.
Elemental analysis: found; C: 59.88%, H: 5.06%, N: 15.32%, F: 5.99%
Diffraction angle (2θ), lattice spacing (d value), and relative intensity of the powder X-ray diffraction spectrum are described in Table 51.

[Table 51]

| Peak number | 2θ | d Value | Relative intensity | Peak number | 2θ | d Value | Relative intensity |
|---|---|---|---|---|---|---|---|
| 1 | 8.08 | 10.93 | 90 | 6 | 15.44 | 5.73 | 67 |
| 2 | 10.32 | 8.56 | 100 | 7 | 19.76 | 4.49 | 34 |
| 3 | 12.90 | 6.86 | 33 | 8 | 23.60 | 3.77 | 35 |
| 4 | 13.48 | 6.56 | 32 | 9 | 24.24 | 3.67 | 53 |
| 5 | 13.82 | 6.40 | 32 | 10 | 25.90 | 3.44 | 48 |

[Example 67] Crystal of 1-(4-{[4-(2-fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one

To the compound described in Example 28 (24.77 g, 41.59 mmol), ethanol (100 mL) was added, and the obtained suspension was stirred under ultrasonic irradiation for 30 minutes. Then, the precipitated crystal was collected by filtration, washed with ethanol (50 mL), and dried at 40°C overnight to obtain the title crystal. For this crystal, powder X-ray diffraction measurements and elemental analysis were performed.
Elemental analysis: found; C: 62.45%, H: 5.71%, N: 15.21%, F: 3.01%
Diffraction angle (2θ), lattice spacing (d value), and relative intensity of the powder X-ray diffraction spectrum are described in Table 52.

[Table 52]

| Peak number | 2θ | d Value | Relative intensity | Peak number | 2θ | d Value | Relative intensity |
|---|---|---|---|---|---|---|---|
| 1 | 8.14 | 10.85 | 58 | 6 | 15.92 | 5.56 | 38 |
| 2 | 10.56 | 8.37 | 100 | 7 | 19.30 | 4.60 | 32 |
| 3 | 13.10 | 6.75 | 25 | 8 | 20.18 | 4.40 | 42 |
| 4 | 15.16 | 5.84 | 28 | 9 | 23.92 | 3.72 | 39 |
| 5 | 15.50 | 5.71 | 20 | 10 | 25.54 | 3.48 | 19 |

[Example 68] N-tert-Butyl-3-{3-fluoro-4-[(7-methoxy-6-{[1-(prop-2-enoyl)piperidin-4-yl]oxy}quinazolin-4-yl)amino]phenoxy}-1H-pyrazole-1-carboxamide methanesulfonate

To the compound (20.19 mg, 32.34 μmol) described in Example 4, 1.0 mol/L aqueous methanesulfonic acid solution (34.0 μL, 1.05 eq.) and water (168 μL) were added at room temperature. The mixture was stirred at 40°C overnight, and then at room temperature for about 30 minutes. The precipitated crystal was collected by filtration and dried at room temperature overnight to obtain the title compound. For this crystal, powder X-ray diffraction measurements were performed.
Elemental analysis: found; C: 50.87%, H: 5.25%, N: 12.78%, F: 4.14%, S: 4.06%

Diffraction angle (2θ), lattice spacing (d value), and relative intensity of the powder X-ray diffraction spectrum are described in Table 53.

[Table 53]

| Peak number | 2θ | d Value | Relative intensity | Peak number | 2θ | d Value | Relative intensity |
|---|---|---|---|---|---|---|---|
| 1 | 6.72 | 13.14 | 100 | 6 | 17.92 | 4.95 | 25 |
| 2 | 8.38 | 10.54 | 37 | 7 | 19.02 | 4.66 | 30 |
| 3 | 11.10 | 7.96 | 34 | 8 | 21.66 | 4.10 | 38 |
| 4 | 13.62 | 6.50 | 45 | 9 | 22.40 | 3.97 | 40 |
| 5 | 16.28 | 5.44 | 55 | 10 | 25.64 | 3.47 | 30 |

[Example 69] 3-{3-Fluoro-4-[(7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide 1,5-naphthalenedisulfonate

To the compound (301.21 mg, 484.55 μmol) described in Example 5, ethanol (753 μL), 0.802 mol/L aqueous 1,5-naphthalenedisulfonic acid solution (317 μL, 0.525 eq.) and water (436 μL) were added at room temperature. The mixture was stirred at 40°C overnight, and then at room temperature for about 30 minutes. The precipitated crystal was collected by filtration and dried at room temperature overnight to obtain the title compound. For this crystal, powder X-ray diffraction measurements and elemental analysis were performed.
Elemental analysis: found; C: 56.14%, H: 5.02%, N: 13.00%, F: 5.31%, S: 3.62%

Diffraction angle (2θ), lattice spacing (d value), and relative intensity of the powder X-ray diffraction spectrum are described in Table 54.

[Table 54]

| Peak number | 2θ | d Value | Relative intensity | Peak number | 2θ | d Value | Relative intensity |
|---|---|---|---|---|---|---|---|
| 1 | 5.74 | 15.38 | 100 | 6 | 18.72 | 4.74 | 20 |
| 2 | 10.32 | 8.56 | 20 | 7 | 19.60 | 4.53 | 23 |
| 3 | 11.58 | 7.64 | 48 | 8 | 20.84 | 4.56 | 33 |
| 4 | 14.62 | 6.05 | 27 | 9 | 22.96 | 3.87 | 27 |
| 5 | 14.94 | 5.92 | 22 | 10 | 26.42 | 3.37 | 19 |

[Example 70] 1-{4-[(4-{2-Chloro-4-[3-(5-methyl-1,3,4-oxadiazol-2-yl)phenoxy]anilino}-7-methoxyquinazolin-6-yl)oxy]piperidin-1-yl}prop-2-en-1-one methanesulfonate

To the compound (20.13 mg, 31.90 μmol) described in Example 13, 1.000 mol/L aqueous methanesulfonic acid solution (33.5 μL, 1.05 eq.) and water (168 μL) were added at room temperature. The mixture was stirred at 40°C overnight, and then at room temperature for about 30 minutes. The precipitated crystal was collected by filtration and dried at room temperature overnight to obtain the title compound. For this crystal, powder X-ray diffraction measurements and elemental analysis were performed.
Elemental analysis: found; C: 55.71%, H: 4.79%, N: 11.62%, Cl: 4.97%, S: 3.36%
Diffraction angle (2θ), lattice spacing (d value), and relative intensity of the powder X-ray diffraction spectrum are described in Table 55.

[Table 55]

| Peak number | 2θ | d Value | Relative intensity | Peak number | 2θ | d Value | Relative intensity |
|---|---|---|---|---|---|---|---|
| 1 | 3.56 | 24.80 | 56 | 6 | 20.26 | 4.38 | 14 |
| 2 | 7.24 | 12.20 | 100 | 7 | 21.88 | 4.06 | 19 |
| 3 | 15.02 | 5.89 | 14 | 8 | 22.92 | 3.88 | 20 |
| 4 | 16.84 | 5.26 | 33 | 9 | 25.76 | 3.46 | 12 |
| 5 | 17.68 | 5.01 | 13 | 10 | 27.08 | 3.29 | 11 |

[Example 71] 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one benzenesulfonate

To the compound (20.72 mg, 33.77 μmol) described in Example 27, ethanol (41 μL), 0.998 mol/L aqueous benzenesulfonic acid solution (35.5 μL, 1.05 eq.) and water (130 μL) were added at room temperature. The mixture was stirred at 40°C overnight, and then at room temperature for about 30 minutes. The precipitated crystal was collected by filtration and dried at room temperature overnight to obtain the title compound. For this crystal, powder X-ray diffraction measurements and elemental analysis were performed.
Elemental analysis: found; C: 57.22%, H: 4.79%, N: 12.30%, F: 4.84%, S: 4.01%
Diffraction angle (2θ), lattice spacing (d value), and relative intensity of the powder X-ray diffraction spectrum are described in Table 56.

[Table 56]

| Peak number | 2θ | d Value | Relative intensity | Peak number | 2θ | d Value | Relative intensity |
|---|---|---|---|---|---|---|---|
| 1 | 6.22 | 14.20 | 100 | 6 | 19.58 | 4.53 | 47 |
| 2 | 12.16 | 7.27 | 29 | 7 | 20.58 | 4.31 | 58 |
| 3 | 13.60 | 6.51 | 35 | 8 | 21.06 | 4.21 | 47 |
| 4 | 16.26 | 5.45 | 49 | 9 | 23.30 | 3.81 | 48 |
| 5 | 18.50 | 4.79 | 74 | 10 | 25.76 | 3.46 | 81 |

[Example 72] 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one tartrate

To the compound (20.21 mg, 32.94 μmol) described in Example 27, ethanol (40 μL), 1.000 mol/L aqueous tartaric acid solution (40 μL, 1.05 eq.) and water (127 μL) were added at room temperature. The mixture was stirred at 40°C overnight, and then at room temperature for about 30 minutes. The precipitated crystal was collected by filtration and dried at room temperature overnight to obtain the title compound. For this crystal, powder X-ray diffraction measurements and elemental analysis were performed.
Elemental analysis: found; C: 51.96%, H: 5.01%, N: 11.64%, F: 4.58%
Diffraction angle (2θ), lattice spacing (d value), and relative intensity of the powder X-ray diffraction spectrum are described in Table 57.

[Table 57]

| Peak number | 2θ | d Value | Relative intensity | Peak number | 2θ | d Value | Relative intensity |
|---|---|---|---|---|---|---|---|
| 1 | 3.58 | 24.66 | 100 | 6 | 24.98 | 3.56 | 55 |
| 2 | 14.50 | 6.10 | 64 | 7 | 25.76 | 3.46 | 51 |
| 3 | 16.50 | 5.37 | 56 | 8 | 26.12 | 3.41 | 53 |
| 4 | 24.28 | 3.66 | 62 | 9 | 26.60 | 3.35 | 48 |
| 5 | 24.70 | 3.60 | 53 | 10 | 27.40 | 3.29 | 55 |

[Example 73] 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one citrate

To the compound (20.57 mg, 33.52 μmol) described in Example 27, ethanol (41 μL), 1.000 mol/L aqueous citric acid solution (35.2 μL, 1.05 eq.) and water (129 μL) were added at room temperature. The mixture was stirred at 40°C overnight, and then at room temperature for about 30 minutes. The precipitated crystal was collected by filtration and dried at room temperature overnight to obtain the title compound. For this crystal, powder X-ray diffraction measurements and elemental analysis were performed.
Elemental analysis: found; C: 55.11%, H: 4.79%, N: 11.92%, F: 4.55%
Diffraction angle (2θ), lattice spacing (d value), and relative intensity of the powder X-ray diffraction spectrum are described in Table 58.

[Table 58]

| Peak number | 2θ | d Value | Relative intensity | Peak number | 2θ | d Value | Relative intensity |
|---|---|---|---|---|---|---|---|
| 1 | 7.36 | 12.00 | 32 | 6 | 17.56 | 5.05 | 100 |
| 2 | 8.74 | 10.11 | 53 | 7 | 19.02 | 4.66 | 44 |
| 3 | 13.62 | 6.50 | 25 | 8 | 19.44 | 4.56 | 32 |
| 4 | 15.32 | 5.78 | 25 | 9 | 21.28 | 4.17 | 74 |
| 5 | 16.32 | 5.43 | 80 | 10 | 25.02 | 3.56 | 40 |

[Example 74] 1-(4-1[4-(2-Fluoro-4-1[1-(6-methylpyridin-3-yl)-1H-pyrazol-3-yl]oxy}anilino)-7-methoxyquinazolin-6-yl]oxy}piperidin-1-yl)prop-2-en-1-one hydrochloride

To the compound (20.24 mg, 31.56 μmol) described in Example 28, ethanol (40 μL), 1.004 mol/L hydrochloric acid (33.0 μL, 1.05 eq.) and water (129 μL) were added at room temperature. The mixture was stirred at 40°C overnight, and then at room temperature for about 30 minutes. The precipitated crystal was collected by filtration and dried at room temperature overnight to obtain the title compound. For this crystal, powder X-ray diffraction measurements and elemental analysis were performed.
Elemental analysis: found; C: 54.64%, H: 5.59%, N: 13.91%, Cl: 4.43%, F: 2.72%
Diffraction angle (2θ), lattice spacing (d value), and relative intensity of the powder X-ray diffraction spectrum are described in Table 59.

[Table 59]

| Peak number | 2θ | d Value | Relative intensity | Peak number | 2θ | d Value | Relative intensity |
|---|---|---|---|---|---|---|---|
| 1 | 5.28 | 16.72 | 25 | 6 | 12.60 | 7.02 | 23 |
| 2 | 5.98 | 14.77 | 17 | 7 | 13.48 | 6.56 | 18 |
| 3 | 7.70 | 11.47 | 22 | 8 | 16.68 | 5.31 | 18 |
| 4 | 8.28 | 10.67 | 100 | 9 | 17.66 | 5.02 | 18 |
| 5 | 10.64 | 8.31 | 41 | 10 | 20.80 | 4.27 | 24 |

[Example 75] 1-(4-{[4-(2-Fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one 1,5-naphthalenedisulfonate

To the compound (20.08 mg, 31.31 μmol) described in Example 28, ethanol (40 μL), 1.000 mol/L aqueous 1,5-naphthalene disulfonic acid solution (32.9 μL, 1.05 eq.) and water (128 μL) were added at room temperature. The mixture was stirred at 40°C overnight, and then at room temperature for about 30 minutes. The precipitated crystal was collected by filtration and dried at room temperature overnight to obtain the title compound. For this crystal, powder X-ray diffraction measurements and elemental analysis were performed.
Elemental analysis: found; C: 57.41%, H: 4.82%, N: 12.57%, F: 2.64%, S: 4.35%
Diffraction angle (2θ), lattice spacing (d value), and relative intensity of the powder X-ray diffraction spectrum are described in Table 60.

[Table 60]

| Peak number | 2θ | d Value | Relative intensity | Peak number | 2θ | d Value | Relative intensity |
|---|---|---|---|---|---|---|---|
| 1 | 8.50 | 10.39 | 100 | 6 | 19.52 | 4.54 | 20 |
| 2 | 13.98 | 6.33 | 21 | 7 | 20.04 | 4.43 | 21 |
| 3 | 15.56 | 5.69 | 21 | 8 | 25.16 | 3.54 | 15 |
| 4 | 16.94 | 5.23 | 43 | 9 | 25.44 | 3.50 | 17 |
| 5 | 18.28 | 4.85 | 21 | 10 | 26.10 | 3.41 | 20 |

[Example 76] 1-(4-{[4-(2-Fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one citrate

To the compound (20.23 mg, 31.55 μmol) described in Example 28, ethanol (40 μL), 1.000 mol/L aqueous citric acid solution (33.1 μL, 1.05 eq.) and water (129 μL) were added at room temperature. The mixture was stirred at 40°C overnight, and then at room temperature for about 30 minutes. The precipitated crystal was collected by filtration and dried at room temperature overnight to obtain the title compound. For this crystal, powder X-ray diffraction measurements and elemental analysis were performed.
Elemental analysis: found; C: 55.53%, H: 5.20%, N: 12.22%, F: 2.97%
Diffraction angle (2θ), lattice spacing (d value), and relative intensity of the powder X-ray diffraction spectrum are described in Table 61.

[Table 61]

| Peak number | 2θ | d Value | Relative intensity | Peak number | 2θ | d Value | Relative intensity |
|---|---|---|---|---|---|---|---|
| 1 | 5.34 | 16.54 | 30 | 6 | 16.26 | 5.45 | 77 |
| 2 | 7.32 | 12.07 | 29 | 7 | 17.50 | 5.06 | 100 |
| 3 | 7.86 | 11.24 | 63 | 8 | 19.36 | 4.58 | 24 |
| 4 | 8.68 | 10.18 | 65 | 9 | 21.22 | 4.18 | 60 |
| 5 | 13.56 | 6.52 | 23 | 10 | 24.90 | 3.57 | 20 |

[Test Example 1]

Cell proliferation suppression test

Ba/F3-Mock, Ba/F3-EGFR WT, Ba/F3-EGFR ins. ASV, and Ba/F3-EGFR ins. SVD cell lines were cultured based on a culture medium of 10% FBS-containing RPMI 1640 supplemented with 1.5 ug/mL puromycin, and 5 ng/mL IL-3 and 100 ng/mL of EGF were further added to the culture solution for Ba/F3-Mock and Ba/F3-EGFR WT, respectively. The cells were cultured in a $CO_2$ incubator set at 37°C, 5% $CO_2$. Specimens prepared by dilution were seeded into 384-well tissue culture plates with Echo555 (Labcyte Inc.), and the cells were seeded thereto at 400 cells/well for Ba/F3-Mock, Ba/F3-EGFR WT, and Ba/F3-EGFR ins. SVD and at 200 cells/well for Ba/F3-EGFR ins. ASV (day 0), then they were cultured for an additional 3 days. On the day of the compound addition (day 0) and 3 days after the compound

addition (day 3), ATP quantity was measured with CellTiter-Glo (registered trademark) 2.0 (Promega Corporation.), and used as an indicator of cell quantity (N = 4). In the test, puromycin-free culture medium was used. Concentrations at which cell proliferation from day 0 to day 3 was inhibited by 50% ($GI_{50}$) were calculated with EXCEL. The results are shown in Table 62.

[Table 62-1]

| Example | Ba/F3 $GI_{50}$ (nM) EGFR-WT | Ba/F3 $GI_{50}$(nM) EGFR-ins. ASV | Ba/F3 $GI_{50}$(nM) EGFR-ins. SVD | Ba/F3 $GI_{50}$ (nM) mock |
|---|---|---|---|---|
| 1 | 115.0 | 15.5 | 8.9 | 7897.5 |
| 2 | 123.4 | 13.9 | 13.7 | 9527.5 |
| 3 | 369.5 | 40.6 | 30.0 | 9927.2 |
| 4 | 78.3 | 7.4 | 5.0 | 7610.9 |
| 5 | 77.1 | 5.4 | 4.3 | >10000 |
| 6 | 175.4 | 19.2 | 19.2 | 5660.0 |
| 7 | 190.6 | 39.4 | 19.6 | 5904.6 |
| 8 | 172.4 | 19.8 | 19.7 | 7156.6 |
| 9 | 280.1 | 17.1 | 15.3 | 9594.2 |
| 10 | 211.7 | 11.9 | 7.6 | 6167.3 |
| 11 | 289.5 | 11.3 | 12.5 | >10000 |
| 12 | 646.2 | 44.8 | 29.8 | >10000 |
| 13 | 853.5 | 44.5 | 54.1 | 8280.2 |
| 14 | 428.6 | 13.7 | 13.5 | 5945.1 |
| 15 | 77.3 | 12.2 | 5.5 | >10000 |
| 16 | 320.4 | 11.4 | 11.3 | >10000 |
| 17 | 185.8 | 10.3 | 8.0 | 8216.9 |
| 18 | 158.5 | 12.2 | 12.2 | 4663.3 |
| 19 | 385.8 | 23.5 | 20.2 | 4923.2 |
| 20 | 171.1 | 18.6 | 9.0 | >10000 |
| 21 | 128.7 | 12.1 | 7.3 | 7994.7 |
| 22 | 101.7 | 14.5 | 12.8 | 8623.1 |
| 23 | 109.7 | 12.4 | 10.9 | 5528.4 |
| 24 | 42.4 | 11.8 | 9.9 | 6466.6 |
| 25 | 180.3 | 18.8 | 12.4 | >10000 |
| 26 | 190.7 | 17.5 | 53.7 | 1056.6 |
| 27 | 249.1 | 11.6 | 11.1 | 8686.3 |
| 28 | 289.8 | 12.4 | 12.1 | 8370.5 |
| 29 | 156.9 | 11.3 | 7.9 | >10000 |
| 30 | 120.9 | 13.5 | 14.0 | >10000 |

[Table 62-2]

| Example | Ba/F3 $GI_{50}$ (nM) EGFR-WT | Ba/F3 $GI_{50}$(nM) EGFR-ins. ASV | Ba/F3 $GI_{50}$(nM) EGFR-ins. SVD | Ba/F3 $GI_{50}$ (nM) mock |
|---|---|---|---|---|
| 31 | 240.6 | 15.5 | 12.8 | >10000 |
| 32 | 161.8 | 11.9 | 12.7 | >10000 |
| 33 | 163.0 | 11.3 | 5.8 | >10000 |
| 34 | 276.5 | 20.5 | 13.7 | >10000 |
| 35 | 212.8 | 14.8 | 12.9 | >10000 |
| 36 | 376.6 | 12.0 | 10.0 | >10000 |
| 37 | 162.8 | 10.5 | 5.7 | >10000 |
| 38 | 174.0 | 17.0 | 18.3 | >10000 |
| 39 | 160.9 | 12.2 | 58.7 | 1149.9 |
| 40 | 80.1 | 5.5 | 32.9 | 764.8 |
| 41 | 113.9 | 8.2 | 27.0 | 841.3 |
| 42 | 103.3 | 5.1 | 17.7 | 764.3 |
| 43 | 69.8 | 5.5 | 14.1 | 805.6 |
| 44 | 167.0 | 15.5 | 52.2 | 724.2 |
| 45 | 411.9 | 10.0 | 11.6 | 2839.4 |
| 46 | 260.7 | 6.5 | 4.7 | 3088.9 |
| 47 | 144.6 | 16.4 | 73.1 | 816.0 |
| 48 | 638.3 | 8.1 | 4.9 | 3166.7 |
| 49 | 731.7 | 20.7 | 21.1 | >10000 |
| 50 | 240.4 | 10.9 | 62.2 | 749.6 |

[Table 62-3]

| Example | Ba/F3 $GI_{50}$ (nM) EGFR-WT | Ba/F3 $GI_{50}$(nM) EGFR-ins. ASV | Ba/F3 $GI_{50}$(nM) EGFR-ins. SVD | Ba/F3 $GI_{50}$ (nM) mock |
|---|---|---|---|---|
| 51 | 104.0 | 11.8 | 20.5 | 805.6 |
| 52 | 244.9 | 11.9 | 19.8 | 2836.7 |
| 53 | 228.7 | 11.0 | 16.7 | 2954.0 |
| 54 | 2894.8 | 33.4 | 50.6 | >10000 |
| 55 | 440.9 | 12.9 | 10.3 | 4293.6 |
| 56 | 635.4 | 8.5 | 4.6 | 4678.2 |
| 57 | 369.1 | 8.5 | 4.1 | 5091.0 |
| 58 | 251.5 | 10.3 | 6.7 | 4014.5 |
| 59 | 468.2 | 11.9 | 7.4 | >10000 |
| 60 | 497.3 | 11.9 | 10.7 | 6027.4 |
| 61 | 731.4 | 25.6 | 88.1 | 3116.2 |
| 62 | 732.9 | 43.9 | 209.6 | 1493.5 |
| 63 | 129.2 | 49.5 | 81.8 | 1055.6 |

[Test Example 2]

Antitumor test

For the compounds of Examples 4, 5, 13, 27, 28, 33, 40, 49, and 55, anti-tumor tests were performed by subcutaneous transplantation of Ba/F3-EGFR ins. ASV or Ba/F3-HER2 ins. YVMA which were produced by gene transduction into mouse proB cell-derived Ba/F3 cells.

Each cell was suspended to 1 x $10^8$ cells/mL with phosphate buffered saline, and 0.1 mL of the prepared cell suspensions were transplanted subcutaneously into nude mice (female, 6 weeks old). Mice used were grouped by estimated tumor volume value when the average estimated tumor volume reached 100 to 300 $mm^3$, and from the same day forced oral administration was started. The administration was performed once or twice daily with 10 mL/kg solution.

Each compound was suspended in 0.5% methylcellulose solution (0.5% MC) or 2 equivalents of methanesulfonic acid-added 0.5% MC, or dissolved in 20% PEG400/3% Tween80/DW. Long diameters (mm) and short diameters (mm) of the tumor were measured with electronic digital calipers over time, and the estimated tumor volume was calculated by the following equation (1) to generate a diagram. Body weight (g) was also measured with an automatic scale for small animals over time, and the body weight change (%) was calculated by the following equation (2) to study the effect of compound administration on body weight, and the latest body weight measurement result was used for dosage calculation.

Estimated Tumor Volume ($mm^3$) = average estimated tumor volume of each individual...(1)

Estimated tumor volume of each individual = $An \times Bn^2/2$

An: Long diameter of tumor on day n
Bn: Short diameter of tumor on day n

Body Weight Change (%) = average body weight change rate of each individual...(2)

Body weight change rate of each individual = $(1 - BWn/BWs) \times 100$

BWn: Body weight on day n
BWs: Body weight on administration starting date

The results are shown in Figures 1-12.

## Claims

1. A compound represented by Formula (I) or a pharmaceutically acceptable salt thereof:

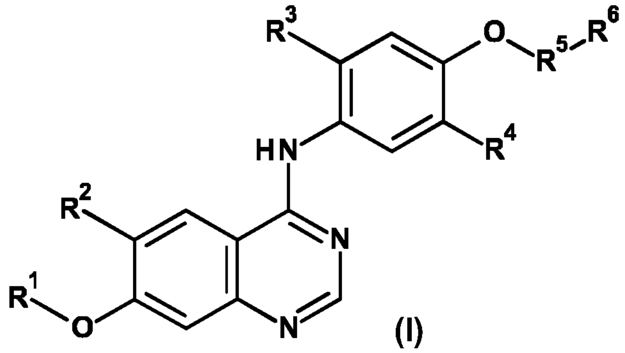

(I)

wherein

$R^1$ represents a $C_1$-$C_3$ alkyl group optionally substituted with 1 to 3 halogen atoms,
$R^2$ represents Formula (II) below:

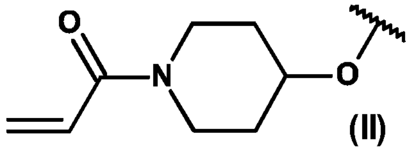

(II)

or -NH-CO-CH=CH-$CH_2$-X;
X represents an amino group optionally having 1 or 2 substituents independently selected from Group A below, a pyrrolidinyl group optionally having 1 or 2 substituents independently selected from Group A below, an azetidinyl group optionally having 1 or 2 substituents independently selected from Group A below, or a morpholyl group;
$R^3$ represents a halogen atom;
$R^4$ represents a hydrogen atom or a halogen atom;
$R^5$ represents a benzene ring, a thiazole ring, or a pyrazole ring optionally having 1 or 2 substituents independently selected from the group consisting of a halogen atom and a $C_1$-$C_3$ alkyl group;
$R^6$ represents an oxadiazolyl group optionally having 1 or 2 substituents independently selected from Group B below, a triazolyl group optionally having 1 or 2 substituents independently selected from Group B below, a pyridyl group optionally having 1 or 2 substituents independently selected from Group B below, a pyrimidyl group optionally having 1 or 2 substituents independently selected from Group B below, a thiadiazolyl group optionally having 1 or 2 substituents independently selected from Group B below, -CO-N(Y)(Z), or -$CH_2$-CON(Y)(Z) ;
Y and Z each independently represents a hydrogen atom, a $C_1$-$C_6$ alkyl group optionally substituted with 1 to 3 halogen atoms, or a $C_3$-$C_6$ cycloalkyl group substituted with a $C_1$-$C_3$ alkyl group optionally substituted with 1 to 3 halogen atoms, or
Y, Z and the nitrogen atom to which they are bonded may be taken together to form a 4- to 6-membered nitrogen-containing saturated heterocyclic ring,
Group A: a halogen atom, a $C_1$-$C_3$ alkyl group, a $C_1$-$C_3$ alkoxy group, and a tetrahydrofuryl group, and
Group B: a halogen atom, a $C_1$-$C_3$ alkyl group, a $C_3$-$C_6$ cycloalkyl group, and a $C_1$-$C_3$ alkoxy group.

2. The compound or a pharmaceutically acceptable salt thereof according to claim 1, wherein $R^1$ is a methyl group.

3. The compound or a pharmaceutically acceptable salt thereof according to claim 1 or 2, wherein $R^2$ is a group represented by Formula (II).

4. The compound or a pharmaceutically acceptable salt thereof according to any one of claims 1 to 3, wherein $R^3$ is a chlorine atom or a fluorine atom, and $R^4$ is a hydrogen atom.

5. The compound or a pharmaceutically acceptable salt thereof according to any one of claims 1 to 4, wherein $R^5$ is a benzene ring or a pyrazole ring.

6. The compound or a pharmaceutically acceptable salt thereof according to any one of claims 1 to 5, wherein

$R^6$ is an oxadiazolyl group optionally substituted with a methyl group, a pyridyl group optionally substituted with 1 or 2 substituents independently selected from the group consisting of a fluorine atom and a methyl group, or -CO-NH-Y; and

Y is a tert-butyl group optionally substituted with 1 to 3 fluorine atoms.

**7.** A compound selected from the group consisting of:

N-tert-butyl-3-{3-fluoro-4-[(7-methoxy-6-{[1-(prop-2-enoyl)piperidin-4-yl]oxy}quinazolin-4-yl)amino]phenoxy}-1H-pyrazole-1-carboxamide,
3-{3-fluoro-4-[(7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide,
1-{4-[(4-{2-chloro-4-[3-(5-methyl-1,3,4-oxadiazol-2-yl)phenoxy]anilino}-7-methoxyquinazolin-6-yl)oxy]piperidin-1-yl}prop-2-en-1-one,
1-{4-[(4-{2-fluoro-4-[3-(5-methyl-1,3,4-oxadiazol-2-yl)phenoxy]anilino}-7-methoxyquinazolin-6-yl)oxy]piperidin-1-yl}prop-2-en-1-one,
1-(4-{[4-(2-fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one; and
1-(4-{[4-(2-fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one,

or a pharmaceutically acceptable salt thereof.

**8.** N-tert-Butyl-3-{3-fluoro-4-[(7-methoxy-6-{[1-(prop-2-enoyl)piperidin-4-yl]oxy}quinazolin-4-yl)amino]phenoxy}-1H-pyrazole-1-carboxamide, or a pharmaceutically acceptable salt thereof.

**9.** N-tert-Butyl-3-{3-fluoro-4-[(7-methoxy-6-{[1-(prop-2-enoyl)piperidin-4-yl]oxy}quinazolin-4-yl)amino]phenoxy}-1H-pyrazole-1-carboxamide methanesulfonate.

**10.** 3-{3-Fluoro-4-[((7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide, or a pharmaceutically acceptable salt thereof.

**11.** 3-{3-Fluoro-4-[(7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide 1,5-naphthalenedisulfonate.

**12.** 1-{4-[(4-{2-Chloro-4-[3-(5-methyl-1,3,4-oxadiazol-2-yl)phenoxy]anilino}-7-methoxyquinazolin-6-yl)oxy]piperidin-1-yl}prop-2-en-1-one, or a pharmaceutically acceptable salt thereof.

**13.** 1-{4-[(4-{2-Chloro-4-[3-(5-methyl-1,3,4-oxadiazol-2-yl)phenoxy]anilino}-7-methoxyquinazolin-6-yl)oxy]piperidin-1-yl}prop-2-en-1-one methanesulfonate.

**14.** 1-{4-[(4-{2-Fluoro-4-[3-(5-methyl-1,3,4-oxadiazol-2-yl)phenoxy]anilino}-7-methoxyquinazolin-6-yl)oxy]piperidin-1-yl}prop-2-en-1-one, or a pharmaceutically acceptable salt thereof.

**15.** 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one, or a pharmaceutically acceptable salt thereof.

**16.** 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one benzenesulfonate.

**17.** 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one tartrate.

**18.** 1-(4-{[4-(2-Fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one citrate.

**19.** 1-(4-{[4-(2-Fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one, or a pharmaceutically acceptable salt thereof.

**20.** 1-(4-{[4-(2-Fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one hydrochloride.

**21.** 1-(4-{[4-((2-Fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one 1,5-naphthalenedisulfonate.

**22.** 1-(4-{[4-(2-Fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one citrate.

**23.** A crystal of 3-{3-fluoro-4-[(7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide, having at least five peaks at diffraction angles (2θ) selected from 5.78±0.2, 15.48±0.2, 16.38±0.2, 17.24±0.2, 19.28±0.2, 19.90±0.2, 20.42±0.2, 20.82±0.2, 22.04±0.2, and 24.50±0.2 in powder X-ray diffraction with CuKα radiation.

**24.** A crystal of 3-{3-fluoro-4-[(7-methoxy-6-{[1-(prop-2-enoyl)piperidin-4-yl]oxy}quinazolin-4-yl)amino]phenoxy}-N-(1-fluoro-2-methylpropan-2-yl)-1H-pyrazole-1-carboxamide 1,5-naphthalenedisulfonate, having at least five peaks at diffraction angles (2θ) selected from 5.74±0.2, 10.32±0.2, 11.58±0.2, 14.62±0.2, 14.94±0.2, 18.72±0.2, 19.60±0.2, 20.84±0.2, 22.96±0.2 and 26.42±0.2 in powder X-ray diffraction with CuKα radiation.

**25.** A crystal of 1-{4-[(4-{2-chloro-4-[3-(5-methyl-1,3,4-oxadiazol-2-yl)phenoxy]anilino}-7-methoxyquinazolin-6-yl)oxy]piperidin-1-yl}prop-2-en-1-one, having at least five peaks at diffraction angles (2θ) selected from 4.26±0.2, 8.66±0.2, 13.64±0.2, 14.34±0.2, 14.98±0.2, 17.60±0.2, 19.08±0.2, 22.10±0.2, 23.02±0.2 and 25.88±0.2 in powder X-ray diffraction with CuKα radiation.

**26.** A crystal of 1-{4-[(4-{2-chloro-4-[3-(5-methyl-1,3,4-oxadiazol-2-yl)phenoxy]anilino}-7-methoxyquinazolin-6-yl)oxy]piperidin-1-yl}prop-2-en-1-one methanesulfonate, having at least five peaks at diffraction angles (2θ) selected from 3.56±0.2, 7.24±0.2, 15.02±0.2, 16.84±0.2, 17.68±0.2, 20.26±0.2, 21.88±0.2, 22.92±0.2, 25.76±0.2 and 27.08±0.2 in powder X-ray diffraction with CuKα radiation.

**27.** A crystal of 1-(4-{[4-(2-fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one, having at least five peaks at diffraction angles (2θ) selected from 8.08±0.2, 10.32±0.2, 12.90±0.2, 13.48±0.2, 13.82±0.2, 15.44±0.2, 19.76±0.2, 23.60±0.2, 24.24±0.2 and 25.90±0.2 in powder X-ray diffraction with CuKα radiation.

**28.** A crystal of 1-(4-{[4-(2-fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one benzenesulfonate, having at least five peaks at diffraction angles (2θ) selected from 6.22±0.2, 12.16±0.2, 13.60±0.2, 16.26±0.2, 18.50±0.2, 19.58±0.2, 20.58±0.2, 21.06±0.2, 23.30±0.2, and 25.76±0.2 in powder X-ray diffraction with CuKα radiation.

**29.** A crystal of 1-(4-{[4-(2-fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one tartrate, having at least five peaks at diffraction angles (2θ) selected from 3.58±0.2, 14.50±0.2, 16.50±0.2, 24.28±0.2, 24.70±0.2, 24.98±0.2, 25.76±0.2, 26.12±0.2, 26.60±0.2 and 27.40±0.2 in powder X-ray diffraction with CuKα radiation.

**30.** A crystal of 1-(4-{[4-(2-fluoro-4-{[1-(5-fluoro-6-methylpyridin-3-yl)-1H-pyrazol-3-yl]oxy}anilino)-7-methoxyquinazolin-6-yl]oxy}piperidin-1-yl)prop-2-en-1-one citrate, having at least five peaks at diffraction angles (2θ) selected from 7.36±0.2, 8.74±0.2, 13.62±0.2, 15.32±0.2, 16.32±0.2, 17.56±0.2, 19.02±0.2, 19.44±0.2, 21.28±0.2, and 25.02±0.2 in powder X-ray diffraction with CuKα radiation.

**31.** A crystal of 1-(4-1[4-(2-fluoro-4-1[1-(6-methylpyridin-3-yl)-1H-pyrazol-3-yl]oxy}anilino)-7-methoxyquinazolin-6-yl]oxy}piperidin-1-yl)prop-2-en-1-one, having at least five peaks at diffraction angles (2θ) selected from 8.14±0.2, 10.56±0.2, 13.10±0.2, 15.16±0.2, 15.50±0.2, 15.92±0.2, 19.30±0.2, 20.18±0.2, 23.92±0.2, and 25.54±0.2 in powder X-ray diffraction with CuKα radiation.

**32.** A crystal of 1-(4-1[4-(2-fluoro-4-1[1-(6-methylpyridin-3-yl)-1H-pyrazol-3-yl]oxy}anilino)-7-methoxyquinazolin-6-yl]oxy}piperidin-1-yl)prop-2-en-1-one hydrochloride, having at least five peaks at diffraction angles (2θ) selected from 5.28±0.2, 5.98±0.2, 7.70±0.2, 8.28±0.2, 10.64±0.2, 12.60±0.2, 13.48±0.2, 16.68±0.2, 17.66±0.2 and 20.80±0.2 in powder X-ray diffraction with CuKα radiation.

**33.** A crystal of 1-(4-{[4-(2-fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one 1,5-naphthalenedisulfonate, having at least five peaks at diffraction angles (2θ) selected from 8.50±0.2, 13.98±0.2, 15.56±0.2, 16.94±0.2, 18.28±0.2, 19.52±0.2, 20.04±0.2, 25.16±0.2, 25.44±0.2 and 26.10±0.2 in powder X-ray diffraction with CuKα radiation.

**34.** A crystal of 1-(4-{[4-(2-fluoro-4-{[1-(6-methylpyridin-3-yl)-1H-pyrazol-3-yl]oxy}anilino)-7-methoxyquinazolin-6-yl]oxy}piperidin-1-yl)prop-2-en-1-one citrate, having at least five peaks at diffraction angles (2θ) selected from 5.34±0.2, 7.32±0.2, 7.86±0.2, 8.68±0.2, 13.56±0.2, 16.26±0.2, 17.50±0.2, 19.36±0.2, 21.22±0.2 and 24.90±0.2 in powder X-ray diffraction with CuKα radiation.

**35.** A crystal of N-tert-butyl-3-{3-fluoro-4-[(7-methoxy-6-{[1-(prop-2-enoyl)piperidin-4-yl]oxy}quinazolin-4-yl)amino]phenoxy}-1H-pyrazole-1-carboxamide methanesulfonate, having at least five peaks at diffraction angles (2θ) selected from 6.72±0.2, 8.38±0.2, 11.10±0.2, 13.62±0.2, 16.28±0.2, 17.92±0.2, 19.02±0.2, 21.66±0.2, 22.40±0.2 and 25.64±0.2 in powder X-ray diffraction with CuKα radiation.

**36.** A pharmaceutical composition, comprising as an active ingredient a compound or a pharmaceutically acceptable salt thereof according to any one of claims 1 to 22, or a crystal according to any one of claims 23 to 35.

**37.** An inhibitor of an EGFR tyrosine kinase having an exon 20 insertion mutation and/or a HER2 tyrosine kinase having an exon 20 insertion mutation, comprising as an active ingredient a compound or a pharmaceutically acceptable salt thereof according to any one of claims 1 to 22, or a crystal according to any one of claims 23 to 35.

**38.** An antitumor agent, comprising as an active ingredient a compound or a pharmaceutically acceptable salt thereof according to any one of claims 1 to 22, or a crystal according to any one of claims 23 to 35.

**39.** The antitumor agent according to claim 38, wherein the tumor is lung cancer, breast cancer, bladder cancer, or ovarian cancer.

**40.** The antitumor agent according to claim 38 or 39, wherein the tumor is a tumor having an exon 20 insertion mutation of an EGFR tyrosine kinase and/or an exon 20 insertion mutation of a HER2 tyrosine kinase.

**41.** A method for treating a tumor, comprising administering a compound or a pharmaceutically acceptable salt thereof according to any one of claims 1 to 22, or a crystal according to any one of claims 23 to 35.

**42.** The method according to claim 41, wherein the tumor is lung cancer, breast cancer, bladder cancer, or ovarian cancer.

**43.** The method according to claim 41 or 42, wherein the tumor is a tumor having an exon 20 insertion mutation of an EGFR tyrosine kinase and/or an exon 20 insertion mutation of a HER2 tyrosine kinase.

**44.** A compound or a pharmaceutically acceptable salt thereof according to any one of claims 1 to 22, or a crystal according to any one of claims 23 to 35, for the treatment of a tumor.

**45.** The compound or a pharmaceutically acceptable salt thereof, or the crystal according to claim 44, wherein the tumor is lung cancer, breast cancer, bladder cancer, or ovarian cancer.

**46.** The compound or a pharmaceutically acceptable salt thereof, or the crystal according to claim 44 or 45, wherein the tumor is a tumor having an exon 20 insertion mutation of an EGFR tyrosine kinase and/or an exon 20 insertion mutation of a HER2 tyrosine kinase.

**47.** Use of a compound or a pharmaceutically acceptable salt thereof according to any one of claims 1 to 22, or a crystal according to any one of claims 23 to 35, in the manufacture of a pharmaceutical composition for use in treating a tumor.

**48.** Use according to claim 47, wherein the tumor is lung cancer, breast cancer, bladder cancer, or ovarian cancer.

**49.** Use according to claim 47 or 48, wherein the tumor is a tumor having an exon 20 insertion mutation of an EGFR tyrosine kinase and/or an exon 20 insertion mutation of a HER2 tyrosine kinase.

[Figure 1]

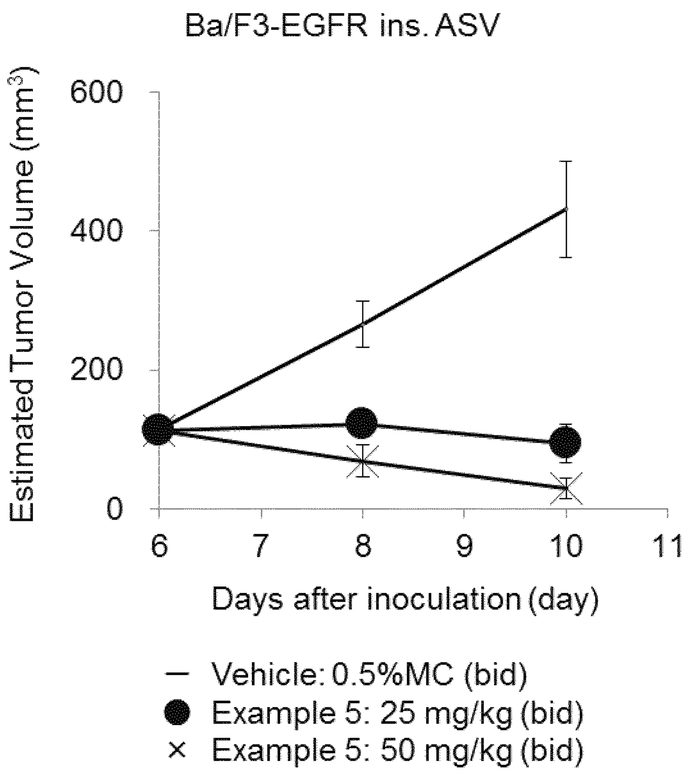
Ba/F3-EGFR ins. ASV
Estimated Tumor Volume (mm3)
600
400
200
0
6
7
8
9
10
11
Days after inoculation (day)
Vehicle: 0.5%MC (bid)
Example 5: 25 mg/kg (bid)
Example 5: 50 mg/kg (bid)

[Figure 2]

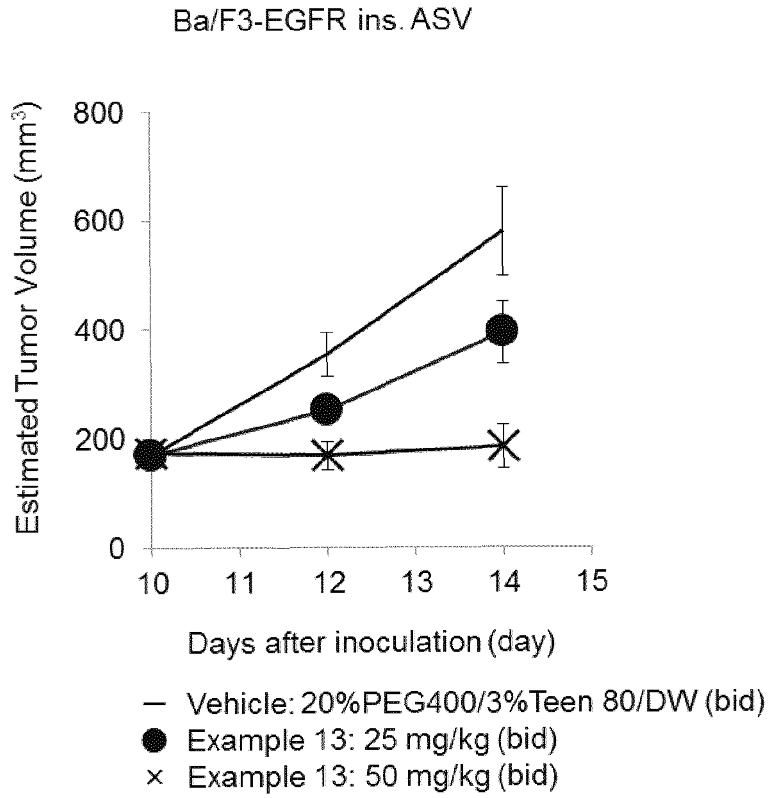
Ba/F3-EGFR ins. ASV
Estimated Tumor Volume (mm3)
800
600
400
200
0
10
11
12
13
14
15
Days after inoculation (day)
— Vehicle: 20%PEG400/3%Teen 80/DW (bid)
● Example 13: 25 mg/kg (bid)
× Example 13: 50 mg/kg (bid)

[Figure 3]

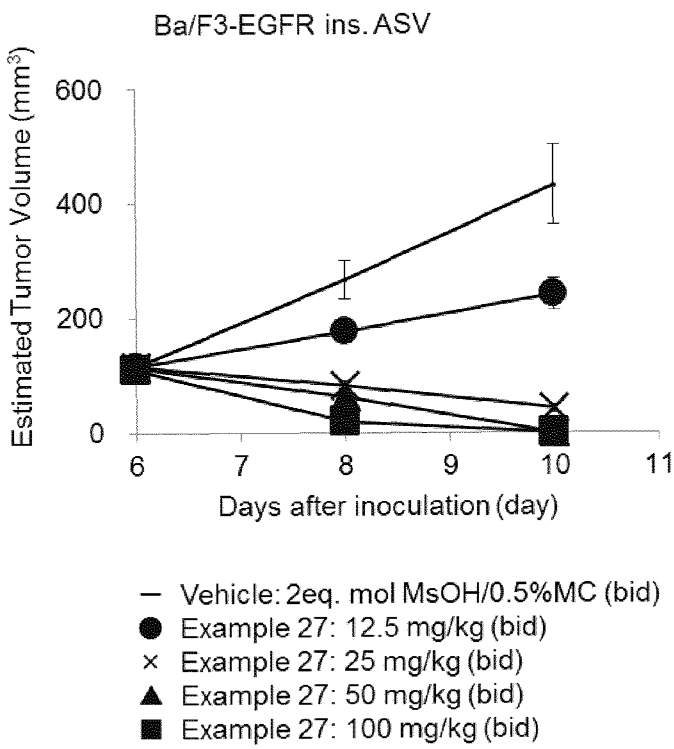
Ba/F3-EGFR ins. ASV
Estimated Tumor Volume (mm3)
600
400
200
0
6
7
8
9
10
11
Days after inoculation (day)
— Vehicle: 2eq. mol MsOH/0.5%MC (bid)
● Example 27: 12.5 mg/kg (bid)
× Example 27: 25 mg/kg (bid)
▲ Example 27: 50 mg/kg (bid)
■ Example 27: 100 mg/kg (bid)

[Figure 4]

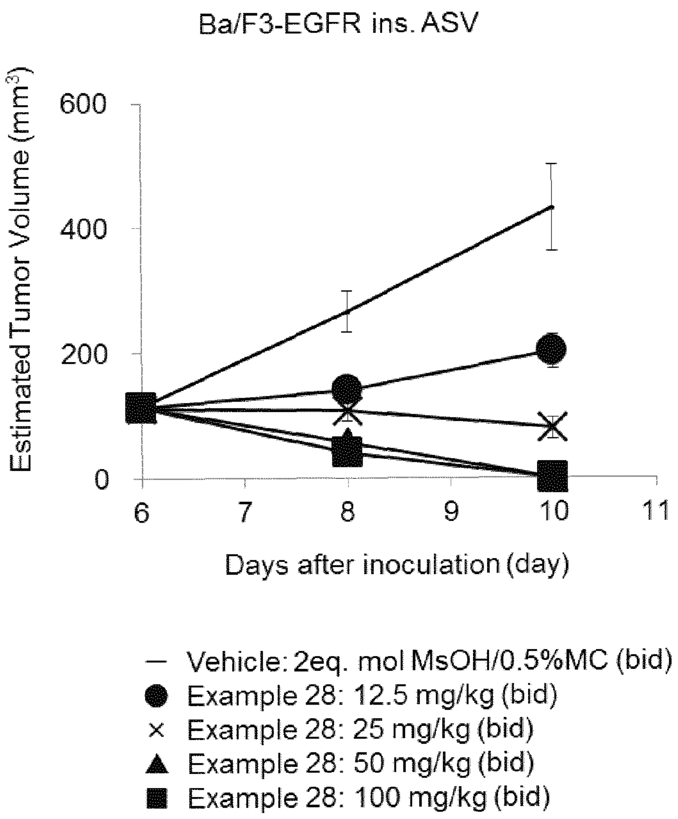
Ba/F3-EGFR ins. ASV
Estimated Tumor Volume (mm3)
600
400
200
0
6
7
8
9
10
11
Days after inoculation (day)
— Vehicle: 2eq. mol MsOH/0.5%MC (bid)
● Example 28: 12.5 mg/kg (bid)
× Example 28: 25 mg/kg (bid)
▲ Example 28: 50 mg/kg (bid)
■ Example 28: 100 mg/kg (bid)

[Figure 5]

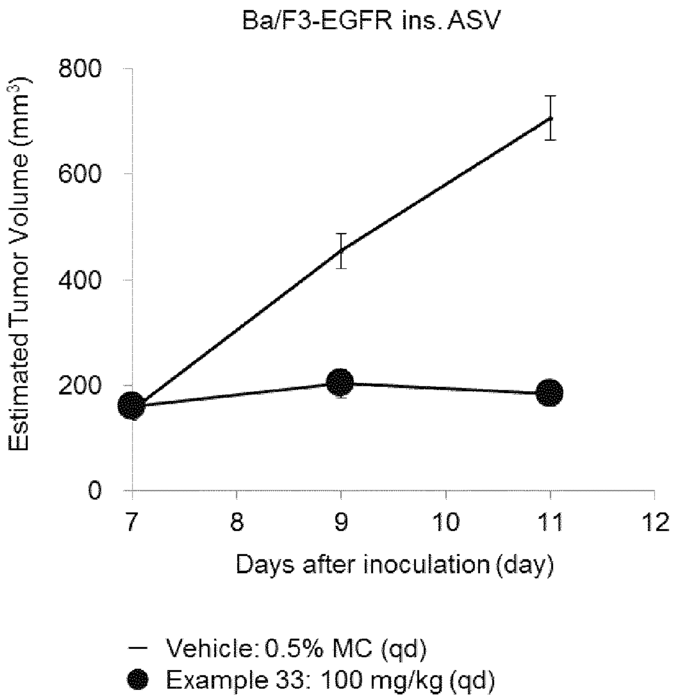
Ba/F3-EGFR ins. ASV
Estimated Tumor Volume (mm3)
800
600
400
200
0
7
8
9
10
11
12
Days after inoculation (day)
Vehicle: 0.5% MC (qd)
Example 33: 100 mg/kg (qd)

[Figure 6]

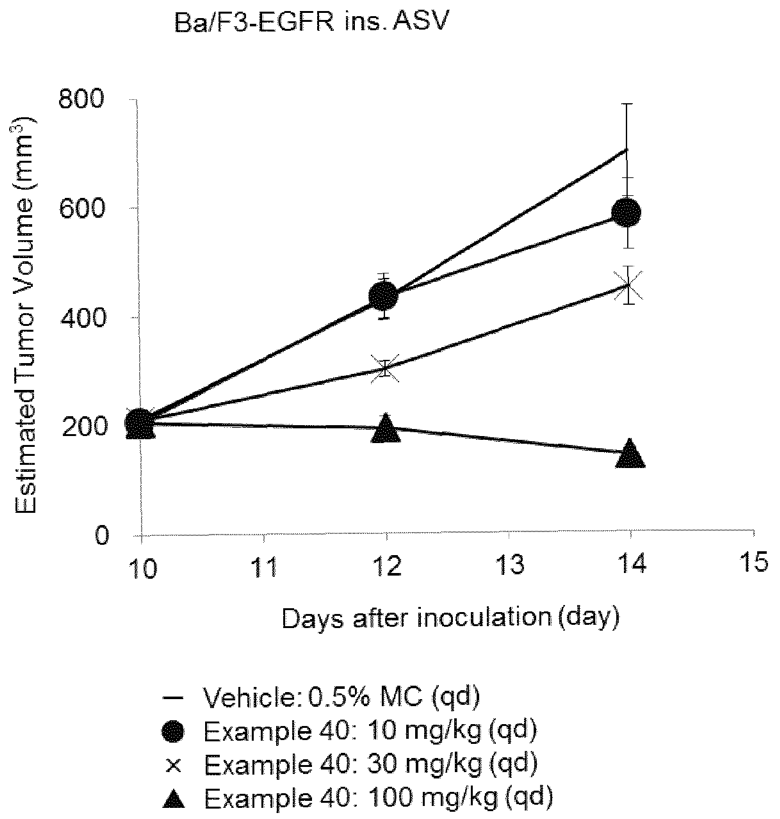
Ba/F3-EGFR ins. ASV
Estimated Tumor Volume (mm3)
800
600
400
200
0
10
11
12
13
14
15
Days after inoculation (day)
Vehicle: 0.5% MC (qd)
Example 40: 10 mg/kg (qd)
Example 40: 30 mg/kg (qd)
Example 40: 100 mg/kg (qd)

[Figure 7]

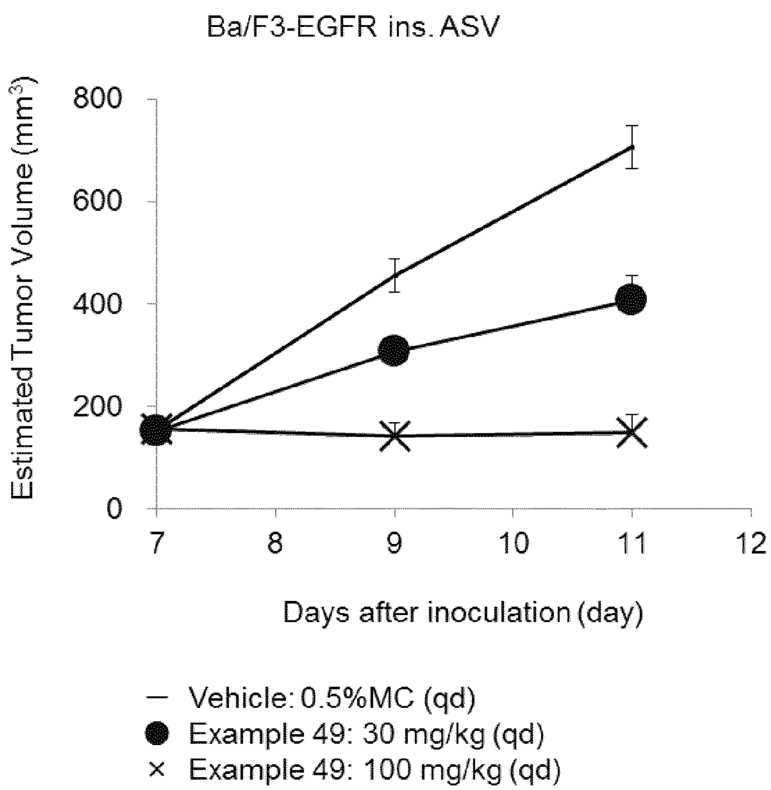
Ba/F3-EGFR ins. ASV
Estimated Tumor Volume (mm3)
800
600
400
200
0
7
8
9
10
11
12
Days after inoculation (day)
Vehicle: 0.5%MC (qd)
Example 49: 30 mg/kg (qd)
Example 49: 100 mg/kg (qd)

[Figure 8]

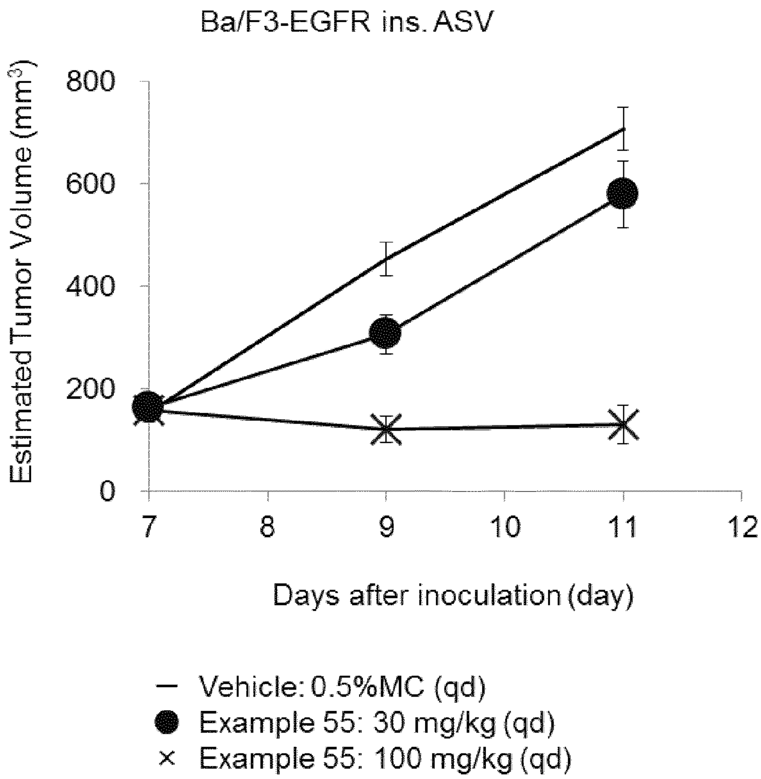
Ba/F3-EGFR ins. ASV
Estimated Tumor Volume (mm3)
800
600
400
200
0
7
8
9
10
11
12
Days after inoculation (day)
— Vehicle: 0.5%MC (qd)
● Example 55: 30 mg/kg (qd)
× Example 55: 100 mg/kg (qd)

[Figure 9]

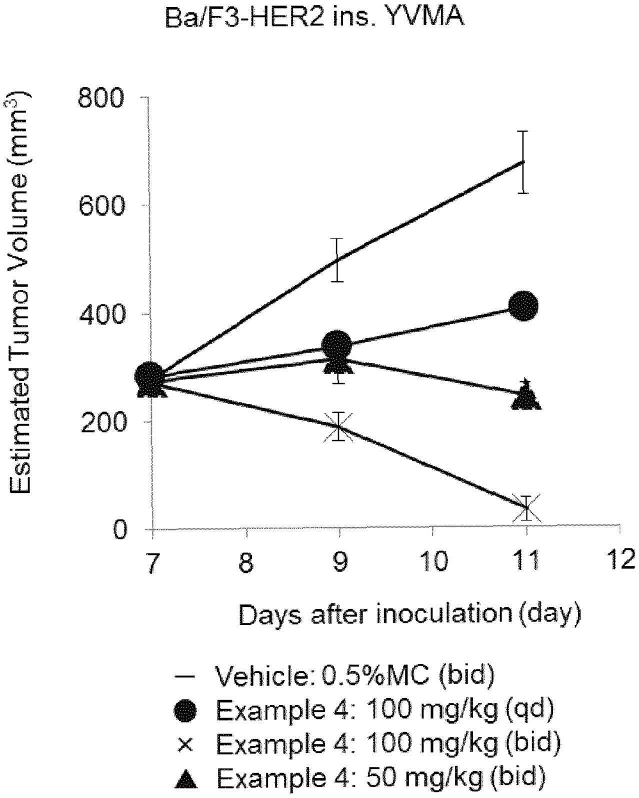
Ba/F3-HER2 ins. YVMA
Estimated Tumor Volume (mm3)
800
600
400
200
0
7
8
9
10
11
12
Days after inoculation (day)
Vehicle: 0.5%MC (bid)
Example 4: 100 mg/kg (qd)
Example 4: 100 mg/kg (bid)
Example 4: 50 mg/kg (bid)

[Figure 10]

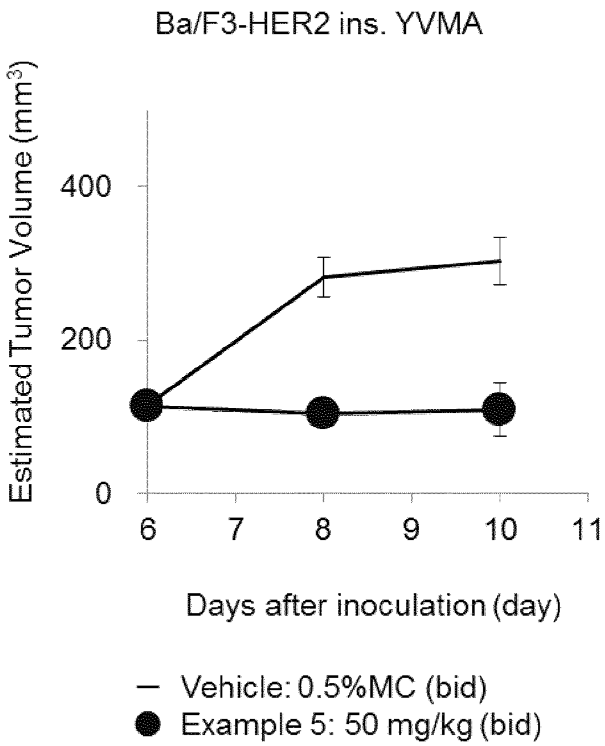
Ba/F3-HER2 ins. YVMA
Estimated Tumor Volume (mm3)
400
200
0
6
7
8
9
10
11
Days after inoculation (day)
Vehicle: 0.5%MC (bid)
Example 5: 50 mg/kg (bid)

[Figure 11]

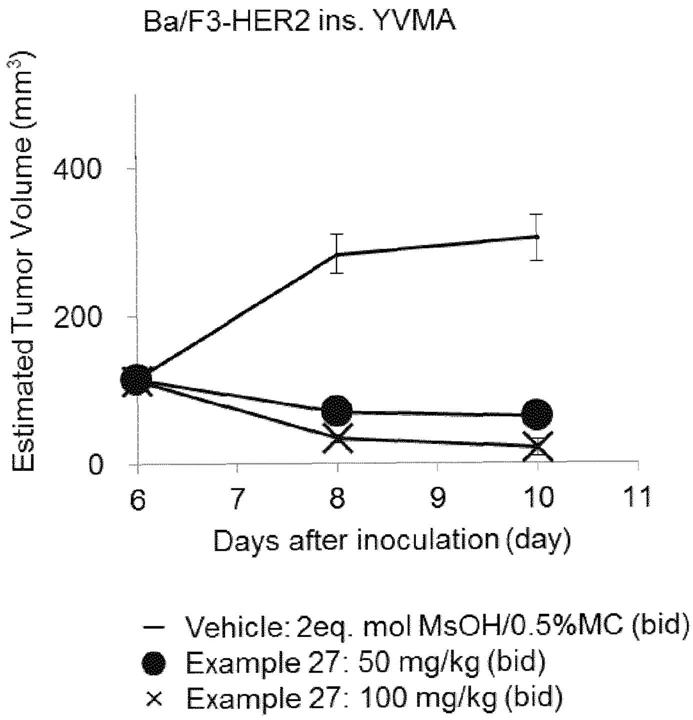
Ba/F3-HER2 ins. YVMA
Estimated Tumor Volume ($mm^3$)
400
200
0
6
7
8
9
10
11
Days after inoculation (day)
Vehicle: 2eq. mol MsOH/0.5%MC (bid)
Example 27: 50 mg/kg (bid)
Example 27: 100 mg/kg (bid)

[Figure 12]

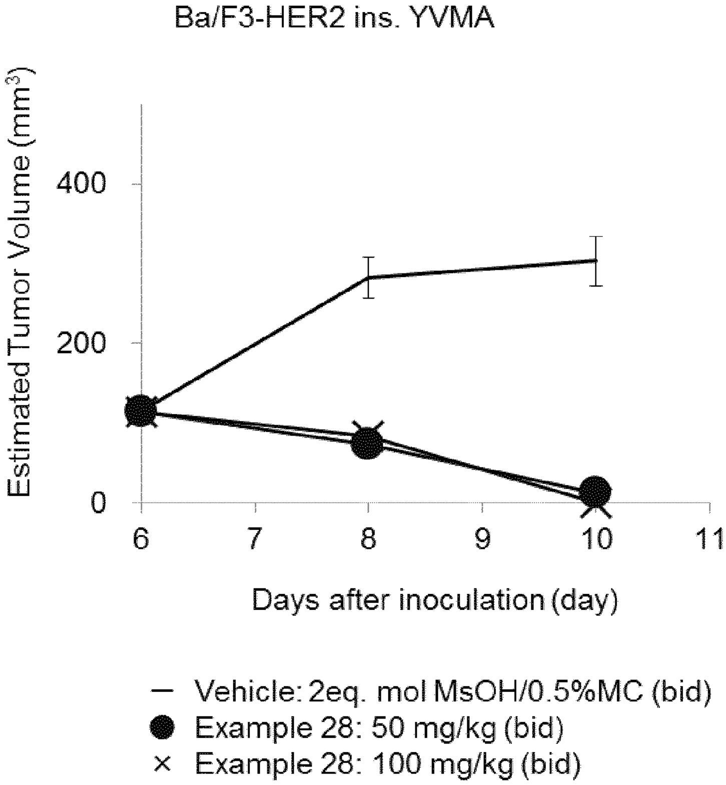


[Figure 13]

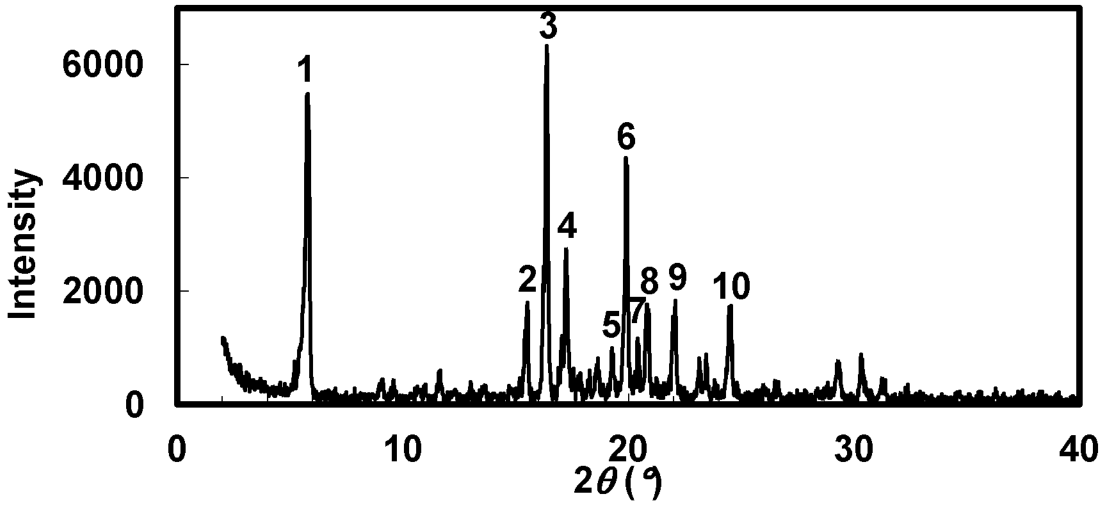

[Figure 14]

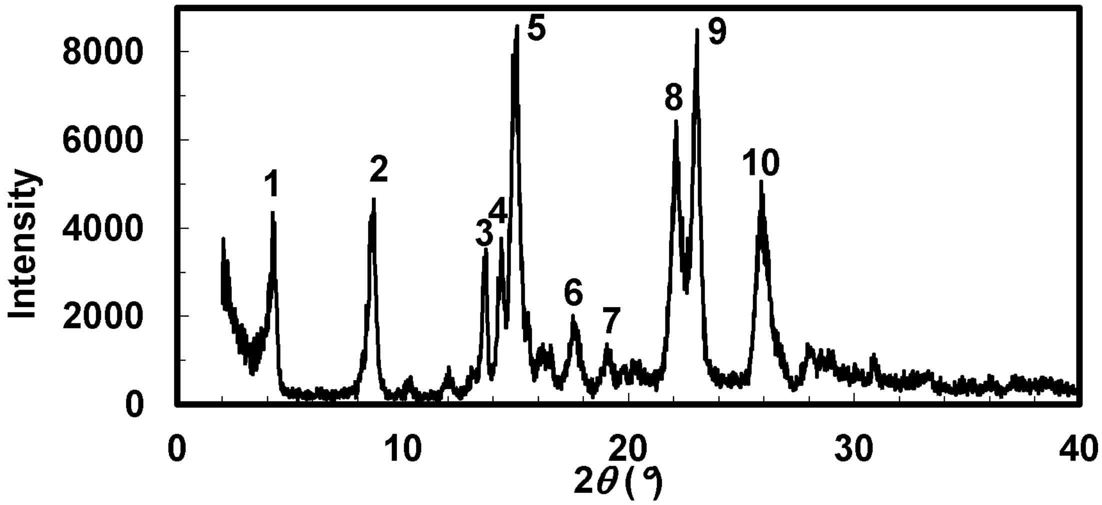


[Figure 15]

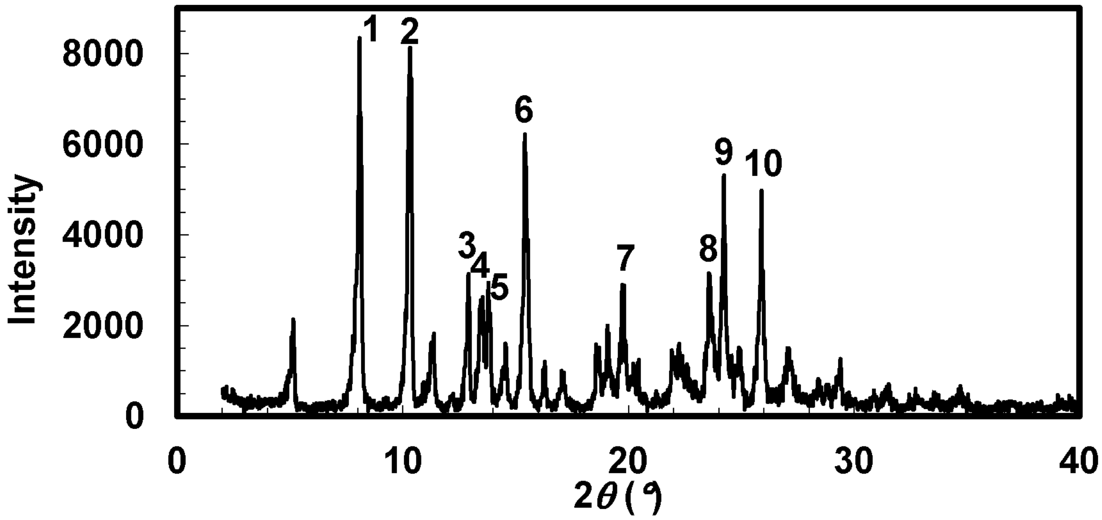

[Figure 16]

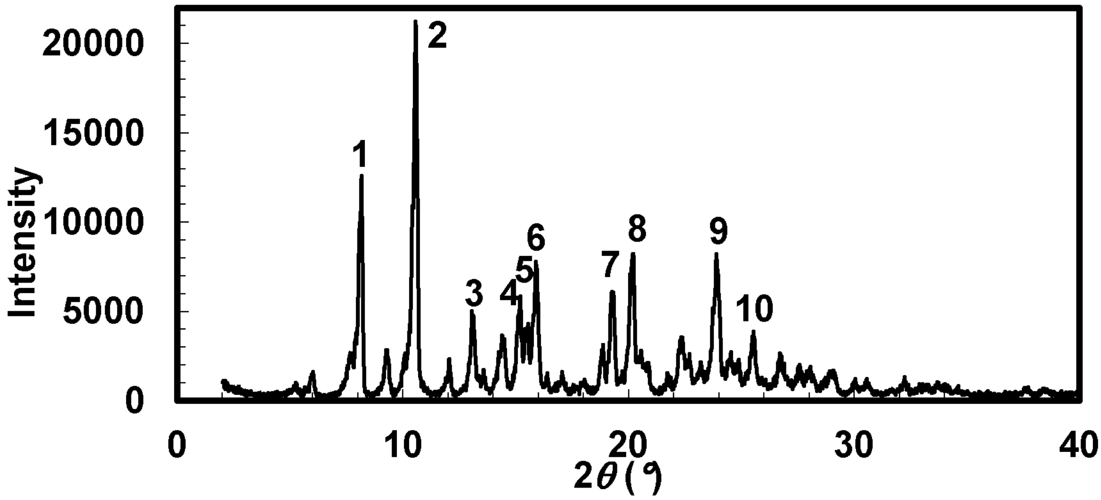


[Figure 17]

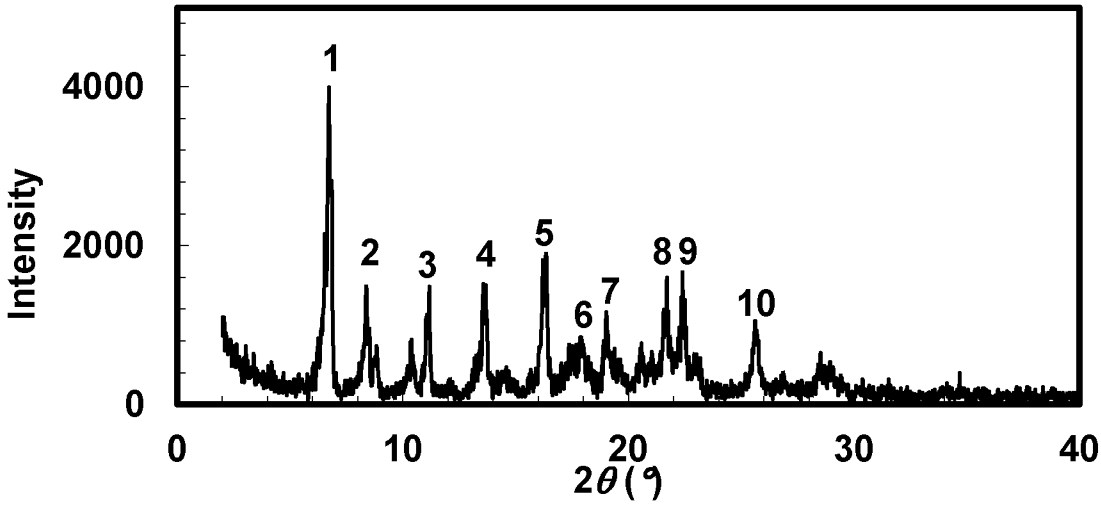

[Figure 18]

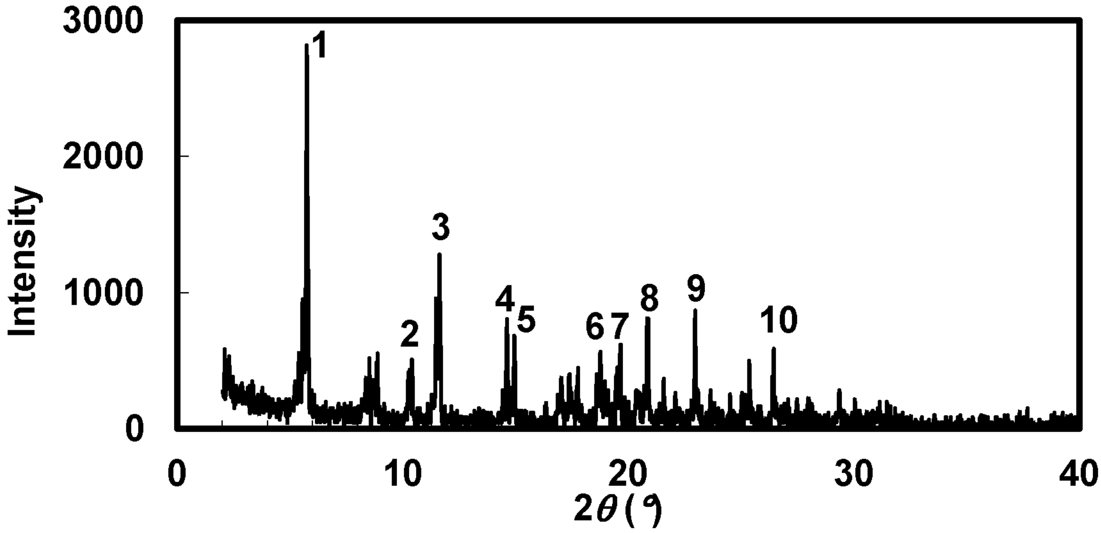


[Figure 19]

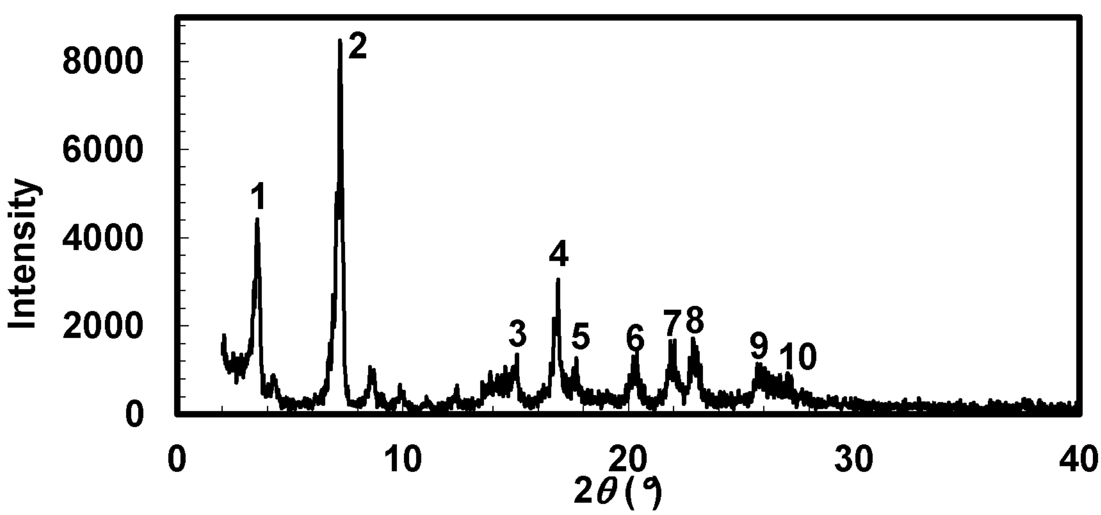

[Figure 20]

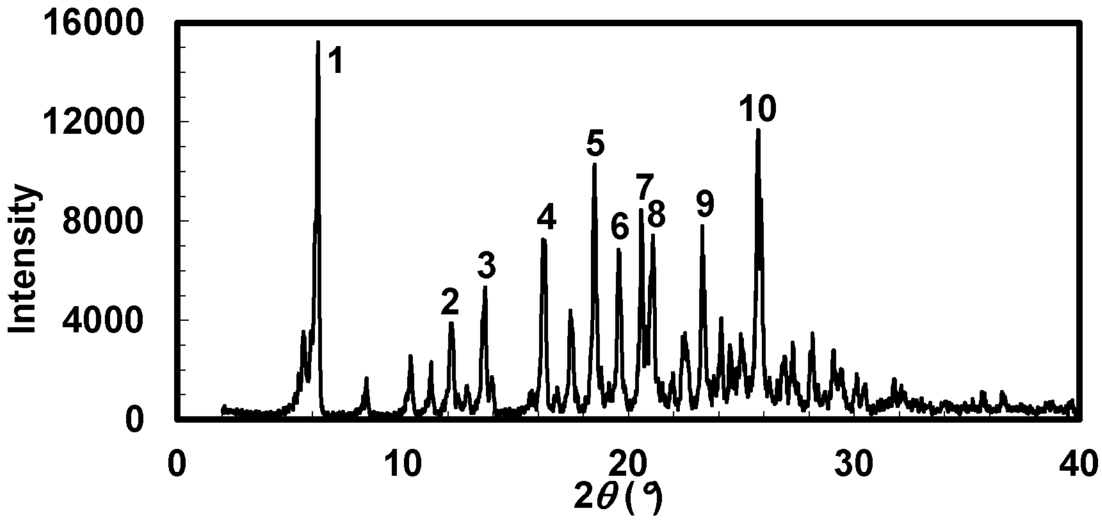


[Figure 21]

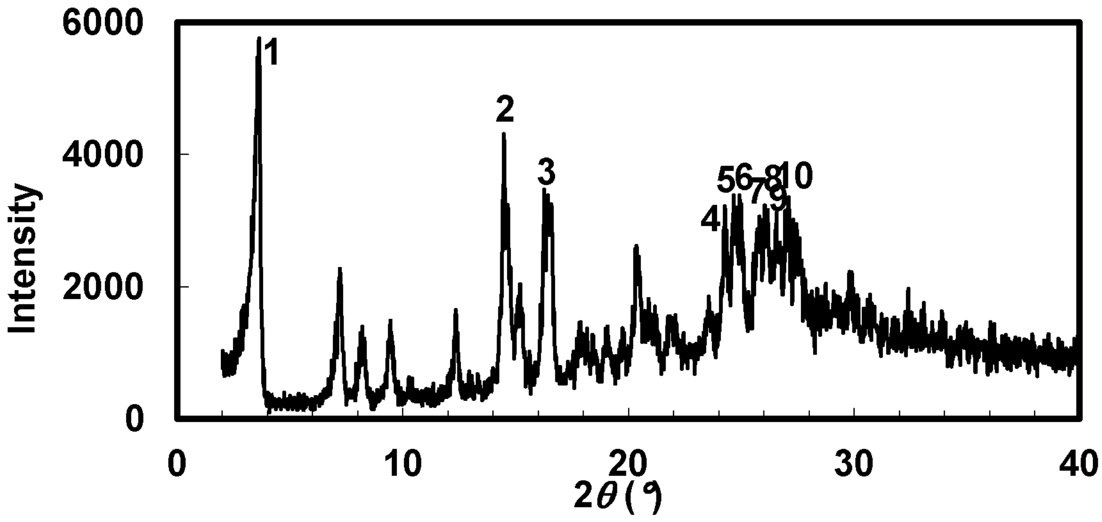

[Figure 22]

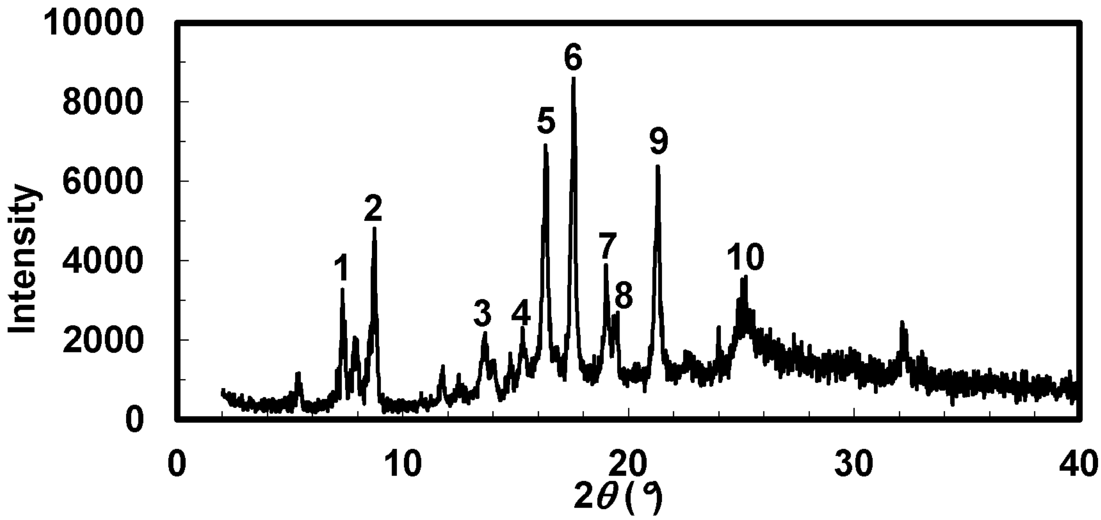


[Figure 23]

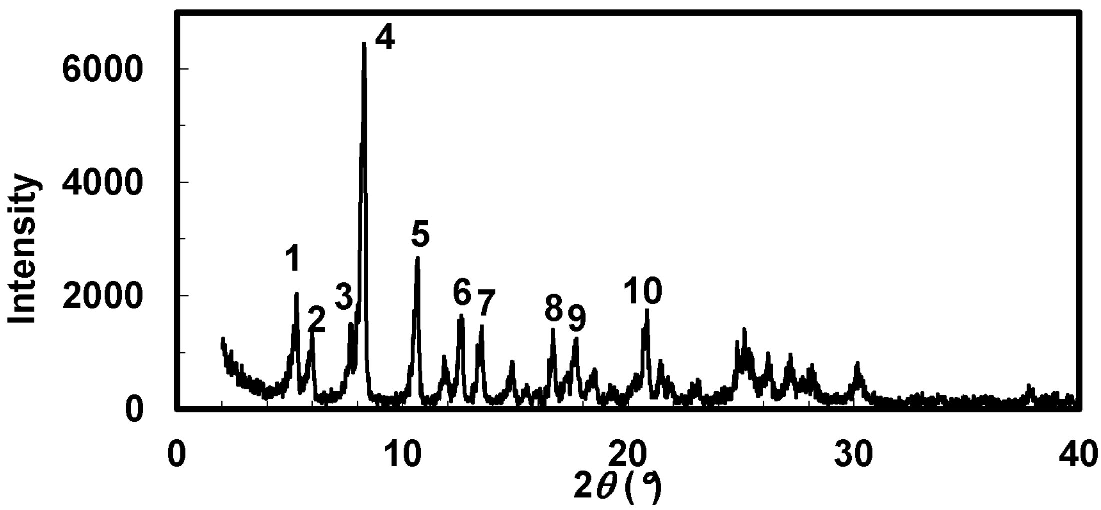

[Figure 24]

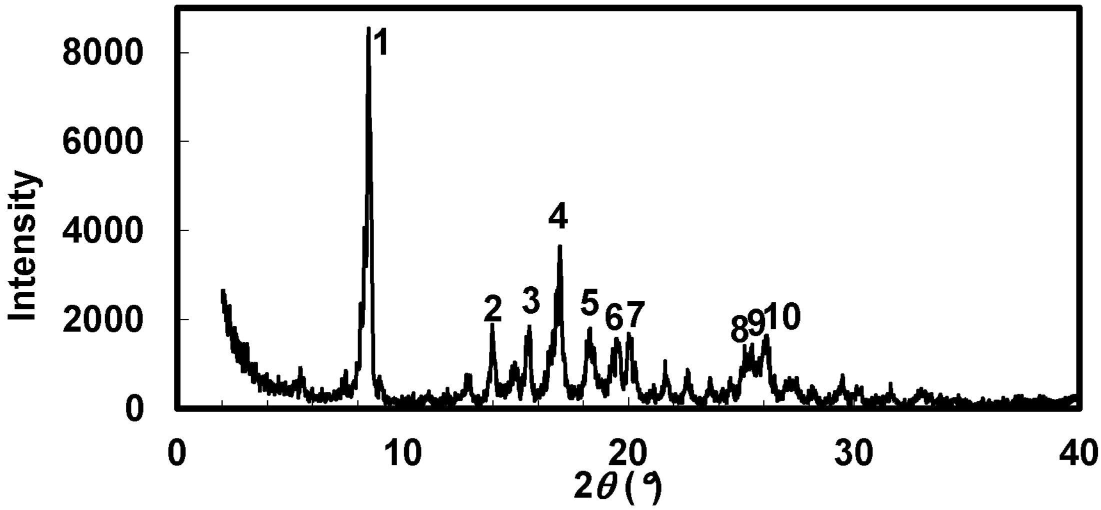


[Figure 25]

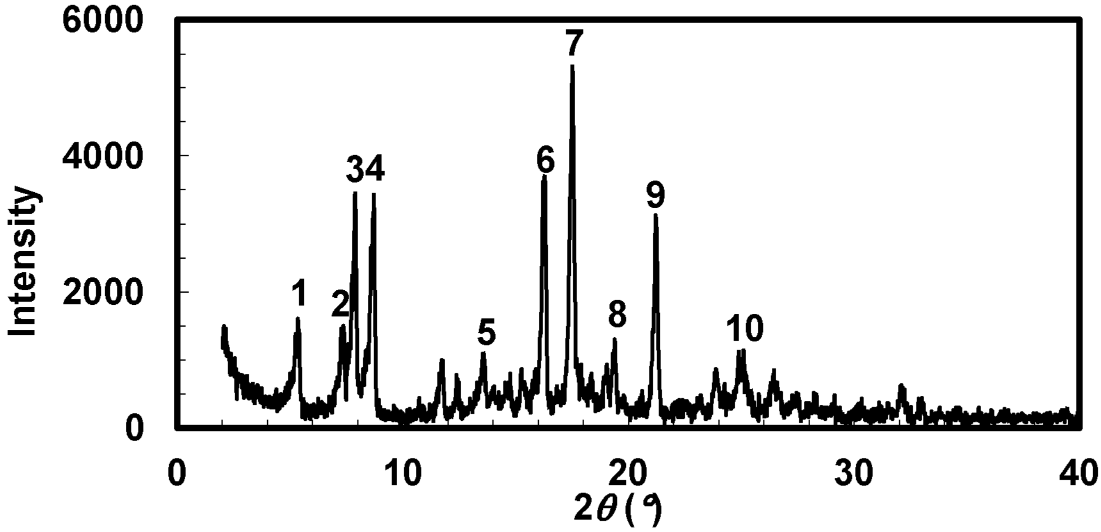

## INTERNATIONAL SEARCH REPORT

International application No. PCT/JP2019/026483

**A. CLASSIFICATION OF SUBJECT MATTER**

Int.Cl. C07D401/12(2006.01)i, A61K31/517(2006.01)i, A61P35/00(2006.01)i, A61P43/00(2006.01)i, C07D401/14(2006.01)i, C07D413/14(2006.01)i, C07D417/14(2006.01)i

According to International Patent Classification (IPC) or to both national classification and IPC

**B. FIELDS SEARCHED**

Minimum documentation searched (classification system followed by classification symbols)
Int.Cl. C07D401/12, A61K31/517, A61P35/00, A61P43/00, C07D401/14, C07D413/14, C07D417/14

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2010-529115 A (HANMI PHARM. CO., LTD.) 26 August 2010 & US 2010/0179120 A1 & WO 2008/150118 A2 & EP 2167492 A2 & KR 10-2008-0107294 A & AR 66876 A & CA 2687180 A & CN 101679384 A & MX 2009012772 A & IL 202154 A & AU 2008260772 A & RU 2009149307 A & TW 200906414 A & NZ 582412 A & MY 144972 A & UA 96045 C | 1-49 |

☒ Further documents are listed in the continuation of Box C.  ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 11 September 2019 (11.09.2019) | 24 September 2019 (24.09.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/026483

C (Continuation). DOCUMENTS CONSIDERED TO BE RELEVANT

| Category* | Citation of document, with indication, where appropriate, of the relevant passages | Relevant to claim No. |
|---|---|---|
| A | JP 2000-508657 A (WARNER-LAMBERT COMPANY) 11 July 2000 & US 2003/0229051 A1 & US 6344459 B1 & US 6602863 B1 & WO 1997/038983 A1 & EP 892789 A1 & DE 69710712 T & PL 329391 A & BR 9708640 A & CA 2249446 A & HU 9901207 A & BG 102811 A & SK 141798 A & EE 9800328 A & NZ 332119 A & HK 1019739 A & EA 199800887 A | 1-49 |
| A | JP 2007-501854 A (PFIZER PRODUCTS INC.) 01 February 2007 & US 2004/0242604 A1 & WO 2004/106308 A1 & EP 1636195 A1 & CA 2527017 A & BR PI0410720 A & MX PA05012839 A | 1-49 |
| A | WO 2018/094225 A1 (BOARD OF REGENTS, THE UNIVERSITY OF TEXAS SYSTEM) 24 May 2018 & CA 3044432 A & AU 2017363199 A & TW 201825098 A | 1-49 |
| A | JI, Hong-Bin, "Epidermal growth factor receptor variant III mutations in lung tumorigenesis and sensitivity to tyrosine kinase inhibitors", Proceedings of the National Academy of Sciences of the United States of America, 2006, vol. 103, no. 20, pp. 7817-7822, ISSN 0027-8424 | 1-49 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008150118 A
- WO 2015041360 A1


### Non-patent literature cited in the description


- **DAS, M. et al.** *Proc. Natl Acad.Sci. USA,* 1977, vol. 74, 2790-2794
- **KANCHA, R. et al.** *Clin. Cancer Res.,* 2009, vol. 15, 460-467
- **LEE, C. K. et al.** *J. Clin. Oncol.,* 2015, vol. 33, 1958-1965
- **WATANABE, S. et al.** *J. Thorac. Oncol.,* 2014, vol. 9, 189-194
- **PAO, W. et al.** *PLoS Med.,* 2005, vol. 2, e73
- **MARIA E. A et al.** *Clin Cancer Res,* 15 September 2012, vol. 18 (18
- **OXNARD G.R.** *J Thorac Oncol.,* February 2013, vol. 8 (2), 179-184
- **YOSHIHISA K. et al.** *Cancer Sci,* September 2016, vol. 107 (9), 1179-1186
- **COUSSENS L et al.** *Science,* 1985, vol. 230 (4730), 1132-1139
- **HARDWICK R et al.** *Eur. J Surg Oncol.,* 1997, vol. 23, 30-35
- **KORKAYA H et al.** *Oncogene,* 2008, vol. 27 (47), 6120-6130
- **YANO T et al.** *OncolRep,* 2006, vol. 15 (1), 65-71
- **SLAMON DJ et al.** *Science,* 1987, vol. 235, 177-182
- **GRAVALOS C et al.** *Ann Oncol,* 2008, vol. 19, 1523-1529
- **FUKUSHIGE S et al.** *Mol Cell Biol,* 1986, vol. 6, 955-958
- **HYMAN D.M. et al.** *Nature,* 08 February 2018, vol. 554 (7691), 189-194
- Iyakuhin no kaihatsu (Drug development). Molecular Design. Hirokawa-Shoten Ltd, 1990, vol. 7, 163-198
- ORGANIC FUNCTIONAL GROUP PREPARATIONS. ACADEMIC PRESS, INC, 1989
- Comprehensive Organic Transformations. VCH Publishers Inc, 1989
- **T.W. GREENE ; P.G. WUTS.** Greene's Protective Groups in Organic Synthesis. John Wiley & Sons, Inc, 2006
- *J. Org. Chem.,* 2003, vol. 68, 770-778
- *J. Med. Chem.,* 1996, vol. 39, 267-276
- *Bioorg. Med. Chem.,* 2009, vol. 17, 3152-3161